# EUROPEAN PATENT APPLICATION

(11) **EP 2 471 933 A2**
(43) Date of publication of application: **04.07.2012**
(21) Application number: 12162150.2
(22) Date of filing: 09.04.2008
(51) Int. Cl.: C12N 15/82, C07K 14/415, A01H 5/00

(54) **Composition and method for modulating plant root hair development**

(30) Priority: 22.05.2007 GB 0709835
(62) Divisional of application: 08736917.9
(71) Applicant: Plant Bioscience Limited, Norwich, Norfolk NR4 7UH (GB)
(72) Inventor: Dolan, Liam, Oxford Oxfordshire OX1 3RB (GB); Menand, Benoit, Norwich Norfolk NR4 7UH (GB); Yi, Keke, Norwich Norfolk NR4 7UH (GB)
(74) Representative: Fleck, Barbara

(57) **Abstract**

This invention relates to the modulation of root hair development in plants by altering the expression of RHD6-related genes, for example to increase the number, length and/or longevity of root hairs in the plant. This may be useful, for example, in improving the ability of plants to extract nutrients from the soil.

## Description

### FIELD OF INVENTION

This invention relates to the modulation of root hair development in plants.

### BACKGROUND OF THE INVENTION

In 1990, Schiefelbein and Somerville²⁷ published a paper describing their work with *Arabidopsis thaliana* mutants in their efforts to understand genetic control of root hair development. They examined roots from 12,000 mutagenized *Arabidopsis* seedlings, leading to identification of more than 40 mutants impaired in root hair morphogenesis. Mutants were characterized as belonging to four phenotypic classes which genetically were produced from single nuclear recessive mutations in four different genes designated *RHD1, RHDP, RHD3,* and *RHD4.* As a result of the phenotypic analysis of the mutants and homozygous double mutants, a model for root hair development was proposed, including the stages at which the genes are normally require. The *RHD1* gene product appears to be necessary for proper initiation of root hairs, whereas the *RHDS, RHD3,* and *RHD4* gene products are required for normal hair elongation. These authors concluded that the results they obtained demonstrate that root hair development in *Arabidopsis* is amenable to genetic dissection and should prove to be a useful model system to study the molecular mechanisms governing cell differentiation in plants.

In 1994, Masucci and Schiefelbein⁷ extended those results by identifying another mutant, the *rhd6* mutant, concluding that root-hair initiation in *Arabidopsis thaliana* provides a model for studying cell polarity and its role in plant morphogenesis. They observed that root hairs normally emerge at the apical end of root epidermal cells, implying that these cells are polarized. The *rhd6* mutant was characterized as displaying three defects: (a) a reduction in the number of root hairs, (b) an overall basal shift in the site of root hair emergence, and (c) a relatively high frequency of epidermal cells with multiple root hairs. They concluded that these defects implicate the *RHD6* gene in root-hair initiation and indicate that *RHD6* is normally associated with the establishment of, or response to, root epidermal cell polarity. Similar alterations in the site of root-hair emergence, although less extreme, were also discovered in roots of the auxin-, ethylene-, abscisic acid-resistant mutant *axr2* and the ethylene-resistant mutant *etrl*. All three *rhd6* mutant phenotypes were rescued when either auxin (indoleacetic acid) or an ethylene precursor (1 -aminocyclopropane-1-carboxylic acid) was included in the growth medium. The *rhd6* root phenotypes could be phenocopied by treating wild-type seedlings with an inhibitor of the ethylene pathway (aminoethoxyvinylglycine). These results indicate that *RHD6* is normally involved in directing the selection or assembly of the root-hair initiation site through a process involving auxin and ethylene.

Root hairs play important roles in plant nutrition and water uptake. In most soils they are important for phosphate and iron uptake. In drought conditions they are important in the uptake of other nutrients such as nitrate. Therefore the manipulation of root hair traits will be important in developing crops that can effectively extract nutrients from the soil. Until now this has been difficult since no gene with a function limited to the root hair has been identified.

EP0803572B1 discloses the identification, isolation, cloning, and characterization of the *CPC* gene of *Arabidopsis thaliana,* for regulating initiation of root hair formation, as well as transgenic plants over-expressing the *CPC* gene. The *CPC* gene is not responsible for the *rhd* mutant phenotypes described above. This is confirmed, for example, in US661749, as well as EP0803572B1 itself.

### SUMMARY OF INVENTION

The present invention relates to the finding that the over-expression of ROOT HAIR DEFECTIVE 6 *(RHD6)* genes in plants alters root hair development, for example leading to plants with an increased number, length and/or longevity of root hairs. Furthermore, over-expression of a different gene family (*ROOT HAIR DEFECTIVE SIX LXKE1 (RSL)* genes) produces a similar effect; Modulation of the expression of these genes (collectively termed '*RHD6*-related genes') in plants may be useful, for example, in manipulating root hair traits in diverse groups of plant species (including crops) to improve their ability to extract nutrients from the soil.

An aspect of the invention provides an isolated ROOT HAIR DEFECTIVE 6 (*RHD6*)-related gene.

*RHD6*-related genes include both *ROOT HAIR DEFECTIVE 6 (RHD6)* genes and *ROOT HAIR DEFECTIVE SIX LIKE1 (RSL1)* genes, and functional homologues thereof, as described herein. *RHD6*-related genes include genes capable of complementing the *rhd6* mutation in plants.

Another aspect of the invention provides an isolated gene encoding an amino acid sequence encoded by the *RHD6*-related gene or a gene product that is sufficiently homologous thereto to permit, on production thereof in an *rhd6* mutant cell a functional complementation of said mutation.

Another aspect of the invention provides an isolated product of the expression of an isolated *RHD6*-related gene.

Another aspect of the invention provides an isolated polynucleotide which encodes a gene product comprising an amino acid sequence shown in SEQ ID NO: 1, 3, 5, 7, 9, 11, 13 to 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112 and 114.

Another aspect of the invention provides an isolated polynucleotide which has at least 40% nucleic acid sequence identity with one or more of SEQ ID NOS: 2, 4, 6, 8, 10, 12, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, and 115.

Other aspects of the invention provide expression constructs, plant cells, and plants or plant progeny, including seeds, which comprise an isolated *RHD6*-related gene or polynucleotide described herein.

Another aspect of the invention provides a method of modulating root hair development in a plant comprising;
increasing the expression of an *RHD6*-related polypeptide within cells of said plant relative to control plants.

An *RHD6*-related polypeptide may, for example, comprise an amino acid sequence shown in SEQ ID NO: 1, 3, 5, 7, 9, 11, 13 to 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112 or 114.

Another aspect of the invention provides a method of improving the tolerance of a plant to nutrient-deficient conditions comprising;
increasing the expression of an *RHD6*-rqlated polypeptide within cells of said plant relative to control plants.

Another aspect of the invention provides a method of increasing the production of a.root-secreted phytochemical in a plant comprising;
increasing the expression of an *RHD6*-related polypeptide within cells of a plant which produces the root-secreted phytochemical.

In some embodiments, expression of an *RHD6*-related polypeptide may be increased in a plant by expressing a heterologous nucleic acid encoding said RHD6-related polypeptide within cells of said plant.

In some embodiments, expression of an *RHD6*-related polypeptide may be increased in a plant by;
crossing a first and a second plant to produce a population of progeny plants;
determining the expression of the *RHD6*-related polypeptide in the progeny plants in the population, and
identifying a progeny plant in the population in which expression of the RHD6-related polypeptide is increased relative to controls.

In some embodiments, expression of an *RHD6*-related polypeptide may be increased in a plant by;
exposing a population of plants to a mutagen,
determining the expression of the *RHD6*-related polypeptide in one or more plants in said population, and;
identifying a plant with increased expression of the *RHD6-*related polypeptide.

Plants identified as having increased expression of the *RHD6*-related polypeptide may be sexually or asexually propagated or grown to produce off-spring or descendants showing increased expression of the *RHD6*-related polypeptide.

Another aspect of the invention provides a method of producing a plant with altered root-hair development comprising:
incorporating a heterologous nucleic acid which alters the expression of a *RHD6*-related polypeptide into a plant cell by means of transformation, and;
regenerating the plant from one or more transformed cells.

Another aspect of the invention provides a plant produced by a method described herein which displays altered root-hair development relative to controls.

### BRIEF DESCRIPTION OF THE DRAWINGS

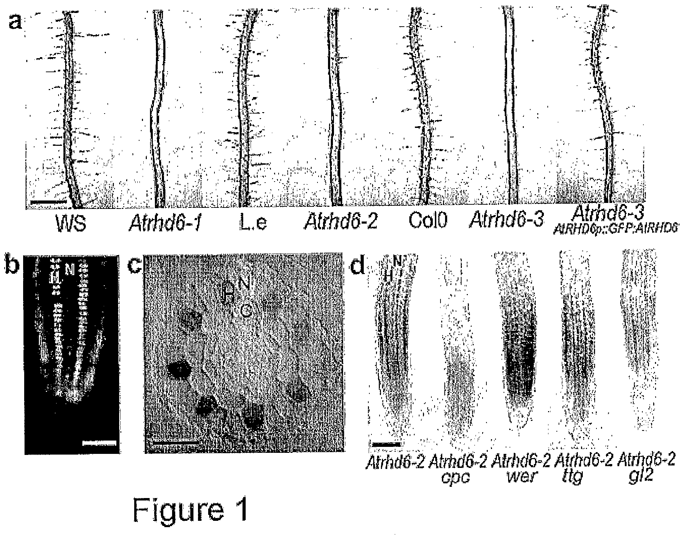

Figure 1 shows that *AtRHD6* is a positive regulator of root hairs development in Arabidopsis. Figure 1a shows roots of *Atrhd6-1, Atrhd6-2* and *Atrhd6-3* mutants with their respective wild type and complementation of the *Atrhd6-3* mutant with a genomic *AtRHD6p::GFP:AtRHD6* fusion. Figure 1b shows a fluorescent image of the genomic *AtRHD6p::GFP:AtRHD6* fusion in the *Atrhd6-3* background showing AtRHD6 protein in hair cells nuclei. Figure 1c shows the expression of the *Atrhd6-2* enhancer trap *GUS* gene in root cross section. Figure 1d shows a whole mount longitudinal view of the expression of the enhancer trap *GUS* gene in *Atrhd6-2* and in different backgrounds (*cpc, wer, ttg1* and *g12*). H, hair cell; N, non hair cells; C, cortex. Scales bars, 500 µm (a), 50 µm (b), 25 µm (c) and 100 µm (d).

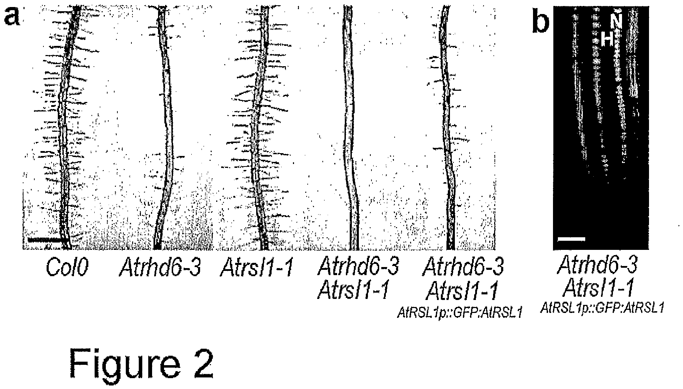

Figure 2 shows that *AtRSL1* positively regulates root hairs development in Arabidopsis. Figure 2a shows roots of WT, *Atrhd6-3* single mutant, *Atrsl1-1* single mutant, *Atrhd6-3 Atrsl1-1* double mutant and *Atrhd6-3 Atrsl1-1* double mutant bearing the *AtRSL1p::GFP:AtRSL1* transgene. Plants were grown on MS media with sucrose overlaid with a cellophane disc to increase root hairs production in the *Atrhd6-3* mutant. Figure 2b shows a fluorescent image of the genomic *AtRSL1p::GFP:AtRSL1* fusion in the *Atrhd6-3 Atrsl1-1* background showing AtRSL1 protein in hair cells nuclei. H, hair cell; N, non hair cells. Scale bars, 500 µm (a) and 50 µm (b).

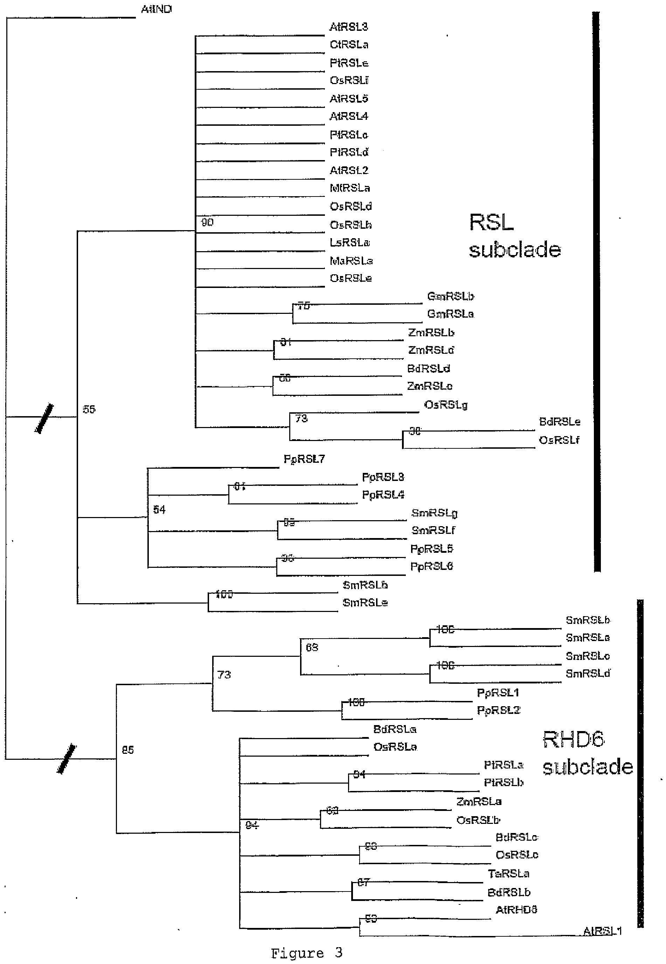

Figure 3 shows the relationship between RHD6-LIKE proteins from Arabidopsis and Physcomitrella. The tree is a strict consensus tree of 12 most parsimonious tree generated using the alignment of bHLH domains amino acids sequences shown in Tables 1 and 2. The Arabidopsis genes used are the members of bHLH subfamily VIIIc, except AtIND (INDEHISCENT)/At4g00120 which was used as out-group and belongs to the bHLH subfamily VIIIb^{8, 10, 26}. Physcomitrella PpRSL 1 to 7 sequences were obtained by BLAST of the Physcomitrella genomic sequence. PpIND1 is a Physcomitrella sequence similar to AtIND and a putative member of family VIIIb in Physcomitrella. Numbers are bootstrap values and indicates an 82 % level of confidence for the occurrence of the AtRHD6 clade. The brackets indicates the AtRHD6 clade and the sister clade.

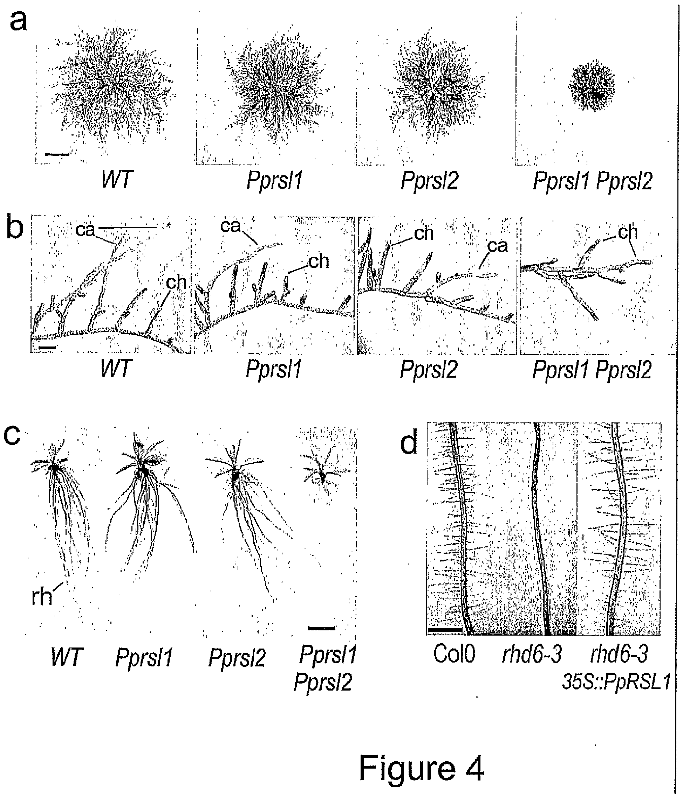

Figure 4 shows that *PpRSL1* and *PpRSL2* positively control the development of caulonemal cells and rhizoids in Physcomitrella and PpRSL1 and AtRHD6 have a conserved molecular function. Figure 4a and b show eighteen day old protonema from WT, *Pprsl1* and *Pprsl2* single mutants, and *Pprsl1 Pprsl2* double mutant, grown from spores on 0.8% agar. Figure 4a shows whole protonema growing from a single spore. Figure 4b shows dissected filaments from protonema shown in Figure 4a. Figure 4c shows isolated one month old gametophores. Figure 4d shows roots of the Arabidopsis *Atrhd6-3* mutant carrying the *35S::PpRSL1* transgene compared to WT and *Atrhd6-3* roots, ca, caulonemal cell; ch, chloronemal cell; rh, rhizoid. Scale bars, 1 mm (a), 100 µm (b), 1 mm (c), and 500 µm (d).

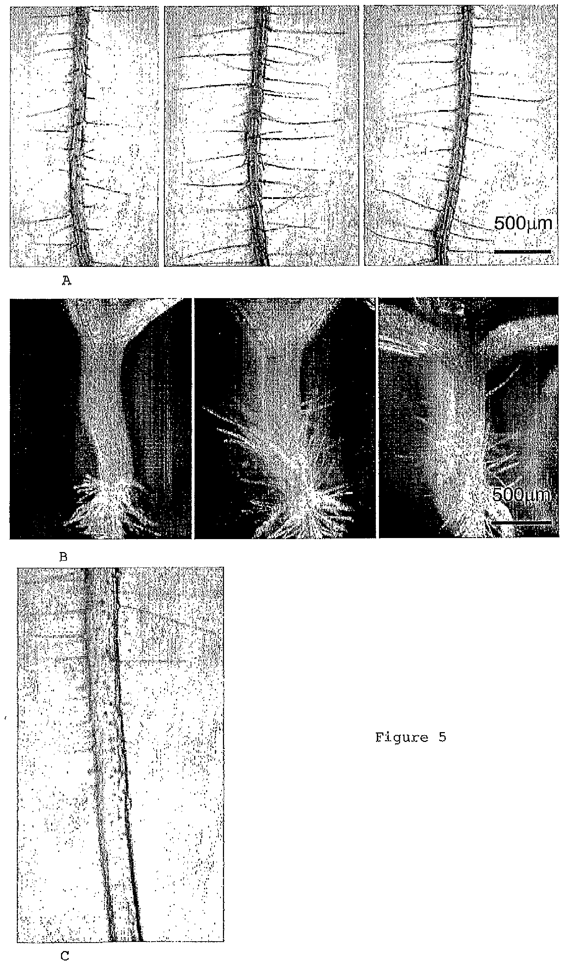

Figure 5 shows the phenotype for the transformants:35S::RHD6 Figure 5A shows col-0 *rhd6*/*rsl1* with 35S::RHDG; Figure 5B shows col-0 *rhd6*/*rsl1* with 35S::RHD6; Figure 5C. *rhd6*/*rsl1* with 35S::RHD6

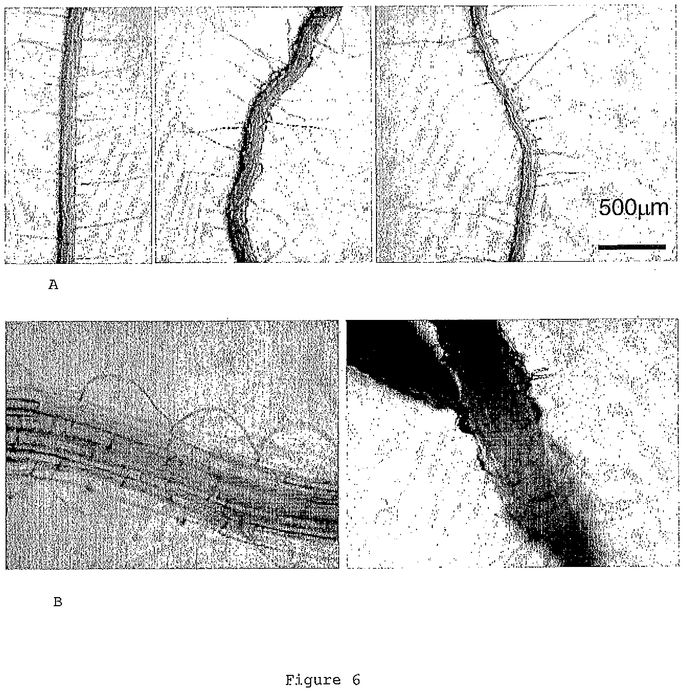

Figure 6 shows the phenotype for the transformants:355::RSL2 and 35S::RSL3 Figure 6A shows col-0 *rhd6*/*rsl1* with 35S::RSL2/3; Figure 6B shows root hypocotyls

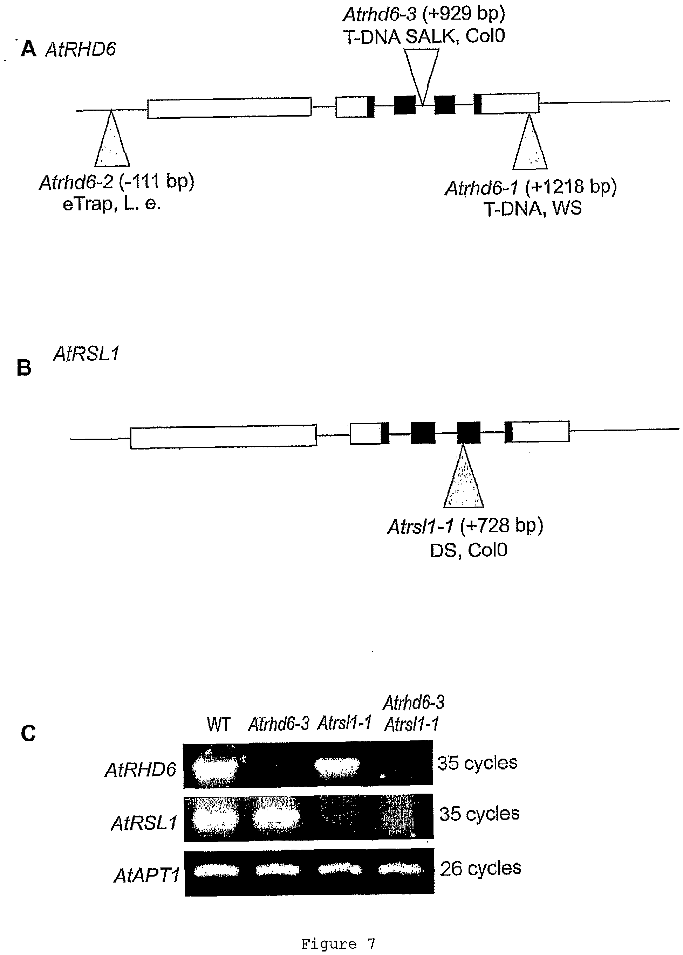

Figure 7 shows the molecular basis of mutations in *A. thaliana AtRHD6* (A) and *AtRSL1* (B) genes. White boxes correspond to coding regions (black boxes for the bHLH domain encoding region). Grey triangles indicate the position of each insertion. Numbers in brackets indicate the distance between each T-DNA insertion and the start codon. (C) RT-PCR showing that *Atrhd6-3, Atrsl1-1* and *Atrhd6-3 Atrsl1-1* are RNA null mutants. AtAPT1, Adenine phosphoribosyltransferase 1.

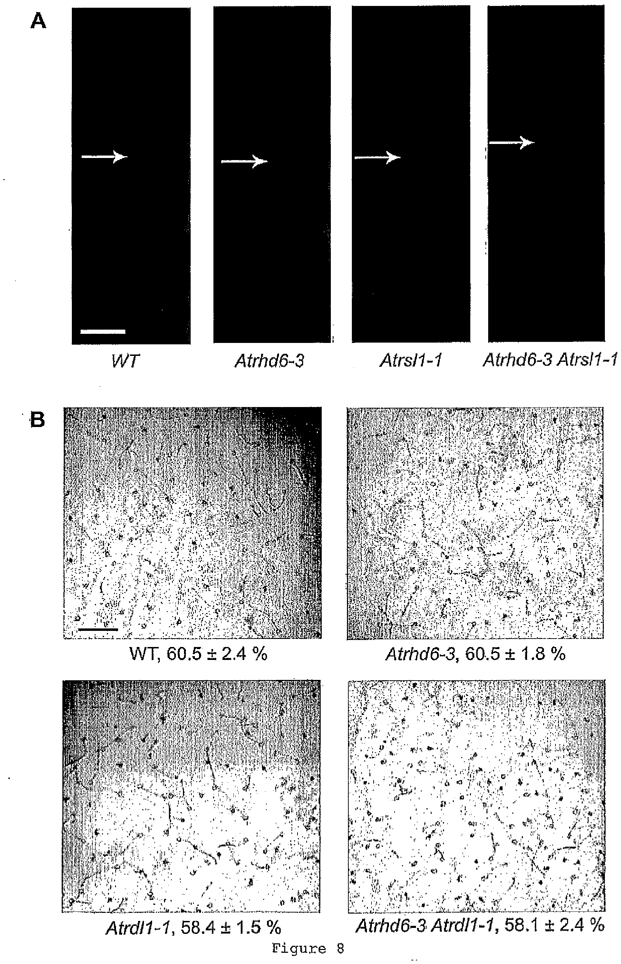

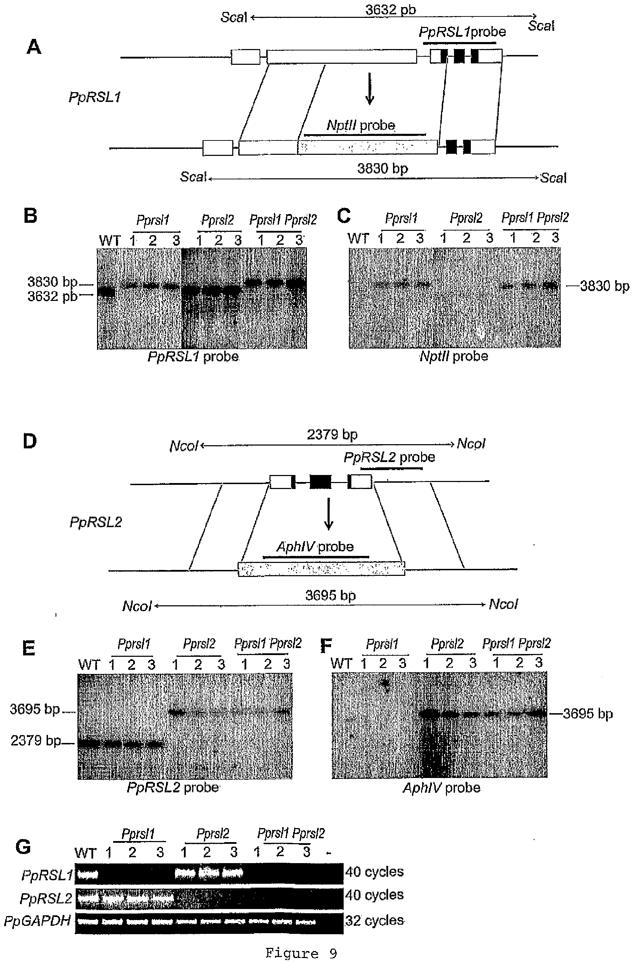

Figure 8 shows that the *Atrhd6-3* and *Atrsl1-1* single mutants and the *Atrhd6 Atrsl1* double mutant have no detectable pollen tube growth defects. Ratios of resistance to antibiotic of F2 plants from *Atrhd6-3 Atrsl1-1* double mutant backcrossed to WT are 76.7 % (n=1404; χ²(3/1) = 2,19; P > 0,05) for Sulfadiazin (resistance carried by the *Atrhd6-3* allele) and 74.9 % (n=1289; χ²(3/1) = 0.0023; P > 0,05) for phosphinothricin (resistance carried by the *Atzsl1-1* allele) showing normal segregation of the single mutants and double mutant gametes. (Figure 8A shows pollen of the genotype indicated below each picture was used to pollinate WT stigma. Carpels were stained with aniline blue 4 hours after pollination. The growth of each mutant pollen tubes in the WT carpel is revealed by callose staining in blue (white arrows). Similar pollen tube growth is observed in WT and mutant pollen tubes. Figure 9B shows *in vitro* pollen tube growth experiment. WT and mutants pollens were germinated on agar plates. Representative plates are shown with the germination ratio (mean of 600 pollen grains per line, with standard error). Similar germination ratios are observed between WT and mutants pollen (Student's-t-test p values are 0.554 for *Atrhd6-3* versus WT, 0.904 for *Atrsl1-1* versus WT and 0.87 for *Atrhd6-3 Atrsl1-1* versus WT). Scale bars, 200 µm (Figure 8A and B).
Figure 9 shows the molecular basis of *P. patens Pprsl1, Pprsl2* and *Pprsl1 Pprsl2* mutations (three independent mutants, named 1 to 3, are shown in each case). Figure 9A and D show the structure of the *PpRSL1* (A) and *PpRSL2* (D) genes (up), and the expected result of the homologous recombination (down). White boxes correspond to coding regions (black boxes for the bHLH domain encoding region) and the grey boxes correspond to the resistance gene cassette (*NptII* and *AphIV*). The regions of homology used for gene replacement are delimited by grey lines. The distance between the restrictions sites used for Southern blots and the position of the probes used are also shown. Figure 9B, C, E and F show southern blots of WT and mutants DNA digested with *Sca*I (B and C) or *Nco*I (E and F) and hybridized with the probe indicated below the picture. Blots C and F are hybridization of the same membrane used for blot B and E respectively, after stripping of the gene specific probe. The replacement of the WT band by a larger band of expected size (see A and D) in mutants lines when hybridization is performed with the gene specific probe (B and E), and the hybridization of only the mutant band with the resistance gene probe (C and F), demonstrate the presence of single insertions in the *PpRSL1* and *PpRSL2* loci. (G) RT-PCR showing that the mutants are RNA null mutants. PpGAPDH, glyceraldehyde 3-phosphate dehydrogenase. In each case, the three independent single insertion mutants presented have the same phenotype and only the mutant 1 is shown in Fig. 4.

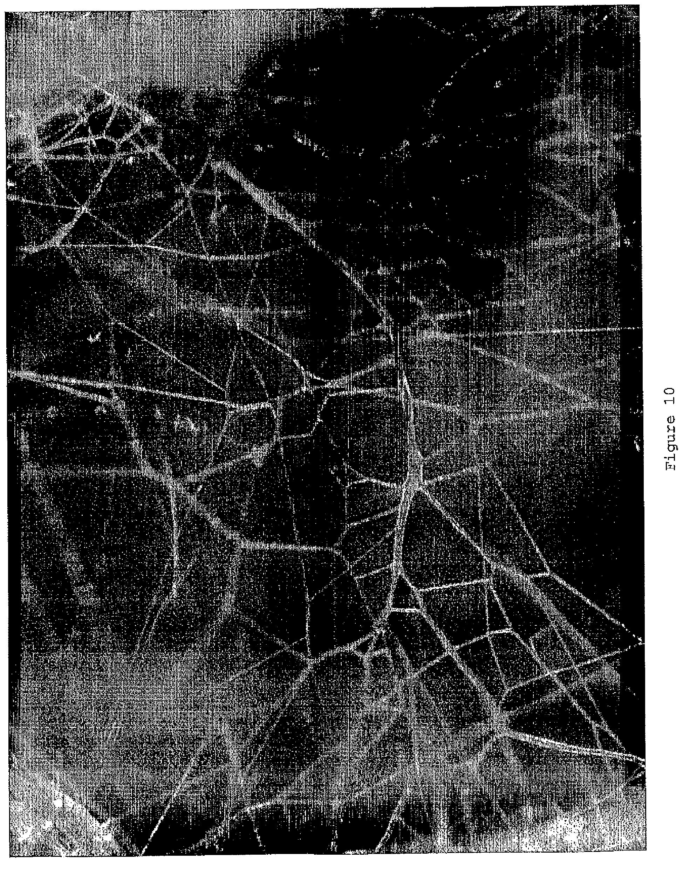

Figure 10 shows the root hair system of an Arabidopsis plant over-expressing RSL4 and displaying a root morphology resembling a fungal symbiont, such as *Mycorrhizae.*

### DETAILED DESCRIPTION OF THE PREFERRED EMBODIMENTS OF THE INVENTION

The present disclosure demonstrates the identification, isolation, cloning and expression of the *ROOT HAIR DEFECTIVE 6* (RHD6) and *ROOT HAIR DEFECTIVE SIX LIKE (RSL)* genes (collectively termed '*RHD6-*related genes' herein) in plants. It shows complementation of mutations by distantly related genes, providing the function of root hair development in plants in which the distantly related gene has been inactivated. Accordingly, those skilled in the art will appreciate that, for the first time, the gene responsible for previously identified mutant phenotypes has been isolated and cloned according to this invention. It will also be appreciated that from this disclosure functional benefits may be conferred on plants by means of introduction into plants and expression of these genes in such plants. Methods known in the art may be utilized for this purpose. Thus, for example, those skilled in the art will appreciate that the methods, for example, for achieving the expression of the *RHD6* and *RSL* genes of this invention may be achieved according methods disclosed herein, and by methods, for example, disclosed in, but not limited to, EP0803572B1, which discloses the cloning and expression of the cpc gene, which, like the *RHD6* and *RSL* genes of this invention, is also related to the control of root hair development in plants, albeit at a different stage of plant and root hair development.

In various aspects, the invention provides ROOT HAIR DEFECTIVE 6 (RHD6)-related polypeptides encoded by *ROOT HAIR DEFECTIVE 6 (RHD6)-*related genes and nucleic acid sequences described herein.

ROOT HAIR DEFECTIVE 6 (RHD6)-related polypeptides include both ROOT HAIR DEFECTIVE 6 (RHD6) polypeptides and ROOT HAIR DEFECTIVE 6-LIKE 1 (RSL1) polypeptides, and functional homologues thereof, as described herein. RHD6-related polypeptides include may be capable of complementing the *rhd6* mutation upon expression in plants.

A ROOT HAIR DEFECTIVE 6 (RHD6)-related polypeptide may fall within the RHD6 clade comprising AtRHD6, AtRSL1, PpRSL1, PpRSL2, BdRSLb, TaRSLa, OsRSLc, BdRSLc, OsRSLb, ZmRSLa, PtRSLa, PrRSLb, OsRSLa, BdRSLa, SmRSLa, SmRSLb, SmRSLc and SmRSLd (the ROOT HAIR DEFECTIVE 6 (RHD6) clade) in a cladogram of protein sequences, for example using the sequences of AtIND and PpINDa as an outgroup (see figure 3).

Alternatively, ROOT HAIR DEFECTIVE 6 (RHD6)-related polypeptide may fall within the RSL clade comprising AtRSL3, CtRSLa, PtRSLe, OsRSLi, AtRSL5, AtRSL4, PtRSLc, PtRSLd, AtRSL2, MtRSLa, OsRSLd, OsRSLh, LsRSLa, MaRSLa, OsRSLe, GmRSLb, GmRSLa, ZmRSLb, ZmRSLd, BdRSLd, ZmRSLc, OsRSLg, BdRSLe, OsRSLf, PpRSL3, PpRSL4, PpRSL5, PpRSL6, PpRSL7, SmRSLg, SmRSLf, SmRSLh and SmRSLe (the *ROOT HAIR DEFECTIVE SIX LIKE (RSL)* clade) in a cladogram of protein sequences, for example using the sequences of AtIND and PpINDa as an outgroup (see figure 3).

A cladogram may be produced using conventional techniques. For example, a cladogram may be calculated using ClustalW to align the protein sequences, Phylip format for tree output, with 1000 bootstrap replicates and TreeViewX (version 0.5.0) for visualisation.

A suitable ROOT HAIR DEFECTIVE 6 (RHD6)-related polypeptide may comprise the amino acid sequence shown of SEQ ID NO: 1, 3, 5, 7, 9, 11, 13 to 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112 or 114 or may be a fragment or variant of one of these sequences which retains RHD6 activity.

In some preferred embodiments, the ROOT HAIR DEFECTIVE 6 (RHD6)-RELATED polypeptide may be a ROOT HAIR DEFECTIVE 6 (RHD6) polypeptide having the amino acid sequence of SEQ ID NO:1 (Atlg66470; NP_176B20.1 GI: 15219658) or may be a fragment or variant of this sequence which retains RHD6 activity.

In other embodiments, the ROOT HAIR DEFECTIVE 6 (RHD6)-RELATED polypeptide may be a *ROOT HAIR DEFECTIVE SIX LIKE (RSL)* polypeptide having the amino acid sequence of any one of SEQ ID NOS: 5, 7, 9, and 11 or may be a fragment or variant of any of these sequences which retains RHD6 activity.

A ROOT HAIR DEFECTIVE 6 (RHD6)-RELATED polypeptide which is a variant a reference sequence set out herein, such as SEQ ID NO: 1, 3, 5, 7, 9, 11, 13 to 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112 or 114, may comprise an amino acid sequence which shares greater than 20% sequence identity with the reference amino acid sequence, preferably greater than 30%, greater than 40%, greater than 50%, greater than 60%, greater than 65%, greater than 70%, greater than 80%, greater than 90% or greater than 95%.

Particular amino acid sequence variants may differ from a RHD6-related polypeptide sequence as described herein by insertion, addition, substitution or deletion of 1 amino acid, 2, 3, 4, 5-10, 10-20 20-30, 30-50, or more than 50 amino acids.

Sequence identity is commonly defined with reference to the algorithm GAP (Wisconsin Package, Accelerys, San Diego USA). GAP uses the Needleman and Wunsch algorithm to align two complete sequences that maximizes the number of matches and minimizes the number of gaps. Generally, default parameters are used, with a gap creation penalty = 12 and gap extension penalty = 4.

Use of GAP may be preferred but other algorithms may be used, e.g. BLAST (which uses the method of Altschul et al. (1990) J. Mol. Biol. 215: 405-410), FASTA (which uses the method of Pearson and Lipman (1988) PNAS USA 85: 2444-2448), or the Smith-Waterman algorithm (Smith and Waterman (1981) J. Mol Biol. 147: 195-197), or the TBLASTN program, of Altschul et al. (1990) supra, generally employing default parameters. In particular, the psi-Blast algorithm (Nucl. Acids Res. (1997) 25 3389-3402) may be used.

Sequence comparison may be made over the full-length of the relevant sequence described herein.

Certain domains of a RHD6-related polypeptide may show an increased level of identity with domains of a reference sequence, such as SEQ ID NO: 1, 3, 5, 7, 9, 11, 13 to 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112 or 114, relative to the RHD6-related polypeptide sequence as a whole. For example, a RHD6-related polypeptide may comprise one or more domains or motifs consisting of an amino acid sequence which has at least 70%, at least 75%, at least 80%, at least 90%, at least 95%, or at least 98% sequence identity or similarity, with an amino acid sequence selected from the group consisting of SEQ ID NOS: 13 to 25 or other RHD6-related polypeptide domain shown in tables 1 and 2.

In some preferred embodiments, a RHD6-related polypeptide may comprise one or more domains or motifs consisting of an amino acid sequence which is selected from the group consisting of SEQ ID NOS: 13 to 25 or other RHD6-related polypeptide domain shown in tables 1 and 2.

In various aspects, the invention provides ROOT HAIR DEFECTIVE 6 (RHD6)-related genes and nucleic acid sequences which encode ROOT HAIR DEFECTIVE 6 (RHD6)-related polypeptides, as described herein.

A nucleic acid encoding a *RHD6*-related polypeptide may comprise or consist of the nucleotide sequence of any one of SEQ ID NOS: 2, 4, 6, 8, 10, 12, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, and 115 or may be a variant or fragment of any one of these sequences which encodes a polypeptide which retains *RHD6* activity.

In some preferred embodiments, a nucleic acid encoding a *RHD6-*related polypeptide may comprise or consist of the nucleotide sequence of SEQ ID NO: 2 or may be a variant or fragment of any one of these sequences which encodes a polypeptide which retains *RHD6* activity.

A variant sequence may be a mutant, homologue, or allele of any one of SEQ ID NOS: 2, 4, 6, 8, 10, 12, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, and 115 and may differ from one of these sequences by one or more of addition, insertion, deletion or substitution of one or more nucleotides in the nucleic acid, leading to the addition, insertion, deletion or substitution of one or more amino acids in the encoded polypeptide. Of course, changes to the nucleic acid that make no difference to the encoded amino acid sequence are included. A nucleic acid encoding a *RHD6*-related polypeptide, which has a nucleotide sequence which is a variant of an *RHD6*-related nucleic acid sequence set out herein may comprise a sequence having at least 30% sequence identity with the nucleic acid sequence of any one of SEQ ID NOS: 2, 4, 6, 8, 10, 12, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, and 115, for example, preferably greater than 40%, greater than 50%, greater than 60%, greater than 65%, greater than 70%, greater than 80%, greater than 90% or greater than 95%. Sequence identity is described above.

A fragment or variant may comprise a sequence which encodes a functional *RHD6*-related polypeptide i.e. a polypeptide which retains one or more functional characteristics of the polypeptide encoded by the wild-type RHD6 gene, for example, the ability to stimulate or increase root hair number, growth or longevity in a plant or to complement the *rhd6* mutation.

In other embodiments, a nucleic acid encoding a RHD6 polypeptide, which has a nucleotide sequence which is a variant of the sequence of any one of SEQ ID NOS: 2, 4, 6, 8, 10, 12, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, and 115 may selectively hybridise under stringent conditions with this nucleic acid sequence or the complement thereof.

Stringent conditions include, e.g. for hybridization of sequences that are about 80-90% identical, hybridization overnight at 42°C in 0.25M Na₂HPO₄, pH 7.2, 6.5% SDS, 10% dextran sulfate and a final wash at 55 °C in 0.1X SSC, 0.1% SDS. For detection of sequences that are greater than about 90% identical, suitable conditions include hybridization overnight at 65°C in 0.25M Na₂HPO₄ pH 7.2, 6.5% SDS, 10% dextran sulfate and a final wash at 60 °C in 0.1X SSC, 0.1% SDS. An alternative, which may be particularly appropriate with plant nucleic acid preparations, is a solution of 5x SSPE (final 0.9 M NaCl, 0.05M sodium phosphate, 0.005M EDTA pH 7.7), 5X Denhardt's solution, 0.5% SDS, at 50°C or 65°C overnight. Washes may be performed in 0.2x SSC/0.1% SDS at 65°C or at 50-60°C in 1x SSC/0.1% SDS, as required.

Nucleic acids as described herein may be wholly or partially synthetic. In particular, they may be recombinant in that nucleic acid sequences which are not found together in nature (do not run contiguously) have been ligated or otherwise combined artificially. Alternatively, they may have been synthesised directly e.g. using an automated synthesiser.

The nucleic acid may of course be double- or single-stranded, cDNA or genomic DNA, or RNA. The nucleic acid may be wholly or partially synthetic, depending on design. Naturally, the skilled person will understand that where the nucleic acid includes RNA, reference to the sequence shown should be construed as reference to the RNA equivalent, with U substituted for T.

ROOT HAIR DEFECTIVE 6 (RHD6)-related polypeptides and nucleic acids may be readily identified by routine techniques of sequence analysis in a range of plants, including agricultural plants selected from the group consisting of *Lithospermum erythrorhizon, Taxus* spp, tobacco, cucurbits, carrot, vegetable brassica, melons, capsicums, grape vines, lettuce, strawberry, oilseed brassica, sugar beet, wheat, barley, maize, rice, soyabeans, peas, sorghum, sunflower, tomato, potato, pepper, chrysanthemum, carnation, linseed, hemp and rye.

A *RHD6*-related nucleic acid as described herein may be operably linked to a heterologous regulatory sequence, such as a promoter, for example a constitutive, inducible, root-specific or developmental specific promoter.

"heterologous" indicates that the gene/sequence of nucleotides in question or a sequence regulating the gene/sequence in question, has been linked to the RHD6 related nucleic acid using genetic engineering or recombinant means, i.e. by human intervention. Regulatory sequences which are heterologous to an RHD6 related nucleic acid may be regulatory sequences which do not regulate the RHD6 related nucleic acid in nature or are not naturally associated with the RHD6 related nucleic acid. "Isolated" indicate that the isolated molecule (e.g. polypeptide or nucleic acid) exists in an environment which is distinct from the environment in which it occurs in nature. For example, an isolated nucleic acid may be substantially isolated with respect to the genomic environment in which it naturally occurs.

Many suitable regulatory sequences are known in the art and may be used in accordance with the invention. Examples of suitable regulatory sequences may be derived from a plant virus, for example the Cauliflower Mosaic Virus 35S (CaMV 35S) gene promoter that is expressed at a high level in virtually all plant tissues (Benfey et al, (1990) EMBO J 9: 1677-1684). Other suitable constitutive regulatory elements include the cauliflower mosaic virus 19S promoter; the Figwort mosaic virus promoter; and the nopaline synthase (nos) gene promoter (Singer et al., Plant Mol. Biol. 14:433 (1990); An, Plant Physiol. 81:86 (1986)). For example, RHD6-related genes such as AtRHD6, AtRSL1 and AtRSL4 may be expressed using constitutive promoters.

Constructs for expression of *RHD6* and *RSL* genes under the control of a strong constitutive promoter (the 35S promoter) are exemplified below. Expression of AtRHD66, AtRSL1 and AtRSL4 from the 35S promoter is shown to modulate root hair development in plants without causing additional phenotypic changes.

However, those skilled in the art will appreciate that a wide variety of other promoters may be employed to advantage in particular contexts. Thus, for example, one might select an epidermal or root-specific promoter to ensure expression of these constructs only in roots. Suitable root-specific promoters are described for example in Qi et al PNAS (2006) 103(49) 18848-18853. For example, RHD6-related genes such as AtRSL2 and ATRSL3, may be expressed using root-specific promoters.

Alternatively, or in addition, one might select an inducible promoter. In this way, for example, in a cell culture setting, production of a particular gene product of interest may be enhanced or suppressed by induction of the promoter driving expression of the genes described herein. Inducible promoters include the alcohol inducible alc gene-expression system (Roslan et al., Plant Journal; 2001 Oct; 28(2):225-35) may be employed.

*RHD6*-related nucleic acid may be contained on a nucleic acid construct or vector. The construct or vector is preferably suitable for transformation into and/or expression within a plant cell.

A vector is, *inter alia*, any plasmid, cosmid, phage or *Agrobacterium* binary vector in double or single stranded linear or circular form, which may ar may not be self transmissible or mobilizable, and which can transform prokaryotic or eukaryotic host, in particular a plant host, either by integration into the cellular genome or exist extrachromasomally (e.g. autonomous replicating plasmid with an origin of replication).

Specifically included are shuttle vectors by which is meant a DNA vehicle capable, naturally or by design, of replication in two different organisms, which may be selected from actinomyces and related species, bacteria and eukaryotic (e.g. higher plant, mammalia, yeast or fungal) cells.

A construct or vector comprising nucleic acid as described above need not include a promoter or other regulatory sequence, particularly if the vector is to be used to introduce the nucleic acid into cells for recombination into the genome.

Constructs and vectors may further comprise selectable genetic markers consisting of genes that confer selectable phenotypes such as resistance to antibiotics such as kanamycin, hygromycin, phosphinotricin, chlorsulfuron, methotrexate, gentamycin, spectinomycin, imidazolinones, glyphosate and d-amino acids.

Those skilled in the art are well able to construct vectors and design protocols for recombinant gene expression, in particular in a plant cell. Suitable vectors can be chosen or constructed, containing appropriate regulatory sequences, including promoter sequences, terminator fragments, polyadenylation sequences, enhancer sequences, marker genes and other sequences as appropriate. For further details see, for example, Molecular Cloning: a Laboratory Manual: 3rd edition, Sambrook & Russell, 2001, Cold Spring Harbor Laboratory Press.

Those skilled in the art can construct vectors and design protocols for recombinant gene expression, for example in a microbial or plant cell. Suitable vectors can be chosen or constructed, containing appropriate regulatory sequences, including promoter sequences, terminator fragments, polyadenylation sequences, enhancer sequences, marker genes and other sequences as appropriate. For further details see, for example, Molecular cloning: a Laboratory Manual: 3rd edition, Sambrook et al, 2001, Cold Spring Harbor Laboratory Press and Protocols in Molecular Biology, Second Edition, Ausubel et al. eds. John Wiley & Sons, 1992. Specific procedures and vectors previously used with wide success upon plants are described by Bevan, Nucl. Acids Res. (1984) 12, 8711-8721), and Guerineau and Mullineaux,(1993) Plant transformation and expression vectors. In: Plant Molecular Biology Labfax (Croy RRD ed) Oxford, BIOS Scientific Publishers, pp 121-148.

When introducing a chosen gene construct into a cell, certain considerations must be taken into account, well known to those skilled in the art. The nucleic acid to be inserted should be assembled within a construct that contains effective regulatory elements that will drive transcription. There must be available a method of transporting the construct into the cell. Once the construct is within the cell membrane, integration into the endogenous chromosomal material either will or will not occur. Finally, the target cell type is preferably such that cells can be regenerated into whole plants.

Those skilled in the art will also appreciate that in producing constructs for achieving expression of the genes according to this invention, it is desirable to use a construct and transformation method which enhances expression of the *RHD6* gene, the *RSL* gene or a functional homolog thereof. Integration of a single copy of the gene into the genome of the plant cell may be beneficial to minimize gene silencing effects. Likewise, control of the complexity of integration may be beneficial in this regard. Of particular interest in this regard is transformation of plant cells utilizing a minimal gene expression construct according to, for example, EP Patent No. EP 1 407 000 B1, herein incorporated by reference for this purpose.

Techniques well known to those skilled in the art may be used to introduce nucleic acid constructs and vectors into plant cells to produce transgenic plants with the properties described herein. Agrobacterium transformation is one method widely used by those skilled in the art to transform woody plant species, in particular hardwood species such as poplar. Production of stable, fertile transgenic plants is now routine in the art: (Toriyama, et al. (1988) Bio/Technology 6, 1072-1074; Zhang, et al. (1988) Plant Cell Rep. 7, 379-384; Zhang, et al. (1988) Theor Appl Genet 76, 835-840; Shimamoto, et al. (1989) Nature 338, 274-276; Datta, et al. (1990) Bio/Technology 8, 736-740; Christou, et al. (1991) Bio/Technology 9, 957-962; Peng, et al. (1991) International Rice Research Institute, Manila, Philippines 563-574; Cao, et al. (1992) Plant Cell Rep. 11, 585-591; Li, et al. (1993) Plant cell Rep. 12, 250-255; Rathore, et al. (1993) Plant Molecular Biology 21, 871-884; Fromm, et al. (1990) Bio/Technology 8, 833-839; Gordon-Kamm, et al. (1990) Plant Cell 2, 603-618; D'Halluin, et al. (1992) Plant Cell 4, 1495-1505; Walters, et al. (1992) Plant Molecular Biology 18, 189-200; Koziel, et al. (1993) Biotechnology 11, 194-200; Vasil, I. K. (1994) Plant Molecular Biology 25, 925-937; Weeks, et al. (1993) Plant Physiology 102, 1077-1084; Somers, et al. (1992) Bio/Technology 1.0, 1589-1594; WO92/14828; Nilsson, O. et al (1992) Transgenic Research 1, 209-220).

Other methods, such as microprojectile or particle bombardment (US 5100792, EP-A-444882, EP-A-434616), electroporation (EP 290395, WO 8706614), microinjection (WO 92/09696, WO 94/00583, EP 331083, EP 175966, Green et al. (1987) Plant Tissue and Cell Culture, Academic Press), direct DNA uptake (DE 4005152, WO 9012096, US 4684611), liposome mediated DNA uptake (e.g. Freeman et al. Plant Cell Physiol. 29: 1353 (1984)), or the vortexing method (e.g. Kindle, PNAS U.S.A. 87: 1228 (1990d)) may be preferred where Agrobacterium transformation is inefficient or ineffective, for example in some gymnosperm species.

Physical methods for the transformation of plant cells are reviewed in Oard, 1991, Biotech. Adv. 9: 1-11.

Alternatively, a combination of different techniques may be employed to enhance the efficiency of the transformation process, e.g. bombardment with Agrobacterium coated microparticles (EP-A-486234) or microprojectile bombardment to induce wounding followed by cocultivation with Agrobacterium (EP-A-486233).

Following transformation, a plant may be regenerated, e.g. from single cells, callus tissue or leaf discs, as is standard in the art. Almost any plant can be entirely regenerated from cells, tissues and organs of the plant. Available techniques are reviewed in vasil et al., Cell Culture and Somatic Cell Genetics of Plants, Vol I, II and III, Laboratory Procedures and Their Applications, Academic Press, 1984, and Weissbach and Weissbach, Methods for Plant Molecular Biology, Academic Press, 1989.

The particular choice of a transformation technology will be determined by its efficiency to transform certain plant species as well as the experience and preference of the person practising the invention with a particular methodology of choice. It will be apparent to the skilled person that the particular choice of a transformation system to introduce nucleic acid into plant cells is not essential to or a limitation of the invention, nor is the choice of technique for plant regeneration.

Other aspects of the invention relate to the modulation of plant root hair development using *RHD6* related polypeptides and nucleic acids as described herein.

A method of modulating root hair development or altering the root hair phenotypes in a plant may comprise;
increasing the expression of a RHD6-related polypeptide within cells of said plant relative to control plants.

Modulation of root hair development in a plant may include increasing one or more of: root-hair growth, number of root-hairs, length of root-hairs, rate of growth of root-hairs, and longevity of individual root-hairs on the plant.

RHD6-related polypeptides are described in more detail above.

Expression of an RHD6-related polypeptide may be increased by any suitable method. In some embodiments, the expression of a RHD6-related polypeptide may be increased by expressing a heterologous nucleic acid encoding the RHD6-related polypeptide within cells of said plant.

Suitable controls will be readily apparent to the skilled person and may include plants in which the expression of the RHD6-related polypeptide is not increased.

A method of producing a plant with altered root hair phenotype may comprise:
incorporating a heterologous nucleic acid which alters the expression of a RHD6-related polypeptide into a plant cell by means of transformation, and;
regenerating the plant from one or more transformed cells.

Suitable RHD6-related polypeptides are described in more detail above.

In some embodiments, the plant may be a plant whose roots are not naturally colonised by symbiotic fungi, such as Mycorrhizae. Plants whose roots are not naturally colonised by fungi include non-mycorrhizal plants such as *Brassicas.*

Plants for use in the methods described herein preferably lack mutations in RHD6-related genes. For example the plant may be a wild-type plant.

A plant with altered root hair phenotype produced as described above may show improved tolerance to nutrient-deficient growth conditions, increased production of phytochemicals and/or increased phytoremediation properties, such as absorption of heavy metals.

Nucleic acid encoding *RHD6*-related polypeptides and their expression in plants is described in more detail above.

In other embodiments, the expression of an RHD6-related polypeptide may be increased by increasing the expression of an endogenous nucleic acid encoding the RHD6-related polypeptide within cells of said plant.

The expression of an endogenous nucleic acid encoding the RHD6-related polypeptide within cells of said plant may be increased by recombinant means, such as the targeted insertion of regulatory factors,

The expression of an endogenous nucleic acid encoding the *RHD6-*related polypeptide within cells of said plant may be increased by non-recombinant means. For example, expression of the RHD6-related polypeptide may be increased in a plant by selective plant breeding methods which employ the RHD6-related amino acid or nucleic acid sequence as a molecular marker in order to.produce a plant having an altered root hair phenotype, for example increased size, number or longevity of root hairs relative to controls.

A method of producing a plant having altered root hair phenotype may comprise:
providing a population of plants,
determining the amount of expression of a *RHD6*-related polypeptide as described herein in one or more plants in the population, and
identifying one or more plants in the population with increased expression of the RHD6-related polypeptide relative to other members of said population.

The identified plants may be further propagated or crossed, for example, with other plants having increased RHD6-related polypeptide expression or self-crossed to produce inbred lines. The expression of an RHD6-related polypeptide in populations of progeny plants may be determined and one or more progeny plants with reduced expression of the *RHD6*-related polypeptide identified.

The expression of an RHD6-related polypeptide in a plant may be determined by any convenient method. In some embodiments, the amount of expression of the RHD6-related polypeptide may be determined at the protein level. A method of producing a plant with altered root hair development may comprise:
providing a population of plants,
determining the amount of RHD6-related polypeptide in one or more plants of said population, and
identifying one or more plants in the population with increased amounts of an RHD6-related polypeptide relative to other members of said population.

The amount of RHD6-related polypeptide may be determined in one or more cells of the plant, preferably cells from a below-ground portion or tissue of the plant, such as the root.

The amount of RHD6-related polypeptide may be determined using any suitable technique. Conveniently, immunological techniques, such as Western blotting may be employed, using antibodies which bind to the RHD6-related polypeptide and show little or no binding to other antigens in the plant. For example, the amount of an RHD6-related polypeptide in a plant cell may be determined by contacting a sample comprising the plant cell with an antibody or other specific binding member directed against the RHD6-related polypeptide, and determining binding of the RHD6-related polypeptide to the sample. The amount of binding of the specific binding member is indicative of the amount of RHD6-related polypeptide which is expressed in the cell.

In other embodiments, the expression of the RHD6-related polypeptide may be determined at the nucleic acid level. For example, the amount of nucleic acid encoding an RHD6-related polypeptide may be determined. A method of producing a plant having altered root hair development may comprise:
providing a population of plants,
determining the level or amount of nucleic acid, for example mRNA, encoding the RHD6-related polypeptide in a cell of one or more plants of said population, and,
identifying one or more plants in the population with increased amount of nucleic acid encoding an RHD6-related polypeptide relative to other members of said population.

The level or amount of encoding nucleic acid in a plant cell may be determined for example by detecting the amount of transcribed encoding nucleic acid in the cell. Numerous suitable methods for determining the amount of a nucleic acid encoding an RHD6-related polypeptide in a plant cell are available in the art, including, for example, Northern blotting or RT-PCR (see for example Molecular Cloning: a Laboratory Manual: 3rd edition, Sambrook & Russell (2001) Cold Spring Harbor Laboratory Press NY; Current Protocols in Molecular Biology, Ausubel et al. eds. John Wiley & Sons (1992); DNA Cloning, The Practical Approach Series (1995); series eds. D. Rickwood and B.D. Hames, IRL Press, Oxford, UK and PCR Protocols: A Guide to Methods and Applications (Innis, et al. 1990. Academic Press, San Diego, Calif.).

A suitable cell may be from a below-ground portion or tissue of the plant, such as the root.

A progeny plant identified as having increased RHD6-related polypeptide expression may be tested for altered root hair development relative to controls, for example increased growth, number or longevity of root hairs, or may be tested for other properties, such as increased resistance to nutrient deficient conditions, increased phytochemical production, increased phytoremediation properties or a consitutive low phosphate response.

A method of producing a plant having an altered root hair phenotype may comprise:
crossing a first and a second plant to produce a population of progeny plants;
determining the expression of a RHD6-related polypeptide in the progeny plants in the population, and
identifying a progeny plant in the population in which expression of the RHD6-related polypeptide is increased relative to controls.

A progeny plant having an altered root hair phenotype may show increased growth, number or longevity of root hairs relative to controls (e.g. other members of the population with a wild-type phenotype).

The identified progeny plant may be further propagated or crossed, for example with the first or second plant (i.e. backcrossing) or self-crossed to produce inbred lines.

The identified progeny plant may be tested for increased tolerance to nutrient-deficient conditions relative to controls.

Other aspects of the invention provide the use of an RHD6-related polypeptide or encoding nucleic acid as described herein as a marker for the selective breeding of a plant which has an altered root hair phenotype relative to control plants, and a method of selective breeding of a plant which has an altered root hair phenotype relative to control plants, which employs the RHD6-related amino acid or encoding nucleic acid sequence.

In some embodiments, plants having reduced expression of the RHD6-related polypeptide may be produced by random mutagenesis, followed by screening of mutants for reduced RHD6-related polypeptide expression. Suitable techniques are well known in the art and include Targeting Induced Local Lesions IN Genomes (TILLING). TILLING is a high-throughput screening technique that results in the systematic identification of non-GMO-derived mutations in specific target genes (Comai and Henikoff, The Plant Journal (2006) 45, 684-694 Till et al_BMC Plant Biol, 2007 Apr 7, 19.

Those skilled in the art will also appreciate that, based on the genetic information disclosed herein, Targeted Induced Local Lesions IN Genomes ("TILLING", e.g. utilizing PCR-based screening of plants generated through chemical mutagenesis (generally via ethyl methane sulfonate (EMS) treatment), often resulting in the isolation of missense and nonsense mutant alleles of the targeted gene(s); TILLING permits the high-throughput identification of mutations in target genes without production of genetically modified organisms and it can be an efficient way to identify mutants in a specific gene that might not confer a strong phenotype by itself), may be carried out to produce plants and offspring thereof with a change in the *RHD6* or *RSL* gene, thereby permitting identification of plants with specific phenotypes relevant to plant root hair production.

A method of producing a plant having an altered root hair phenotype may comprise:
exposing a population of plants to a mutagen,
determining the expression of a RHD6-related polypeptide or nucleic acid in one or more plants in said population, and
identifying a plant with increased expression of the RHD6-related polypeptide relative to other members of said population.

Suitable mutagens include ethane methyl sulfonate (EMS).

Methods for determining the expression of RHD6-related polypeptide or nucleic acid in plants is described in more detail above.

The identified plant may be further tested for increased tolerance or resistance to low-nutrient conditions relative to controls, increased production of phytochemicals or increased phytoremediation.

A plant identified as having increased expression of the RHD6-related polypeptide relative to controls (e.g. other members of the population) may display increased growth, number or longevity of root hairs relative to the controls.

A plant produced or identified as described above may be sexually or asexually propagated or grown to produce off-spring or descendants. Off-spring or descendants of the plant regenerated from the one or more cells may be sexually or asexually propagated or grown. The plant or its off-spring or descendents may be crossed with other plants or with itself.

Expression of RHD6-related genes such as RSL4 is shown herein to produce a phenotype in which the root hairs display a fungus-like morphology. This morphology is characterised by extensive indeterminate masses of growing cells which resemble fungal like colonies (figure 10) and results in a greatly increased surface area of root hairs. This phenotype may confer significantly enhanced root uptake of phosphate and iron, which is largely limited by the length and surface area of the root hair.

By means of expression of the genes described herein in a plant, a plant may be made to exhibit enhanced absorption of otherwise less efficiently absorbed nutrients. Thus, for example, it is known in the art that phosphate and iron absorption from the soil is achieved primarily by plant root hairs. By enhancing the number, length, time of production or duration of survival of plant root hairs, by expression, overexpression, or targeted misexpression (expression in cells that otherwise may not produce root hairs) of RHD6-related genes, absorption of iron or phosphate or both, as well as other nutrients, may be enhanced. In particular, absorption may be increased by the fungus-like root hair morphology produced by overexpression of RHD6-related genes such as AtRSL4 (see figure 10). Thus, it has been shown in the literature that *rhd6* mutants are compromised in their ability to absorb phosphate, see Plant Growth and Phosphorus Accumulation of Wild Type and Two Root Hair Mutants of Arabidopsis thaliana (Brassicaceae)", Terence R. Bates and Jonathan P. Lynch, American Journal of Botany 87(7): 958-963. 2000. This effect may be reversed by supplementation with the functional gene described herein. Aspects of the invention would be of particular benefit, for example, in low-iron or low-phosphate containing soils, such as those found in China, sub-Saharan Africa and Australia

A method of improving the tolerance or resistance of a plant to nutrient deficient conditions may comprise;
increasing the expression of an RHD6-related polypeptide within cells of said plant relative to control plants.

Nutrient deficient conditions include conditions which contain levels of one or more nutrients such as nitrate, phosphate and/or iron, which are insufficient to fulfil the nutritional requirements of the wild-type plant. A wild-type plant subjected to nutrient deficient conditions may adopt a nutrient deficient phenotype, such as reduced growth resulting in greatly reduced yield and crop quality.

For example, the plant may show improved growth in soil which contains low levels of one or more nutrients such as nitrate, phosphate and/or iron, relative to control plants (i.e. plants in which RHD6-related polypeptide expression is unaltered). Furthermore, phosphate deficiency increases the expression of RHD6-related polypeptides such as AtRHD6 and AtRSL1 in the root epidermis of a plant. Expression of a RHD6-related polypeptide in the root epidermis may therefore be useful in producing a constitutive "low phosphate" response in a plant.

The genes described herein may be utilized to achieve enhanced production of compounds of interest, including medicinally relevant compounds. Thus, for example, it is known that plant root hairs are responsible for production of antibiotic compounds. In nature, these compounds are secreted by plant roots and especially plant root hairs, to thereby modify or otherwise control the microflora and microfauna surrounding the plant roots. Production of these phytochemicals is enhanced in plants in which the number, length, duration of production, time of production and other characteristics of root hair development and growth may be modified at will according to the methods of this invention.

A method of increasing the production or secretion of a root-secreted phytochemical in a plant may comprise;
increasing the expression of a RHD6-related polypeptide within cells of a plant which secretes the phytochemical through its roots.

Root-secreted phytochemicals include shikonin (Brigham LA, et al Plant Physiol. 1999 Feb;119(2):417-28) which may be produced by *Lithospermum erythrorhizon,* and paclitaxel, which may be produced by *Taxus* spp.

Heavy metals are an important environmental pollutant and may be removed by growing plants on contaminated soils. In phytoremediation or phytoextraction, plants absorb contaminating substances such as heavy metals from the soil and the plants are harvested at maturity, thereby removing these contaminants from the area. The long root hair phenotypes conferred by increased expression of RHD6-related polypeptides may enhance the phytoremediation properties of plant species.

A method of reducing the amount of a contaminating substance in soil comprising;
increasing the expression of a RHD6-related polypeptide within cells of a plant which absorbs the contaminating substance through its roots,
growing the plant or a descendent thereof in soil which comprises the contaminating substance such that the plant or descendent absorbs the contaminating substance from the soil, and
harvesting said plant or descendent thereof.

Contaminating substances include uranium, polychlorinated biphenyls, salt, arsenic and heavy metals such as cadmium, zinc and lead.

A plant suitable for use in the present methods is preferably a higher plant, for example an agricultural plant selected from the group consisting of *Lithospermum erythrorhizon, Taxus* spp, tobacco, cucurbits, carrot, vegetable brassica, melons, capsicums, grape vines, lettuce, strawberry, oilseed brassica, sugar beet, wheat, barley, maize, rice, soyabeans, peas, sorghum, sunflower, tomato, potato, pepper, chrysanthemum, carnation, linseed, hemp and rye.

In embodiments relating to phytochemical production, *Lithospermum erythrorhizon* and *Taxus* spp may be preferred.

In embodiments relating to phytoremediation, sunflower (*Helianthus annuus*), Chinese Brake fern, alpine pennycress (*Thlaspi caerulescens*), Indian mustard (*Brassica juncea*), Ragweed (*Ambrosia artemisifolia*) Hemp Dogbane *(Apocymun cannabinum)* and Poplar may be preferred.

Another aspect of the invention provides a plant which is produced by a method described herein, wherein said plant shows altered root hair phenotype relative to controls.

For example, a plant may display increased growth, number or longevity of root hairs relative to controls (e.g. other members of the population with a wild-type phenotype).

A plant may display increased tolerance to nutrient deficient conditions and/or increased production of root-secreted phytochemicals.

Also provided is any part or propagule of such a plant, for example seeds, selfed or hybrid progeny and descendants.

A plant according to the present invention may be one which does not breed true in one or more properties. Plant varieties may be excluded, particularly registrable plant varieties according to Plant Breeders Rights.

In addition to a plant produced by a method described herein, the invention encompasses any clone of such a plant, seed, selfed or hybrid progeny and descendants, and any part or propagule of any of these, such as cuttings and seed, which may be used in reproduction or propagation, sexual or asexual. Also encompassed by the invention is a plant which is a sexually or asexually propagated off-spring, clone or descendant of such a plant, or any part or propagule of said plant, off-spring, clone or descendant.

While the foregoing disclosure provides a general description of the subject matter encompassed within the scope of the present invention, including methods, as well as the best mode thereof, of making and using this invention, the following examples are provided to further enable those skilled in the art to practice this invention and to provide a complete written description thereof. However, those skilled in the art will appreciate that the specifics of these examples should not be read as limiting on the invention, the scope of which should be apprehended from the claims and equivalents thereof appended to this disclosure. Various further aspects and embodiments of the present invention will be apparent to those skilled in the art in view of the present disclosure.

All documents mentioned in this specification are incorporated herein by reference in their entirety.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

Certain aspects and embodiments of the invention will now be illustrated by way of example and with reference to the figures described above and table described below.

Tables 1 and 2 show a sequence alignment of bHLH amino acid sequences (Heim et al. Mol. Biol. Evol. 2003) generated by ClustalW (http://www.ebi.ac.uk).

Table 3 shows % identities of RHD6-related proteins as determined by DNA Strider (Christain Mark, Center. d'Etudes de Saclay).

Table 4 shows relative identities of the bHLH domains of RHD6-related proteins to RHD6.

Table 5 shoes the correspondence between the names on the tree of figure 3 and the alignments of tables 1 and 2 with respective species and locus or GI accession number.

### EXAMPLES

Root hairs are highly polarised cells that increase the surface area of the plant that is in contact with the soil. They play important roles in nutrient acquisition and anchorage in those land plants that have roots^{1, 2}. Other tip growing cells such as rhizoids and caulonemal cells have a similar function in more basal groups of land plants that lack roots^{3, 4}. Here we identify and characterise two basic helix loop helix transcription factors that control the development of root hair cells in Arabidopsis sporophyte and show that their closest homologs in *Physcomitrella patens* are required for the development of both rhizoids and caulonemal cells in the gametophyte of this moss. This indicates that an ancient mechanism controls the development of functionally and morphologically similar but non-homologous cell types in these divergent groups of land plants. This suggests that the evolution of the land plant body over the past 475 million years^{5, 6} has resulted at least in part from the independent recruitment of genes from the gametophyte to the sporophyte.

Unless, stated otherwise, standard techniques were as follows:

### RT-PCR

Total RNA (5 µg for A. thaliana and 1 µg for P. patens) was reverse transcribed with the Superscript First Strand synthesis system (Invitrogen, Carlsbad, USA) in a 20 µl reaction containing oligo d (T)12-18 primer. One µl of this product was used for PCR in 20 µl reactions containing primers described herein.

### Pollen growth experiments

In vivo and in vitro pollen tube growth experiments were done as described previously (44, 45).

### Southern analysis of Inserts

Southern blots were performed with the DIG System for PCR labelling of DNA probes (Roche Diagnostics, Penzberg, Germany) according to manufacturer protocol Hybridization was done at 42°C in DIG Easy Hyb hybridization buffer.

### Arabidopsis growth conditions.

Arabidopsis thaliana (L.) Heyn. lines were grown vertically for 4 days on MS medium + 2% sucrose solidified with 0.5% Phytagel at 24 °C under continuous illumination. For the cellophane disc experiment, the agar was overlaid with a cellophane disk (AA packaging, Preston, UK) before application of the seeds.

### Enhancer trapping and cloning of the AtRHD6 gene

*Atrhd6-2* is an enhancer trap line (1261) of Arabidopsis (ecotype Lansberg erecta) generated with the *DsE* element. ¹⁸ that was screened for root hairless phenotype and reporter gene expression in hair cells. Failure to complement *Atrhd6-1* ⁷ indicated that line 1261 carries a mutation that is allelic to *Atrhd6-1.* The DNA sequence flanking the *DsE* element insertion was identified by inverse-PCR ¹⁹. Genomic DNA of the 1261 line was digested by Sau3A I and subsequently ligated using T4 DNA ligase. The ligated DNA was used for PCR with Ds element-specific primers. This showed that the *DsE* is inserted 111 bp upstream the ATG site of *At1g66470* gene (Fig. 7).

### GUS staining of Arabidopsis thaliana roots and embedding

Four-days-old seedlings were stained for 12 hr at 37°C in 1 mM 5-bromo-4-chloro-3-indolyl-glucuronide, 0.5 mM potassium ferricyanide, 0.5 mM potassium ferrocyanide, and 10 mM sodium phosphate buffer (pH 7). Seedlings were embedding in Technovit 7100^{®} resin (Kulzer GmbH, Germany) according to the manufacturer instructions and 10 µm transverse sections were taken from roots.

### Identification of A. thaliana mutants and generation of transgenic plants

Verification of the T-DNA insertion sites in mutants used in this work (Fig. 7) was carried out by sequencing PCR fragments amplified with primers described herein. Atrhd6-3 (ecotype Columbia 0) correspond to the GABI-Kat line 475E09 (10). Atrs11-1 (ecotype Columbia 0) corresponding to line WiscDsLox356A02 comes from the Biotechnology centre of the University of Wisconsin. cpc, wer, ttg1 and g12 mutants have been described previously (32-35).

The genomic constructs AtRHD6p::GFP:AtRHD6 and AtRSL1p::GFP:AtRSL1 contain the promoter and 5'UTR of AtRHD6 or ATRSL1 upstream of the GFP coding sequence fused in N-terminal to the AtRHD6 or AtRSL1 coding region including introns and the AtRHD6 or AtRSL1 3'UTR with terminator. These constructs were generated using the Gateway system (Invitrogen, Carlsbad, USA). The AtRHD6 or AtRSL1 promoter + 5'UTR and the AtRHD6 or AtRSL1 coding region + 3'UTR + terminator were amplified with PCR primers containing recombination sequences and cloned into pDONR P4-P1R and pDONR P2R-P3. A GATEWAY multisite reaction was then performed with the two resulting pDONR plasmids, the plasmid p207-GFP2.5 and the binary vector pGWBmultisite (destination vector). The binary vector pGWBmultisite was generated by replacing the R1-CmR-ccdB-R2 cassette of pGWB1 into R4-CmR-ccdB-R3 (pGWB1 is from Tsuyoshi Nakagawa, Shimane University, Japan). AtRHD6p::GFP:AtRHD6 and AtRSL1p::GFP:AtRSL1 were transformed respectively in Atrhd6-3 and in Atrhd6-3 Atrsl1-1 double mutant by floral dip (36) and transformants were selected on kanamycin (50 µg/ml) and hygromycin (50 µl/ml). Nine independent transgenic lines containing the AtRHD6p::GFP:AtRHD6 construct in the Atrhd6-3 background were obtained. They show different levels of complementation of the AtRhd6- hairless phenotype but all lines express the GFP in hair cells before the emergence of root hair. Five independent lines having the same GFP expression pattern and restore the Atrhd6-3 phenotype were obtained for AtRSL1p::GFP:AtRSL1 transformation in Atrhd6 Atrsl1.

For the p35S::PpRSL1 construct, the PpRSL1 coding sequence was amplified from protonema cDNA. This fragment was cloned between the BamHI and SalI sites of a modified pCAMBIA1300 plasmid containing the CaMV 35S promoter and the terminator of pea Rubisco small subunit E9 from 35S-pCAMBIA1301 cloned into its EcoRI and PstI sites (37). The p35S::PpRSL1 construct was transformed by floral dip in Atrhd6-3 and transformants were selected on hygromycin (50 µg/ml). Ten independent transgenic lines that complement the Atrhd6-3 hairless phenotype were obtained.

### Physcomitrella genes isolation and phylogenetic analyses

Physcomitrella *RSLs* and the *PpIND1* genome sequences where obtained from BLAST of the available genome sequence assembled into contigs (http://moss.nibb.ac.jp/). The splice sites were predicted with NetPlantGene 20 and the bHLH coding sequences were confirmed by RTPCR and sequencing. The full length coding sequence of *PpRSL1* was obtained by sequencing EST clone pdp31414, provided by the RIKEN BioResource Center ²¹ and the full length coding sequence of *PpRSL2* was obtained by RT-PCR. Sequences have been deposited to GenBank as follows: PpRSL1 (EF156393), PpRSL2 (EF156394), PpRSL3 (EF156395), PpRSL4 (EF156396), PpRSL5 (EF156397) PpRSL6 (EF156398), PpRSL7 (EF156399) and PpIND1 (EF156400). The phylogenetic analysis was performed with PAUP* software as described previously ²².

### Physcomitrella growth conditions

The Gransden wild type strain of *Physcomitrella patens* (Hedw.) Bruch and Schimp 23 was used in this study. Cultures were grown at 25°C and illuminated with a light regime of 16 h light /8h darkness and a quantum irradiance of 40 µE m-2s-1. For the analysis of protonema phenotype, spores kept at 4°C for at least 1 month were germinated in a 5 ml top agar (0.8%) plated on 9 cm Petri dish containing 25 ml of 0.8% agar overlaid with a cellophane disk (AA packaging, Preston, UK). Leafy gametophores were grown on 100 times diluted minimal media ²⁴ supplemented with 5 mg/L NH4 tartrate and 50 mg/L Glucose.

### Constructing mutants in Physcomitrella genes

The constructs for Physcomitrella transformation were made in plasmids pBNRF and pBHSNR. pBNRF carries a *NptII* gene driven by a 35S promoter cloned in the *Eco*RI site of pBilox, a derivative of pMCS5 (MoBiTec, Goettingen Germany) carrying two direct repeats of the loxP sites cloned in the *Xho*I-*Kpn*I and *Bgl*II-*Spe*I sites. pBHSNR contains a *AphIV* gene driven by a 35S promoter clone between the 2 loxP sites of pBilox using *Sac*I and *Not*I. pPpRSL1-KO was made by cloning *PpRSL1* genomic fragment 1 in pBNRF digested with *Xba*I and *Xho*I and then cloning *PpRSL1* genomic fragment 2 in the resulting plasmid digested with *Hpa*I and *Asc*I. pPpRSL2-KO was made by cloning *PpRSL2* genomic fragment 1 in pBHSNR digested with *Mlu*I and *Spe*I and then cloning *PpRSL2* genomic fragment 2 in the resulting plasmid digested with *Bam*HI and *Hind*III.

### PEG transformation of protoplasts

PEG transformation of protoplasts was done as described previously ²⁵. pPpRSL1-KO was linearised with *Sca*I and *Ssp*I before protoplast transformation and transformants were selected on G418 (50 µl/ml). pPpRSL2-KO was linearised with *Bcl*I and *Ssp*I before protoplast transformation and transformants were selected on Hygromycin B (25 µl/ml). The *Pprsl1 Pprsl2* double mutants were obtained by transformation of *Pprsl1* line 1 with the pPpRSL2-KO construct. Stable transformants were first selected by PCR using primers flanking the recombination-sites and then analysed by Southern blot and RT-PCR. For each transformation, three independent lines having the expected single insertion pattern and being RNA null mutants were selected (Fig. 9). In each case, the 3 transformants selected had the same phenotype.

### Plants overexpressing RHD6 or RSL Genes

For the 35S::RHD6 and RSL2, 3, and 4 constructs, the coding sequence of each gene was amplified from root cDNA with primers as listed below. This fragment was subcloned into a modified pCAMBIA1300 plasmid containing the CaMV 35S promoter and the terminator of pea Rubisco small subunit E9. All these overexpression constructs were transformed by floral dip in *rhd6*/*rsl1* and transformants were selected on hygromycin (50 µl/ml)
35S::RHD6
   CCAGGATCCATGGCACTCGTTAATGACCAT
   CCAGTCGACTTAATTGGTGATCAGATTCGAA
35S::RSL2
   CCAGGATCCATGGGAGAATGGAGCAACAA
   CCAGTCGACTCATCTCGGTGAGCTGAGA
35S::RSL3
   CGGGGTACC ATGGAAGCCATGGGAGAAT
   CGCGGATCC TCATCTGGTCAGTGCATTGAG
35S::RSL4
   CGGGGTACCATGGACGTTTTTGTTGATGGT
   CGCGGATCCTCACATAAGCCGAGACAAAAG

### Results

The Arabidopsis root epidermis is organised in alternate rows of hair forming cells (H cells) that produce a tip growing protuberance (root hairs) and rows of non-hair cells (N cells) that remain hairless. *AtRHD6* (*ROOT HAIR DEFECTIVE 6*) positively regulates the development of H cells - *Atrhd6* mutants develop few root hairs (Fig. 1a)⁷.

We cloned *AtRHD6* using an enhancer trap line (*Atrhd6-2*) in which the *GUS* reporter gene is expressed in H cells but not in N cells (Fig.1 c, d, Fig. 7). *AtRHD6* encodes the basic-Helix-loop-helix (bHLH) transcription factor At1g66470⁸. The identification of another independent allele (*Atrhd6-3*) with a similar phenotype and the complementation of the *Atrhd6-3* mutation with a whole gene *AtRHD6p::GFP:AtRHD6* fusion confirmed that the defect in root hair development observed in this mutant is due to mutation of *At1g66470* (Fig. 1a). This complementing *AtRHD6p::GFP:AtRHD6* fusion indicates that AtRHD6 protein accumulates in H cell nuclei in the meristem and elongation zones (Fig. 1b) but disappears before the emergence of the root hair. The spatial pattern of N cells and H cells in the Arabidopsis root epidermis is controlled by a transcriptional network including the positive regulator of H cell identity *CPC* and the negative regulators of H cell identity *WER, TTG* and *GL2*⁹.

To determine if *AtRHD6* is regulated by these genes we analysed the promoter activity of the *Atrhd6*-2 enhancer trap in different mutant backgrounds. While the *Atrhd6-2* enhancer trap expresses GUS in cells in the H position this expression spreads to the cells in the N position in the *wer, ttg* and *gl2* mutant backgrounds indicating that *WER, TTG* and *GL2* negatively regulate transcription of *AtRHD6* in the N position (Fig. 1d). No expression was observed in the cpc mutant indicating that CPC positively regulates *AtRHD6* expression (Fig. 1d). Thus, *AtRHD6* controls the development of root hair cells and acts downstream of the genes involved in epidermal pattern formation.

AtRHD6 is a member of sub family VIIIc of bHLH transcription factors that comprises five other members ^{8,10}. One of these genes, *At5g37800,* hereafter named *RHD SIX-LIKE1* (*AtRSL1*), is very similar to *AtRHD6* and these two genes may derive from a relatively recent duplication event ⁸. This provides indication that *AtRHD6* and *AtRSL1* might have redundant functions. To determine if AtRSL1 is also required for root hair development we identified a line (*Atrsl1-1*) carrying a complete loss of function mutation in the *AtRSL1* gene and created the *Atrhd6-3 Atrsl1-1* double mutant (Fig. 7).

Because no new phenotypes were observed when these mutants were grown in our standard growth conditions, we grew them on the surface of cellophane discs, where small numbers of root hairs develop in the *Atrhd6-3* single mutant (Fig. 2a). Plants homozygous for the *Atrsl1-1* mutation had wild type root hair morphology when grown on cellophane discs (Fig. 2a). However, the *Atrhd6-3 Atrsl1-1* double mutant did not develop root hairs, indicating that *AtRHD6* and *AtRSL1* have partially redundant functions in root hair development (Fig. 2a). *Atrhd6-3 Atrsl1-1* double mutant plants carrying the genomic construct *AtRSL1p::GFP:AtRSL1* displayed the AtRhd6-3- mutant phenotype, confirming that the extreme hairless phenotype of the *Atrhd6-3 Atrsl1-1* double mutant is the result of a loss of function of both *AtRHD6* and *AtRSL1* genes (Fig. 2a). The complementing GFP:AtRSL1 fusion protein accumulates in hair cells nuclei in the meristem and elongation zones, indicating that *AtRHD6* and *AtRSL1* have similar expression patterns (Fig. 2b). These data indicate that *AtRSL1* and *AtRHD6* act together to positively regulate root hair development.

To determine if *AtRHD6* and *AtRSL1* are required for the development of the only other tip growing cell in flowering plants, the pollen tube, we characterised the phenotypes of pollen tubes in *Atrhd6-3*, *Atrsl1-1* and *Atrhd6-3 Atrsl1-1* mutants both *in vitro* and *in vivo*. We detected neither a defect in pollen tube growth nor in the segregation of mutant alleles in the F2 progeny of backcrosses to wild type (Fig. 8). No other defective phenotype was detected in any other part of *Atrhd6-3, Atrsl1-1* or *Atrhd6-3 Atrsl1-1* mutants.

Together these data indicate that AtRHD6 and AtRSL1 are bHLH transcription factors that are specifically required for the development of root hairs and act downstream of the genes that regulate epidermal pattern formation in the flowering plant Arabidopsis.

The most ancestral grade of land plants are the bryophytes - the earliest micro fossils of land plants from the middle Ordovician circa 475 Ma have bryophyte characteristics ⁶. Bryophytes do not have roots but possess tip-growing cells that are morphologically similar to root hairs and fulfil rooting functions. In mosses, caulonemal cells increase the surface area of the filamentous protonema tissue in contact with the substrate and rhizoids anchor the leafy gametophore to their growth substrate ^{3, 4} and both cell types are hypothesised to be involved in nutrient acquisition ³. However, rhizoids and caulonema develop from the gametophyte of mosses whereas root hairs develop from the sporophyte of modern vascular plants. Thus, according to the current view that land plants evolved by the intercalation of a sporophytic generation from a haplontic algal ancestor followed by the progressive increase of size and complexity of the sporophyte in parallel to a reduction of the gametophyte ^{11, 12}, neither rhizoids nor caulonema are homologous to root hairs.

To determine if the developmental mechanism that controls the development of root hairs in angiosperms also controls the development of non-homologous tip growing cells with a rooting function in bryophytes, we identified *RHD6-LIKE* genes from the moss *Physcomitrella patens.* We identified seven members of the *AtRHD6* subfamily of bHLH genes from the publicly available Physcomitrella genomic sequence (http://moss.nibb.ac.jp/) providing indication that these genes have been conserved through the land plant evolution. These were designated *Physcomitrella patens RHD6-LIKE 1* to 7 (*PpRSL1* to *PpRSL7*). To analyse the relationship between Physcomitrella and Arabidopsis *RSL* genes we constructed phylogenetic trees by maximum parsimony. A strict consensus tree is presented in Fig. 3. This shows that *AtRHD6, AtRSL1* and the two *Physcomitrella PpRSL1* and *PpRSL2* genes are closely related and together form a monophyletic clade (AtRHD6 clade) that is sister to the clade comprising all the other members of the subfamily (sister clade) (Fig. 3). This indicates that the AtRHD6 clade evolved before the separation of the bryophytes and the vascular plants from a common ancestor.

To characterize the function of the *RHD6-LIKE* genes in moss we constructed deletion mutants that lacked the function of *PpRSL1* and *PpRSL2* genes and determined if they develop morphological defects. Three independent RNA null mutants with single insertions in *PpRSL1* and in *PpRSL2* were made. Double mutants with single insertions into both genes were also generated (Fig. 9). The phenotypes of each of these mutants were then analysed. A haploid protonema develops upon germination of a wild type Physcomitrella spore 3. This filamentous tissue comprises two cell types, the chloronema and the caulonema (Fig. 4a, b). Chloronemal cells contain large chloroplasts and grow by a slow tip growth mechanism. Caulonemal cells are more elongated, contain few smaller chloroplasts, grow by rapid tip growth and are involved in the colonization of the substrate. Leafy gametophores usually develop from caulonema and are anchored to their substrate by tip growing multicellular rhizoids that are morphologically similar to caulonema (Fig. 4c). The *Pprsl1* and *Pprsl2* single mutants have slightly smaller and greener protonema cultures than WT and this phenotype is much stronger in the *Pprsl1 Pprsl2* double mutant which produces small dark green protonema (Fig. 4a). *Pprsl1* and *Pprsl2* single mutants produce fewer caulonemal cells than the WT indicating that the greener protonema phenotype is the result of a defect in the development of caulonemal cells (Fig. 4b). No caulonemal cells develop in the *Pprsl1 Pprsl2* double mutant.and the protonema of this mutant comprises chloronemal cells only (Fig. 4b). In wild type plants gametophores develop from caulonema but in the *Pprsl1 Pprsl2* double mutants the gametophores develop from chloronema, as previously observed in another caulonema defective mutant ¹³. The gametophores of the *Pprsl1 Pprsl2* double mutant develop few very short rhizoids (Fig. 4c). No other defective phenotypes were detected in the chloronema, in the leafy part of the gametophore or in the sporophyte in the single or double mutants. This indicates that *PpRSL1* and *PpRSL2* together regulate the development of caulonemal cells and rhizoids in the moss gametophyte. To determine if protein function is conserved across the land plants we performed a cross-species complementation experiment. Expression of *PpRSL1* under the CaMV35S promoter in the *Atrhd6-3* mutant resulted in the formation of wild type root hairs (Fig. 4d). Thus, the moss *PpRSL1* gene can substitute for loss of *AtRHD6* function in Arabidopsis. This indicates that the molecular function of *PpRSL1* and *AtRHD6* has been conserved since the divergence of seed plants and mosses from a common ancestor and suggests that the same molecular mechanism controls the development of Arabidopsis root hairs and Physcomitrella caulonema and rhizoids.

Plants were engineered to over-express RHD6 or RSL Genes using a constitutive promoter, as described above. The phenotype of these transformants is described below:

### 35S::RHD6

The deficient of root hair phenotype of *rhd6*/*rsl1* can be rescued by the over-expression of RHD6. The transformants get longer root hair and higher percent of ectopic root hair (root hairs developed on the non hair cells) than col-0. A few root hairs can be observed on the hypocotyls.

Phosphate deficiency alters *AtRHD6* and *AtRSL1* gene expression. When grown in the presence of sufficient phosphate these genes are expressed in the meristem and elongation zone and transcription is down regulated in the regions where hairs form. When growing in conditions where phosphate is limiting, *AtRHD6* and *AtRSL1* are expressed in the root hair forming zone where they positively regulate the development of root hairs. This shows that phosphate deficiency promotes expression. Therefore, expressing high levels of *AtRSL1* and *AtRHD6* in the root epidermis results in a constitutive "low phosphate" response.

### 35S::RSL2 and 35S::RSL3

The *rhd6*/*rsl1* plants harbouring *35S::RSL2* or *35S::RSL3* constructs also develop some root hair. The root hairs are longer than the col-0. Some transgenic lines showed swollen epidermal cells on the roots and hypocotyls. There are also some root hairs on the hypocotyls.

Over-expression of *AtRSL2* and *AtRSL3* using CaMV35S promoter in wild type plants results in the development of long root hairs. These hairs are not as long as those that form fungal like colonies upon over expression of AtRSL4. Plants over-expressing AtRSL2 and AtRSL3 develop stunted phenotypes. This may be due to expression in non-root hair cells.

### 35S::RSL4

The deficient of root hair phenotype of *rhd6*/*rsl1* can also be partially complemented by introducing the over expression of RSL4. The transformants show longer root hair than col-0. A few root hairs were also detected on the hypocotyls, which is quite similar with that of RHD6 overexpression transformants.

Over-expression of *AtRSL4* using CaMV35S promoter in wild type plants, causes the formation of long root hairs which can form extensive indeterminate growing masses of cells resembling fungal like colonies (figure 10). The root hair system of a plant overexpressing RSL4 is shown in figure 10. The root is surrounded by a mass of fungus-like cells, which resemble mycorrhizae, the nutrient scavanging fungi that form associations with roots. Furthermore, when the RSL4 is expressed by the 35S promoter, this phenotypic effect (long root hairs) was found to be restricted to the root hair cells. No defective phenotypes resulting from the RSL4 expression were observed elsewhere in the plant.

Plants overexpressing RHD6-related genes may therefore have increased nutrient uptake ability because of their increased surface area resulting from enhanced root hair growth. This effect may be marked in plants, such as Brassicas, which are devoid of mycorrhizae throughout their entire life cycle.

Here we show how closely related transcription factors control the development of tip growing cells that have a rooting function in the seed plant sporophyte and the bryophyte gametophyte. These data indicate that we have identified an ancient developmental mechanism that was present in the common ancestor of the mosses and vascular plants (tracheophytes). These genes will have been important for the invasion of land by plants when nutrient acquisition and anchorage to the solid substrate of the continental surface was necessary. The observation that rhizoids have been found on some of the oldest macro fossils of land plants is consistent with this view ¹⁴⁻¹⁶.

Our results provide indication that *RHD6-LIKE* genes functioned in the haploid generation (gametophyte) of the early land plant life cycle which may have been bryophyte-like ¹⁴, where they controlled the formation of cells with a rooting function. We propose that during the subsequent radiation of the land plants these genes were deployed in the development of the diploid generation (sporophyte) of vascular plants where they control the development of root hairs in angiosperms and we predict they control the development of root hairs and rhizoids in lycophytes (clubmosses and allies) and monilophytes (ferns and horse tails). It is likely that such independent recruitment of genes from haploid to diploid phases of the life cycle was in part responsible for the explosion in morphological diversity of the diploid stage of the life cycle (sporophyte) that occurred in the middle Palaeozoic when green plants colonised the continental surfaces of the planet ¹⁷.

### REFERENCES

1. Carol, R. J. & Dolan, L. Philos. Trans. R. Soc. Lond. B Biol. Sci. 357, 815-21 (2002).
2. Gahoonia, T. S., Care, D. & Nielsen, N. E. Plant Soil 191, 181-188 (1997).
3. Duckett, J. G. et al. Protonemal morphogenesis. In Bryology for the twenty-first century (eds. Bates, J. W., Ashton, N. W. & Duckett, J. G.) 223-245 (British Bryological Society, 1998).
4. Sakakibara, K. et al. Development 130, 4835-4846 (2003).
5. Kenrick, P. & Crane, P. R. Nature 389, 33-39 (1997).
6. Wellman, C. H. et al Nature 425, 282-285 (2003).
7. Masucci, J. D. et al Plant Physiol. 106, 1335-1346 (1994).
8. Heim, M. A. et al. Mol. Biol. Evol. 20, 735-747 (2003).
9. Schiefelbein, J. Curr. Opin. Plant Biol. 6, 74-78 (2003).
10. Bailey, P. C. et al. Plant Cell 15, 2497-2501 (2003).
11. Blackwell, W. H. Bot. Rev. 69, 125-148 (2003).
12. Graham, L. E. et al Proc. Natl Acad. Sci. USA 97, 4435-4540 (2000).
13. Thelander, M. et al J. Exp. Bot. 56, 653-662 (2005).
14. Edwards, D. et al Nature 374, 635-636 (1995).
15. Kerp, H. et al New Data on Nothia aphylla Lyon 1964 ex El-Saadawy et Lacey 1979, a Poorly Known Plant from the Lower Devonian Rynie Chert. In Plants Invade the Land (eds. Gensel, P. & Edwards, D.) 52-82 (Columbia University Press, New York, 2001).
16. Kerp, H. et al T. Roy. Soc. Edin-Earth 94, 411-428 (2004).
17. Davis, P. & Kenrick, P. Fossil Plants (The Natural History, Museum, London, 2004).
18. Sundaresan, V. et al. Genes Dev. 9, 1797-1810 (1995).
19. Long, D. et al. Mol. Gen. Genet. 241, 627-636 (1993).
20. Hebsgaard, S. M. et al. Nucleic Acids Res. 24, 3439-3452 (1996).
21. Nishiyama, T. et al. PNAS. USA 100, 8007-8012 (2003).
22. Harrison, C. J. & Langdale, J. A. Plant J. 45, 561-572 (2006).
23. Ashton, N. W. & Cove, D. J. Mol. Gen. Genet. 154, 87-95 (1977).
24. Ashton, N. W. et al Planta 144, 427-435 (1979).
25. Schaefer, D. G. & Zryd, J. P. Plant J. 11, 1195-1206 (1997).
26. Liljegren, S. J. et al. Cell 116, 843-853 (2004).
27. Schiefelbein, J. W. et al The Plant Cell, 2, 235-243 (1990).
28. V. Sundaresan et al., Genes Dev. 9, 1797 (1995).
29. J. D. Masucci et al Plant Physiol. 106, 1335 (1994).
30. D. Long et al., Mol. & Gen. Genet. 241, 627 (1993).
31. M. G. Rosso et al., Plant Mol. Biol. 53, 247 (2003).
32. M. E. Galway et al., Dev. Biol. 166, 740 (1994).
33. M. M. Lee, J. Schiefelbein, Cell 99, 473 (1999).
34. J. D. Masucci, J. W. Schiefelbein, Plant Cell 8, 1505 (1996).
35. T. Wada et al Science 277, 1113 (1997).
36. S. J. Clough, A. F. Bent, Plant J. 16, 735 (1998).
37. K. K. Yi et al., Plant Physiol. 138, 2087 (2005).
38. S. M. Hebsgaard et al., Nucleic-Acids Res. 24, (Sep 1, 1996).
39. T. Nishiyama et al. PNAS USA 100, 8007 (2003).
40. C. J. Harrison, J. A. Langdale, Plant J. 45, 561 (2006).
41. N. W. Ashton, D. J. Cove, Mol. & Gen. Genet. 154, 87 (1977).
42. N. W. Ashton, N. H. Grimsley, D. J. Cove, Planta 144, 427 (1979).
43. D. G. Schaefer, J. P. Zryd, Plant J. 11, 1195 (1997).
44. L. M. Fan et al J. Exp. Bot. 52, 1603 (2001).
45. E. Ryan, et al New Phytol. 138, 49 (1998).

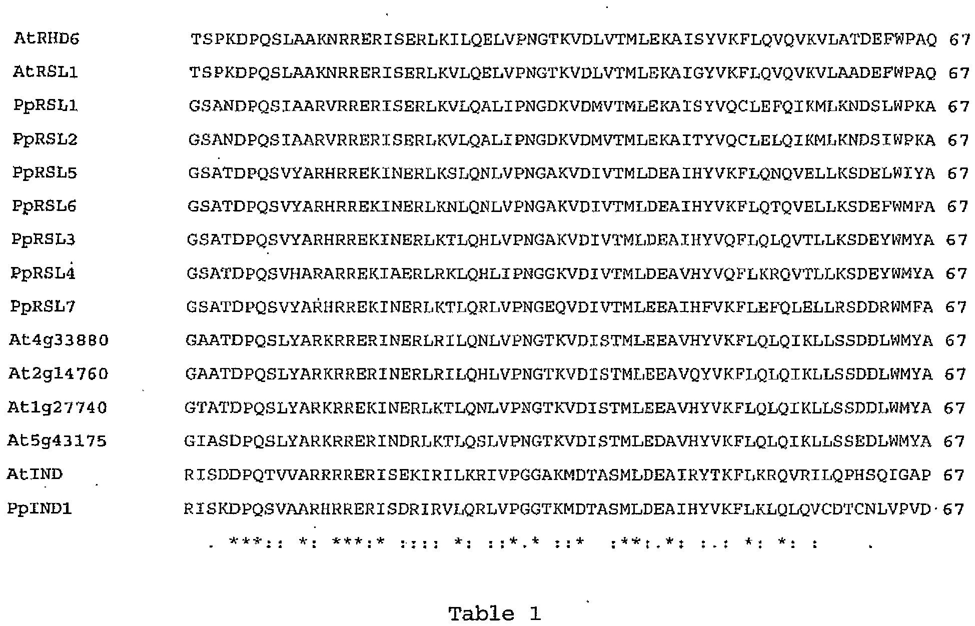

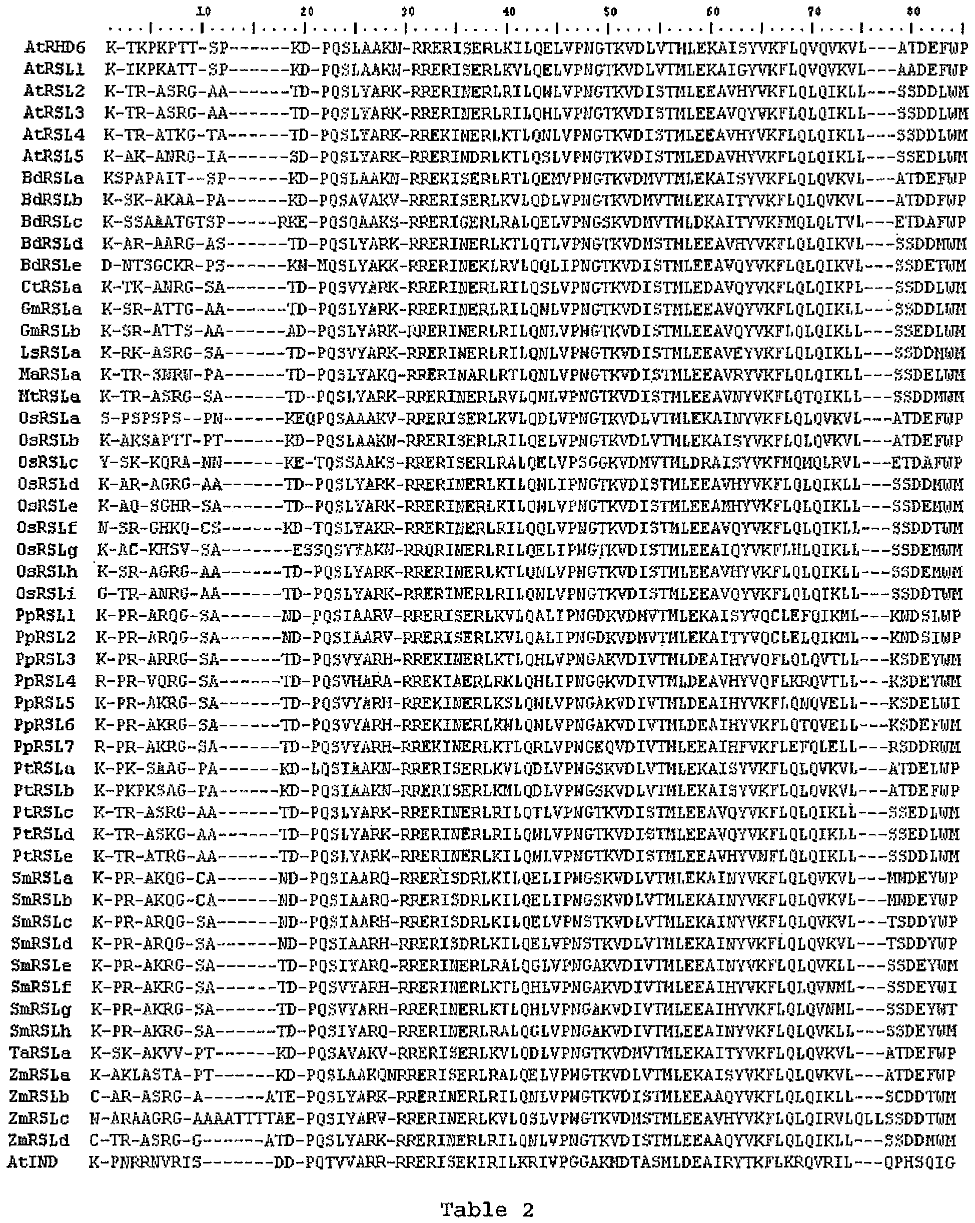

**Table 3**

| **Gene** | **Database reference** | **% Identity with RHD6** | |
|---|---|---|---|
| | | **over full length** | **over bHLH domain** |
| RHD6 | At1g66470 | 100 | 100 |
| RSL1 | At5g37800 | 58.8 | 95.5 |
| RSL2 | At4g33880 | 19.1 | 75 |
| RSL3 | At2g14760 | 22.7 | 75 |
| RSL4 | At1g27740 | 23.2 | 73.1 |
| RSL5 | At5g43175 | 22.3 | 73.1 |

**Table 4**

| Protein | **Sequence Identity AtRHD6** | Protein | **Sequence Identity AtRHD6** |
|---|---|---|---|
| AtRHD6 | 1 | PpRSL1 | 0.625 |
| AtRSL1 | 0.93 | PpRSL2 | 0.611 |
| AtRSL2 | 0.611 | PpRSL3 | 0.583 |
| AtRSL3 | 0.611 | PpRSL4 | 0.513 |
| AtRSL4 | 0.597 | PpRSL5 | 0.597 |
| AtRSL5 | 0.583 | PpRSL6 | 0.611 |
| BdRSLa | 0.835 | PpRSL7 | 0.527 |
| BdRSLb | 0.763 | PtRSLa | 0.791 |
| BdRSLc | 0.675 | PtRSLb | 0.847 |
| BdRSLd | 0.611 | PtRSLc | 0.597 |
| BdRSLe | 0.569 | PtRSLd | 0.597 |
| CtRSLa | 0.625 | PtRSLe | 0.611 |
| GmRSLa | 0.611 | SmRSLa | 0.708 |
| GmRSLb | 0.597 | SmRSLb | 0.708 |
| LsRSLa | 0.611 | SmRSLc | 0.722 |
| MaRSLa | 0.611 | SmRSLd | 0.722 |
| MtRSLa | 0.611 | SmRSLe | 0.625 |
| OsRSLa | 0.78 | SmRSLf | 0.625 |
| OsRSLb | 0.902 | SmRSLg | 0.625 |
| OsRSLc | 0.652 | SmRSLh | 0.625 |
| OsRSLd | 0.597 | TaRSLa | 0.777 |
| OsRSLe | 0.638 | ZmRSLa | 0.835 |
| OsRSLf | 0.597 | ZmRSLb | 0.561 |
| OsRSLg | 0.602 | ZmRSLc | 0.493 |
| OsRSLh | 0.611 | ZmRSLd | 0.589 |
| OsRSLi | 0.597 | *AtIND* | *0.424* |

**Table 5**

| **Name** | **Species** | **Locus name** | **GI** | **accession** |
|---|---|---|---|---|
| AtRHD6 | *Arabidopsis thaliana* | AT1G66470 | | |
| AtRSL1 | | AT5G37800 | | |
| AtRSL2 | | AT4G33880 | | |
| AtRSL3 | | AT2G14760 | | |
| AtRSL4 | | AT1G27740 | | |
| AtRSL5 | | AT5G43175 | | |
| BdRSLa | *Brachypodium distachyon* | | | |
| BdRSLb | | | | |
| BdRSLc | | | N/A | |
| BdRSLd | | | | |
| BdRSLe | | | | |
| CtRSLa | *Carthamus tinctorius* | | | 125399878 |
| GmRSLa | *Glycine max* | | | 26056905 |
| GmRSLb | | | | 15663066 |
| LsRSLa | *Lactuca saligna* | | | 83790803 |
| MaRSLa | *Musa acuminata* | | | 102139852 |
| MtRSLa | *Medicago truncatula* | | | 92870204 |
| OsRSLa | *Oryza sativa ssp japonica* | Os01g02110 | | |
| OsRSLb | | Os02g48060 | | |
| OsRSLc | | Os06g30090 | | |
| OsRSLd | | Os03g10770 | | |
| OsRSLe | | Os03g42100 | | |
| OsRSLi | | Os07g39940 | | |
| OsRSLf | | Os11g41640 | | |
| OsRSLg | | Os12g32400 | | |
| OsRSLh | | Os12g39850 | | |
| PpRSL1 | | | | 140084326 |
| PpRSL2 | | | | 140084333 |
| PpRSL3 | | | | |
| PpRSL4 | *Physcomitrella patens* | | | |
| PpRSL5 | | | | |
| PpRSL6 | | | | |
| PpRSL7 | | | | |
| PtRSLa | | | | |
| PtRSLb | | | | |
| PtRSLc | *Populus trichocarpa* | | | |
| PtRSLd | | | | |
| PtRSLe | | | N/A | |
| SmRSLa | | | | |
| SmRSLh | | | | |
| SmRSLb | | | | |
| SmRSLc | *Selaginella moelendorfii* | | | |
| SmRSLd | | | | |
| SmRSLe | | | | |
| SmRSLf | | | | |
| SmRSLg | | | | |
| TaRSLa | *Triticum aestivum* | | | |
| ZmRSLa | *Zea mays* | AZM4_60871 | | |
| ZmRSLb | | AZM4_70092 | | |
| ZmRSLc | | AZM4_91750 | | |
| ZmRSLd | | AZM4_86104 | | |

### PRIMERS

*A*,*Amplification and sequencing of the insertion sites of A. thaliana mutants*
   *rhd6-1* insertion site
      AtRHD6-A: 5'-GGATTGATT`I'AATTACCATATTTAT-3'
      LB2: 5'-CAAGTATCAAACGATGTG- 3'
   rhd6-2 insertion site (inverse PCR)
      DL3: 5'-CACCG GTACCGACCGTTACCGACCG-3'
      Ds3I2: 5'-TACCGGTACCGAAAACGAACGGGA-3'
   rhd6-3 insertion site
      AtRHD6-B: 5'-GTTCCCAATGGCACCAAGGTACA-3'
      GABI-LB: 5'-CCCAT`I`TGGACGTGAATGTAGACAC-3'
   *rsl1-1* insertion site
      AtRSL1-A: 5'-CGTGTGGACCGACGTCTGA
      JL-202: 5'-CATTTTATAATAACGCTGCGGACATCTAC-3'
*B*, *Construction of the pRHD6::GFP-RHD6::RHD6t plasmid* Amplification of the *AtRHD6* promoter + 5'UTR fragment from BAC F28G11 
RHD6prom-attB4F: 5'-GGGACAACTTTGTATAGAAAAGTTGTTCTCAAAGAGGG
ACAAGACCAAAGCCCATGAC-3'
 
RHD6prom-attB1R: 5'-GGGGACTGCTTTTTTGTACAAACTTGCTAGACACTAATA
AGTTTGATAAGTGATTTTTTGT-3'
   Amplification of the *AtRHD6* coding region + 3'UTR + terminator fragment from BAC F28G11 
RHD6term-attB2F: 5'-GGGGACAGCTTTCTTGTACAAAGTGGCCATGGCACTCG
TTAATGACCATCCCAACGAGA-3'
 
RHD6term-attB3R: 5'-GGGGACAACTTTGTATAATAAAGTTGCTGATAAATCGAG
ATCTTAGGTATGTCGTCC-3'
*C*, *Construction of the pRSL1::GFP-RSL1::RSL1t plasmid*
   Amplification of the *AtRSL1* promoter + 5'UTR fragment from Col0 genomic DNA 
RSL1prom-attB4F: 5'- GGGGACAACTTTGTATAGAAAAGTTGTGATAATGGATTG
GAGAAAAATTAAAG-3'
 
RSL1prom-attB1R: 5'- GGGGACTGCTTTTTTGTACAAACTTGTATTGCAATGTTC
GTTAATGAGTGAC -3'
   Amplification of the *AtRSL1* coding region + 3'UTR + terminator fragment from Col0 genomic DNA 
RSL1term-attB2F: 5'-GGGGACAGCTTTCTTGTACAAAGTGGGTAATTACAT
CTCAACCCCAAATTCTT-3'
 
RSL1term-attB3R: 5'- ACAACTTTGTATAATAAAGTTGATGTATAATTTCCGAA
GATGCTTAAAA -3'
*D, Construction of the p35S::PpRSL1 plasmid*
   Amplification of *RSL1* coding sequence from Co10 cDNA
      35SPpRDL1 F: 5'-CCAGGATCCATGGCAGGTCCAGCAGGA-3'
      35SPpRDL2 R: 5'-CCAGTCGACTTAGTCAGCAGAAGGCTGATT-3'
*E*, *Construction of* t*he pPpRSL1-KO plasmid*
   Amplification of *PpRSL1* fragment 1 from P. *patens* WT DNA
      PpRSL1KO 1 F: 5`-CCTCTAGAAGTACTTGTGATCCACAGCCTA-3'
      PpRSL1KO 1 R: 5'-GGCTCGAGCCGTACTGGGTGGTTTG-3'
   Amplification of *PpRSL1* fragment 2 from *P. patens* WT DNA
      PpRSL1KO 2 F: 5'-CCGTTAACTTCTACATGTTGCGTTATTTATGGT-3'
      PpRSL1KO 2 R: 5'-CCGGCGCGCCAATATTTATATAAATAAGCATAATACACTTCGA-3'
*F*, *Construction of the pPpRSL2-KO plasmid*
   Amplification of *PpRSL2* fragment 1 from *P. patens* WT DNA
      PpRSL2KO 1 F: 5'-GCAACGCGTGGGTTTGATCAAAGACGGAA-3'
      PpRSL2KO 1 R: 5'-GCTACTAGTCGTCAACCTAACCCAAACAT-3'
   Amplification of *PpRSL2* fragment 2 from P. *patens* WT DNA
      PpRSL2KO 2 F: 5'-GAGGGATCCGTGAGGTGAAAGCAGTGAAA-3'
      PpRSL2KO 2 R: 5'-CCAAAGCTTAGGCCTGTGAACTCGGACA-3'
*G*, *Amplification of Dig labelled probes for southern blots*
   Amplification of the *PpRSL1* probe from *P. patens* WT DNA
      PpRSL1 probe F: 5'-GCTGCTAGGGTAACATAAACATTCTT-3'
      PpRSL1 probe R: 5'-CTGGACACTGGAATGAACCTA-3'
   Amplification of *NptII (NEOMYCIN PHOSPHOTRANSFERASE* II) probe from pBNRF
      NptII probe F: 5'-CCCATGGAGTCAAAGATTCA-3'
      NptII probe R: 5'-CCGCGAATTCGAGCTCGGT-3'
   Amplification of the *PpRSL2* probe from *P. patens* WT DNA
      PpRSL2 probe F: 5'-CCCAAATATGCATTTTTAATCTTT-3'
      PpRSL2 probe R: 5'-GCGACAATCCAGCAGCCTCTAT-3'
   Amplification of *AphIV(AMINOGLYCOSIDE PHOSPHOTRANSFERASE IV)* probe from pBHSNR
      AphIV probe F: 5'-GTAGGAGGGCGTGGATATGT-3'
      AphIV probe R: 5'-CGAGTGCTGGGGCGT-3'
*H*, *amplification of PpRSL2 coding sequence from protonema cDNA*
   PpRSL2 RT-PCR F: 5`-GGGACCTCAAGGATGCAGCA-3'
   PpRSL2 RT-PCR R: 5'-CGAACTCAATAACGTCAGGA-3'
*I*, *RT-PCRs*
   RT-PCR of *AtAPT1 (ADENINE PHOSPHORIBOSYLTRANSFERASE 1)*
      AtAPT1 RT-PCR F: 5'-TCCCAGAATCGCTAAGATTGCC-3'
      AtAPT1 RT-PCR R: 5'-CCTTTCCCTTAAGCTCTG-3'
   RT-PCR of *AtRHD6*
      AtRHD6 RT-PCR F: 5'-CTCACACGGGAGAGAGCA-3'
      AtRHD6 RT-PCR R: 5'-CTTCGATTCTTGGCTGCTA-3'
   RT-PCR of *AtRSL1*
      ATRSL1 RT-PCR F: 5'-GCCTAGCAGCCAAGAACCGAA-3'
      ATRSL1 RT-PCR R: 5'-CTCATCGGCTGCAAGTACCTTA-3'
   RT-PCR of *PpRSL1*
      PpRSL1 RT-PCR F: 5'-CTGGTTGGTTAGGAGATCTTGCAT-3'
      PpRSL1 RT-PCR R: 5'-GTTGTAATTTGGTCCATTTCTGCT-3'
   RT-PCR of *PpRSL2*
      PpRSL2 RT-PCR F: 5'-GGGACCTCAAGGATGCAGCA-3'
      PpRSL2 RT-PCR R: 5'-CGAACTCAATAACGTCAGGA-3'
   RT-PCR of *PpGAPDH (GLYCERALDEHYDE 3-PHOSPHATE DEHYDROGENASE)*
      PpGAPDH RT-PCR F: 5'-GAGATAGGAGCATCTGTACCGCTTGT-3'
      PpGAPDH RT-PCR R: 5'-CGCATGGTGGGATCGGCT-3'

### SEQUENCES

MALVNDHPNETNYLSKQNSSSSEDLSSPGLDQPDAAYAGGGGGGSASSSSTMNSDHQQH
QGFVFYPSGEDHHNSLMDFNGSSFLNFDHHESFPPPAISCGGSSGGGGFSFLEGNNMSYG
FTNWNHQHHMDIISPRSTETPQGQKDWLYSDSTVVTTGSRNESLSPKSAGNKRSHTGEST
QPSKKLSSGVTGKTKPKPTTSPKDPQSLAAKNRRERISERLKILQELVPNGTKVDLVTML
EKAISYVKFLQVQVKVLATDEFWPAQGGKAPDISQVKDAIDAILSSSQRDRNSNLITN

RHD6 amino acid sequence (At1g66470; NP_176820.1 GI: 15219658 SEQ ID NO: 1) 
atggcactcgttaatgaccatcccaacgagaccaattacttgtcaaaacaaaattcctcc
tcttccgaagatctctcctcgccgggactggatcagccagatgcagcttatgccggtgga
ggaggaggaggaggctcggcttcgagcagtagcacgatgaattcagatcatcaacaacat
caggggtttgtatttacccatccggtgaagatcatcacaactctttgatggatttcaac
ggatcatcatttcttaactttgatcatcacgagagctttcctcctccagccataagctgt
ggtggtagtagcggtgggggcggcttctccttcttggagggcaacaacatgagctacggc
ttcacaaactggaatcatcaacatcatatggatattattagccctagatccaccgaaact
ccccaaggccagaaagactggttatattctgattcaactgttgtaaccactggttctaga
aacgagtctctttcgcctaaatccgctggaaacaaacgttctcacacggagagagcact
caaccgtcgaagaaactgagtagcggtgtgaccggaaagaccaagcctaagccaacaact
tcacctaaagatccacaaagcctagcagccaagaatcgaagagaaaggataagtgaacgt
ctcaagatattgcaagaacttgttcccaatggcaccaaggttgatttggtgacaatgctt
gaaaaggctattagttatgtcaagttccttcaagtacaagttaaggtattagcgaccgat
gagttttggccggctcaaggaggaaaagctcctgacatttctcaagttaaagacgccatt
gatgccattctctcctcatcacaacgagacaggaattcgaatctgatcaccaattaa
 RHD6 nucleotide sequence (NM_10S318.2 GI: 30697352 SEQ ID NO: 2) 
MSLINEHCNE RNYISTPNSS EDLSSPQNCG LDEGASASSS STINSDHQNN QGFVFYPSGE
TIEDHNSLMD FNASSFFTFD NHRSLISPVT NGGAFPVVDG NMSYSYDGWS HHQVDSISPR
VIKTPNSFET TSSFGLTSNS MSKPATNHGN GDWLYSGSTI VNIGSRHEST SPKLAGNKRP
FTGENTQLSK KPSSGTNGKI KPKATTSPKD PQSLAAKNRR ERISERLKVL QELVPNGTKV
DLVTMLEKAI GYVKFLQVQV KVLAADEFWP AQGGKAPDIS QVKEAIDAIL SSSQRDSNST
RETSIAE
 RSL1 amino acid sequence (At5g37800 SEQ ID NO: 3) 
atgtcactcattaacgaacattgcaatgagcgtaattacatctcaaccccaaattcttca
gaagatctctcttcaccacagaattgcggattagacgaaggagcttcagcttcaagcagt
agcaccataaattctgatcatcaaaataatcaaggtttgtgttttacccttccggggaa
accattgaagatcataattctttgatggatttcaatgcttcatcattcttcacctttgat
aatcaccgaagccttatctctcccgtgaccaacggtggtgccttcccggtcgtggacggg
aacatgagttacagctatgatggctggagtcatcatcaagtggatagtattagccctaga
gtcatcaaaactccaaatagctttgaaacaacgagcagttttggattgacttcaaactcc
atgagtaaaccggccacaaaccatggaaatggagactggttatactctggttcaactatt
gtaaacatcggttcaaggcacgagtccacgtcccctaaactggctggcaataaacggcct
ttcacgggagagaacacacaactttcaaagaagccgagtagcggtacgaatggaaagatc
aagcctaaggcaacaacttcacctaaagatccacaaagcctagcagccaagaaccgaaga
gaaaggataagcgaacgcctcaaggtattgcaagaacttgtaccgaatggtaccaaggtg
gatttggtaactatgcttgagaaagcaattggctatgtaaagtttcttcaagtacaagtt
aaggtacttgcagccgatgagttttggccggcacaaggagggaaagctccggacatttct
caagttaaagaagctattgacgcaatcctctcatcatcacaacgagatagtaactcaact
agagaaacaagtatagcagaataa
 RSL1 nucleotide sequence (SEQ ID NO: 4) 
MEAMGEWSNN LGGMYTYATE EADFMNQLLA SYDHPGTGSS SGAAASGDHQ GLYWNLGSHH
NHLSLVSEAG SFCFSQESSS YSAGNSGYYT VVPPTVEENQ NETMDFGMED VTINTNSYLV
GEETSECDVE KYSSGKTLMP LETVVENHDD EESLLQSEIS VTTTKSLTGS KKRSRATSTD
KNKRARVNKR AQKNVEMSGD NNEGEEEEGE TKLKKRKNGA MMSRQNSSTT FCTEEESNCA
DQDGGGEDSS SKEDDPSKAL NLNGKTRASR GAATDPQSLY ARKRRERINE RLRILQNLVP
NGTKVDISTM LEEAVHYVKF LQLQIKLLSS DDLWMYAPIA FNGMDIGLSS PR
 RSL2 amino acid sequence (At4g33880; SEQ ID NO: 5) 
atggaagccatgggagaatggagcaacaacctcggaggaatgtacacttatgcaaccgag
gaagccgatttcatgaaccagcttctcgcctcttatgatcatcctggcaccggctcatcc
tccggcgcagcagccagtggtgaccaccaaggcttgtattggaaccttggttctcatcac
aaccaccttagcctcgtgtctgaagccggtagcttctgtttctctcaagagagcagcagc
tacagcgctgggaacagcggatattacaccgttgttccacccacggttgaagagaaccaa
aatgagacaatggactttgggatggaagatgtgaccatcaatacaaactcataccttgtt
ggtgaggagacaagtgagtgtgacgttgagaaatactcttctggaaagactcttatgcct
ttggaaaccgtagtggagaaccacgatgacgaggaaagcttgttgcaatctgagatctct
gtgactactacaaaatctctcaccggctccaaaaagagatcccgtgccacatctactgat
aaaaacaagagagcaagagtgaataagagggcccagaagaacgtagagatgagtggggat
aacaatgaaggagaagaggaagaaggagagacgaagttgaagaaaagaaagaatggggca
 
atgatgagtagacagaactcaagcaccactttctgtacggaggaagaatcaaactgcgct
gatcaagacggtggaggagaagactcatcctctaaggaagatgatccctcaaaggccctc
aacctcaatggtaaaacaagagccagtcgtggtgcagccaccgatcctcaaagcctctat
gcaaggaaaagaagagaaaggattaacgagagactaaggatttacaaatctcgtcccc
aatggaacaaaggtcgatattagtacaatgcttgaggaagcagttcattacgtcaaattt
ttgcagctccaaattaagttattgagctctgatgatctatggatgtatgcgccgattgct
ttcaatgggatggacattggtctcagctcaccgagatga
 RSL2 nucleotide sequence (SEQ ID NO: 6) 
MEAMGEWSTG LGGIYTEEAD FMNQLLASYE QPCGGSSSET TATLTAYHHQ GSQWNGGFCF
SQESSSYSGY CAAMPRQEED NNGMEDATIN TNLYLVGEET SECDATEYSG KSLLPLETVA
ENHDHSMLQP ENSLTTTTDE KMFNQCESSK KRTRATTTDK NKRANKARRS QKCVEMSGEN
ENSGEEEYTE KAAGKRKTKP LKPQKTCCSD DESNGGDTFL SKEDGEDSKA LNLNGKTRAS
RGAATDPQSL YARVDISTML EEAVQYVKFL QLQIKRLLAI GTNHRNRSIP LWTARNRQIS
KAHSRKRLRL RAVAKIIWSD EMTRFLLELI TLEKQAGNYR GKSLIEKGKE NVLVKFKKRF
PITLNWNKVK NRLDTLKKQY EIYPAKLRSH PLRFIPLLDV VFRDETVVVE ESWQPRRGVH
RRAPVLDLSD SECPNNNGDE REDLMQNRER DHMRPPTPDW MSQTPMENSP TSANSDPPFA
SQERSSTHTQ VKNVSRNRKR KQNPADSTLD RIAATMKKI
 RSL3 amino acid sequence (At2g14760; SEQ ID NO: 7) 
atggaagccatgggagaatggagcaccggcctaggcggaatatatacagaggaagctgac
tttatgaatcagctccttgcctcctatgagcaaccttgtggcggttcatcttcagagaca
accgccacactcacggcctaccaccaccagggttctcaatggaatggtggcttttgcttc
tctcaggagagcagtagttatagtggttactgcgcggcgatgccacggcaagaagaagat
aacaatgggatggaggacgcgacaatcaacacgaacttgtaccttgttggtgaagagaca
agtgaatgtgatgcgacggaatactccggtaaaagcctcttgcctttggagactgtcgca
gaaaaccacgaccatagtatgctacagcctgagaactccttgaccacgaccactgatgag
aaaatgttcaaccaatgtgagagttcaaagaagaggacgcgtgccacaacaactgataag
aacaagagagccaacaaggcacgaaggagccagaaatgcgtagagatgagtggcgaaaat
gaaaatagcggcgaagaagaatatacggagaaggctgcggggaagagaaagaccaaacca
cttaagccgcaaaagacttgttgttcggatgacgaatcaaacggtggagacactttcttg
tccaaagaagatggcgaggactctaaggctctcaacctcaacggcaagactagggccagc
cgcggcgcggccacagatcctcaaagcctttacgcaaggaaaagaagagagaggataaac
gagaggctaaggatttgcaacatctcgtccctaatggaacaaaggttgatattagcacg
atgttggaagaagcagtacaatacgtcaaatttctacagctccaaattaagttattgagc
tctgatgatctatggatgtatgcgcctattgcttacaacggaatggacattggccttgac
ctaaaactcaatgcactgaccagatga
 RSL3 nucleotide sequence (SEQ ID NO: 8) 
MDVFVDGELE SLLGMFNFDQ CSSSKEERPR DELLGLSSLY NGHLHQHQHH NNVLSSDHHA
FLLPDMFPFG AMPGGNLPAM LDSWDQSHHL QETSSLKRKL LDVENLCKTN SNCDVTRQEL
AKSKKKQRVS SESNTVDESN TNWVDGQSLS NSSDDEKASV TSVKGKTRAT KGTATDPQSL
YARKRREKIN ERLKTLQNLV PNGTKVDIST MLEEAVHYVK FLQLQIKLLS SDDLWMYAPL
AYNGLDMGFH HNLLSRLM
 RSL4 amino acid sequence (At1g27740; SEQ ID NO: 9) 
atggacgtttttgttgatggtgaattggagtctctcttggggatgttcaactttgatcaa
tgttcatcatctaaagaggagagaccgcgagacgagttgcttggcctctctagcctttac
aatggtcatcttcatcaacatcaacaccataacaatgtcttatcttctgatcatcatgct
ttcttgctccctgatatgttcccatttggtgcaatgccgggaggaaatcttccggccatg
cttgattcttgggatcaaagtcatcacctccaagaaacgtcttctcttaagaggaaacta
cttgacgtggagaatctatgcaaaactaactctaactgtgacgtcacaagacaagagctt
gcgaaatccaagaaaaaacagagggtaagctcggaaagcaatacagttgacgagagcaac
actaattgggtagatggtcagagtttaagcaacagttcagatgatgagaaagcttcggtc
acaagtgttaaaggcaaaactagagccaccaaagggacagccactgatcctcaaagcctt
tatgctcggaaacgaagagagaagattaacgaaaggctcaagacactacaaaaccttgtg
ccaaacggacaaaagtcgatataagcacgatgcttgaagaagcggtccattacgtgaag
ttcttgcagcttcagattaagttgttgagctcggatgatctatggatgtacgcaccattg
gcttacaacggcctggacatggggttccatcacaaccttttgtctcggcttatgtga
 RSL4 nucleotide sequence (SEQ ID NO: 10) 
MENEAFVDGELESLLGMFNFDQCSSNESSFCNAPNETDVFSSDDFFPFGTILQSNYAAVL
DGSNHQTNRNVDSRQDLLKPRKKQKLSSESNLVTEPKTAWRDGQSLSSYNSSDDEKALGL
VSNTSKSLKRKAKANRGIASDPQSLYARKRRERINDRLKTLQSLVPNGTKVDISTMLEDA
VHYVKFLQLQIKLLSSEDLWMYAPLAHNGLNMGLHHNLLSRLI
 RSL5 amino acid sequence (At5g43175; SEQ ID NO: 11) 
atggagaatgaagcttttgtagatggtgaattggagtctcttttggggatgttcaacttt
gatcaatgttcatctaacgaatcgagcttttgcaatgctccaaatgagactgatgttttc
tcttctgatgatttcttcccatttggtacaattctgcaaagtaactatgcggccgttctt
gatggttccaaccaccaaacgaaccgaaatgtcgactcaagacaagatctgttgaaacca
aggaagaagcaaaagttaagctcggaaagcaatttggttaccgagcctaagactgcttgg
 
agagatggtcaaagcctaagcagttataatagttcagatgatgaaaggctttaggttta
gtgtctaatacatcaaaaagcctaaacgcaaagcgaaagccaacagaggatagcttcc
gatcctcagagcctatacgctaggaaacgaagagaaaggataaacgataggctaaagaca
ttgcagagcctagttcctaatgggacaaaggtcgatataagcacaatgctggaagatgct
gtccattacgtgaagttcctgcagcttcaaatcaagctcttgagttcagaagatctatgg
atgtatgcacctcttgctcacaatggtctgaatatgggactacatcacaatcttttgtct
cggcttatttaa
 RSL5 nucleotide sequence (SEQ ID NO: 12)
TSPKDPQSLAAKNRRERISERLKILQELVPNGTKVDLVTMLEKAISYVKFLQVQVKVLATDEFWPAQ AtRHD6 bHLH amino acid sequence (SEQ ID NO: 13)
TSPKDPQSLAAKNRRERISERLKVLQELVPNGTKVDLVTMLEKAIGYVKFLQVQVKVIAADEFWPAQ AtRLD1 bHLH amino acid sequence (SEQ ID NO: 14)
GSANDPQSIAARVRRERISERLKVLQALIPNGDKVDMVTMLEKAISYVQCLEFQIKMLKNDSLWPKA PpRSL1 bHLH amino acid sequence (SEQ ID NO: 15)
GSANDPQSIAARVRRERISERLKVLQALIPNGDKVDMVTMLEKAITYVQCLELQIKMLKNDSIWPKA PpRSL2 bHLH amino acid sequence (SEQ ID NO: 16)
GSATDPQSVYARHRREKINERLKSLQNLVPNGAKVDIVTMLDEAIHYVKFLQNQVELLKSDELWIYA PpRSL5 bHLH amino acid sequence (SEQ ID NO: 17)
GSATDPQSVYARHRREKINERLKNLQNLVPNGAKVDIVTMLDEAIHYVKFLQTQVELLKSDEFWMFA PpRSL6 bHLH amino acid sequence (SEQ ID NO: 18)
GSATDQSVYARHRREKINERLKTLQHLVPNGAKVDIVTMLDEAIHYVQFLQTQVTLLKSDEYWMYA PpRSL3 bHLH amino acid sequence (SEQ ID NO: 19)
GSATDPQSVHARARREKIAERLRKLQHLIPNGGKVDIVTMLDEAVHYVQFLKRQVTLLKSDEYWMYA PpRSL4 bHLH amino acid sequence (SEQ ID NO: 20)
GSATDPQSVYARHRREKINERLKTLQRLVPNGEQVDIVTMLEEAIHFVKFLEFQLELLRSDDRWMFA PpRSL7 bHLH amino acid sequence (SEQ ID NO: 21)
GAATDPQSLYARKRRERINERLRILQNLVPNGTKVDISTMLEEAVHYVKFLQLQIKLLSSDDLWMYA At4g33880 bHLH amino acid sequence (SEQ ID NO: 22)
GAATDPQSLYARKRRERINERLRILQHLVPNGTKVDISTMLEEAVQYVKFLQLQIKLLSSDDLWMYA At2g14760 bHLH amino acid sequence (SEQ ID NO: 23)
GTATDPQSLYARKRREKINERLKTLQNLVPNGTKVDISTMLEEAVHYVKFLQLQIKLLSSDDLWMYA Atg27740 bHLH amino acid sequence (SEQ ID NO: 24)
GIASDPQSLYARKRRERINDRLKTLQSLVPNGTKVDISTMLEDAVHYVKFLQLQIKLLSSEDLWMYA At5g43175 bHLH amino acid sequence (SEQ ID NO: 25) 
MAGPAGALWSTCDPQPIQQAEIFSGPDNQAGLMSFHVDTPFHWGSEPWALHSRSDDIALMSPSLVHDISPYDSVL
HLSGVSGDVQDLVCGNPKFRQSGQWGQSEFSYSVQDNMQDLLTNQFIPYNTSSLGLNHLSPNFTDLDCAPVYNDT
KAFGTVTHNRAVPSTNTQSAQHGSSSMVSSNRPITSTASPTTQYGGPRTPSQTTQYGGSSMVTNSMEMFASAAPQ
GIMTTSGLSGGCNSDLMHLPKRQHAHSLPPTTGRDLTASEVVSGNSISNISGVGSFNSSQKSSASVMMSPLAASS
 
HMHKAAAVSEELKMASFNPGPFVPTQKKQHEQQDTMTSNRIWADKNNLGKISSSPIPIMGFEQSQQQSMSNSSP
VTSLGFEQRQKMSMGSSPSITIIGFEQRQKQPMSSSSPISNMVFEPRQKQPMSSSSPISNIVFEQRQLPTVGSSP
PISISGFEPKKQPSLSNSPPLSNLGFEQRLQPMSNASPISNLPFEQQRQQATMSNTRSAEPDSVESTTKWPLRMD
GAIGGCAGLPSSQKAPVIMQPETGTMKCPIPRTMPSNAKACPAVQNANSVNKRPLTVDDKDQTGSMNKKSMQKFL
GPQGCSRLESISALAHQKVSQSTTSGRALGPALNTNLKPRARQGSANDPQSIAARVRRERISERLKVLQALIPNG
DKVDMVTMLEKAISYVQCLEFQIKMLKNDSLWPKALGPLPNTLQELLELAGPEFAGIDGKNTEESSEKPKKSALE
VIELDGNQPSAD*
 Physcomitrella RHD SIX LIKE 1 (PpRSL1) amino acid sequence (SEQ ID NO: 26; ABOB4930.1.GI: 140084327) 
atggcaggtccagcaggagctttatggagtacttgtgatccacagcctattcaacaggcagagatatttagtggt
cctgacaaccaagctggtttgatgtcttttcatgtggataccccgttccattggggatctgaaccatgggctctc
cactctcggtcagatgacatcgccttgatgtcccctcgcttgttcacgacatatcaccttatgattctgtcttg
catctttccggagtgtctggggatgtgcaagatttagtttgcggaatcccaaattcgccaaagtgggcaatgg
gggcagagcgagttttcatactctgttcaggacaacatgcaagatctcctaaccaaccagttcataccgtacaac
acatcttcattgggtttaaatcatctctccccgaatttcaccgacttggattgcgcaccggtatacaatgatacc
aaggcttttggcactgttacacacaacagggcagtcccgagcactaatacccagagtgctcagcacgggagttcg
tctatggtttcaagtaacaggccaatcactagcacagcttctcctactactcagtatggaggtccgaggactcca
tcccaaaccacccagtacggggggttcatctatggttaccaactcgatggaaatgtttgcttcagctgcacctcag
ggtattatgactacatctggcttgagtggcggttgcaactcagacttgatgcatctgccgaagcgccagcatgct
cactctcttcctcctaccactggcagagatttaactgcatctgaagtggtatctggaaattcgatatcaaacatt
tccggggttggatcttttaacagcagccagaaaagcagtgcatccgtgatgatgtctcctttagctgcttcttct
cacatgcacaaggctgctgctgtatctgaagaacttaagatggcaagtttcaaccctggtccattcgtacctacg
cagaaaaagcagcaacatgagcagcaggatacgatgacctctaatcgtatatgggcggataagaacaacttggga
aaaattagttcatcgcccattccgatcatggggtttgagcagagtcaacagcaatccatgagcaattcctcccct
gttaccagtttggggtttgagcaaaggcaaaaaatgtccatgggtagctctccctccatcacgatcattggattt
gagcaaagacagaagcaacctatgagtagttcttcccccatttcaaacatggttttgaaccaagacaaaaacag
ccaatgagtagctcttctcctatctctaatattgtctttgagcaaagacaactcccaactgtgggtagctctcct
ccgatttcaatctcaggatttgagccaaagaaacaaccatctttgagcaattctcctcccctctctaatctgggt
tttgagcaaaggctacaacccatgagtaatgcatctcctatttccaacttaccctttgagcaacaaagacaacaa
gcaaccatgagtaacaccagatctgcagaacccgattctgtcgagtctaccacgaagtggcccttgcggatggat
ggtgccataggtggatgtgctggcttaccaagcagtcagaaagctcctgttatcatgcagcctgagactgggact
atgaagtgtcctattccgaggaccatgcccagcaatgctaaggcttgcccagctgtgcagaatgctaattccgta
aacaagcgccctcttacggttgatgacaaggaccaaactggatcgatgaataagaagtcgatgcaaaagttttg
ggacctcaaggttgtagcagacttgaaagtatcagtgctttagctcaccaaaaagtgagtcaaagtacaacaagc
ggtcgtgctctagggcctgctttgaacaccaatctcaagcctcgtgcacgccaagggagtgccaatgatccgcag
agcattgctgctagggtgcgaagagaaagaataagtgagcggctcaaagttttgcaagccttgatacctaacggt
gataaagtggatatggtcaccatgctggagaaggctatcagctacgtgcagtgtttggaatttcagattaagatg
ttaaaaaatgactctttgtggcctaaggcgcttggccctctaccgaacactttgcaagagcttctcgaacttgct
gggccagagtttgccggcatagatggcaagaatactgaggagtcgtcagagaaaccgaagaaatctgctcttgaa
gtaattgagttggacggcaatcagccttctgctgactaa
 Physcomitrella RHD SIX LIKE 1 (PpRSL1) nucleotide sequence (SEQ ID NO: 27; EF156393.1 GI: 140084326) 
MNKKPMQKALGPQGCSRLESISALAHQKVSQSASGRALGPALNTNLKPRARQGSANDPQSIAARVRRERISERLK
VLQALIPNGDKVDMVTMLEKAITYVQCLELQIKMLKNDSIWPKALGPLPNTLQELLELAGPEFSGTESKNVEEPP
AKPKKSAPDVIEFDGNQPSADKE*
 Physcomitrella RHD SIX LIKE 2 (PpRSL2) amino acid sequence (SEQ ID NO: 28 ABO84931.1 GI: 140084334) 
atgaataagaagcctatgcaaaaagctttgggacctcaaggatgcagcaggctagaaagcatcagtgctttagct
catcaaaaagtgagtcagagtgcaagtggtcgtgcactagggcctgctctgaacaccaacctcaagcctcgtgct
cgtcaagggagtgccaatgacccacagagcattgccgctagggttcgaagagaaaggataagtgagcggctgaaa
gttttgcaagccttgatacctaatggtgataaggtagatatggtgaccatgctggagaaggctatcacctacgtg
cagtgtctggaactccagattaagatgttaaagaatgattctatctggcccaaggcgcttggacctctaccaaac
 
actcttcaagagcttctggagcttgctggaccagaattttctggaacggaaagcaagaatgtagaggagcccca
gcgaagccaaagaaatcagctcctgacgttattgagttcgacggcaatcaaccttctgccgacaaagagtag
 Physcomitrella RHD SIX LIKE 2 (PpRSL2) nucleotide sequence (SEQ ID NO: 29; EF156394.1 GI: 140084333)
GSATDPQSVYARHRREKINERLKTLQHLVPNGAKVDIVTMLDEAIHYVQFLQLQVTLLKSDEYWMYA
Physcomitrella RHD SIX LIKE 3 (PpRSL3) amino acid sequence (SEQ ID NO: 30; ABO84932.1 GI: 140084346) 
ggttcagcgactgatccgcagagtgtatatgccaggcatagaagggagaagatcaacgagcgcttgaagacatta
cagcacttggtaccaaatggagctaaggtagacatcgtgaccatgcttgacgaagccattcactacgtccaattt
ctgcagctccaagtgacgctgttgaagtcggatgaatattggatgtacgcc
 Physcomitrella RHD SIX LIKE 3 (PpRSL3) nucleotide sequence (SEQ ID NO: 31; EF156395.1 GI;140084345) 
MTDLISILESSGSSREEMCPVAVPSSVASSCERLIWEGWTAQPSPVEESTTSKLLPKLLPELETSSYSALTLQQP
DALSSILSVLHPFSHYSSASLELARNPDWSLKSSNPLRESSSEAGIRTSSFEGLYSGQHTTKKIHLGVIPYHLSE
DQRQCAVSPPENECRLLSANSSGSLHWWHSIGPESPSSTLAFHNIGIQHSTFEKCEPRGQSHSSWPAASGTSPTV
QYFHAHSADNEGVEVVKQDDSQISKALATYQPHGDHSLVLNSDRIASTTSHSEDPCGPKPGRRPAASYDTEMILS
PSESFLTTPNMLSTLECVISGASNISDQYMNFVREPQEQRLSSISDLSLIPDSHADPHSIGFISGTFRTDSHGTG
IRKNRIFLSDEESDFLPKKRSKYTVRGDFQMDRFDAVWGNTGLRGSSCPGNSVSQMMAIYEFGPALNRNGRPRVQ
RGSATDPQSVHARARREKIAERLRKLQHLIPNGGKVDIVTMLDEAVHYVQFLKRQVTLLKSDEYWMYATPTSYRS
KFDDCSLVPGENN*
 Physcomitrella RHD SIX LIKE 4 (PpRSL4) amino acid sequence (SEQ ID NO: 32; ABO84933.1 GI:140084359) 
ATGACCGATCTGATTTCGATCTTGGAGTCATCAGGGTCATCACGAGAGGAGATGTGCCCTGTTGCTGTGCCAAGC
TCCGTGGCTTCTTCTTGTGAAAGGTTGATATGGAGGGGTGGACTGCACAACCATCTCCTGTCGAAGAAAGCACC
ACCAGCAAGTTACTTCCAAAGCTACTTCCAGAGCTCGAGACATCATCCTACTCTGCACTCACCCTTCAGCAACCT
GATGCGCTCTCCAGCATACTTTCAGTCCTCCACCCTTTTTCTCATTACAGTTCGGCCAGTTTAGAACTCGCTCGC
AATCCTGACTGGAGCTTGAAATCTTCAAATCCTCTGCGGAAAGCAGCTCGGAGGCTGGCATCCGAACCTCATCT
TTCGAAGGCTTGTACTCTGGTCAGCACACCACCAAAAAGATTCATTTGGGGGTCATACCCTACCACTTGTCCGAA
GATCAGCGCCAGTGCGCTGTCAGTCCTCCGGAAAATGAGTGCCGCCTACTGTCTGCAAATTCCTCTGGATCCCTT
CACTGGTGGCATTCCATAGGCCCCGAGTCTCCTTCCTCTACTCTTGCATTCCATAATATTGGGATCCAACACTCT
ACCTTCGAAAAGTGTGAGCCTAGGGGCCAGTCGCACTCATCATGGCCAGCGGCCAGCGGCACGTCGCCAACAGTT
CAATACTTTCATGCCCATTCTGCAGATAATGAAGGTGTCGAGGTCGTCAAGCAAGATGACTCGCAGATATCCAAG
GCTCTGGCGACCTATCAACCCCACGGCGACCATAGTCTCGTGCTAAATTCAGACCGCATTGCAAGCACAACCAGC
CACTCAGAAGATCCTTGCGGCCCTAAACCTGGACGCAGACCAGCTGCATCATACGACACCGAGATGATTCTTAGC
CCAAGTGAGAGTTTCTTGACAACTCCCAATATGTTATCAACGTTGGAGTGCGTAATATCCGGTGCAAGTAACATA
TCTGATCAGTATATGAACTTCGTCAGAGAACCGCAGGAGCAAAGGCTGTCCTCTATCTCCGATCTGTCCCTTATT
CCTGACAGCCACGCGGATCCGCACAGTATCGGATTTATCTCTGGGACCTTTAGAACAGACTCCCACGGAACTGGA
ATAAGAAAGAACCGCATCTTTCTCAGTGATGAGGAATCCGACTTCTTGCCTAAGAAGCGATCCAAGTACACGGTC
CGCGGCGATTTTCAGATGGATCGCTTCGACGCAGTTTGGGGGAATACCGGTCTTCGGGGATCTAGCTGTCCTGGA
AATTCAGTATCCCAGATGATGGCGATTTACGAATTCGGACCCGCACTGAACAGGAACGGCAGGCCGCGAGTACAA
CGTGGTTCGGCGACTGATCCGCAGAGTGTACACGCCAGGGCGCGGAGGAGAAAATCGCCGAGCGCTTGAGAAAG
TTGCAGCACCTCATTCCAAACGGCGGGAAGGTGGACATCGTAACCATGCTCGACGAAGCCGTTCACTATGTTCAG
TTTTTGAAGCGACAAGTTACGCTTCTGAAATCCGACGAGTATTGGATGTACGCCACGCCGACCTCGTACCGGAGC
AAATTCGACGACTGCAGTCTGGTTCCCGGCGAGAACAACTGA
 Physcomitrella RHD SIX LIKE 4 (PpRSL4) nucleotide sequence (SEQ ID NO: 33; EF156396.1 GI: 140084358) 
MVQLYMSSVEEQRETMVQPYVSSMDSGSTSGRQTPSCVVQQGSNTFETSNLWEEWTQASNGDDTVSTSNFLPEIS
SFTSSRLSFQQSDSLTTWMSGFPPLSQTALSPDLSHSSDPVDHPPAFMQEGLGPGDSILDYSPALTEMYPKSSSK
 
HNSSDCLPYPAASAPDKKMTDHELGSAISLAYDRGTVSRQLLRALGPLSPSSPLALQNGLQNPLGDPWDASPSAM
PWPMATTGHAYGPGATRTSIPDHLANAINHLEGIAPSSASHASKPRHTDIFIAPNGTFDSTPGGWTPQYYDGSVT
TDESVKAMKLIASLREAGHAEATIGFCTESKPSFLRGGDRTTSPVDSFFGKCVGAKTSIKQACSGKHPLELEEIV
DSENSELNPTQLKRSKLFENHPNALWSDQSMNGRELRSYSHLVGSSLTASQPMDIIAIGPALNTDGKPRAKRGSA
TDPQSVYARHRREKINERLKSLQNLVPNGAKVDIVTMLDEAIHYVKFLQNQVELLKSDELWIYATPNKYNGMDIS
DLSDMYLQELESRA*
 Physcomitrella RHD SIX LIKE 5 (PpRSL5) amino acid sequence (SEQ ID NO: 34: ABO84934.1 GI: 140084368) 
ATGGTGCAGTTATACATGTCCTCAGTTGAAGAGCAGCGGGAAACAATGGTACAGCCATACGTCTCAAGCATGGAC
TCAGGCTCAACGTCGGGGCGCCAGACGCCATCTTGCGTCGTTCAGCAGGGAAGTAACACATTTGAGACTTCGAAT
CTGTGGGAGGAATGGACGCAAGCATCGAACGGCGACGATACAGTCTCCACCAGCAATTTCCTCCCCGAAATCAGT
TCCTTCACGTCGAGTCGTCTCTCCTTCCAGCAAAGCGACTCTCTCACCACTTGGATGTCAGGGTTCCCTCCCCTC
TCCCAAACTGCCTTGAGCCCGGATCTTAGTCACTCCTCCGACCCCGTGGATCATCCCCCAGCATTCATGCAGGAG
GGTTTAGGCCCCGGTGATTCTATTCTGGACTATTCCCCCGCTCTCACAGAGATGTACCCGAAAAGTAGCTCCAAA
CATAATTCCTCGGATTGTTTACCTTACCCTGCGGCCAGTGCACCAGACAAAAAAATGACTGATCACGAACTAGGT
TCGGCTATTTCCCTCGCGTATGATAGAGGCACCGTTTCCCGCCAGCTTCTTCGAGCCTTGGCCCATTGTCGCCT
TCATCGCCTCTAGCATTGCAGAATGGGCTGCAAAACCCGCTTGGGGACCCCTGGGATGCTTCTCCATCTGCAATG
CCGTGGCCAATGGCAACAACCGGTCATGCTTATGGACCAGGCGCCACCAGGACTTCTATTCCAGATCACTTAGCA
AATGCAATTAATCACCTGGAGGGCATTGCACCGTCCAGTGCCAGTCATGCATCGAAACCTCGTCACACTGATATT
TTCATTGCACCCAATGGCACGTTCGATTCGACGCCGGGAGGTTGGACACCGCAGTATTACGATGGGTCCGTGACG
ACAGATGAGTCTGTGAAGGCGATGAAGCTGATTGCGTCCCTACGTGAAGCAGGCCACGCAGAGGCTACAATTGGA
TTCTGTACAGAGAGCAAGCCTAGTTTTCTCAGGGGTGGGGACAGAACAACCTCGCCAGTGGACAGCTTCTTCGGC
AAATGTGTAGGGGCCAAAACGAGTATAAAGCAAGCCTGTTCTGGGAAACACCCTCTTGAACTTGAGGAGATCGTT
GATAGTGAAAACAGTGAATTAAATCCCACCCAGCTCAAACGCTCTAAACTTTTTGAGAATCATCCGAATGCCTTG
TGGAGCGATCAGAGTATGAATGGAAGAGAACTGAGATCGTACTCTCATTTGGTTGGCAGCAGTCTTACTGCATCG
CAGCCCATGGACATAATTGCAATTGGCCCAGCGCTCAACACTGATGGCAAACCACGAGCAAAGCGGGGTTCAGCA
ACCGATCCTCAGAGTGTTTACGCTAGACATAGGAGAGAAAAAATCAACGAACGATTGAAGAGTTTACAAAACCTA
GTACCTAATGGAGCCAAGGTTGACATAGTAACCATGCTGGACGAAGCTATACATTACGTCAAATTTTTACAAAAT
CAAGTTGAGCTGCTGAAGTCCGACGAGTTGTGGATTTACGCAACACCAAATAAGTACAACGGCATGGACATTTCC
GACCTCTCTGACATGTATTTGCAGGAGCTGGAGTCACGTGCGTGA
 Physcomitrella RHD SIX LIKE 5 (PpRSL5) nucleotide sequence (SEQ ID NO: 35; EF156397.1 GI: 140084367) 
MVRFNYMYPVQEQLEAMTDQHTPSMDSVSSAGEKTSSCIVQQGGNASETSNLWEEWTQGSNGDDSVSTSNFLPEL
NSSTSSRLAFHQSDILSTWISGYHPLSQSSLSSEFSHTSDRENHPPAFMQEGLIPSGLILDSDPALTDIYTRSSS
SDSLPYPTARIMDKALTDHELESAVPLAYEKGCVPPQVLRNLGPLSPSSPLAFQNGLLNPLRDPWDSCPSALPWS
NVTTASQTYGQVTTRTFIPDHSASAIDKLEAVATITAGYGASKPQHTDVFIEPNGTFQSTPAGWAPQFYDGSEAT
GLLVKPMRAIASLGEAGCGEATSEFCTKTKPGLLKGGDTITSPVGSLLGDCKKAESSMKQVWPGKHRLELVELVD
GEDTKSSPTQLKRPKHSTDYANVLLSDHILKGAELRSYFHSGDVGLNASQAMDIIVIGPALNTNGKPRAKRGSAT
DPQSVYARHRREKINERLKNLQNLVPNGAKVDIVTMLDEAIHYVKFLQTQVELLKSDEFWMFANPHNYNGIDISD
PSSMHSPELESNI*
 Physcomitrella RHD SIX LIKE 6 (PpRSL6) amino acid sequence (SEQ ID NO: 36; ABO84935 GI: 140084376) 
ATGGTGCGGTTTAACTACATGTACCCGGTTCAAGAGCAGCTGGAAGCCATGACGGACCAACACACCCCAAGCATG
GATTCGGTCTCGTCGGCCGGAGAGAAGACATCCTCTTGCATCGTCCAGCAGGGAGGAAATGCATCCGAAACTTCA
AACTTGTGGGAAGAATGGACACAAGGGTCGAACGGCGACGATTCTGTCTCTACCAGCAACTTCCTCCCCGAACTG
AATTCCTCCACCTCCAGTCGTCTCGCATTCCACCAAAGCGACATTCTTTCCACTTGGATCTCAGGCTACCACCCA
CTCTCGCAAAGCAGCCTGAGTTCCGAATTCAGCCACACCTCCGACCGCGAGAATCACCCCCCAGCATTCATGCAA
GAGGGTTTAATCCCCAGTGGTTTAATTCTTGACTCTGATCCTGCTCTCACAGATATTTATACGAGAAGCAGCTCC
TCGGACTCTTTGCCATACCCCACGGCTAGGATCATGGACAAAGCATTGACCGATCACGAGCTTGAGTCTGCTGTC
CCACTTGCATATGAAAAAGGCTGCGTTCCTCCCCAGGTTCTGCGTAACCTAGGGCCATTGTCACCTTCTTCGCCT
CTGGCATTCCAGAATGGACTGCTAAACCCCCTCAGGGACCCTTGGGATTCGTGTCCATCTGCATTGCCATGGTCA
AATGTGACCACAGCCAGCCAGACTTACGGTCAAGTGACAACCAGGACTTTCATTCCAGATCACTCTGCAAGTGCA
 
ATCGACAAGTTGGAGGCCGTCGCAACGATCACTGCCGGATACGGCGCGTCGAAACCACAACATACTGACGTCTTC
ATAGAACCCAACGGACGTTTCAGTCGACTCCGGCAGGGTGGCACCGCAGTTTTACGATGGATCCGAGGCGACG
GGCCTGTTGGTCAAGCCAATGAGGGCCATCGCATCTCTGGGTGAAGCCGGCTGTGGGAGGCCACTAGTGAATTC
TGCACAAAGACCAAGCCAGGACTTCTCAAAGGTGGGGACACAATAACCTCGCCGGTGGGTAGCCTGTTGGGCGAT
TGCAAAAAAGCTGAGTCAAGTATGAAGCAAGTTTGGCCTGGAAAACACCGTCTTGAACTCGTGGAACTAGTCGAT
GGTGAAGACACCAAATCAAGTCCCACCCAGCTCAAACGGCCGAAACATTCTACGGATTATGCGAATGTCCTGTTG
AGCGATCATATTCTGAAAGGAGCGGAGCTGCGGTCCTACTTCCATTCTGGTGATGTTGGTCTAAATGCATCTCAA
GCGATGGACATTATTGTAATTGGCCCAGCCTTGAATACTAATGGCAAGCCGCGAGCTAAACGGGGTTCAGCCACC
GATCCCCAGAGTGTGTACGCTAGACATAGGCGAGAAAAAATCAACGAACGACTGAAGAATTTACAAAATCTCGTG
CCAAATGGAGCCAAGGTTGACATTGTGACCATGCTAGACGAAGCCATACACTACGTCAAATTCTTGCAAACTCAA
GTTGAGCTGCTGAAATCCGACGAGTTCTGGATGTTCGCAAATCCACACAACTACAACGGCATAGATATCTCCGAT
CCCTCTAGCATGCATTCGCCGGAGCTGGAGTCGAATATTTAG
 Physcomitrella RHD SIX LIKE 6 (PpRSL6) nucleotide sequence (SEQ ID NO: 37: EF156398.1 GI: 140084375)
GSATDPQSVYARHRREKINERLKTLQRLVPNGEQVDIVTMLEEAIHFVKFLEFQLELLRSDDRWMFA
Physcomitrella RHD SIX LIKE 7 (PpRSL7) amino acid sequence (SEQ ID NO: 38; ABOS4936.1 GI: 140084384) 
Gggtcagctactgatcctcagagtgtgtacgcaaggcatcgccgggagaagattaacgagcgcctaaagacattg
cagcggttggttcctaacggagaacaggtcgacattgtgaccatgctggaagaagccattcactttgtcaaattt
ttggagttccaactggagctgttgcgatccgatgatcgctggatgttcgcc
 Physcomitrella RHD SIX LIKE 7 (PpRSL7) nucleotide sequence (SEQ ID NO: 39; EF156399.1 GI: 140084383) 
LNTNLKPRAKQGCANDPQSIAARQRRERISDRLKILQELIPNGSKVDLVTMLEKAINYVKFLQLQVKVLMNDEYW
PPKGD
 Selaginella moelendorfii SmRSLa amino acid sequence (SEQ ID NO: 40) 
CTCAACACTAATCTTAAGCCGCGAGCAAAGCAAGGTTGTGCTAATGATCCACAAAGCATTGCTGCCAGACAACGA
AGAGAACGGATAAGTGACCGGCTTAAAATCCTGCAGGAGCTCATACCAAATGGATCCAAGGTCGATCTGGTAACC
ATGCTGGAGAAGGCCATCAACTACGTCAAGTTCTTGCAATTGCAAGTCAAAGTTCTTATGAACGATGAGTATTGG
CCACCAAAGGGAGAT
 Selaginella moelendorfii SmRSLa nucleotide sequence(SEQ ID NO: 41) 
LNTNLKPRAKQGCANDPQSIAARQRRERISDRLKILQELIPNGSKVDLVTMLEKAINYVKFLQLQVKVLMNDEYW
PPKGD
 Selaginella moelendorfii SmRSLb amino acid sequence (SEQ ID NO: 42) 
CTCAACACTAATCTTAAGCCGCGAGCAAAGCAAGGTTGTGCTAATGATCCACAAAGCATTGCTGCCAGACAACGA
AGAGAACGGATAAGTGACCGGCTTAAAATCCTGCAGGAGCTCATACCAAATGGATCCAAGGTCGATCTGGTAACC
ATGTTGGAGAAGGCCATCAACTACGTCAAGTTCTTGCAATTGCAAGTCAAAGTTCTTATGAACGATGAGTATTGG
CCACCAAAGGGAGAT
 Selaginella moelendorfii SmRSLb nucleotide sequence (SEQ ID NO: 43) 
LNTNFKPRARQGSANDPQSIAARHRRERISDRLKILQELVPNSTKVDLVTMLEKAINYVKFLQLQVKVLTSDDYW
P
 Selaginella moelendorfii SmRSLc amino acid sequence (SEQ ID NO: 44) 
CTCAACACCAATTTCAAGCCTCGAGCCAGGCAGGGAAGCGCCAATGATCCCCAGAGCATCGCTGCTAGACATCGC
CGGGAGAGGATCAGTGACAGGCTCAAGATCTTGCAAGAGCTCGTTCCAAACAGCACAAAGGTTGATCTAGTGACG
ATGCTGGAGAAGGCCATCAATTACGTCAAGTTCCTCCAGCTGCAAGTTAAGGTGCTTACGTCGGACGACTACTGG
CCA
 Selaginella moelendorfii SmRSLc nucleotide sequence (SEQ ID NO: 45) 
LNTNFKPRARQGSANDPQSIAARHRRERISDRLKILQELVPNSTKVDLVTMLEKAINYVKFLQLQVKVLTSDDYW
P
 Selaginella moelendorfii SmRSLd amino acid sequence (SEQ ID NO: 46) 
CTCAACACCAATTTCAAGCCTCGAGCCAGGCAGGGAAGCGCCAATGATCCCCAGAGCATCGCTGCTAGACATCGC
CGGGAGAGGATCAGTGACAGGCTCAAGATCTTGCAAGAGCTCGTTCCAAACAGCACAAAGGTTGATCTAGTGACG
ATGCTGGAGAAGGCCATCAATTACGTCAAGTTCCTCCAGCTGCAAGTTAAGGTGCTTACGTCGGACGACTATTGG
CCA
 Selaginella moelendorfii SmRSLd nucleotide sequence (SEQ ID NO: 47) 
LNTDGKPRAKRGSATDPQSIYARQRRERINERLRALQGLVPNGAKVDIVTMLEEAINYVKFLQLQVKLLSSDEYW
MYAPT
 Selaginella moelendorfii SmRSLe amino acid sequence (SEQ ID NO: 48) 
CTAAACACCGACGGAAAGCCACGCGCAAAGCGTGGATCTGCCACGGACCCGCAAAGCATCTACGCTCGGCAAAGA
AGAGAAAGGATCAACGAGCGTTTGAGAGCGCTACAAGGACTCGTACCAAACGGAGCGAAGGTTGACATTGTGACG
ATGCTCGAGGAAGCCATCAACTATGTCAAGTTTTTGCAGCTGCAAGTAAAGCTGCTCAGCTCGGACGAGTATTGG
ATGTACGCCCCCACA
 Selaginella moelendorfii SmRSLe nucleotide sequence (SEQ ID NO: 49) 
LNTNGKPRAKRGSATDPQSVYARHRRERINERLKTLQHLVPNGAKVDIVTMLEEAIHYVKFLQLQVNMLSSDEYW
IYAPT
 Selaginella moelendorfii SmRSLf amino acid sequence (SEQ ID NO: 50) 
CTCAACACGAATGGCAAGCCCAGAGCAAAGCGTGGATCTGCAACAGATCCCCAAAGCGTTTACGCAAGGCACCGG
AGAGAGAGGATCAACGAGAGGCTCAAAACTTTACAACACCTTGTTCCAAATGGTGCAAAGGTTGACATAGTGACA
ATGCTTGAAGAAGCAATACATTACGTGAAGTTTCTACAGCTGCAAGTCAACATGTTAAGCTCTGATGAGTACTGG
ATTTATGCACCCACA
 Selaginella moelendorfii SmRSLf nucleotide sequence (SEQ ID NO: 51) 
LNTNGKPRAKRGSATDPQSVYARHRRERINERLKTLQHLVPNGAKVDIVTMLEEAIHYVKFLQLQVNMLSSDEYW
TYAPT
 Selaginella moelendorfii SmRSLg amino acid sequence (SEQ ID NO: 52) 
CTCAACACGAATGGCAAGCCCCGAGCAAAGCGTGGATCTGCAACAGATCCCCAAAGCGTTTATGCAAGGCACCGG
AGAGAGAGGATCAACGAGAGGCTCAAAACTTTACAACACCTTGTTCCAAATGGTGCAAAGGTTGACATTGTGACA
ATGCTTGAAGAAGCAATACATTACGTGAAGTTTCTACAGCTGCAAGTCAACATGTTAAGCTCTGATGAGTACTGG
ACTTATGCACCCACA
 Selaginella moelendorfii SmRSLg nucleotide sequence (SEQ ID NO: 53) 
LNTDGKPRAKRGSATDPQSIYARQRRERINERLRALQGLVPNGAKVDIVTMLEEAINYVKFLQLQVKLLSSDEYW
MYAPT
 Selaginella moelendorfii SmRSLh amino acid sequence (SEQ ID NO: 54) 
CTAAACACCGACGGAAAGCCACGCGCAAAGCGTGGATCTGCCACGGACCCGCAAAGTATCTACGCTCGGCAAAGA
AGAGAAAGGATCAACGAGCGTTTGAGAGCGCTACAAGGACTCGTACCAAACGGAGCGAAGGTTGACATTGTGACG
ATGCTCGAGGAAGCCATCAACTATGTCAAGTTTTTGCAGCTGCAAGTAAAGCTGCTCAGCTCGGACGAGTATTGG
ATGTACGCCCCCACA
 Selaginella moelendorfii SmRSLh nucleotide sequence (SEQ ID NO: 55) 
MMAAQASSKRGMLLPREAVLYDDEPSMPLEILGYHGNGVGGGGCVDADYYYSWSGSSSSSSSSVLSFDQAAVGGS
GGGCARQLAFHPGGDDDDCAMWMDAAAGAMVENTSVVAGGGNNYCHRLQFHGGAAGFGLASPGSSVVDNGLEIHE
SNVSKPPPPAAKKRACPSGEARAAGKKQCRKGSKPNKAASASSPSPSPSPSPSPNKEQPQSAAAKVRRERISERL
KVLQDLVPNGTKVDLVTMLEKAINYVKFLQLQVKVLATDEFWPAQGGKAPELSQVKDALDAILSSQHPNK*
 Rice (Oryza sativa subsp. Japonica) OsRSLa amino acid sequence (SEQ ID NO: 56; LOC_Os01g02110.1 11971.m06853) 
ATGATGGCAGCTCAGGCAAGCAGCAAGCGCGGCATGCTGCTGCCACGGGAGGCGGTGCTCTACGACGAGCCC
TCCATGCCGCTGGAGATCTTGGGCTACCACGGCAATGGCGTCGGCGGCGGTGGCTGCGTTGACGCCGATTACTAC
TACAGCTGGTCGGGGTCCAGCTCCAGCTCCAGCTCGTCGGTGCTCAGCTTTGACCAGGCGGCGGTCGGCGGCAGC
GGCGGCGGCTGCGCCCGGCAGCTGGCTTTCCATCCCGGCGGCGACGACGACGACTGCGCCATGTGGATGGACGCC
GCCGCCGGCGCCATGGTCGAGAACACGTCTGTCGTCGCCGGCGGCGGCAACAACTACTGTCATCGCCTGCAGTTC
CACGGCGGCGCCGCCGGTTTCGGACTCGCGAGCCCAGGCTCGTCGGTCGTTGACAACGGCCTCGAAATCCACGAG
AGCAACGTCAGCAAGCCGCCACCGCCGGCAGCCAAGAAGCGCGCATGCCCGAGCGGCGAGGCGAGAGCAGCGGGG
AAGAAGCAGTGCAGGAAAGGGAGCAAGCCAAACAAGGCTGCTTCTGCTTCTTCTCCTTCTCCTTCTCCTTCTCCT
TCTCCTTCTCCTAACAAGGAACAACCTCAAAGCGCCGCTGCAAAGGTAAGAAGAGAGCGGATCAGTGAGAGGCTC
AAAGTTCTTCAGGATCTCGTGCCTAATGGCACAAAGGTAGACTTGGTCACCATGCTAGAAAAGGCGATCAACTAC
GTCAAATTCCTCCAGCTGCAAGTGAAGGTTTTGGCTACTGATGAGTTCTGGCCGGCACAAGGAGGGAAAGCACCA
GAGCTCTCTCAAGTCAAGGACGCCTTGGACGCCATCCTATCTTCTCAGCATCCAAACAAATGA
 Rice OsRSLa nucleotide sequence (SEQ ID NO: 57; LOC_Os01g0110.1 11971.m06853) 
MRMALVRERAMVYGGGCDAEAFGGGFESSQMGYGHDALLDIDAAALFGGYEAAASAGCALVQDGAAGWAGAGASS
SVLAFDRAAQAEEAECDAWIEAMDQSYGAGGEAAPYRSTTAVAFDAATGCFSLTERATGGGGGAGGRQFGLLFPS
TSGGGVSPERAAPAPAPRGSQKRAHAESSQAMSPSKKQCGAGRKAGKAKSAPTTPTKDPQSLAAKNRRERISERL
RILQELVPNGTKVDLVTMLEKAISYVKFLQLQVKVLATDEFWPAQGGKAPEISQVKEALDAILSSSSPLMGQLMN
*
 Rice OsRSLb amino acid sequence (SEQ ID NO: 58; LOC_Os02g4B060.1 11972.m09840) 
ATGCGCATGGCGCTGGTGCGGGAGCGCGCGATGGTGTACGGTGGAGGGTGCGACGCCGAGGCGTTCGGCGGCGGG
TTCGAGTCGTCCCAGATGGGGTACGGCCACGACGCGCTGCTCGACATCGACGCGGCGGCGCTGTTCGGGGGTAC
GAGGCGGCCGCCAGCGCCGGGTGCGCCCTCGTGCAGGACGGCGCCGCGGGTGGCGGCGCGGGCGCGTCGTCC
TCGGTGCTGGCGTTCGACCGCGCCGCTCAGGCGGAGGAGGCCGAGTGCGACGCGTGGATCGAAGCCATGGACCAG
AGCTACGGCGCCGGCGGCGAGGCGGCGCCGTACCGGTCGACGACGGCCGTCGCCTTCGACGCGGCCACCGGCTGC
TTCAGCCTGACGGAGAGAGCCACCGGCGGCGGCGGCGCGGGTGGCGGCAGTTCGGGCTGCTGTTCCCGAGC
ACGTCGGGCGGCGGCGTCTCCCCCGAACGCGCCGCGCCGGCGCCGGCGCCCCGCGGCTCGCAGAAGCGGCCCAC
GCGGAGTCGTCGCAGGCCATGAGCCCTAGCAAGAAGCAGTGCGGCGCCGGCAGGAAGGCGGGCAAGGCCAAGTCG
GCGCCGACCACCCCAACCAAGGACCCGCAAAGCCTCGCGGCCAAGAATCGGCGCGAGAGGATCAGCGAGCGGCTG
CGGATCCTGCAGGAGCTCGTGCCCAACGGCACCAAGGTCGACCTCGTCACCATGCTCGAGAAGGCCATCAGCTAC
GTCAAGTTCCTCCAGCTTCAAGTCAAGGTTCTTGCGACGGACGAGTTCTGGCCGGCGCAGGGAGGGAAGGCGCCG
GAGATATCCCAGGTGAAGGAGGCGCTCGACGCCATCTTGTCGTCGTCGTCGCCGCTGATGGGACAACTCATGAAC
TGA
 Rice OsRSLb nucleotide sequence (SEQ ID NO: 59; LOC_Os02g48060.1 11972.m09840) 
MAMVAGDEAMSVPWHDVGVVVDPEAAGTAPFDAGAGYVPSYGQCQYYYYYDDHHHHPCSTELIHAGDAGSAVAVA
YDGVDGWVHAAAAATSPSSSSALTFDGHGAEEHSAVSWMDMDMDAHGAAPPLIGYGPTAATSSPSSCFSSGGSGD
SGMVMVTTTTPRSAAASGSQRRARPPPSPLQGSELHEYSKKQRANNKETQSSAAKSRRERISERLRALQELVPSG
GKVDMVTMLDRAISYVKFMQMQLRVLETDAFWPASDGATPDISRVKDALDAIILSSSSPSQKASPPRSG*
 Rice OsRSLc amino acid sequence (SEQ ID NO: 60; LOC_Os06g30090.1 11976.m07553) 
ATGGCTATGGTGGCCGGCGACGAGGCGATGTCAGTGCCATGGCACGACGTCGGCGTCGTCGTCGACCCCGAGGCG
GCCGGGACGGCGCCGTTCGACGCCGGCGCCGGCTATGTCCCATCGTACGGTCAGTGCCAATACTACTACTACTAC
GACGACCACCACCACCACCCGTGCAGCACGGAGCTGATCCACGCGGGCGACGCTGGCAGTGCGGTTGCGGTTGCG
TACGACGGCGTCGACGGCTGGGTTCACGCCGCCGCCGCAGCCACCTCCCCGTCCTCGTCATCTGCGCTCACCTTC
GATGGTCACGGCGCCGAGGAGCACAGCGCAGTGTCGTGGATGGACATGGACATGGACGCGCACGGCGCCGCGCCT
CCCCTAATCGGCTACGGCCCGACGGCGGCGACCTCCTCCCCCTCCTCCTGCTTCAGCTCCGGCGGCTCCGGCGAC
AGCGGCATGGTGATGGTGACCACCACCACCCCGAGGAGCGCCGCCGCCTCTGGTTCGCAGAGGCGGCACGCCCG
CCGCCGTCGCCGTTGCAGGGATCAGAGCTGCACGAGTACTCCAAGAAGCAGCGCGCCAACAACAAGGAGACACAG
AGCTCAGCTGCCAAGAGCCGGCGGGAGAGGATCAGCGAGCGGCTGAGGGCGCTGCAGGAGCTGGTGCCGAGCGGC
GGGAAGGTGGACATGGTGACCATGCTGGACAGGGCCATCAGCTACGTCAAGTTCATGCAGATGCAGCTCAGGGTG
CTGGAGACCGACGCGTTCTGGCCGGCGTCCGACGGCGCCACGCCGGACATCTCCCGGGTCAAGGACGCGCTCGAC
GCCATCATCCTCTCCTCGTCCTCGCCCTCGCAAAAGGCTTCTCCTCCTCGGTCGGGCTAG
 Rice OsRSLc nucleotide sequence (SEQ ID NO: 61; LOC_Os06g30090.1 11976.m07553) 
MEDSEAMAQLLGVQYFGNDQEQQQPAAAAPPAMYWPAHDAADQYYGSAPYCYMQQQQHYGCYDGGAMVAGGDFFV
PEEQLVADPSFMVDLNLEFEDQHGGDAGGAGSSAAAAAAATKMTPACKRKVEDHKDESCTDNVARKKARSTAATV
VQKKGNKNAQSKKAQKGACSRSSNQKESNGGGDGGNVQSSSTNYLSDDDSLSLEMTSCSNVSSASKKSSLSSPAT
GHGGAKARAGRGAATDPQSLYARKRRERINERLKILQNLIPNGTKVDISTMLEEAVHYVKFLQLQIKLLSSDDMW
MFAPIAYNGVNVGLDLKISPPQQQ*
 Rice OsRSLd amino acid sequence (SEQ ID NO: 62; LOC_Os03g10770.1 11973.m06529) 
ATGGAGGACTCGGAGGCGATGGCGCAGCTGCTCGGCGTGCAGTACTTCGGCAATGACCAGGAGCAGCAGCAGCCG
GCGGCGGCGGCGCCGCCGGCGATGTACTGGCCGGCGCACGACGCGGCCGACCAGTACTACGGCTCGGCGCCATAC
TGCTACATGCAGCAGCAGCAGCATTACGGGTGCTACGACGGCGGCGCGATGGTGGCCGGCGGCGACTTCTTCGTG
CCGGAGGAGCAGCTGGTGGCCGACCCGAGCTTCATGGTGGACCTGAACCTCGAGTTCGAGGACCAGCACGGCGGC
GATGCTGGCGGCGCTGGGAGCAGCGCCGCCGCCGCCGCCGCCACCAAGATGACACCGGCGTGCAAGAGGAAG
GTTGAGGATCACAAGGATGAGAGCTGCACGGACAACGTCGCGAGGAAGAAGGCGCGCTCCACGGCAGCAACAGTG
GTGCAGAAGAAGGGTAATAAGAACGCGCAGTCAAAGAAGGCGCAGAAGGGCGCGTGCAGCCGGAGCAGCAACCAG
AAGGAGAGCAATGGCGGCGGCGACGGCGGCAATGTGCAGAGCTCGAGCACCAACTACCTCTCTGATGACGACTCG
CTGTCGCTGGAGATGACTTCGTGCAGCAACGTGAGCTCGGCGTCCAAGAAGTCGTCGTTGTCATCGCCGGCGACC
GGGCACGGCGGCGCGAAGGCGAGGGCCGGGCGCGGGGCGGCGACCGATCCGCAAAGCCTCTATGCCAGGAAGAGG
AGAGAAAGGATCAATGAACGGCTAAAGATACTGCAGAATCTTATCCCAAATGGAACCAAGGTGGACATCAGCACG
ATGCTTGAAGAAGCAGTTCACTACGTCAAGTTCTTGCAGCTCCAAATCAAGCTTCTGAGCTCGGATGATATGTGG
ATGTTCGCGCCGATCGCGTACAACGGGGTCAACGTCGGGCTCGACCTCAAGATCTCTCCACCGCAGCAGCAATGA
 Rice OsRSLd nucleotide sequence (SEQ ID NO: 63; LOC_Os03g10770.1 11973.m06529) 
MESGGVIAEAGWSSLDMSSQAEESEMMAQLLGTCFPSNGEDDHHQELPWSVDTPSAYYLHCNGGSSSAYSSTTSS
NSASGSFTLIAPRSEYEGYYVSDSNEAALGISIQEQGAAQFMDAILNRNGDPGFDDLADSSVNLLDSIGASNKRK
IQEQGRLDDQTKSRKSAKKAGSKRGKKAAQCEGEDGSIAVTNRQSLSCCTSENDSIGSQESPVAAKSNGKAQSGH
 
RSATDPQSLYARKRRERINERLKILQNLVPNGTKVDISTMLEEAMHYVKFLQLQIKLLSSDEMWMYAPIAYNGMN
IGIDLNLSQH*
 Rice OsRSLe amino acid sequence (SEQ ID NO: 64; LOC_Os03g42100.1 11973.m09268) 
ATGGAGTCCGGAGGGGTGATCGCGGAGGCGGGGTGGAGCTCGCTCGACATGTCGTCGCAGGCCGAGGAGTCGGAG
ATGATGGCGCAGCTGCTTGGAACCTGCTTCCCCTCCAATGGCGAGGATGATCATCACCAAGAGCTTCCTTGGTCG
GTTGACACCCCCAGTGCCTACTACCTCCATTGCAATGGAGGTAGCTCAAGTGCATACAGCTCTACCACTAGCAGC
AACAGTGCTAGTGGTAGCTTCACTCTCATTGCACCAAGATCTGAGTATGAGGGGTACTATGTGAGTGACTCTAAT
GAGGCGGCCCTCGGGATCAGCATCCAGGAGCAAGGTGCAGCTCAGTTCATGGATGCCATTCTCAACCGGAACGGC
GATCCGGGCTTCGATGATCTCGCTGACTCGAGCGTTAATCTGCTGGATTCCATCGGCGCTTCTAACAAGAGAAAG
ATTCAGGAGCAAGGCAGGCTAGATGACCAAACGAAAAGTAGGAAATCTGCGAAGAAGGCTGGCTCGAAGCGGGGA
AAGAAGGCGGCGCAATGTGAAGGTGAAGATGGCAGCATTGCTGTCACCAACAGGCAAAGCTTGAGCTGCTGCACC
TCTGAAAATGATTCGATTGGTTCTCAAGAATCTCCTGTTGCTGCTAAGTCGAATGGCAAGGCTCAATCTGGCCAT
CGGTCAGCAACCGATCCCCAGAGCCTCTATGCAAGGAAAAGAAGAGAGAGGATCAATGAGAGGCTCAAGATTCTG
CAGAACCTTGTACCAAATGGAACCAAAGTAGATATCAGCACTATGCTTGAAGAGGCAATGCATTACGTGAAGTTC
TTGCAGCTTCAAATCAAGCTCCTCAGCTCTGATGAAATGTGGATGTACGCACCGATTGCTTACAACGGATGAAC
ATCGGATCGATTTGAACCTCTCTCAGCATTGA
 Rice OsRSLe nucleotide sequence (SEQ ID NO: 65; LOC_Os03g42100.1 11973.m09268) 
MDARCANIWSSADARSEESEMIDQLKSMFWSSTDAEINFYSPDSSVNSCVTTSTMPSSLFLPLMDDEGFGTVQLM
HQVITGNKRMFPMDEHFEQQQKKPKKKTRTSRSVSSSSTITDYETSSELVNPSCSSGSSVGEDSIAATDGSVVLK
QSDNSRGHKQCSKDTQSLYAKRRRERINERLRILQQLVPNGTKVDISTMLEEAVQYVKFLQLQIKLLSSDDTWMF
APLAYNGMNMDLGHTLAENQE*
 Rice OsRSLf amino acid sequence (SEQ ID NO: 66; LOC_Os11g41640.1 11981.m08005) 
ATGGATGCAAGGTGTGCAAACATCTGGAGCTCTGCTGATGCAAGGAGTGAGGAATCTGAGATGATTGATCAACTA
AAGTCCATGTTCTGGAGCAGCACTGATGCTGAAATCAACTTTTATTCTCCTGACAGTAGTGTAAATTCTTGTGTC
ACAACTAGCACAATGCCTAGCAGCTTGTTTCTTCCTCTGATGGATGATGAGGGATTTGGCACAGTGCAATTGATG
CATCAGGTCATCACTGGGAACAAGAGGATGTTCCCCATGGATGAGCACTTTGAGCAGCAGCAGAAGAAGCCGAAG
AAGAAAACCCGAACTTCTCGCTCGGTATCAAGTAGTTCAACCATTACTGACTATGAGACTAGCTCTGAACTTGTC
AATCCTAGCTGTTCCTCCGGGAGCAGCGTCGGAGAGGATTCAATTGCTGCAACTGATGGATCTGTAGTGCTGAAA
CAAAGTGACAATTCAAGAGGCCATAAGCAGTGCTCCAAGGATACACAAAGCCTCTATGCTAAGAGGAGAAGGGAA
AGGATTAATGAGAGACTGAGAATACTTCAGCAGCTTGTTCCCAATGGCACTAAAGTTGACATCAGCACAATGCTG
GAGGAAGCAGTTCAGTATGTCAAGTTTTTGCAGTTGCAAATAAAGCTATTGAGCTCTGACGACACATGGATGTTT
GCGCCCCTAGCCTATAATGGCATGAACATGGATCTCGGTCATACTCTTGCTGAAAACCAAGAATGA
 Rice OsRSLf nucleotide sequence (SEQ ID NO: 67; LoC_Os11g41640.1 11981.m08005) 
MECSSFEAICNESEMIAHLQSLFWSSSDADPCFGSSSFSLISSEGYDTMTTEFVNSSTNVCFDYQDDSFVSAEET
TIGNKRKVQMDTENELMTNRSKEVRTKMSVSKACKHSVSAESSQSYYAKNRRQRINERLRILQELIPNGTKVDIS
TMLEEAIQYVKFLHLQIKLLSSDEMWMYAPLAFDSGNNRLYQNSLSQE*
 Rice OsRSLg amino acid sequence (SEQ ID NO: 68; LOC_Os12g32400.1 11982.m07043) 
ATGGAATGCAGCTCCTTTGAAGCAATCTGCAATGAGTCGGAGATGATTGCGCATTTGCAGTCATTGTTCTGGAGC
AGCAGCGATGCTGATCCTTGTTTTGGTAGCTCATCATTTTCTCTCATCAGTAGTGAGGGCTACGACACAATGACC
ACAGAGTTTGTGAATAGCAGCACAAATGTATGTTTTGATTACCAAGATGATAGCTTCGTTTCAGCAGAGGAGACT
ACCATTGGTAACAAGAGAAAAGTTCAGATGGATACTGAGAATGAGCTGATGACGAACCGCAGCAAGGAAGTTCGC
ACCAAGATGTCGGTGTCAAAAGCATGCAAACATTCTGTTTCTGCAGAGAGCTCACAGTCTTATTATGCAAAGAAC
 
AGGAGACAGAGGATCAATGAGAGATTGAGAATACTGCAAGAACTGATCCCTAATGGAACAAAAGTTGACATCAGC
ACAATGTTGGAGGAAGCAATTCAGTATGTCAAGTTTCTACACCTGCAAATCAAGCTCTTGAGCTCTGATGAAATG
TGGATGTATGCGCCCCTTGCTTTTGACAGTGGTAACAACAGGCTCTATCAGAACTCTCTGTCACAAGAGTAG
 Rice OsRSLg nucleotide sequence (SEQ ID NO: 69; LOC_Os12g32400.1 11982.m07043) 
MEGGGLIADMSWTVFDLPSHSDESEMMAQLFSAFPIHGEEEGHEQLPWFDQSSNPCYYSCNASSTAYSNSNASSI
PAPSEYEGYCFSDSNEALGVSSSIAPHDLSMVQVQGATEFLNVIPNHSLDSFGNGELGHEDLDSVSGTNKRKQSA
EGEFDGQTRGSKCARKAEPKRAKKAKQTVEKDASVAIPNGSCSISDNDSSSSQEVADAGATSKGKSRAGRGAATD
PQSLYARKRRERINERLKTLQNLVPNGTKVDISTMLEEAVHYVKFLQLQIKLLSSDEMWMYAPIAYNGMNIGLDL
NIDT*
 Rice OsRSLh amino acid sequence (SEQ ID NO: 70; LOC_Os12g39850.1 11982.m07769) 
ATGGAGGGTGGAGGACTGATCGCCGATATGAGCTGGACCGTCTTCGACTTGCCATCGCACAGCGATGAGTCGGAG
ATGATGGCGCAGCTCTTCAGTGCATTCCCCATCCATGGTGAGGAGGAAGGCCATGAGCAGCTCCCATGGTTTGAT
CAATCTTCCAATCCATGCTACTATAGCTGCAATGCTAGCAGCACTGCATACAGCAACAGCAATGCTAGTAGCATT
CCTGCTCCATCTGAGTATGAAGGATACTGCTTCAGTGACTCAAATGAGGCCCTGGGTGTCAGCTCCAGCATTGCA
CCACATGACCTGAGCATGGTCCAGGTGCAAGGTGCAACTGAGTTTCTGAATGTGATCCCAAACCATTCCCTTGAT
TCATTCGGTAATGGCGAGCTGGGCCACGAGGATCTTGATTCGGTTAGTGGGACTAACAAGAGAAAACAGTCGGCA
GAAGGAGAATTTGATGGCCAAACAAGAGGTTCAAAATGCGCGAGAAAGGCTGAACCGAAGCGAGCGAAGAAGGCC
AAGCAAACTGTGGAGAAGGATGCAAGTGTTGCCATCCCAAATGGGAGCTGTTCCATTTCTGACAATGATTCCAGT
TCATCCCAGGAGGTTGCAGATGCTGGTGCTACTTCGAAAGGCAAATCCCGGGCTGGCCGCGGAGCAGCCACTGAT
CCCCAGAGCCTCTATGCAAGGAAAAGGAGAGAGAGGATCAATGAGAGGCTCAAGACACTTCAGAACCTTGTGCCC
AATGGCACCAAAGTTGATATCAGCACCATGCTTGAGGAGGCAGTCCACTATGTGAAGTTCCTGCAGCTTCAGATC
AAGCTCCTCAGCTCCGATGAAATGTGGATGTATGCGCCAATTGCGTACAACGGGATGAACATTGGGCTCGATCTG
AACATTGATACATGA
 Rice OsRSLh nucleotide sequence (SEQ ID NO: 71; LOC_Os12g39850.1 11982.m07769) 
MAQFLGAHGDHCFTYEQMDESMEAMAAMFLPGLDTDSNSSSGCLNYDVPPQCWPQHGHSSSVTSFPDPAHSYGSF
EFPVMDPFPIADLDAHCAIPYLTEDLISPPHGNHPSARVEEATKVVTPVATKRKSSAAMTASKKSKKAGKKDPIG
SDEGGNTYIDTQSSSSCTSEEGNLEGNAKPSSKKMGTRANRGAATDPQSLYARKRRERINERLRILQNLVPNGTK
VDISTMLEEAVQYVKFLQLQIKLLSSDDTWMYAPIAYNGVNISNIDLNISSLQK*
 Rice OsRSLi amino acid sequence (SEQ ID NO: 72; LOC_Os07g39940.1 11977.m08236) 
ATGGCGCAGTTTCTTGGAGCTCATGGTGATCACTGCTTCACCTACGAGCAAATGGATGAGTCCATGGAGGCAATG
GCAGCGATGTTCTTGCCTGGCCTTGACACCGACTCCAATTCTTCTTCTGGTTGTCTCAACTACGATGTGCCTCCA
CAATGCTGGCCTCAGCATGGCCATAGCTCTAGCGTCACCAGCTTCCCTGATCCAGCTCATAGCTATGGAAGCTTT
GAGTTCCCGGTCATGGATCCGTTCCCGATCGCCGATCTCGACGCGCATTGCGCCATCCCCTACCTTACTGAGGAT
CTGATCAGCCCTCCACATGGCAACCATCCATCAGCAAGAGTGGAAGAAGCTACAAAGGTTGTTACACCAGTGGCT
ACCAAGAGGAAGTCTAGTGCTGCCATGACGGCATCAAAGAAGAGCAAGAAGGCTGGCAAAAAAGATCCTATTGGC
AGCGACGAAGGCGGCAACACCTACATTGATACGCAAAGTTCTAGCAGTTGCACCTCAGAGGAAGGAAACCTGGAG
GGCAACGCGAAGCCGAGCTCGAAGAAGATGGGTACTAGGGCCAACCGTGGGGCGGCAACCGATCCCCAGAGTCTC
TATGCAAGGAAGAGGAGAGAGAGGATCAATGAAAGATTGAGGATCCTGCAGAACTTGGTTCCCAATGGAACAAAG
GTTGACATCAGTACAATGCTGGAGGAAGCAGTGCAGTATGTCAAATTTTTGCAACTTCAGATTAAGTTGCTAAGC
TCTGATGACACGTGGATGTATGCACCAATCGCTTACAATGGAGTCAACATCAGCAATATTGATCTGAACATCTCT
TCTCTGCAAAAATAA
 Rice OsRSLi nucleotide sequence (SEQ ID NO: 73; LOC_Os07g39940.1 11977.m08236) 
MALAKDRMGSVQTCPYNGNVMGDFSSMGSYGFDEYQKVAFYEEGNSTFEKTSGLMIKNLAMTSSPSSLGSPSSAI
SGELVFQATDHQAEEAHSLISFKGIGFDNIMHNNGSLLSFEQSSRVSQTSSQKDDYSAWEGNLSYNYQWNEMNPK
CNTSPRLMEDFNCFQRAGNFISMTGKENHGDWLYAESTIVADSIQDSATPDASSFHKRPNMGESMQALKKQCNNA
TKKPKPKSAAGPAKDLQSIAAKNRRERISERLKVLQDLVPNGSKVDLVTMLEKAISYVKFLQLQVKVLATDELWP
VQGGKAPDISQVKEAIDALLSSQTKDGNSSSSPK*
 Populus trichocarpa PtRSLa amino acid sequence (SEQ ID NO: 74) 
ATGGCACTTGCCAAGGACCGTATGGGATCGGTTCAAACTTGCCCCTATAATGGAAATGTGATGGGGGATTTTTCC
TCCATGGGGTCTTACGGATTTGATGAATATCAGAAGGTAGCATTTTATGAAGAGGGAAATAGCACCTTTGAGAAA
ACCAGTGGGCTTATGATCAAGAATTTAGCTATGACCTCTTCTCCTTCTTCTCTTGGCAGTCCGAGCAGCGCGATT
TCTGGTGAATTAGTGTTTCAGGCTACTGACCATCAAGCTGAGGAAGCTCATTCTTTGATCAGCTTCAAAGGTATC
GGATTCGATAACATCATGCATAATAATGGATCTTTGCTTAGCTTTGAGCAAAGTAGTAGGGTTTCTCAAACTAGT
AGCCAGAAAGATGACTACTCAGCCTGGGAGGGTAATTTGAGTTACAACTACCAGTGGAACGAAATGAATCCAAAA
TGTAACACAAGTCCTCGGTTGATGGAAGATTTTAATTGCTTTCAAAGAGCTGGCAACTTCATTTCCATGACTGGA
AAGGAAAATCATGGTGATTGGTTATACGCTGAATCCACAATTGTTGCTGATAGCATTCAGGATTCTGCAACACCA
GATGCCAGCAGCTTCCATAAGCGTCCTAATATGGGAGAGAGTATGCAGGCTCTAAAGAAGCAATGCAACAATGCA
ACAAAAAAGCCAAAACCGAAGTCCGCAGCAGGTCCAGCTAAGGATCTACAGAGTATTGCTGCCAAGAATCGACGA
GAGAGGATTAGCGAGAGGCTTAAGGTATTGCAGGATTTAGTCCCTAATGGCTCAAAGGTTGATTTGGTTACTATG
CTAGAGAAAGCCATTAGTTATGTTAAGTTTCTTCAATTGCAAGTAAAGGTGTTAGCCACTGATGAATTATGGCGA
GTTCAAGGTGGTAAAGCTCCTGATATTTCTCAAGTAAAGGAAGCCATCGATGCCCTACTCTCATCTCAGACTAAA
GACGGAAACTCAAGCTCAAGCCCAAAGTAA
 Populus trichocarpa PtRSLa nucleotide sequence (SEQ ID NO: 75) 
MALAKDRMDSVQTCALYGNVMGDLSSLGPNYRFDEEGDRNFEKNSALMIKNLAMSPSPPSLGSPSSANSGELVFQ
ATDNQVEEAHSLINFKGTGFDSIMHANGSLISFEQSNRVSQTSSHKDDYSAWEGNLSCNYQWNQINPKCNANPRL
MEDLNCYQSASNFNSITNSAEKENHGDWLYTHESTIVTDSIPDSATPDASSFHKRPNMGESMQALKKQRDSATKK
PKPKSAGPAKDPQSIAAKNRRERISERLKMLQDLVPNGSKVDLVTMLEKAISYVKFLQLQVKVLATDEFWPVQGG
KAPDISQVKGAIDATLSSQTKDRNSNSSSK*
 Populus trichocarpa PtRSLb amino acid sequence (SEQ ID NO: 76) 
ATGGCACTTGCCAAGGACCGTATGGATTCGGTTCAAACTTGCGCCCTTTATGGAAATGTGATGGGGGATCTTTCC
TCCTTGGGGCCTAATTATAGATTTGATGAAGAGGGAGATAGGAACTTTGAGAAAAATAGTGCGCTTATGATCAAG
AATTTAGCTATGAGCCCTTCTCCTCCTTCTCTTGGCAGTCCAAGCAGTGCAAATTCTGGTGAACTAGTGTTTCAG
GCTACTGACAATCAAGTTGAGGAAGCTCATTCTTTGATCAACTTCAAAGGTACCGGATTTGATAGTATCATGCAT
GCTAATGGATCTTTGATTAGCTTTGAGCAAAGTAATAGGGTTTCTCAAACTAGTAGTCACAAAGATGACTACTCT
GCTTGGGAGGGTAATTTGAGTTGCAATTACCAGTGGAACCAAATCAATCCAAAATGTAACGCAAATCCTCGGTTG
ATGGAAGATCTTAATTGCTATCAAAGTGCAAGCAACTTCAACTCCATAACCAACAGTGCTGAAAAGGAAAACCAT
GGTGATTGGTTATACACTCATGAATCCACAATTGTTACTGATAGCATTCCCGATTCTGCAACACCAGATGCCAGC
AGCTTCCATAAGCGTCCCAATATGGAGAGAGTATGCAGGCTCTAAAGAAGCAACGCGACAGCGCCACAAAAAAG
CCGAAACCCAAGTCTGCTGGTCCAGCTAAGGATCCACAAAGTATTGCTGCCAAGAATCGACGAGAGCGGATTAGC
GAGCGCCTTAAGATGTTGCAGGATTTAGTCCCTAACGGCTCCAAGGTTGATTTGGTTACTATGCTAGAGAAAGCC
ATTAGTTATGTTAAGTTTCTTCAATTGCAAGTAAAGGTGTTGGCCACTGATGAATTCTGGCCAGTTCAAGGTGGT
AAAGCTCCTGATATTTCTCAAGTAAAGGGAGCCATTGATGCCACACTCTCATCTCAGACTAAAGACAGAAATTCA
AACTCAAGCTCAAAGTGA
 Populus trichocarpa PtRSLb nucleotide sequence (SEQ ID NO: 77) 
MAEGEWSSLGGMYTSEEADFMAQLLGNCPNQVDSSSNFGVPSSFWPNHEPTTDMEGANECLFYSLDFANINLHHF
SQGSSSYSGGSGILFPNTSQDSYYMSDSHPILANNNSSMSMDFCMGDSYLVEGDDCSNQEMSNSNEEPGGNQTVA
ALPENDFRAKREPEMPASELPLEDKSSNPPQISKKRSRNSGDAQKNKRNASSKKSQKVASTSNNDEGSNAGLNGP
ASSGCCSEDESNASHELNRGASSSLSSKGTATLNSSGKTRASRGAATDPQSLYARKRRERINERLRILQTLVPNG
TKVDISTMLEEAVQYVKFLQLQIKLLSSEDLWMYAPIAYNGMDIGLDHLKVTAP*
 Populus trichocarpa PtRSLc amino acid sequence (SEQ ID NO: 78) 
ATGGCAGAGGGAGAGTGGAGTTCTCTTGGTGGAATGTACACTAGTGAGGAGGCTGATTTCATGGCACAGTTGCTT
GGTAACTGTCCTAATCAGGTTGATTCAAGTTCAAACTTTGGAGTTCCATCTAGTTTCTGGCCTAACCACGAACCA
ACAACGGACATGGAAGGGGCTAATGAATGTTTATTTATTCTTTGGATTTTGCTAATATTAATTTGCACCATTTT
TCACAAGGGAGTAGTAGTTATAGTGGTGGCAGTGGCATTCTTTTTCCCAACACAAGCCAAGATAGCTACTACATG
AGTGATTCTCATCCAATTTTGGCTAACAATAATAGCTCAATGTCAATGGATTTTTGCATGGGAGACTCATATCTC
GTTGAAGGCGATGACTGCTCAAACCAAGAAATGAGCAATAGCAATGAGGAGCCTGGTGGAAACCAGACTGTAGCT
GCTCTTCCTGAAAACGATTTTCGGGCCAAGAGAGAACCAGAGATGCCAGCTTCTGAACTACCCCTGGAAGACAAA
AGCAGCAACCCACCTCAGATTTCTAAGAAAAGATCACGAAATTCAGGAGATGCTCAAAAGAACAAGAGGAATGCA
AGTTCAAAGAAGAGCCAGAAGGTTGCCTCGACTAGCAACAATGATGAAGGAAGTAATGCTGGCCTTAATGGGCCT
GCCTCAAGCGGTTGCTGCTCAGAGGATGAATCCAATGCCTCTCATGAGCTCAATAGAGGAGCGAGTTCAAGTTTG
AGCTCGAAAGGGACTGCAACTCTCAACTCAAGTGGCAAAACAAGAGCCAGCAGGGGGGCAGCCACTGATCCCCAG
AGTCTCTATGCAAGGAAAAGAAGAGAAAGAATAAATGAGAGGCTGAGAATTCTACAAACCCTTGTCCCCAACGGA
ACAAAGGTTGACATTAGCACAATGCTTGAAGAAGCTGTCCAGTATGTGAAGTTTTTGCAACTCCAAATTAAGCTG
CTAAGCTCTGAGGACTTGTGGATGTATGCGCCTATCGCTTACAACGGATGGACATCGGTCTTGATCATCTGAAG
GTTACCGCACCATGA
 Populus trichocarpa PtRSLc nucleotide sequence (SEQ ID NO: 79) 
MEPIGATAEGEWSSLSGMYTSEEADFMEQLLVNCPPNQVDSSSSFGVPSSFWPNHESTMNMEGANECLLYSLDIA
DTNLYHFSQVSSGYSGELSNGNVEESGGNQTVAALPEPESNLQPKRESKMPASELPLEDKSRKPPENSKKRSRRT
GDAQKNKRNVRSKKSQKVASTGNNDEESNGGLNGPVSSGCCSEDESNASQELNGGASSSLSSKGTTTLNSSGKTR
ASKGAATDPQSLYARKRRERINERLRILQNLVPNGTKVDISTMLEEAVQYVKFLQLQIKLLSSEDLWMYAPIAYN
GMDIGLDHLKLTTPRRL*
 Populus trichocarpa PtRSLd amino acid sequence (SEQ ID NO: 80) 
ATGGAGCCTATTGGAGCCACTGCGGAGGGAGAGTGGAGTTCTCTTAGTGGAATGTACACAAGTGAGGAGGCTGAT
TTCATGGAACAGTTGCTTGTCAACTGTCCTCCTAATCAGGTTGATTCAAGTTCAAGCTTTGGAGTTCCATCTAGT
TTTTGGCCTAACCATGAATCAACAATGAACATGGAAGGGGCCAATGAATGTTTATTGTATTCTTTGGATATTGCT
GATACTAATCTGTACCATTTTTCACAAGTGAGCAGTGGTTATAGTGGTGAATTGAGCAATGGAAATGTGGAAGAG
TCTGGTGGAAACCAGACTGTAGCTGCTCTTCCTGAACCTGAAAGCAATTTGCAACCCAAGAGAGAATCAAAGATG
CCAGCATCTGAACTACCCCTGGAAGATAAAAGCAGAAAGCCACCTGAGAATTCCAAGAAAAGATCACGACGTACG
GGAGATGCCCAAAAGAACAAGAGGAATGTAAGGTCAAAGAAGAGCCAGAAGGTTGCCTCGACTGGCAACAATGAT
GAAGAAAGCAATGGTGGCCTTAATGGTCCTGTCTCAAGCGGTTGCTGCTCAGAGGATGAATCCAATGCCTCCCAG
GAGCTCAATGGAGGAGCGAGTTCAAGTTTGAGCTCAAAAGGGACAACAACTCTCAACTCAAGTGGCAAAACAAGA
GCCAGTAAGGGGGCAGCCACTGATCCCCAGAGCCTCTATGCAAGGAAAAGAAGAGAAAGAATAAATGAGAGGCTG
AGAATTCTACAAAACCTTGTCCCCAATGGAACAAAGGTTGACATTAGCACAATGCTTGAAGAGGCTGTCCAGTAT
GTGAAGTTTTTGCAACTCCAAATTAAGCTGCTAAGCTCTGAAGACCTGTGGATGTATGCTCCTATCGCGTACAAT
GGTATGGACATCGGTCTTGATCATCTGAAGCTTACCACACCAAGACGATTGTAG
 Populus trichocarpa PtRSLd nucleotide sequence (SEQ ID NO: 81) 
MNTQAMEAFRDGELWNFSRMFSMEEPDCTPELLGQCSFLQDTDEGLHFTIPSAFFPAPESDASMAEDESLFYSWH
TPNPNLHFDSQESSNNSNSSSSVFLPYSSHESYFFNDSNPIQATNNNSMSMDIMDEENIGLFMPLFPEIAMAETA
CMNGDMSGDKTGDLDDNLKPAANDVLAKGLQLKRKLDVPEPIANTLDDMKKKARVTRNVQKTRKVGQSKKNQKNA
PDISHDEEESNAGPDGQSSSSCSSEEDNASQDSDSKVSGVLNSNGKTRATRGAATDPQSLYARKRRERINERLKI
LQNLVPNGTKVDISTMLEEAVHYVNFLQLQIKLLSSDDLWMYAPLAYNGIDIGLNQKLSMFL*
 Populus trichocarpa PtRSLe amino acid sequence (SEQ ID NO: 82) 
ATGAATACGCAGGCTATGGAAGCCTTTCGTGATGGAGAATTATGGAACTTCAGCAGAATGTTCTCCATGGAAGAG
CCTGATTGCACCCCAGAATTACTTGGTCAGTGCTCTTTTCTTCAGGATACTGATGAAGGATTGCATTTACAATC
CCATCAGCTTTCTTCCCTGCTCCTGAATCCGACGCGAGCATGGCTGAGGACGAGAGTTTGTTTATTCTTGGCAT
ACTCCCAACCCCAATTTGCATTTTGATTCTCAAGAAAGTAGTAATAACAGTAATTCTAGCAGTAGTGTATTTCTT
CCCTATTCCAGCCATGAATCCTACTTCTTCAATGATTCTAATCCCATTCAAGCTACGAACAATAACTCTATGTCC
ATGGATATTATGGATGAGGAAAATATTGGCTTGTTTATGCCACTTTTTCCTGAAATTGCAATGGCAGAAACTGCC
TGTATGAATGGAGATATGAGCGGTGACAAAACAGGAGATTTAGATGATAATCTGAAGCCAGCAGCTAATGATGTT
 
CTGGCCAAGGGATTGCAGCTCAAAAGGAAGCTTGATGTTCCAGAACCAATAGCCAACACATTGGACGACATGAAG
AAAAAAGCCCGGGTTACAAGAAATGTGCAAAAGACTAGGAAGGTTGGACAGTCAAAAAAAATCAGAAGAACGCA
CCAGATATTAGCCATGATGAAGAAGAGAGTAATGCTGGACCAGACGGACAAAGTTCCAGCAGTTGTAGTTCAGAA
GAGGACAATGCCTCTCAGGATTCTGATTCCAAGGTTTCTGGAGTTCTCAATTCCAATGGAAAAACAAGAGCTACT
AGGGGAGCTGCCACAGACCCCCAGAGCCTTTATGCAAGGAAAAGAAGGGAGAGGATAAACGAGAGACTGAAAATC
TTGCAGAATCTTGTCCCTAACGGAACCAAGGTTGATATCAGCACGATGCTAGAAGAGGCAGTCCATTACGTAAAC
TTTTTGCAGCTTCAAATCAAGCTTTTGAGCTCGGATGATCTATGGATGTATGCACCTCTGGCTTACAATGGAATA
GATATTGGACTCAACCAGAAGCTCTCTATGTTTCTATGA
 Populus trichocarpa PtRSLe nucleotide sequence (SEQ ID NO: 83) 
MAQESTWSSFDATMLAEEESRMIAQLLSNYQCFGEQDRDVGCCELPPSSCCSSHAADSCYCWSANENSNPGLCYW
SQSGDESDGAHAIGTVPVFTNHCLVGDQVAVNQTLSIHEPTAAHAEMPKRKIESHASEDDFRRQSSKKKLQAPTN
ALKSVKKARPGRNQKSIVCGDEEENNARSSGRSCCSYSSEEDSQAFQADLNAKTRSNRWPATDPQSLYAKQRRER
INARLRTLQNLVPNGTKVDISTMLEEAVRYVKFLQLQIKLLSSDELWMYAPVVHSGMIDGQVNSEIFVSANTRNE
WF*
 Musa acuminata MaRSLa amino acid sequence (SEQ ID NO: 84; GI102139852, ABF70010.1) 
atggctcaggagtcaacttggagctcgtttgatgctacaatgcttgctgaggaggagtcccgaatgatcgcacaa
ttgctcagcaactaccagtgttttggcgagcaagatcgagatgttggatgctgtgaactcccgccatcgtcttgt
tgttcttctcatgcagctgattcatgttactgttggtcagcaaatgagaacagtaacccggtttgtgctactgg
tctcagagtggagatgaatccgatggagcacatgcaatcggcactgtgccggtcttcacgaaccattgcttggtg
ggagatcaagtcgctgtgaatcaaactttgagcattcacgaacctactgctgctcatgcagagatgccaaagcgc
aagatagagtctcatgcttctgaagatgatttccgtcgtcaaagttctaagaaaagcttcaggctccgacgaat
gctctgaagagcgtgaagaaggcacgacctgggaggaaccagaagagcattgtgtgtggtgatgaggaagagaac
aatgccaggagcagtggccggagttgctgcagctacagctctgaggaagactcacaagctttccaggctgatctt
aatgcaaaaacacgatcgaatcgatggccagccacagatcctcaaagcctctatgcaaagcaaagaaggaaaga
atcaatgctagattgaggacattgcagaacctggtgcctaatggaactaaagttgacattagcacaatgctcgaa
gaagctgttcgttacgtcaagttcttgcagctgcagataaagcttttgagctcggatgagctgtggatgtacgct
cctgttgtccacagtgggatgattgatggccaagtcaactcagagatatttgtgtctgcaaatactcgtaatgag
tggttctga
 *Musa* acuminata MaRSLa nucleotide sequence (SEQ ID NO: 85). 
MEPIGTFPEGEWDFFRKMFASEDHEYYSQQFLDQNSLLLGENDGLNNGTQSTFCTAEIGENERMFYSFDHAHIQN
SNYIPQTQENSYNSNSSASDDTNYYFSYPNHVLENNINNCISNDFRMDENLFASSVPSLNEIVMEENVRMNEDSA
SDDHIVEKNGYNTQIMEPFDLHTKHEMQMKLKRKLDVIEVEVPVEEKINNNPKKKPRVSNDGQGCMKNARSKKNH
KVIASHEEEMTEEINRGSNGNSSSSNISEDDNASQENSGGTTLNSNGKTRASRGSATDPQSLYARKRRERINERL
RVLQNLVPNGTKVDISTMLEEAVNYVKFLQTQIKLLSSDDMWMYAPLAYNGLDLGLNLNLNSSLPL*
 *Medicago truncatula* MtRSLa amino acid sequence (SEQ ID NO: 86; AC140548.11 GI:156231148) 
atggaacctataggtactttccctgaaggagaatggatttctttcgcaaaatgtttgcaagtgaagatcatgaa
tattactcacaacaatttcttgatcaaaattcacttcttctaggggaaaatgatgggttgaacaatggaacacag
tccacattttgcactgctgaaattggtgaaaatgagcgtatgtttattcttttgatcatgctcatatccaaaac
tctaactatattcctcaaactcaagagaatagttacaatagcaattctagtgctagtgatgatacaaattactat
tttagttatcctaatcatgtactagaaaataatattaataattgtatatccaatgatttcgcatggatgagaat
ttgtttgcttcttctgttccatcccttaatgagattgtaatggaagagaatgtgagaatgaatgaagattctgca
agtgatgatcatattgtggagaaaaatggttacaatactcaaataatggaacctttgatcttcacaccaagcat
gagatgcaaatgaagctcaaaaggaaacttgatgtgatagaagtggaggttcccgttgaagaaaaattaacaac
aatccgaagaaaaaacctcgtgtttcgaatgatggccaaggatgcatgaaaaatgcaaggtcaaagaagaaccac
aaagttattgctagccatgaagaggagatgacagaagagattaatagaggatcaaatggaaatagttctagtagt
aacatttctgaggatgataatgcttctcaagaaaatagtggaggaactactctcaactcaaatgggaagacaaga
gctagtagaggatctgcaacagatccccaaagtctatatgcaaggaaaagaagagagagaataaatgaacgacta
agagtcttacaaaatcttgtaccaaacggaacaaaggttgatatcagtacaatgcttgaagaggcagtcaattat
 
gtgaaatttttacagactcaaatcaagcttttgagctctgatgatatgtggatgtatgcaccacttgcttacaat
ggacttgaccttggactcaatctcaacctcaacagctctctaccactatga
 Medicago truncatula nucleotide sequence (SEQ ID NO:87, AC140548.11 GI:156231148) 
XFLCFSQGSSSSTDNSGNNIFSITSSGAYSCDPEANFDSVSMVLCLGDAKFSPHSFQCDDNSNQQINENTDEESS
LDPWKLAIADNNLQAKREYEMMVSEPVEVDRSRNLENLAKRLKSSIEVSKTLRSAKSGKNSKSASVSNDEDDRSL
SLQAQRNSCFSQSDSNAYLEPNGGASKDPAPPNLHRKSRATTGAATDPQSLYARKRRERINERLRILQNLVPNGT
KVDISTMLEEAVQYVKFLQLQIKLLSSDDLWMY
 Soybean GmRSLa amino acid sequence (SEQ ID NO: 88) (gi|26056905|gb|CA799819.1|CA799819) 
ATTTTTGTGTTTCTCACAAGGGAGTAGCTCCAGTACTGATAATAGTGGTAATAATATCTTTTCCATTACAAGTA
GTGGAGCCTACTCCTGTGATCCAGAAGCAAACTTTGATTCTGTGTCCATGGTTTTGTGCCTTGGAGATGCCAAAT
TTAGTCCCCATAGTTTTCAATGTGATGACAACTCAAACCAACAGATAAATGAAAACACTGATGAAGAGTCAAGTC
TAGACCCATGGAAGTTGGCTATAGCTGACAATAATTTGCAGGCTAAGAGGGAGTATGAAATGATGGTTTCTGAAC
CTGTAGAAGTGGATAGAAGCAGAAACCTGGAGAACCTAGCAAAAAGACTAAAGAGTTCAATAGAGGTTTCAAAAA
CATTGAGGAGTGCTAAATCAGGGAAAAATTCAAAATCTGCTTCAGTGAGCAACGATGAAGATGATAGAAGCTTGA
GCCTCCAAGCCCAAAGGAATAGCTGTTTTTCACAGAGTGACTCTAATGCTTATCTGGAGCCAAATGGAGGGGCAT
CAAAAGATCCTGCACCTCCCAATTTGCATAGAAAATCAAGAGCAACTACCGGTGCTGCCACTGATCCACAGAGCC
TCTATGCAAGAAAGAGAAGAGAAAGAATAAATGAAAGGTTGAGAATACTGCAAAATCTTGTTCCCAACGGAACTA
AGGTGGATATCAGCACCATGCTTGAGGAAGCTGTCCAATACGTGAAGTTTTTACAGCTCCAAATTAAGCTTCTGA
GCTCTGACGATCTGTGGATGTAT
 Soybean GmRSLa nucleotide sequence (SEQ ID NO: 89) (gi|2605905|gb|CA799819.1|CA799819) 
XNLENLPKRLKSSIEVPKTSRNAKSRKNSKSASTSNDEDDRSLSLQVQRNNSCFSQSDSNAYLEPNGGASKDPAP
PNLDRKSRATTSAAADPQSLYARKRRERINERLRILQNLVPNGTKVDISTMLEEAVQYVKFLQLQIKLLSSEDLW
MYAPIVYNGINIGLDLGISPTKGRSM*
 Soybean GmRSLb amino acid sequence (SEQ ID NO: 90) (gi|15663066|gb|BI700437.1|1700437) 
GAAACCTGGAGAACCTACCAAAAAGACTAAAGAGCTCAATAGAGGTCCCAAAAACATCGAGGAATGCTAAATCAA
GGAAAAATTCAAAATCTGCTTCAACTAGCAACGATGAAGATGATAGAAGCTTGAGCCTCCAAGTCCAAAGGAATA
ATAGCTGTTTTTCACAGAGTGACTCTAATGCTTATCTTGAGCCAAATGGAGGGGCATCAAAAGATCCTGCACCTC
CTAATTTGGATAGAAAATCAAGAGCAACTACCAGTGCCGCCGCTGATCCACAGAGCCTCTATGCAAGAAAGAGAA
GAGAAAGAATAAATGAAAGGCTGAGAATACTGCAAAATCTTGTCCCCAACGGAACTAAGGTGGATATCAGCACCA
TGCTTGAAGAAGCTGTCCAATACGTTAAGTTTTTACAGCTCCAAATTAAGCTTCTGAGCTCTGAAGATTTGTGGA
TGTATGCTCCAATTGTTTACAATGGAATAAACATTGGACTAGACCTCGGTATTTCTCCAACCAAAGGAAGATCAA
TGTGATAGCATAGCAATTAAAGAGGATATAATATTTCATTAACTTA
 Soybean GmRSLb nucleotide sequence (SEQ ID NO: 91). (gi|15663066|gb|BI700437.1|BI700437) 
XRSKEAEILSSNGKRKASRGSATDPQSVYARKRRERINERLRILQNLVPNGTKVDISTMLEEAVEYVKFLQLQIK
LLSSDDMWMYAPIAYDGMDIGLHSTTIPSSSTR*
 Lettuce saligna LsRSLa amino acid sequence (SEQ ID NO: 92) (gi|83790803|gb|DWO51020.1|DW051020 CLLX3812.b1_Hl8.ab1) 
TGAGATCAAAAGAGGCTGAAATTCTGAGCTCAAATGGCAAGAGAAAAGCAAGTAGGGGGTCAGCAACTGATCCAC
AAAGTGTCTATGCACGGAAAAGAAGAGAAAGAATTAACGAACGTTTAAGAATATTACAAAATCTTGTTCCTAATG
GTACAAAGGTTGATATAAGCACAATGCTTGAAGAGGCTGTTGAGTACGTGAAGTTTTTGCAGCTTCAAATCAAGC
TCTTGAGCTCCGATGATATGTGGATGTATGCTCCGATTGCATACGATGGAATGGACATTGGGCTTCATTCAACAA
 
CCATCCCATCATCGTCAACAAGATAATGCAAAGTTGGGCTATCCATATTGTCACATTTTTGTTGAATAAAAGGCA
ATCGATAACAAAATTCAAAGTTTATAAAGAGTACACATTTATGC
 Lettuce saligna LsRSLa nucleotide sequence (SEQ ID NO:93) (gi|83790803|gb|DW051020.1|DW051020 CLLX3812.b1_Hl8.ab1) 
MASKRATTRELRAMYDDEPSSMSLELFGYHGVVVDGDDENDDTATALPQLSFVDNFKGGCGSAADYYSWAYNASG
GTPGASSSSTSSVLSFEHAGGAGHQLAYNSGTGDDDCALWMDSMADHQHGAARFGFMNPGSADVVPEIQESSIKQ
PAKSAQKRSSSGGEAQAAAKKQCGGGRKSKAKVVPTKDPQSAVAKVRRERISERLKVLQDLVPNGTKVDMVTMLE
KAITYVKFLQLQVKVLATDEFWPVQGGKAPELSQVKTALDAILSSQQQP*
 Triticum aestivum TaRSLa amino acid sequence (SEQ ID NO: 94) (gi|25232820|gb|CA654295.1|CA654295) 
ATGGCGAGCAAGCGGGCCACCACGCGGGAGCTCCGGGCGATGTACGACGAGCCCTCCTCCATGTCCCTCGAG
CTCTTCGGCTACCATGGCGTGGTCGTCGACGGTGACGATGAAAACGACGACACTGCCACCGCCCTGCCCCAGCTC
TCCTTCGTCGACAACTTCAAAGGTGGGTGCGGGTCGGCGGCGGACTACTACAGCTGGGCGTACAACGCCTCCGGC
GGGACGCCGGGCGCCTCCTCCAGCTCCACCTCGTCGGTGCTCAGCTTTGAGCATGCCGGCGGTGCCGGTCATCAG
CTGGCTTATAATTCCGGCACAGGCGACGATGACTGCGCGCTCTGGATGGACAGCATGGCCGATCATCAGCACGGC
GCGGCCAGGTTTGGGTTCATGAACCCAGGGTCGGCCGATGTCGTCCCAGAAATCCAGGAGAGCAGCATCAAGCAG
CCGGCCAAGTCTGCGCAGAAGCGCTCGAGCTCGGGTGGTGAGGCGCAAGCAGCGGCGAAGAAGCAGTGTGGAGGA
GGCAGGAAGAGCAAGGCCAAAGTTGTCCCTACCAAGGATCCTCAGAGCGCTGTTGCAAAGGTCCGAAGAGAGCGC
ATCAGTGAGAGGCTCAAAGTTCTGCAGGATCTTGTACCCAACGGCACGAAGGTGGACATGGTCACCATGCTCGAG
AAGGCAATCACCTATGTCAAGTTCCTGCAGCTGCAAGTCAAGGTGTTGGCGACCGACGAGTTCTGGCCGGTGCAA
GGAGGGAAGGCGCCGGAGCTCTCCCAAGTGAAGACCGCGCTGGACGCCATCCTTTCTTCCCAGCAGCAACCCTAG
 Triticum aestivum TaRSLa nucleotide sequence (SEQ ID NO:95) (gi|25232820|gb|CA654295.1|CA654295) 
DSQIIHPMPCDELHKSLI*LYHIRRRYPYWVFTDGESTSFARPLLNDSRIRGELLLTLSTTKHCKVTASSMRRSY
SMMHDHEKS*KIQRRKSQKLVSKGNESEADHDAVFGQIMKMCGSDNDSNWPRESSTSPRPKEAANLNSNGKTKAN
RGSATDPQSVVARKRRERINERLRILQSLVPNGTKVDISTMLEDAVQYVKFLQLQIKPLSSDDLWMYAPIAYNGM
ETGLDSTIPSPR*RLSKVAASFFLKKGKPGA
 Safflower Carthamus tinctorius CtRSLa amino acid sequence (SEQ ID NO: 96) (gi|25399878|gb|EL411863.1|EL411863 CFFS9477.b1_I18.ab1) 
GATTCACAGATAATCCACCCTATGCCGTGTGATGAACTCCACAAATCCTTAATTTAATTGTACCACATCAGGCGA
CGTTATCCATATTGGGTGTTCACTGATGGTGAAAGCACATCTTTCGCGCGACCTCTACTCAATGACTCAAGAATT
AGAGGTGAACTATTGCTTACACTATCTACTACTAAACATTGTAAAGTGACTGCCAGTTCTATGAGACGTTCGTAT
AGCATGATGCATGATCATGAGAAAAGCTAAAAGATACAGCGCAGAAAGAGCCAGAAGCTCGTTTCTAAAGGCAAC
GAAAGTGAAGCTGACCATGATGCAGTTTTTGGGCAAATAATGAAAATGTGTGGATCTGACAATGACTCGAATTGG
CCTCGGGAGTCGAGCACAAGTCCAAGACCAAAAGAGGCTGCAAATCTGAACTCAAATGGAAGACAAAAGCAAAT
AGGGGGTCAGCAACGGATCCACAAAGTGTCTACGCACGGAAGAGAAGAGAACGAATTAATGAACGGTTAAGAATA
CTACAGAGTCTGGTTCCTAATGGTACAAAGGTTGATATAAGCACAATGCTTGAAGATGCTGTCCAGTATGTGAAA
TTTTTGCAGCTCCAAATCAAGCCGTTGAGCTCTGATGATCTGTGGATGTATGCCCCCATCGCGTACAACGGGATG
GAGACGGGGCTTGATTCTACGATCCCCTCGCCAAGGTGAAGACTATCCAAAGTTGCCGCATCTTTTTTCTTGAAA
AAAGGGAAGCCTGGGGCAA
 Safflower Carthamus tinctorius CtRSLa nucleotide sequence (SEQ ID NO: 97) (gi|125399878|gb|EL411863.1|EL411863 CFFS9477.b1_I18.ab1) 
MALVREPMVLYDGGFDASEASAFDSIGCFGHGHGHDALLGGVDAAALFGGYAHDEPAGASASAYVKDGSHWAGVG
ASVLAFDRAARGHGAQAMATAAAQEEEECDAWIDAMDEDNGEAAPAPSIGFDPATGCFSLTQRPGAGARRPFGLL
FPSASGGAPSPDSAAPAPASRGSQKRPSAGIARAQDAEPRASKKQCGASRKTTAKAKSPAPAITSPKDPQSLAAK
NRREKISERLRTLQEMVPNGTKVDMVTMLEKAISYVKFLQLQVKVLATDEFWPAQGGMAPEISQVKEALDAILSS
QRGQFNCSS*
 BdRSLa amino acid sequence (SEQ ID NO: 98) 
ATGGCATTAGTGCGGGAGCCGATGGTACTGTATGACGGCGGTTTCGACGCCTCGGAGGCGTCGGCATTCGACTCC
ATCGGCTGCTTCGGCCACGGCCACGGCCACGACGCTCCTAGGCGGCGTCGACGCGGCCGCGCTGTTCGGGGGC
TACGCGCACGACGAGCCGGCCGGCGCCAGCGCCAGCGCCTACGTGAAGGACGGCTCGCACTGGGCCGGCGTGGGT
GCGTCCGTGCTCGCGTTCGACCGTGCCGCTCGGGGCCACGGCGCGCAGGCCATGGCGACCGCGGCCGCTCAGGAG
GAGGAAGAATGCGACGCGTGGATCGACGCCATGGACGAGGACAATGGCGAGGCGGCGCCGGCGCCGTCCATCGGC
TTCGACCCGGCCACGGGCTGCTTCAGCCTCACGCAGCGGCCCGGCGCCGGCGCGGCGCCCGTTCGGGCTCCTG
TTCCCGAGCGCGTCCGGTGGCGCGCCCTCGCCCGACAGCGCCGCGCCAGCGCCGGCATCCCGCGGTTCCCAGAAG
CGGCCATCCGCCGGGATTGCGCGCGCAGGACGCGGAGCCGCGGGCCAGCAAGAAGCAGTGCGGCGCGAGCAGG
AAGACGACGGCCAAGGCGAAGTCGCCTGCGCCTGCCATCACCTCGCCCAAGGACCCGCAGAGCCTCGCTGCAAAG
AACCGGAGGGAGAAGATCAGCGAGCGGCTCCGGACGTTGCAGGAGATGGTGCCCAACGGCACCAAGGTGGACATG
GTCACCATGCTCGAGAAGGCCATCAGCTACGTCAAGTTCCTGCAGCTGCAAGTCAAGGTGCTCGCGACGGACGAG
TTCTGGCCGGCGCAGGGAGGGATGGCGCCGGAGATCTCCCAGGTGAAGGAGGCGCTCGACGCCATCCTGTCGTCG
CAGAGGGGGCAATTCAACTGCTCCAGCTAG
 BdRSLa nucleotide sequence (SEQ ID NO: 99) 
MASRHATTREPHLRTMYDDEPSMSLELFGYHGVVVDGDDDGDTATDLPQLTFVDNFKGGCGSADYYGWAYSASGG
ASGACSSSSSSVLSFEQAGGAGHQLAYNAGTGDDDCALWMDGMADQHDTAKFGFMDPGMSDVSLEIQESSMKPPA
KMAQKRACQGGETQAAAKKQCGGSKKSKAKAAPAKDPQSAVAKVRRERISERLKVLQDLVPNGTKVDMVTMLEKA
ITYVKFLQLQVKVLATDDFWPVQGGKAPELSQVKDALDAILSSQNQS*
 BdRSLb amino acid sequence (SEQ ID NO: 100) 
ATGGCAAGCAGGCACGCCACTACACGGGAGCCACACCTCCGGACCATGTACGACGACGAGCCATCCATGTCCCTC
GAGCTCTTCGGCTACCATGGCGTCGTCGTCGACGGTGACGACGATGGCGACACCGCCACCGACCTTCCCCAGCTC
ACCTTTGTTGACAACTTCAAAGGCGGGTGTGGTCAGCCGACTACTACGGCTGGGCGTACAGCGCCTCCGGTGGT
GCGTCAGGCGCCTGCTCCAGCTCCAGCTCGTCGGTGCTCAGCTTTGAGCAGGCGGGTGGTGCCGGTCATCAGCTG
GCTTATAACGCCGGCACAGGTGACGATGACTGCGCGCTCTGGATGGACGGCATGGCTGACCAGCATGACACAGCC
AAGTTTGGGTTCATGGACCCAGGCATGTCTGATGTCAGCCTAGAAATCCAGGAGAGCAGCATGAAACCGCCGGCC
AAGATGGCACAGAAGCGCGCTTGCCAGGGTGGTGAGACGCAAGCAGCGGCGAAGAAGCAGTGTGGAGGAAGCAAG
AAGAGCAAGGCAAAAGCTGCCCCTGCCAAGGATCCTCAAAGCGCCGTTGCAAAGGTCCGAAGAGAGCGCATCAGC
GAGAGGCTCAAAGTTCTGCAGGATCTCGTGCCCAATGGCACAAAGGTTGACATGGTCACCATGCTCGAAAAGGCA
ATCACCTATGTCAAGTTCCTGCAGCTGCAAGTCAAGGTATTGGCGACTGATGACTTCTGGCCGGTGCAAGGAGGG
AAAGCTCCGGAGCTCTCCCAAGTGAAGGACGCTCTGGACGCGATCCTGTCTTCCCAGAATCAATCCTAG
 BdRSLb nucleotide sequence (SEQ ID NO: 101) 
MALVGQATKLCYDGFAGDGVPPFMDAACLAFDHGYDYNNPHAWEFPTGAEPGNSSAFDVAWTGVSSTSPVLTFDA
AEWMDATATDRLSSYSPSAATVPASYKRPRAHVQPQQEAEEQESITPNPKKQCGDGKVVIKSSAAATGTSPRKEP
QSQAAKSRRERIGERLRALQELVPNGSKVDMVTMLDKAITYVKFMQLQLTVLETDAFWPAQGGAAPEISQVKAAL
DAIILSSSQKPRQWS*
 BdRSLc amino acid sequence (SEQ ID NO: 102) 
ATGGCTCTAGTGGGTCAGGCAACGAAGCTCTGCTACGACGGCTTCGCCGGAGACGGTGTGCCGCCGTTCATGGAC
GCAGCTTGTCTGGCATTCGACCACGGGTATGATTACAACAATCCCCACGCATGGAATTCCCCACCGGCGCCGAG
CCAGGCAACAGCAGCGCGTTCGACGTTGCCTGGACCGGCGTCTCCTCCACTTCTCCGGTGCTCACATTCGACGCC
GCCGAGTGGATGGACGCCACGGCCACGGACCGGCTGAGCTCCTACAGCCCGTCTGCGGCCACCGTGCCGGCCTCT
TACAAGCGGCCTCGTGCGCACGTGCAGCCACAGCAGGAAGCAGAAGAACAGGAAAGCATTACTCCCAATCCCAAG
AAGCAGTGCGGCGATGGGAAAGTAGTTATCAAGTCATCGGCGGCTACCGGCACCAGTCCACGCAAGGAACCC
CAAAGCCAAGCTGCCAAGAGCCGTCGTGAGCGGATCGGCGAGCGGCTGAGAGCGCTGCAGGAGCTGGTGCCCAAC
GGCAGCAAGGTGGACATGGTCACCATGCTCGACAAGGCCATCACTTATGTCAAGTTCATGCAGCTCCAGCTCACG
GTGCTCGAGACAGACGCGTTCTGGCCTGCGCAGGGTGGCGCGGCGCCGGAGATCTCCCAGGTGAAGGCGGCGCTC
GACGCCATCATCCTCTCCTCGTCGCAGAAGCCTCGTCAGTGGAGCTAG
 BdRSLc nucleotide sequence (SEQ ID NO: 103) 
MEAGGLISEAGWTMFDFPSQGEESEIMSQLLGAFPSHLEEGHQDLPWYQASDPSYYDCNLNTSSESNASSLAVPS
ECMGYYLGDSSESLDLSSCIAPNDLNLVQEQDATEFLNMTPNLSLDLRGNGESSCEDLTSVGPTNKRKHSSAEEG
IDCQARGQKFARKAEPKRTKKTKQSGWEVAVATRNGSTASCCTSDDDSNASQESADTGVCPKGKARAARGASTDP
QSLYARKRRERINERLKTLQTLVPNGTKVDMSTMLEEAVHYVKFLQLQIKVLSSDDMWMYAPLAYNGMNIGLDLN
IYTPERWRTASAAPSTEGREYAGVDRISDLPDGILGDIVSLLPTAEGARTQILKRRWRHIWRCSAPLNLDCCTLV
ARGGGREAEDELVGLIPSILSSHQGTGRRFHVPSSRHSDRAATIEAWLQSAALDNLQELDLWCTHTYLYDYVPLP
PAVFRFSATVRVVTIANCNLRDSAVQGLQFPQLKQLGFKDIIIMEDSLHHMIAACPDLECLMIERSLGFACVRIN
SLSLRSIGVSTDHPHPHELQFVELVIDNAPCLKRLLHLEMCYHLDMHITVISAPKLETLSCCSSVSRSSTKLSFG
SAAIQGLHIDSLTTVVRTVQILAVEMHSLCLDTIIDFMKCFPCLQKLYIKSFVSGNNWWQRKHRNVIKSLDIRLK
TIALESYGGNQSDINFVTFFVLNARVLELMTFDVCSEHYTVEFLAEQYRKLQLDKRASRAARFHFTSNRCVRGIP
YIGRAELFLPIKCSHVDTSPNLSSFRLSAVFSVCITRNLLRLKKAMWVISLYYSPEFTKQVAVHNPNEMPF*
 BdRSLd amino acid sequence (SEQ ID NO: 104) 
ATGGAGGCTGGAGGGCTGATTTCTGAGGCTGGCTGGACCATGTTTGACTTCCCGTCGCAAGGCGAGGAATCAGAG
ATCATGTCGCAGCTGCTAGGCGCCTTCCCCTCCCATCTTGAGGAAGGCCATCAGGATCTGCCTTGGTACCAGGCT
TCTGACCCATCCTACTATGACTGTAATCTTAATACAAGTAGTGAAAGCAATGCTAGTAGTCTTGCTGTTCCATCC
GAGTGTATGGGCTACTATTTGGGTGATTCAAGTGAGTCCCTGGACCTGAGCTCCTGCATTGCACCAAATGACCTG
AACTTGGTCCAGGAGCAAGATGCAACTGAGTTTCTGAATATGACACCAAATCTTTCCCTTGATTTACGTGGAAT
GGTGAGTCGAGCTGCGAGGATCTCACTTCGGTCGGTCCTACTAACAAGCGAAAGCACTCCTCGGCAGAAGAAGGA
ATCGACTGCCAAGCAAGAGGCCAGAAATTCGCCAGAAAGGCTGAACCGAAGCGAACAAAGAAGACCAAGCAAAGC
GGATGGAGGTTGCTGTTGCCACCAGGAATGGAAGCACAGCGAGCTGCTGCACCTCTGATGATGACTCAAACGCT
TCTCAAGAATCTGCAGATACCGGTGTTTGTCCGAAAGGCAAGGCTCGGGCTGCCCGTGGCGCATCAACTGATCCC
CAGAGCCTCTATGCAAGGAAAAGGAGGGAAAGGATCAATGAGAGACTGAAGACACTGCAGACCCTTGTGCCCAAT
GGAACCAAAGTAGATATGAGCACCATGCTTGAGGAGGCAGTCCACTACGTGAAGTTCCTGCAGCTTCAGATCAAG
GTCTTGAGCTCTGATGATATGTGGATGTATGCGCCGCTAGCATACAACGGGATGAACATTGGGCTTGATCTGAAC
ATATATACTCCGGAGAGGTGGAGGACAGCGTCCGCGGCGCCCTCAACCGAAGGGCGTGAATACGCCGGCGTCGAC
CGCATCAGCGACCTCCCCGACGGCATCCTCGGCGACATCGTCTCGTTGCTCCCCACCGCCGAAGGAGCCCGCACC
CAGATCCTCAAGCGCAGGTGGCGCCACATCTGGCGCTGCTCCGCCCCTCTCAACCTCGATTGCTGTACCTTGGTC
GCCCGTGGCGGCGGCCGTGAGGCTGAAGATGAACTCGTCGGTCTCATACCGTCCATCCTTTCTTCTCACCAAGGC
ACCGGCCGCCGCTTCCACGTCCCCTCGTCGCGCCACTCTGACCGAGCTGCTACCATTGAAGCCTGGCTCCAATCT
GCTGCCCTCGACAATCTCCAGGAGCTCGATTTATGGTGCACCCACACCTATCTTTACGACTATGTTCCGCTGCCA
CCCGCCGTCTTTCGCTTCTCCGCCACCGTCCGTGTTGTCACCATCGCAAATTGTAACCTCCGTGACAGCGCCGTC
CAAGGCCTTCAATTCCCACAACTTAAACAGCTCGGATTCAAAGATATCATCATCATGGAGGATTCGCTGCACCAC
ATGATTGCTGCGTGTCCAGATCTCGAGTGCTTGATGATTGAAAGGAGCTTAGGTTTTGCTTGCGTCCGGATCAAT
TCCCTTAGTCTTAGAAGCATCGGTGTGAGCACTGACCACCCTCACCCACATGAGCTCCAGTTTGTGGAACTCGTC
ATTGATAATGCACCTTGTCTTAAGAGATTGCTCCATCTTGAAATGTGTTATCACCTTGACATGCATATAACAGTA
ATCTCCGCGCCTAAACTGGAGACCTTGAGCTGCTGTTCTTCTGTGAGTCGCTCCTCCACCAAACTCTCGTTTGGC
TCCGCGGCCATTCAGGGATTGCACATTGATAGCCTAACAACAGTGGTGCGCACTGTCCAAATTTTAGCTGTAGAG
ATGCATTCTCTTTGTCTAGACACAATTATTGACTTCATGAAATGCTTTCCATGTCTGCAGAAGTTGTACATTAAG
TCATTTGTAAGTGGAAACAATTGGTGGCAACGTAAACACCGGAACGTTATCAAATCCCTTGACATCCGTCTCAAG
ACAATAGCGTTGGAAAGTTATGGGGGCAATCAGTCTGACATCAACTTTGTCACATTCTTTGTCTTGAACGCGAGA
GTGCTAGAGTTGATGACATTTGACGTTTGTTCTGAGCATTACACTGTGGAGTTCTTGGCAGAGCAATATAGGAAG
CTTCAGCTAGATAAGAGGGCTTCAAGAGCCGCTCGGTTCCATTTTACAAGTAACCGATGTGTCCGTGGTATTCCG
TATATCGGACGTGCCGAGCTATTCTTGCCTATCAAATGTTCTCATGTTGACACCAGTCCAAACTTGAGTAGTTTC
CGTTTGTCTGCAGTATTTTCAGTTTGTATTACCCGGAACCTTTTGCGTTTAAAAAAAGCTATGTGGGTCATTAGT
TTGTATTATTCTCCAGAATTTACAAAACAAGTGGCCGTGCACAATCCCAATGAAATGCCGTTTTAG
 BdRSLd nucleotide sequence (SEQ ID NO: 105) 
MEAKCGAIWSSIDARSEDSEMIAHLQSMFWSNSDVALNLCSSNTSGNSCVTASTLPSSLFLPLVDNESYGAAPSV
DTGMDSCFDHQHQSITGHKRISHMDEQMKKTRKKSRTVPSVSKALGSSLVDNQMNADIFNQSSSCCSSGEDSIGT
SEKSIVANQSDNTSGCKRPSKNMQSLYAKKRRERINEKLRVLQQLIPNGTKVDISTMLEEAVQYVKFLQLQIKVL
SSDETWMYAPLAYNGMDIGLTLALRTAANQE*
 BdRSLe amino acid sequence (SEQ ID NO: 106) 
ATGGAGGCCAAGTGTGGAGCTATTTGGAGCTCTATCGATGCGAGGAGCGAGGACTCTGAGATGATTGCTCACCTG
CAGTCCATGTTCTGGAGCAACAGTGATGTTGCTCTCAACCTCTGTTCGTCAAACACCAGTGGCAATTCTTGTGTC
ACAGCTAGCACATTGCCTAGCAGCTTGTTCCTTCCTCTTGTCGATAATGAGAGCTATGGTGCAGCGCCATCGGTG
GACACCGGCATGGATTCATGCTTTGATCACCAGCATCAGAGCATTACTGGTCACAAGAGGATATCGCACATGGAT
GAGCAGATGAAGA[?][?]ACGAGAAAGAAGTCCCGGACTGTTCCATCGGTATCAAAGGCTCTGGGTTCCAGCCTAGTC
GATAATCAGATGAATGCTGACATTTTCAATCAGAGCTCCTCCTGCTGCAGCTCGGGAGAAGATTCAATTGGAACA
TCTGAGAAATCCATTGTTGCAAACCAGAGTGACAATACGAGTGGTTGTAAGCGGCCTTCAAAGAATATGCAAAGC
CTTTATGCAAAGAAGAGAAGAGAGAGGATCAACGAGAAGTTGAGAGTACTGCAGCAGCTGATTCCCAATGGCACC
AAAGTTGACATCAGCACAATGTTGGAGGAAGCAGTTCAGTATGTCAAGTTTCTGCAGCTGCAAATAAAGGTCTTA
AGCTCTGACGAGACATGGATGTATGCGCCCCTCGCCTACAATGGTATGGACATCGGTCTCACTCTCGCTCTGAGA
ACTGCTGCAAACCAAGAGTGA
 BdRSLe nucleotide sequence (SEQ ID NO: 107) 
MALVREHGGYYGGFDSVEAAAFDTLGYGHGASLGFDASSALFGEGGYAAGGGDAWAGAGASTVLAFNRTTAAAAV
GVEEEEEECDAWIDAMDEDDQSSGPAAAAPEARHALTASVGFDASTGCFTLTERASSSSGGAGRPFGLLFPSTSS
SGGTPERTAPVRVPQKRTYQAVSPNKKHCGAGRKASKAKLASTAPTKDPQSLAAKQNRRERISERLRALQELVPN
GTKVDLVTMLEKAISYVKFLQLQVKVLATDEFWPAQGGKAPEISQVREALDAILSSAS
 Zea mays ZmRSLa amino acid sequence (AZM4_60871: SEQ ID NO: 108) 
ATGGCGTTGGTGAGGGAGCACGGTGGGTACTACGGAGGCTTCGACAGCGTCGAGGCGGCGGCCTTCGACACGCTC
GGCTACGGCCACGGCGCGTCGCTGGGCTTTGACGCGTCGTCGGCGCTGTTCGGGAAGGCGGTTATGCGGCGGGC
GGCGGGGACGCCTGGGCGGGCGCGGGGGCGTCGACCGTCCTGGCGTTCAACCGCACAACGGCAGCGGCGGCCGTG
GGTGTGGAAGAGGAGGAGGAGGAGTGCGACGCGTGGATCGACGCTATGGACGAGGACGACCAGAGCTCCGGCCCC
GCCGCGGCGGCGCCAGAGGCGCGCCACGCGCTGACGGCCTCCGTGGGTTTCGACGCCTCCACGGGGTGCTTCACC
CTGACGGAGAGGGCGTCGTCGTCGTCAGGCGGAGCGGGGCGCCCGTTCGGCCTGCTGTTCCCGAGCACGTCGTCG
TCGGGCGGCACGCCCGAGCGCACGGCGCCGGTGCGCGTCCCGCAGAAACGGACCTACCAGGCTGTGAGCCCCAAC
AAGAAGCACTGCGGCGCGGGCAGGAAGGCGAGCAAGGCCAAGCTCGCGTCCACAGCCCCAACCAAAGATCCCCAG
AGCCTCGCGGCCAAGCAGAACCGGCGCGAGCGGATCAGCGAGCGGCTGCGGGCGCTGCAGGAGCTGGTGCCCAAC
GGCACCAAGGTCGACCTGGTCACCATGCTCGAGAAGGCCATCAGCTACGTTAAGTTCCTCCAGTTGCAAGTCAAG
GTTCTGGCAACAGACGAATTCTGGCCGGCACAGGGAGGAAGGCGCCGGAGATCTCCCAGGTGAGGGAGGCGCTC
GACGCCATCTTGTCGTCGGCGTCG
 Zea mays ZmRSLa nucleotide sequence (AZM4_60871: SEQ ID NO: 109) 
MAQFLGAADDHCFTYEYEHVDESMEAIAALFLPTLDTDSANFSSSCFNYAVPPQCWPQPDHSSSVTSLLDPAENF
EFPVRDPLPPSGFDPHCAVAYLTEDSSPLHGKRSSVIEEEAANAAPAAKKRKAGAAMQGSKKSRKASKKDNIGDA
DDDGGYACVDTQSSSSCTSEDGNFEGNTNSSSKKTCARASRGAATEPQSLYARKRRERINERLRILQNLVPNGTK
VDISTMLEEAAQYVKFLQLQIKLLSCDDTWMYAPIAYNGINIGNVDLNIYSLQK*
 Zea mays ZmRSLb amino acid sequence (AZM4_70092: SEQ ID NO: 110) 
ATGGCTCAGTTTCTTGGGGCGGCTGATGATCACTGCTTCACCTACGAGTATGAGCATGTGGATGAGTCCATGGAA
GCAATAGCAGCCCTGTTCTTGCCTACCCTTGACACCGACTCCGCCAACTTCTCCTCTAGCTGTTTCAACTATGCT
GTCCCTCCACAGTGCTGGCCTCAGCCAGACCATAGCTCTAGCGTTACCAGTTTGCTTGATCCAGCCGAGAACTTT
GAGTTTCCAGTCAGGGACCCGCTCCCCCCAAGCGGCTTCGATCCACATTGCGCTGTCGCCTACCTCACTGAGGAT
TCGAGCCCTCTGCATGGCAAACGTTCATCAGTCATTGAGGAAGAAGCAGCCAACGCCGCACCTGCTGCTAAGAAG
AGGAAGGCTGGTGCTGCAATGCAGGGATCAAAGAAATCCAGGAAGGCGAGCAAAAAGGATAACATCGGCGACGCC
GACGATGATGGCGGCTATGCCTGTGTTGACACGCAAAGCTCCAGTAGCTGCACCTCCGAGGACGGGAACTTCGAA
GGAAATACGAATTCAAGCTCCAAGAAGACCTGCGCCAGGGCCAGCCGCGGAGCAGCAACTGAACCTCAGAGTCTC
TATGCAAGGAAGAGGAGAGAGAGGATCAACGAAAGGTTGAGAATCTTGCAGAACTTGGTTCCAAATGGAACAAAA
GTAGACATTAGCACGATGCTCGAGGAAGCGGCGCAGTATGTCAAGTTTTTACAGCTCCAGATTAAGCTGTTGAGC
 
TGTGACGACACATGGATGTATGCGCCAATCGCGTACAATGGAATTAACATCGGCAATGTTGATCTGAACATCTAC
TCTCTGCAAAAGTAA
 Zea mays ZmRSLb nucleotide sequence (AZM4_70092: SEQ ID NO: 111) 
MEDGGLXSEAGAWAELGTGGDESEELVAQLLGAFFRSHGEEGRHQLLWSDDQASSDDVHGDGSLAVPLAYDGCCG
YLSYSGSNSDELPLGSSSRAAPAGGPPEELLGAAETEYLNNVAAADHPFFKWCGNGEGLDGPTSVVGTLGLGSGR
KRARKKSGDEDEDPSTAIASGSGPTSCCTTSDSDSNASPLESADAGARRPKGNENARAAGRGAAAATTTTAEPQS
IYARVRRERINERLKVLQSLVPNGTKVDMSTMLEEAVHYVKFLQLQIRVLQLLSSDDTWMYAPIAYNGMGIGIDL
RMHGQDR*
 Zea mays ZmRSLc amino acid sequence (AZM4_91750: SEQ ID NO: 112) 
ATGGAGGACGGAGGGTTGRTCAGCGAGGCCGGCGCCTGGGCCGAGCTCGGCACCGGCGGCGACGAGTCGGAGGAG
CTGGTGGCGCAGCTGCTGGGCGCCTTCTTCCGGTCCCACGGCGAGGAAGGCCGGCACCAGCTGCTTTGGTCTGAC
GACCAAGCTTCTTCCGACGACGTGCACGGCGACGGCAGCCTTGCCGTGCCGCTCGCATACGACGGCTGCTGCGGC
TATCTGAGCTACTCAGGTAGCAACTCGGACGAGCTCCCCCTCGGGAGCAGCTCCCGCGCTGCGCCAGCAGGTGGC
CCACCGGAGGAGCTGCTCGGTGCAGCTGAGACTGAGTACCTGAATAATGTGGCCGCCGCAGACCATCCCTTCTTC
AAATGGTGTGGGAATGGTGAGGGTCTGGATGGTCCGACGAGCGTCGTGGGCACGCTTGGGCTTGGCTCGGGCCGG
AAACGCGCGCGCAAGAAGAGCGGGGACGAAGACGAAGACCCGAGCACGGCCATCGCCAGCGGAAGCGGCCCCACG
AGCTGCTGCACTACCTCCGACAGCGACTCAAACGCGTCTCCTCTGGAGTCCGCGGACGCCGGCGCTCGTCGCCCC
AAGGGCAACGAGAATGCCCGGGCAGCTGGCCGCGGCGCGGCGGCGACGACGACGACAGCGGAGCCCCAGAGC
ATCTACGCAAGGGTACGGAGGGAGCGGATCAACGAGAGGCTCAAGGTGCTGCAGAGCCTGGTGCCCAACGGCACC
AAGGTGGACATGAGCACCATGCTCGAGGAGGCCGTCCACTACGTCAAGTTCCTGCAGCTTCAGATCAGGGTGCTG
CAGCTCCTGAGCTCCGACGACACGTGGATGTACGCGCCCATCGCGTACAACGGGATGGGCATCGGGATCGACCTC
CGCATGCATGGACAGGACAGATGA
 Zea mays ZmRSLc nucleotide sequence (AZM4_91750: SEQ ID NO: 113) 
SKKSRKASKKDCIVDDDDVYVDPQSSGSCTSEEGNFEGNTYSSAKKTCTRASRGGATDPQSLYARKRRERINERL
RILQNLVPNGTKVDISTMLEEAAQYVKFLQLQIKLLSSDDMWMYAPIAYNGINISNVDLNIPALQK*
 Zea mays amino acid sequence (AZM4_86104: SEQ ID NO: 114) 
TCAAAGAAATCCAGGAAGGCGAGCAAAAAAGATTGTATTGTCGATGACGACGATGTCTATGTTGACCCGCAAAGC
TCCGGTAGCTGCACCTCCGAGGAGGGAATTTTGAAGGGAATACGTATTCAAGCGCGAAAAAGACCTGCACCAGG
GCCAGCCGCGGAGGAGCAACTGATCCTCAGAGTCTCTATGCAAGGAAGAGGAGAGAGAGGATCAATGAAAGGTTG
AGAATCTTGCAGAACTTGGTCCCCAATGGAACAAAGGTTGACATTAGTACGATGCTCGAGGAAGCAGCACAGTAT
GTCAAATTTTTACAGCTTCAGATTAAGCTGTTGAGCTCTGACGACATGTGGATGTATGCGCCAATCGCGTACAAT
GGGATCAACATCAGCAATGTTGATCTGAACATCCCTGCA
 Zea mays ZmRSLd nucleotide sequence (AZM4_86104: SEQ ID NO: 115)

The invention is further described in the following clauses:
1. An isolated *ROOT HAIR DETECTIVE 6 (RHD6)*-related gene.
2. An isolated gene according to clause 1 selected from the group consisting of a *ROOT HAIR DEFECTIVE 6 (RHD6)* gene, a *ROOT HAIR DEFECTIVE 6-LIKE 1 (RSL1)* gene, and functional homologues thereof.
3. An isolated gene encoding an amino acid sequence encoded by the *RHD6* gene, the *RSL1* gene or a gene product that is sufficiently homologous thereto to permit, on production thereof in an *rhd6* mutant cell a functional complementation of said mutation.
4. An isolated product of the expression of the gene according to any one of clauses 1 to 3.
5. An expression construct comprising the gene according to any one of clauses 1 to 4.
6. An expression construct according to clause 5 wherein the gene is operably linked to a heterologous regulatory sequence.
7. A plant cell comprising a gene according to any one of clauses 1 to 4 or an expression construct according to clause 5 or clause 6.
8. A plant or its progeny, including a seed, comprising the cell according to clause 7.
9. An isolated nucleic acid which encodes a RHD6-related polypeptide comprising an amino acid sequence having at least 50% sequence identity to an amino acid sequence selected from the group consisting of SEQ ID NOS: 13 to 25.
10. An isolated nucleic acid according to clause 9 which encodes a polypeptide comprising an amino acid sequence selected from the group consisting of SEQ ID NOS: 13 to 25.
11. An isolated nucleic acid which has at least 50% nucleic acid sequence identity with SEQ ID NO: 2, SEQ ID NO: 4, or both.
12. An isolated nucleic acid according to clause 1 i which encodes an amino acid product which functionally complements the *rhd6* mutation.
13. A method of modulating root hair development in a plant comprising;
   increasing the expression of a RHD6-related polypeptide comprising an amino acid sequence having at least 50% sequence identity to an amino acid sequence selected from the group consisting of SEQ ID NOS: 13 to 25 within cells of said plant relative to control plants.
14. A method of increasing the tolerance of a plant to nutrient-deficient conditions comprising;
   increasing the expression of a RHD6-related polypeptide comprising an amino acid sequence having at least 50% sequence identity to an amino acid sequence selected from the group consisting of SEQ ID NOS: 13 to 25 within cells of said plant relative to control plants.
15. A method according to clause 14 wherein the nutrient-deficient conditions are selected from the group consisting of nitrate-deficient, phosphate-deficient or iron-deficient conditions.
16. A method of increasing the production or secretion of a root-secreted phytochemical in a plant comprising;
   increasing the expression of a RHD6-related polypeptide within cells of a plant which secretes the phytochemical through its roots.
17. A method according to any one of clauses 13 to 16 wherein the RHD6-related polypeptide comprising an amino acid sequence having at least 55% sequence identity to SEQ ID NO:1 or SEQ ID NO:3.
18. A method according to any one of clauses 13 to 16 wherein the RHD6-related polypeptide comprising an amino acid sequence having at least 55% sequence identity to any one of SEQ ID NOS: 5, 7, 9, or 11, 26, 28, 30, 32, 34, 36, 38, 40, 42, 44, 46, 48, 50, 52, 54, 56, 58, 60, 62, 64, 66, 68,70, 72, 74, 76, 78, 80, 82, 84, 86, 88, 90, 92, 94, 96, 98, 100, 102, 104, 106, 108, 110, 112 and 114.
19. A method according to any one of clauses 13 to 18 wherein one or more of the length, number and/or longevity of root hairs is increased in the plant.
20. A method according to any one of clauses 13 to 19 wherein expression is increased by expressing a heterologous nucleic acid encoding said RHD6-related polypeptide within cells of said plant.
21. A method according to clause 20 wherein the heterologous nucleic acid comprises a nucleotide sequence which has at least 40% sequence identity with any one of SEQ ID NO: 2 or SEQ ID NO: 4.
22. A method according to clause 20 wherein the heterologous nucleic acid comprises a nucleotide sequence which has at least 40% sequence identity with any one of SEQ ID NOS: 6, 8, 10, 12, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113 and 115.
23. A method according to any one of clauses 20 to 22 wherein the heterologous nucleic acid is operably linked to a promoter.
24. A method according to clause 23 wherein the promoter is a constitutive promoter.
25. A method according to clause 23 wherein the promoter is a root-specific promoter.
26. A method according to clause 23 wherein the promoter is a inducible promoter.
27. A method according to any one of clauses 23 to 26 wherein the heterologous nucleic acid is comprised in a vector.
28. A method according to any one of clauses 13 to 19 wherein expression is increased by a method comprising;
   crossing a first and a second plant to produce a population of progeny plants;
   determining the expression of the RHD6-related polypeptide in the progeny plants in the population, and
   identifying a progeny plant in the population in which expression of the RHD6-related polypeptide is increased relative to controls.
29. A method according to any one of clauses 13 to 19 wherein expression is increased by a method comprising;
   exposing a population of plants to a mutagen,
   determining the expression of the RHD6-related polypeptide in one or more plants in said population, and
   identifying a plant with increased expression of the RHD6-related polypeptide.
30. A method according to any one of clauses 13 to 29 comprising sexually or asexually propagating or growing off-spring or descendants of the plant having increased RHD6-1-elated polypeptide expression.
31. A method of producing a plant with an altered root hair phenotype comprising:
   incorporating a heterologous nucleic acid which encodes a RHD6-related polypeptide comprising an amino acid sequence having at least 50% sequence identity to an amino acid sequence selected from the group consisting of SEQ ID NOS: 13 to 25 into a plant cell by means of transformation, and;
   regenerating the plant from one or more transformed cells.
32. A method according to clause 31 wherein the plant has increased tolerance to nutrient-deficient conditions relative to control plants.
33. A method according to clause 32 wherein the nutrient-deficient conditions are selected from the group consisting of nitrate-deficient, phosphate-deficient or iron-deficient conditions.
34. A method according to clause 31 wherein the plant has increased production or secretion of a root-secreted phytochemical.
35. A method according to any one of clauses 31 to 34 wherein the RHD6-related polypeptide comprising an amino acid sequence having at least 55% sequence identity to SEQ ID NO:1 or SEQ ID NO:3.
36. A method according to any one of clauses 31 to 35 wherein the RED6-related polypeptide comprising an amino acid sequence having at least 55% sequence identity to any one of SEQ ID NOS: 5, 7, 9, or 11, 26, 28, 30, 32, 34, 36, 38, 40, 42, 44, 46, 48, 50, 52, 54, 56, 58, 60, 62, 64, 66, 68,70, 72, 74, 76, 78, 80, 82, 84, 86, 88, 90, 92, 94, 96, 98, 100, 102, 104, 106, 108, 110, 112 and 114.
37. A method according to any one of clauses 31 to 36
   wherein the length, number and/or longevity of root hairs is increased in the plant.
38. A method according to any one of clauses 31 to 37 wherein expression is increased by expressing a heterologous nucleic acid encoding said RHD6-related polypeptide within cells of said plant.
39. A method according to clause 38 wherein the heterologous nucleic acid comprises a nucleotide sequence which has at least 40% sequence identity with any one of SEQ ID NO: 2 or SEQ ID NO: 4.
40. A method according to clause 38 wherein the heterologous nucleic acid comprises a nucleotide sequence which has at least 40% sequence identity with any one of SEQ ID NOS: 6, 8, 10, 12, 27, 29, 31, 33, 35, 37, 39, 41, 43, 45, 47, 49, 51, 53,55, 57, 59, 61, 63, 65, 67, 69, 71, 73, 75, 77, 79, 81, 83, 85, 87, 89, 91, 93, 95, 97, 99, 101, 103, 105, 107, 109, 111, 113 and 115.
41. A method according to any one of clauses 38 to 40 wherein the heterologous nucleic acid is operably linked to a promoter.
42. A methods according to clause 41 wherein the promoter is a consitutive promoter.
43. A method according to clause 41 wherein the promoter is a root-specific promoter.
44. A method according to clause 41 wherein the promoter is a inducible promoter.
45. A method according to any one of clauses 38 to 44 wherein the heterologous nucleic acid is comprised in a vector.
46. A method according to any one of clauses 31 to 37 wherein expression is increased by a method comprising;
   crossing a first and a second plant to produce a population of progeny plants;
   determining the expression of the RHD6-related polypeptide in the progeny plants in the population, and
   identifying a progeny plant in the population in which expression of the RHD6-related polypeptide is increased relative to controls.
47. A method according to any one of clauses 31 to 37 wherein expression is increased by a method comprising;
   exposing a population of plants to a mutagen,
   determining the expression of the RHD6-related polypeptide in one or more plants in said population, and
   identifying a plant with increased expression of the RHD6-related polypeptide.
48. A method according to any one of clauses 31 to 47 comprising sexually or asexually propagating or growing off-spring or descendants of the plant having increased RHD6-related polypeptide expression.
49. A method according to any one of clauses 13 to 48 wherein the plant is a higher plant.
50. A method according to clause 49 wherein the plant is an agricultural plant selected from the group consisting of *Lithospermum erythrorhizon, Taxus* spp, tobacco, cucurbits, carrot, vegetable brassica, melons, capsicums, grape vines, lettuce, strawberry, oilseed brassica, sugar beet, wheat, barley, maize, rice, soyabeans, peas, sorghum, sunflower, tomato, potato, pepper, chrysanthemum, carnation, linseed, hemp and rye.
51. A plant produced by a method according to any one of clauses 31 to 50.
52. A plant according to clause 51 which has an altered root hair phenotype.
53. A plant according to clause 51 or clause 52 which has an increased tolerance to nutrient-deficient conditions.
54. A plant according to clause 51 or clause 52 which has an increased production of root secreted phytochemical.
55. A plant according to clause 51 or clause 52 which has an increased uptake of a soil contaminant.
56. A method of reducing the amount of contaminant in contaminated soil comprising;
   increasing the expression of a RHD6-related polypeptide within cells of a plant which absorbs the contaminant through its roots,
   growing the plant or a descendent thereof in soil which comprises the contaminant such that the plant or descendent absorbs the contaminant from the soil, and
   harvesting said plant or descendent thereof.

## Claims

1. A method of producing a plant with an altered root hair phenotype comprising:
increasing expression of an RHD6-related polypeptide by incorporating a heterologous nucleic acid which encodes a RHD6-related polypeptide comprising an amino acid sequence having greater than 80% sequence identity to an amino acid sequence selected from the group consisting of SEQ ID NOS: 102 (BdRSLc), 98 (BdRSLa), 100 (BdRSLb), 104 (BdRSLd) or 106 (BdRSLe) into a plant cell by means of transformation, expressing said heterologous nucleic acid within cells of said plant and
regenerating the plant from one or more transformed cells wherein the root hair phenotype is altered relative to control plants in which expression of the RHD6-related polypeptide is not increased.

2. A method according to claim 1 wherein the amino acid sequence has greater than 90% or greater than 95% sequence identity to an amino acid sequence selected from the group consisting of SEQ ID NOS: 102, 98, 100, 104 or 106.

3. A method according to claim 1 or 2 wherein the plant has increased tolerance to nutrient-deficient conditions relative to the control plants.

4. A method according to claim 3 wherein the nutrient-deficient conditions are selected from the group consisting of nitrate-deficient, phosphate-deficient or iron-deficient conditions.

5. A method according to claim 1 or 2 wherein the plant has increased production or secretion of a root-secreted phytochemical relative to the control plants.

6. A method according to any one of claims 1 to 5 wherein the length, number and/or longevity of root hairs is increased in the plant.

7. A method according to any one of claims 1 to 6 wherein the heterologous nucleic acid is operably linked to a promoter.

8. A method according to claim 7 wherein the promoter is a constitutive promoter, a root-specific promoter or an inducible promoter.

9. A method according to any one of claims 1 to 8 wherein the heterologous nucleic acid is comprised in a vector.

10. A method for producing a plant with an altered root hair phenotype comprising increasing expression of an RHD6-related polypeptide comprising an amino acid sequence having greater than 80% sequence identity to an amino acid sequence selected from the group consisting of SEQ ID NOS: 102 (BdRSLc), 98 (BdRSLa), 100 (BdRSLb), 104 (BdRSLd) or 106 (BdRSLe) wherein expression is increased by a method comprising;
exposing a population of plants to a mutagen, determining the expression of the RHD6-related polypeptide in one or more plants in said population, and
identifying a plant with increased expression of the RHD6-related polypeptide wherein the root hair phenotype is altered relative to control plants in which expression of the RHD6-related polypeptide is not increased.

11. A method of increasing the tolerance of a plant to nutrient-deficient conditions comprising;
increasing expression of an RHD6-related polypeptide by incorporating a heterologous nucleic acid which encodes a RHD6-related polypeptide comprising an amino acid sequence having greater than 80% sequence identity to an amino acid sequence selected from the group consisting of SEQ ID NOS: 102 (BdRSLc), 98 (BdRSLa), 100 (BdRSLb), 104 (BdRSLd) or 106 (BdRSLe) into a plant cell by means of transformation, expressing said heterologous nucleic acid within cells of said plant and
regenerating the plant from one or more transformed cells wherein the root hair phenotype is altered relative to control plants in which expression of the RHD6-related polypeptide is not increased.

12. A method of increasing the production or secretion of a root-secreted phytochemical in a plant comprising;
increasing expression of an RHD6-related polypeptide by incorporating a heterologous nucleic acid which encodes a RHD6-related polypeptide comprising an amino acid sequence having greater than 80% sequence identity to an amino acid sequence selected from the group consisting of SEQ ID NOS: 102 (BdRSLc), 98 (BdRSLa), 100 (BdRSLb), 104 (BdRSLd) or 106 (BdRSLe) into a plant cell by means of transformation, expressing said heterologous nucleic acid within cells of said plant and
regenerating the plant from one or more transformed cells wherein the root hair phenotype is altered relative to control plants in which expression of the RHD6-related polypeptide is not increased.

13. A plant produced by a method of any of claims 1 to 12.

14. A plant cell comprising an expression construct comprising a nucleic acid which encodes a RHD6-related polypeptide comprising an amino acid sequence selected from the group consisting of SEQ ID NOS: 102 (BdRSLc), 98 (BdRSLa), 100 (BdRSLb), 104 (BdRSLd) or 106 (BdRSLe).

15. A plant or its progeny, including seed, comprising the cell according to claim 14.


SEQUENCE LISTING

<110> Plant Bioscience Limited
Dolan, Liam
Menand, Benoit
Yi, Keke

<120> Composition and Method for Modulating Plant Root Hair Development

<130> PC780301EPA

<140> PCT/GB2008/001246
<141> 2008-04-09

<150> GB 0709835.3
<151> 2007-05-22

<160> 228

<170> PatentIn version 3.3

<210> 1
<211> 298
<212> PRT
<213> Arabidopsis thaliana

<400> 1

Met Ala Leu Val Asn Asp His Pro Asn Glu Thr Asn Tyr Leu Ser Lys
1               5                   10                  15

Gln Asn Ser Ser Ser Ser Glu Asp Leu Ser Ser Pro Gly Leu Asp Gln
            20                  25                  30

Pro Asp Ala Ala Tyr Ala Gly Gly Gly Gly Gly Gly Gly Ser Ala Ser
        35                  40                  45

Ser Ser Ser Thr Met Asn Ser Asp His Gln Gln His Gln Gly Phe Val
    50                  55                  60

Phe Tyr Pro Ser Gly Glu Asp His His Asn Ser Leu Met Asp Phe Asn
65                  70                  75                  80

Gly Ser Ser Phe Leu Asn Phe Asp His His Glu Ser Phe Pro Pro Pro
                85                  90                  95

Ala Ile Ser Cys Gly Gly Ser Ser Gly Gly Gly Gly Phe Ser Phe Leu
            100                 105                 110

Glu Gly Asn Asn Met Ser Tyr Gly Phe Thr Asn Trp Asn His Gln His
        115                 120                 125

His Met Asp Ile Ile Ser Pro Arg Ser Thr Glu Thr Pro Gln Gly Gln

130                135                140

Lys Asp Trp Leu Tyr Ser Asp Ser Thr Val Val Thr Thr Gly Ser Arg
145                150                155                160

Asn Glu Ser Leu Ser Pro Lys Ser Ala Gly Asn Lys Arg Ser His Thr
                165                170                175

Gly Glu Ser Thr Gln Pro Ser Lys Lys Leu Ser Ser Gly Val Thr Gly
            180                185                190

Lys Thr Lys Pro Lys Pro Thr Thr Ser Pro Lys Asp Pro Gln Ser Leu
        195                200                205

Ala Ala Lys Asn Arg Arg Glu Arg Ile Ser Glu Arg Leu Lys Ile Leu
    210                215                220

Gln Glu Leu Val Pro Asn Gly Thr Lys Val Asp Leu Val Thr Met Leu
225                230                235                240

Glu Lys Ala Ile Ser Tyr Val Lys Phe Leu Gln Val Gln Val Lys Val
                245                250                255

Leu Ala Thr Asp Glu Phe Trp Pro Ala Gln Gly Gly Lys Ala Pro Asp
            260                265                270

Ile Ser Gln Val Lys Asp Ala Ile Asp Ala Ile Leu Ser Ser Ser Gln
        275                280                285

Arg Asp Arg Asn Ser Asn Leu Ile Thr Asn
    290                295

<210> 2
<211> 897
<212> DNA
<213> Arabidopsis thaliana

<400> 2
atggcactcg ttaatgacca tcccaacgag accaattact tgtcaaaaca aaattcctcc      60

tcttccgaag atctctcctc gccgggactg gatcagccag atgcagctta tgccggtgga     120

ggaggaggag gaggctcggc ttcgagcagt agcacgatga attcagatca tcaacaacat     180

caggggtttg tattttaccc atccggtgaa gatcatcaca actctttgat ggatttcaac     240

ggatcatcat ttcttaactt tgatcatcac gagagctttc ctcctccagc cataagctgt     300

ggtggtagta gcggtggggg cggcttctcc ttcttggagg gcaacaacat gagctacggc     360

ttcacaaact ggaatcatca acatcatatg gatattatta gccctagatc caccgaaact 420

ccccaaggcc agaaagactg gttatattct gattcaactg ttgtaaccac tggttctaga 480

aacgagtctc tttcgcctaa atccgctgga aacaaacgtt ctcacacggg agagagcact 540

caaccgtcga agaaactgag tagcggtgtg accggaaaga ccaagcctaa gccaacaact 600

tcacctaaag atccacaaag cctagcagcc aagaatcgaa gagaaaggat aagtgaacgt 660

ctcaagatat tgcaagaact tgttcccaat ggcaccaagg ttgatttggt gacaatgctt 720

gaaaaggcta ttagttatgt caagttcctt caagtacaag ttaaggtatt agcgaccgat 780

gagttttggc cggctcaagg aggaaaagct cctgacattt ctcaagttaa agacgccatt 840

gatgccattc tctcctcatc acaacgagac aggaattcga atctgatcac caattaa 897

<210> 3
<211> 307
<212> PRT
<213> Arabidopsis thaliana

<400> 3

Met Ser Leu Ile Asn Glu His Cys Asn Glu Arg Asn Tyr Ile Ser Thr
1               5                   10                  15

Pro Asn Ser Ser Glu Asp Leu Ser Ser Pro Gln Asn Cys Gly Leu Asp
            20                  25                  30

Glu Gly Ala Ser Ala Ser Ser Ser Ser Thr Ile Asn Ser Asp His Gln
        35                  40                  45

Asn Asn Gln Gly Phe Val Phe Tyr Pro Ser Gly Glu Thr Ile Glu Asp
    50                  55                  60

His Asn Ser Leu Met Asp Phe Asn Ala Ser Ser Phe Phe Thr Phe Asp
65                  70                  75                  80

Asn His Arg Ser Leu Ile Ser Pro Val Thr Asn Gly Gly Ala Phe Pro
                85                  90                  95

Val Val Asp Gly Asn Met Ser Tyr Ser Tyr Asp Gly Trp Ser His His
            100                 105                 110

Gln Val Asp Ser Ile Ser Pro Arg Val Ile Lys Thr Pro Asn Ser Phe
        115                 120                 125

Glu Thr Thr Ser Ser Phe Gly Leu Thr Ser Asn Ser Met Ser Lys Pro
    130                 135                 140

Ala Thr Asn His Gly Asn Gly Asp Trp Leu Tyr Ser Gly Ser Thr Ile
145                 150                 155                 160

Val Asn Ile Gly Ser Arg His Glu Ser Thr Ser Pro Lys Leu Ala Gly
                165                 170                 175

Asn Lys Arg Pro Phe Thr Gly Glu Asn Thr Gln Leu Ser Lys Lys Pro
            180                 185                 190

Ser Ser Gly Thr Asn Gly Lys Ile Lys Pro Lys Ala Thr Thr Ser Pro
        195                 200                 205

Lys Asp Pro Gln Ser Leu Ala Ala Lys Asn Arg Arg Glu Arg Ile Ser
    210                 215                 220

Glu Arg Leu Lys Val Leu Gln Glu Leu Val Pro Asn Gly Thr Lys Val
225                 230                 235                 240

Asp Leu Val Thr Met Leu Glu Lys Ala Ile Gly Tyr Val Lys Phe Leu
                245                 250                 255

Gln Val Gln Val Lys Val Leu Ala Ala Asp Glu Phe Trp Pro Ala Gln
            260                 265                 270

Gly Gly Lys Ala Pro Asp Ile Ser Gln Val Lys Glu Ala Ile Asp Ala
        275                 280                 285

Ile Leu Ser Ser Ser Gln Arg Asp Ser Asn Ser Thr Arg Glu Thr Ser
    290                 295                 300

Ile Ala Glu
305

<210>  4
<211>  924
<212>  DNA
<213>  Arabidopsis thaliana

<400>  4
atgtcactca ttaacgaaca ttgcaatgag cgtaattaca tctcaacccc aaattcttca      60

gaagatctct cttcaccaca gaattgcgga ttagacgaag gagcttcagc ttcaagcagt     120

agcaccataa attctgatca tcaaaataat caagggtttg tgttttaccc ttccggggaa     180

accattgaag atcataattc tttgatggat ttcaatgctt catcattctt cacctttgat     240

aatcaccgaa gccttatctc tcccgtgacc aacggtggtg ccttcccggt cgtggacggg     300

aacatgagtt acagctatga tggctggagt catcatcaag tggatagtat tagccctaga 360

gtcatcaaaa ctccaaatag ctttgaaaca acgagcagtt ttggattgac ttcaaactcc 420

atgagtaaac cggccacaaa ccatggaaat ggagactggt tatactctgg ttcaactatt 480

gtaaacatcg gttcaaggca cgagtccacg tcccctaaac tggctggcaa taaacggcct 540

ttcacgggag agaacacaca actttcaaag aagccgagta gcggtacgaa tggaaagatc 600

aagcctaagg caacaacttc acctaaagat ccacaaagcc tagcagccaa gaaccgaaga 660

gaaaggataa gcgaacgcct caaggtattg caagaacttg taccgaatgg taccaaggtg 720

gatttggtaa ctatgcttga gaaagcaatt ggctatgtaa agtttcttca agtacaagtt 780

aaggtacttg cagccgatga gttttggccg gcacaaggag ggaaagctcc ggacatttct 840

caagttaaag aagctattga cgcaatcctc tcatcatcac aacgagatag taactcaact 900

agagaaacaa gtatagcaga ataa 924

<210> 5
<211> 352
<212> PRT
<213> Arabidopsis thaliana

<400> 5

Met Glu Ala Met Gly Glu Trp Ser Asn Asn Leu Gly Gly Met Tyr Thr
1               5                   10                  15

Tyr Ala Thr Glu Glu Ala Asp Phe Met Asn Gln Leu Leu Ala Ser Tyr
            20                  25                  30

Asp His Pro Gly Thr Gly Ser Ser Ser Gly Ala Ala Ala Ser Gly Asp
        35                  40                  45

His Gln Gly Leu Tyr Trp Asn Leu Gly Ser His His Asn His Leu Ser
    50                  55                  60

Leu Val Ser Glu Ala Gly Ser Phe Cys Phe Ser Gln Glu Ser Ser Ser
65                  70                  75                  80

Tyr Ser Ala Gly Asn Ser Gly Tyr Tyr Thr Val Val Pro Pro Thr Val
                85                  90                  95

Glu Glu Asn Gln Asn Glu Thr Met Asp Phe Gly Met Glu Asp Val Thr
            100                 105                 110

Ile Asn Thr Asn Ser Tyr Leu Val Gly Glu Glu Thr Ser Glu Cys Asp
        115                 120                 125

Val Glu Lys Tyr Ser Ser Gly Lys Thr Leu Met Pro Leu Glu Thr Val
    130                 135                 140

Val Glu Asn His Asp Asp Glu Glu Ser Leu Leu Gln Ser Glu Ile Ser
145                 150                 155                 160

Val Thr Thr Thr Lys Ser Leu Thr Gly Ser Lys Lys Arg Ser Arg Ala
                165                 170                 175

Thr Ser Thr Asp Lys Asn Lys Arg Ala Arg Val Asn Lys Arg Ala Gln
            180                 185                 190

Lys Asn Val Glu Met Ser Gly Asp Asn Asn Glu Gly Glu Glu Glu Glu
        195                 200                 205

Gly Glu Thr Lys Leu Lys Lys Arg Lys Asn Gly Ala Met Met Ser Arg
    210                 215                 220

Gln Asn Ser Ser Thr Thr Phe Cys Thr Glu Glu Glu Ser Asn Cys Ala
225                 230                 235                 240

Asp Gln Asp Gly Gly Gly Glu Asp Ser Ser Ser Lys Glu Asp Asp Pro
                245                 250                 255

Ser Lys Ala Leu Asn Leu Asn Gly Lys Thr Arg Ala Ser Arg Gly Ala
            260                 265                 270

Ala Thr Asp Pro Gln Ser Leu Tyr Ala Arg Lys Arg Arg Glu Arg Ile
        275                 280                 285

Asn Glu Arg Leu Arg Ile Leu Gln Asn Leu Val Pro Asn Gly Thr Lys
    290                 295                 300

Val Asp Ile Ser Thr Met Leu Glu Glu Ala Val His Tyr Val Lys Phe
305                 310                 315                 320

Leu Gln Leu Gln Ile Lys Leu Leu Ser Ser Asp Asp Leu Trp Met Tyr
                325                 330                 335

Ala Pro Ile Ala Phe Asn Gly Met Asp Ile Gly Leu Ser Ser Pro Arg
            340                 345                 350

<210> 6
<211> 1059
<212> DNA
<213> Arabidopsis thaliana

<400> 6
atggaagcca tgggagaatg gagcaacaac ctcggaggaa tgtacactta tgcaaccgag    60

gaagccgatt tcatgaacca gcttctcgcc tcttatgatc atcctggcac cggctcatcc    120

tccggcgcag cagccagtgg tgaccaccaa ggcttgtatt ggaaccttgg ttctcatcac    180

aaccacctta gcctcgtgtc tgaagccggt agcttctgtt tctctcaaga gagcagcagc    240

tacagcgctg gaaacagcgg atattacacc gttgttccac ccacggttga agagaaccaa    300

aatgagacaa tggactttgg gatggaagat gtgaccatca atacaaactc ataccttgtt    360

ggtgaggaga caagtgagtg tgacgttgag aaatactctt ctggaaagac tcttatgcct    420

ttggaaaccg tagtggagaa ccacgatgac gaggaaagct tgttgcaatc tgagatctct    480

gtgactacta caaaatctct caccggctcc aaaaagagat cccgtgccac atctactgat    540

aaaaacaaga gagcaagagt gaataagagg gcccagaaga acgtagagat gagtggggat    600

aacaatgaag gagaagagga agaaggagag acgaagttga agaaaagaaa gaatggggca    660

atgatgagta gacagaactc aagcaccact ttctgtacgg aggaagaatc aaactgcgct    720

gatcaagacg gtggaggaga agactcatcc tctaaggaag atgatccctc aaaggccctc    780

aacctcaatg gtaaaacaag agccagtcgt ggtgcagcca ccgatcctca aagcctctat    840

gcaaggaaaa gaagagaaag gattaacgag agactaagga ttttacaaaa tctcgtcccc    900

aatggaacaa aggtcgatat tagtacaatg cttgaggaag cagttcatta cgtcaaattt    960

ttgcagctcc aaattaagtt attgagctct gatgatctat ggatgtatgc gccgattgct    1020

ttcaatggga tggacattgg tctcagctca ccgagatga                    1059

<210>  7
<211>  519
<212>  PRT
<213>  Arabidopsis thaliana

<400>  7

Met Glu Ala Met Gly Glu Trp Ser Thr Gly Leu Gly Gly Ile Tyr Thr
1               5                   10                  15

Glu Glu Ala Asp Phe Met Asn Gln Leu Leu Ala Ser Tyr Glu Gln Pro
            20                  25                  30

Cys Gly Gly Ser Ser Ser Glu Thr Thr Ala Thr Leu Thr Ala Tyr His
        35                  40                  45

His Gln Gly Ser Gln Trp Asn Gly Gly Phe Cys Phe Ser Gln Glu Ser
    50                  55                  60

Ser Ser Tyr Ser Gly Tyr Cys Ala Ala Met Pro Arg Gln Glu Glu Asp
65                  70                  75                  80

Asn Asn Gly Met Glu Asp Ala Thr Ile Asn Thr Asn Leu Tyr Leu Val
                85                  90                  95

Gly Glu Glu Thr Ser Glu Cys Asp Ala Thr Glu Tyr Ser Gly Lys Ser
            100                 105                 110

Leu Leu Pro Leu Glu Thr Val Ala Glu Asn His Asp His Ser Met Leu
        115                 120                 125

Gln Pro Glu Asn Ser Leu Thr Thr Thr Thr Asp Glu Lys Met Phe Asn
    130                 135                 140

Gln Cys Glu Ser Ser Lys Lys Arg Thr Arg Ala Thr Thr Thr Asp Lys
145                 150                 155                 160

Asn Lys Arg Ala Asn Lys Ala Arg Arg Ser Gln Lys Cys Val Glu Met
                165                 170                 175

Ser Gly Glu Asn Glu Asn Ser Gly Glu Glu Glu Tyr Thr Glu Lys Ala
            180                 185                 190

Ala Gly Lys Arg Lys Thr Lys Pro Leu Lys Pro Gln Lys Thr Cys Cys
        195                 200                 205

Ser Asp Asp Glu Ser Asn Gly Gly Asp Thr Phe Leu Ser Lys Glu Asp
    210                 215                 220

Gly Glu Asp Ser Lys Ala Leu Asn Leu Asn Gly Lys Thr Arg Ala Ser
225                 230                 235                 240

Arg Gly Ala Ala Thr Asp Pro Gln Ser Leu Tyr Ala Arg Val Asp Ile
                245                 250                 255

Ser Thr Met Leu Glu Glu Ala Val Gln Tyr Val Lys Phe Leu Gln Leu
            260                 265                 270

Gln Ile Lys Arg Leu Leu Ala Ile Gly Thr Asn His Arg Asn Arg Ser
        275                 280                 285

Ile Pro Leu Trp Thr Ala Arg Asn Arg Gln Ile Ser Lys Ala His Ser
    290                 295                 300

Arg Lys Arg Leu Arg Leu Arg Ala Val Ala Lys Ile Ile Trp Ser Asp
305                 310                 315                 320

Glu Met Thr Arg Phe Leu Leu Glu Leu Ile Thr Leu Glu Lys Gln Ala
                325                 330                 335

Gly Asn Tyr Arg Gly Lys Ser Leu Ile Glu Lys Gly Lys Glu Asn Val
            340                 345                 350

Leu Val Lys Phe Lys Lys Arg Phe Pro Ile Thr Leu Asn Trp Asn Lys
        355                 360                 365

Val Lys Asn Arg Leu Asp Thr Leu Lys Lys Gln Tyr Glu Ile Tyr Pro
    370                 375                 380

Ala Lys Leu Arg Ser His Pro Leu Arg Phe Ile Pro Leu Leu Asp Val
385                 390                 395                 400

Val Phe Arg Asp Glu Thr Val Val Val Glu Glu Ser Trp Gln Pro Arg
                405                 410                 415

Arg Gly Val His Arg Arg Ala Pro Val Leu Asp Leu Ser Asp Ser Glu
            420                 425                 430

Cys Pro Asn Asn Asn Gly Asp Glu Arg Glu Asp Leu Met Gln Asn Arg
        435                 440                 445

Glu Arg Asp His Met Arg Pro Pro Thr Pro Asp Trp Met Ser Gln Thr
    450                 455                 460

Pro Met Glu Asn Ser Pro Thr Ser Ala Asn Ser Asp Pro Pro Phe Ala
465                 470                 475                 480

Ser Gln Glu Arg Ser Ser Thr His Thr Gln Val Lys Asn Val Ser Arg
                485                 490                 495

Asn Arg Lys Arg Lys Gln Asn Pro Ala Asp Ser Thr Leu Asp Arg Ile
            500                 505                 510

Ala Ala Thr Met Lys Lys Ile
        515

<210>  8
<211>  987
<212>  DNA
<213>  Arabidopsis thaliana

<400> 8
atggaagcca tgggagaatg gagcaccggc ctaggcggaa tatatacaga ggaagctgac 60

tttatgaatc agctccttgc ctcctatgag caaccttgtg gcggttcatc ttcagagaca 120

accgccacac tcacggccta ccaccaccag ggttctcaat ggaatggtgg cttttgcttc 180

tctcaggaga gcagtagtta tagtggttac tgcgcggcga tgccacggca agaagaagat 240

aacaatggga tggaggacgc gacaatcaac acgaacttgt accttgttgg tgaagagaca 300

agtgaatgtg atgcgacgga atactccggt aaaagcctct tgcctttgga gactgtcgca 360

gaaaaccacg accatagtat gctacagcct gagaactcct tgaccacgac cactgatgag 420

aaaatgttca accaatgtga gagttcaaag aagaggacgc gtgccacaac aactgataag 480

aacaagagag ccaacaaggc acgaaggagc cagaaatgcg tagagatgag tggcgaaaat 540

gaaaatagcg gcgaagaaga atatacggag aaggctgcgg ggaagagaaa gaccaaacca 600

cttaagccgc aaaagacttg ttgttcggat gacgaatcaa acggtggaga cactttcttg 660

tccaaagaag atggcgagga ctctaaggct ctcaacctca acggcaagac tagggccagc 720

cgcggcgcgg ccacagatcc tcaaagcctt tacgcaagga aaagaagaga gaggataaac 780

gagaggctaa ggattttgca acatctcgtc cctaatggaa caaaggttga tattagcacg 840

atgttggaag aagcagtaca atacgtcaaa tttctacagc tccaaattaa gttattgagc 900

tctgatgatc tatggatgta tgcgcctatt gcttacaacg gaatggacat tggccttgac 960

ctaaaactca atgcactgac cagatga 987

<210> 9
<211> 258
<212> PRT
<213> Arabidopsis thaliana

<400> 9

Met Asp Val Phe Val Asp Gly Glu Leu Glu Ser Leu Leu Gly Met Phe
1               5                   10                  15

Asn Phe Asp Gln Cys Ser Ser Ser Lys Glu Glu Arg Pro Arg Asp Glu
            20                  25                  30

Leu Leu Gly Leu Ser Ser Leu Tyr Asn Gly His Leu His Gln His Gln
        35                  40                  45

His His Asn Asn Val Leu Ser Ser Asp His His Ala Phe Leu Leu Pro
    50                  55                  60

Asp Met Phe Pro Phe Gly Ala Met Pro Gly Gly Asn Leu Pro Ala Met
65                  70                  75                  80

Leu Asp Ser Trp Asp Gln Ser His His Leu Gln Glu Thr Ser Ser Leu
                85                  90                  95

Lys Arg Lys Leu Leu Asp Val Glu Asn Leu Cys Lys Thr Asn Ser Asn
            100                 105                 110

Cys Asp Val Thr Arg Gln Glu Leu Ala Lys Ser Lys Lys Lys Gln Arg
        115                 120                 125

Val Ser Ser Glu Ser Asn Thr Val Asp Glu Ser Asn Thr Asn Trp Val
    130                 135                 140

Asp Gly Gln Ser Leu Ser Asn Ser Ser Asp Asp Glu Lys Ala Ser Val
145                 150                 155                 160

Thr Ser Val Lys Gly Lys Thr Arg Ala Thr Lys Gly Thr Ala Thr Asp
                165                 170                 175

Pro Gln Ser Leu Tyr Ala Arg Lys Arg Arg Glu Lys Ile Asn Glu Arg
            180                 185                 190

Leu Lys Thr Leu Gln Asn Leu Val Pro Asn Gly Thr Lys Val Asp Ile
        195                 200                 205

Ser Thr Met Leu Glu Glu Ala Val His Tyr Val Lys Phe Leu Gln Leu
    210                 215                 220

Gln Ile Lys Leu Leu Ser Ser Asp Asp Leu Trp Met Tyr Ala Pro Leu
225                 230                 235                 240

Ala Tyr Asn Gly Leu Asp Met Gly Phe His His Asn Leu Leu Ser Arg
                245                 250                 255

Leu Met

<210> 10
<211> 777
<212> DNA
<213> Arabidopsis thaliana

<400> 10
atggacgttt ttgttgatgg tgaattggag tctctcttgg ggatgttcaa ctttgatcaa 60

tgttcatcat ctaaagagga gagaccgcga gacgagttgc ttggcctctc tagcctttac 120

aatggtcatc ttcatcaaca tcaacaccat aacaatgtct tatcttctga tcatcatgct 180

ttcttgctcc ctgatatgtt cccatttggt gcaatgccgg gaggaaatct tccggccatg 240

cttgattctt gggatcaaag tcatcacctc caagaaacgt cttctcttaa gaggaaacta 300

cttgacgtgg agaatctatg caaaactaac tctaactgtg acgtcacaag acaagagctt 360

gcgaaatcca agaaaaaaca gagggtaagc tcggaaagca atacagttga cgagagcaac 420

actaattggg tagatggtca gagtttaagc aacagttcag atgatgagaa agcttcggtc 480

acaagtgtta aaggcaaaac tagagccacc aaagggacag ccactgatcc tcaaagcctt 540

tatgctcgga aacgaagaga gaagattaac gaaaggctca agacactaca aaaccttgtg 600

ccaaacggga caaaagtcga tataagcacg atgcttgaag aagcggtcca ttacgtgaag 660

ttcttgcagc ttcagattaa gttgttgagc tcggatgatc tatggatgta cgcaccattg 720

gcttacaacg gcctggacat ggggttccat cacaaccttt tgtctcggct tatgtga 777

<210> 11
<211> 223
<212> PRT
<213> Arabidopsis thaliana

<400> 11

Met Glu Asn Glu Ala Phe Val Asp Gly Glu Leu Glu Ser Leu Leu Gly
1               5                   10                  15

Met Phe Asn Phe Asp Gln Cys Ser Ser Asn Glu Ser Ser Phe Cys Asn
            20                  25                  30

Ala Pro Asn Glu Thr Asp Val Phe Ser Ser Asp Asp Phe Phe Pro Phe
        35                  40                  45

Gly Thr Ile Leu Gln Ser Asn Tyr Ala Ala Val Leu Asp Gly Ser Asn
    50                  55                  60

His Gln Thr Asn Arg Asn Val Asp Ser Arg Gln Asp Leu Leu Lys Pro
65                  70                  75                  80

Arg Lys Lys Gln Lys Leu Ser Ser Glu Ser Asn Leu Val Thr Glu Pro
                85                  90                  95

Lys Thr Ala Trp Arg Asp Gly Gln Ser Leu Ser Ser Tyr Asn Ser Ser
            100                 105                 110

Asp Asp Glu Lys Ala Leu Gly Leu Val Ser Asn Thr Ser Lys Ser Leu
        115                 120                 125

Lys Arg Lys Ala Lys Ala Asn Arg Gly Ile Ala Ser Asp Pro Gln Ser
130                 135                 140

Leu Tyr Ala Arg Lys Arg Arg Glu Arg Ile Asn Asp Arg Leu Lys Thr
145                 150                 155                 160

Leu Gln Ser Leu Val Pro Asn Gly Thr Lys Val Asp Ile Ser Thr Met
                165                 170                 175

Leu Glu Asp Ala Val His Tyr Val Lys Phe Leu Gln Leu Gln Ile Lys
            180                 185                 190

Leu Leu Ser Ser Glu Asp Leu Trp Met Tyr Ala Pro Leu Ala His Asn
        195                 200                 205

Gly Leu Asn Met Gly Leu His His Asn Leu Leu Ser Arg Leu Ile
    210                 215                 220

<210>  12
<211>  672
<212>  DNA
<213>  Arabidopsis thaliana

<400>  12
atggagaatg aagcttttgt agatggtgaa ttggagtctc ttttggggat gttcaacttt      60

gatcaatgtt catctaacga atcgagcttt tgcaatgctc caaatgagac tgatgttttc     120

tcttctgatg atttcttccc atttggtaca attctgcaaa gtaactatgc ggccgttctt     180

gatggttcca accaccaaac gaaccgaaat gtcgactcaa gacaagatct gttgaaacca     240

aggaagaagc aaaagttaag ctcggaaagc aatttggtta ccgagcctaa gactgcttgg     300

agagatggtc aaagcctaag cagttataat agttcagatg atgaaaaggc tttaggttta     360

gtgtctaata catcaaaaag cctaaaacgc aaagcgaaag ccaacagagg gatagcttcc     420

gatcctcaga gcctatacgc taggaaacga agagaaagga taaacgatag gctaaagaca     480

ttgcagagcc tagttcctaa tgggacaaag gtcgatataa gcacaatgct ggaagatgct     540

gtccattacg tgaagttcct gcagcttcaa atcaagctct tgagttcaga agatctatgg     600

atgtatgcac ctcttgctca caatggtctg aatatgggac tacatcacaa tcttttgtct     660

cggcttattt aa                                                         672

<210>  13
<211>  67
<212>  PRT
<213>  Arabidopsis thaliana

<400>  13

Thr Ser Pro Lys Asp Pro Gln Ser Leu Ala Ala Lys Asn Arg Arg Glu
1               5                   10                  15

Arg Ile Ser Glu Arg Leu Lys Ile Leu Gln Glu Leu Val Pro Asn Gly
            20                  25                  30

Thr Lys Val Asp Leu Val Thr Met Leu Glu Lys Ala Ile Ser Tyr Val
        35                  40                  45

Lys Phe Leu Gln Val Gln Val Lys Val Leu Ala Thr Asp Glu Phe Trp
    50                  55                  60

Pro Ala Gln
65

<210>  14
<211>  67
<212>  PRT
<213>  Arabidopsis thaliana

<400>  14

Thr Ser Pro Lys Asp Pro Gln Ser Leu Ala Ala Lys Asn Arg Arg Glu
1               5                   10                  15

Arg Ile Ser Glu Arg Leu Lys Val Leu Gln Glu Leu Val Pro Asn Gly
            20                  25                  30

Thr Lys Val Asp Leu Val Thr Met Leu Glu Lys Ala Ile Gly Tyr Val
        35                  40                  45

Lys Phe Leu Gln Val Gln Val Lys Val Leu Ala Ala Asp Glu Phe Trp
    50                  55                  60

Pro Ala Gln
65

<210>  15
<211>  67
<212>  PRT
<213>  Physcomitrella patens

<400>  15

Gly Ser Ala Asn Asp Pro Gln Ser Ile Ala Ala Arg Val Arg Arg Glu
1               5                   10                  15

Arg Ile Ser Glu Arg Leu Lys Val Leu Gln Ala Leu Ile Pro Asn Gly
            20                  25                  30

Asp Lys Val Asp Met Val Thr Met Leu Glu Lys Ala Ile Ser Tyr Val
        35                  40                  45

Gln Cys Leu Glu Phe Gln Ile Lys Met Leu Lys Asn Asp Ser Leu Trp
    50                  55                  60

Pro Lys Ala
65

<210> 16
<211> 67
<212> PRT
<213> Physcomitrella patens

<400> 16

Gly Ser Ala Asn Asp Pro Gln Ser Ile Ala Ala Arg Val Arg Arg Glu
1               5                   10                  15

Arg Ile Ser Glu Arg Leu Lys Val Leu Gln Ala Leu Ile Pro Asn Gly
            20                  25                  30

Asp Lys Val Asp Met Val Thr Met Leu Glu Lys Ala Ile Thr Tyr Val
        35                  40                  45

Gln Cys Leu Glu Leu Gln Ile Lys Met Leu Lys Asn Asp Ser Ile Trp
    50                  55                  60

Pro Lys Ala
65

<210> 17
<211> 67
<212> PRT
<213> Physcomitrella patens

<400> 17

Gly Ser Ala Thr Asp Pro Gln Ser Val Tyr Ala Arg His Arg Arg Glu
1               5                   10                  15

Lys Ile Asn Glu Arg Leu Lys Ser Leu Gln Asn Leu Val Pro Asn Gly
            20                  25                  30

Ala Lys Val Asp Ile Val Thr Met Leu Asp Glu Ala Ile His Tyr Val
        35                  40                  45

Lys Phe Leu Gln Asn Gln Val Glu Leu Leu Lys Ser Asp Glu Leu Trp

    50                  55                  60

Ile Tyr Ala
65

<210> 18
<211> 67
<212> PRT
<213> Physcomitrella patens

<400> 18

Gly Ser Ala Thr Asp Pro Gln Ser Val Tyr Ala Arg His Arg Arg Glu
1               5                   10                  15

Lys Ile Asn Glu Arg Leu Lys Asn Leu Gln Asn Leu Val Pro Asn Gly
            20                  25                  30

Ala Lys Val Asp Ile Val Thr Met Leu Asp Glu Ala Ile His Tyr Val
        35                  40                  45

Lys Phe Leu Gln Thr Gln Val Glu Leu Leu Lys Ser Asp Glu Phe Trp
    50                  55                  60

Met Phe Ala
65

<210> 19
<211> 67
<212> PRT
<213> Physcomitrella patens

<400> 19

Gly Ser Ala Thr Asp Pro Gln Ser Val Tyr Ala Arg His Arg Arg Glu
1               5                   10                  15

Lys Ile Asn Glu Arg Leu Lys Thr Leu Gln His Leu Val Pro Asn Gly
            20                  25                  30

Ala Lys Val Asp Ile Val Thr Met Leu Asp Glu Ala Ile His Tyr Val
        35                  40                  45

Gln Phe Leu Gln Leu Gln Val Thr Leu Leu Lys Ser Asp Glu Tyr Trp
    50                  55                  60

Met Tyr Ala
65

<210> 20
<211> 67
<212> PRT
<213> Physcomitrella patens

<400> 20

Gly Ser Ala Thr Asp Pro Gln Ser Val His Ala Arg Ala Arg Arg Glu
1               5                   10                  15

Lys Ile Ala Glu Arg Leu Arg Lys Leu Gln His Leu Ile Pro Asn Gly
            20                  25                  30

Gly Lys Val Asp Ile Val Thr Met Leu Asp Glu Ala Val His Tyr Val
        35                  40                  45

Gln Phe Leu Lys Arg Gln Val Thr Leu Leu Lys Ser Asp Glu Tyr Trp
    50                  55                  60

Met Tyr Ala
65

<210> 21
<211> 67
<212> PRT
<213> Physcomitrella patens

<400> 21

Gly Ser Ala Thr Asp Pro Gln Ser Val Tyr Ala Arg His Arg Arg Glu
1               5                   10                  15

Lys Ile Asn Glu Arg Leu Lys Thr Leu Gln Arg Leu Val Pro Asn Gly
            20                  25                  30

Glu Gln Val Asp Ile Val Thr Met Leu Glu Glu Ala Ile His Phe Val
        35                  40                  45

Lys Phe Leu Glu Phe Gln Leu Glu Leu Leu Arg Ser Asp Asp Arg Trp
    50                  55                  60

Met Phe Ala
65

<210> 22
<211> 67
<212> PRT
<213> Arabidopsis thaliana

<400> 22

Gly Ala Ala Thr Asp Pro Gln Ser Leu Tyr Ala Arg Lys Arg Arg Glu
1               5                   10                  15

Arg Ile Asn Glu Arg Leu Arg Ile Leu Gln Asn Leu Val Pro Asn Gly
            20                  25                  30

Thr Lys Val Asp Ile Ser Thr Met Leu Glu Glu Ala Val His Tyr Val
        35                  40                  45

Lys Phe Leu Gln Leu Gln Ile Lys Leu Leu Ser Ser Asp Asp Leu Trp
    50                  55                  60

Met Tyr Ala
65

<210>  23
<211>  67
<212>  PRT
<213>  Arabidopsis thaliana

<400>  23

Gly Ala Ala Thr Asp Pro Gln Ser Leu Tyr Ala Arg Lys Arg Arg Glu
1               5                   10                  15

Arg Ile Asn Glu Arg Leu Arg Ile Leu Gln His Leu Val Pro Asn Gly
            20                  25                  30

Thr Lys Val Asp Ile Ser Thr Met Leu Glu Glu Ala Val Gln Tyr Val
        35                  40                  45

Lys Phe Leu Gln Leu Gln Ile Lys Leu Leu Ser Ser Asp Asp Leu Trp
    50                  55                  60

Met Tyr Ala
65

<210>  24
<211>  67
<212>  PRT
<213>  Arabidopsis thaliana

<400>  24

Gly Thr Ala Thr Asp Pro Gln Ser Leu Tyr Ala Arg Lys Arg Arg Glu
1               5                   10                  15

Lys Ile Asn Glu Arg Leu Lys Thr Leu Gln Asn Leu Val Pro Asn Gly
            20                  25                  30

Thr Lys Val Asp Ile Ser Thr Met Leu Glu Glu Ala Val His Tyr Val
        35                  40                  45

Lys Phe Leu Gln Leu Gln Ile Lys Leu Leu Ser Ser Asp Asp Leu Trp
    50                  55                  60

Met Tyr Ala
65

<210> 25
<211> 67
<212> PRT
<213> Arabidopsis thaliana

<400> 25

Gly Ile Ala Ser Asp Pro Gln Ser Leu Tyr Ala Arg Lys Arg Arg Glu
1               5                   10                  15

Arg Ile Asn Asp Arg Leu Lys Thr Leu Gln Ser Leu Val Pro Asn Gly
            20                  25                  30

Thr Lys Val Asp Ile Ser Thr Met Leu Glu Asp Ala Val His Tyr Val
        35                  40                  45

Lys Phe Leu Gln Leu Gln Ile Lys Leu Leu Ser Ser Glu Asp Leu Trp
    50                  55                  60

Met Tyr Ala
65

<210> 26
<211> 762
<212> PRT
<213> Physcomitrella patens

<400> 26

Met Ala Gly Pro Ala Gly Ala Leu Trp Ser Thr Cys Asp Pro Gln Pro
1               5                   10                  15

Ile Gln Gln Ala Glu Ile Phe Ser Gly Pro Asp Asn Gln Ala Gly Leu
            20                  25                  30

Met Ser Phe His Val Asp Thr Pro Phe His Trp Gly Ser Glu Pro Trp
        35                  40                  45

Ala Leu His Ser Arg Ser Asp Asp Ile Ala Leu Met Ser Pro Ser Leu
    50                  55                  60

Val His Asp Ile Ser Pro Tyr Asp Ser Val Leu His Leu Ser Gly Val
65                  70                  75                  80

Ser Gly Asp Val Gln Asp Leu Val Cys Gly Asn Pro Lys Phe Arg Gln
                85                  90                  95

Ser Gly Gln Trp Gly Gln Ser Glu Phe Ser Tyr Ser Val Gln Asp Asn
            100                 105                 110

Met Gln Asp Leu Leu Thr Asn Gln Phe Ile Pro Tyr Asn Thr Ser Ser
        115                 120                 125

Leu Gly Leu Asn His Leu Ser Pro Asn Phe Thr Asp Leu Asp Cys Ala
    130                 135                 140

Pro Val Tyr Asn Asp Thr Lys Ala Phe Gly Thr Val Thr His Asn Arg
145                 150                 155                 160

Ala Val Pro Ser Thr Asn Thr Gln Ser Ala Gln His Gly Ser Ser Ser
                165                 170                 175

Met Val Ser Ser Asn Arg Pro Ile Thr Ser Thr Ala Ser Pro Thr Thr
            180                 185                 190

Gln Tyr Gly Gly Pro Arg Thr Pro Ser Gln Thr Thr Gln Tyr Gly Gly
        195                 200                 205

Ser Ser Met Val Thr Asn Ser Met Glu Met Phe Ala Ser Ala Ala Pro
    210                 215                 220

Gln Gly Ile Met Thr Thr Ser Gly Leu Ser Gly Gly Cys Asn Ser Asp
225                 230                 235                 240

Leu Met His Leu Pro Lys Arg Gln His Ala His Ser Leu Pro Pro Thr
                245                 250                 255

Thr Gly Arg Asp Leu Thr Ala Ser Glu Val Val Ser Gly Asn Ser Ile
            260                 265                 270

Ser Asn Ile Ser Gly Val Gly Ser Phe Asn Ser Ser Gln Lys Ser Ser
        275                 280                 285

Ala Ser Val Met Met Ser Pro Leu Ala Ala Ser Ser His Met His Lys
    290                 295                 300

Ala Ala Ala Val Ser Glu Glu Leu Lys Met Ala Ser Phe Asn Pro Gly
305                 310                 315                 320

Pro Phe Val Pro Thr Gln Lys Lys Gln Gln His Glu Gln Gln Asp Thr
                325                 330                 335

Met Thr Ser Asn Arg Ile Trp Ala Asp Lys Asn Asn Leu Gly Lys Ile
            340                 345                 350

Ser Ser Ser Pro Ile Pro Ile Met Gly Phe Glu Gln Ser Gln Gln Gln
        355                 360                 365

Ser Met Ser Asn Ser Ser Pro Val Thr Ser Leu Gly Phe Glu Gln Arg
    370                 375                 380

Gln Lys Met Ser Met Gly Ser Ser Pro Ser Ile Thr Ile Ile Gly Phe
385                 390                 395                 400

Glu Gln Arg Gln Lys Gln Pro Met Ser Ser Ser Ser Pro Ile Ser Asn
                405                 410                 415

Met Val Phe Glu Pro Arg Gln Lys Gln Pro Met Ser Ser Ser Ser Pro
            420                 425                 430

Ile Ser Asn Ile Val Phe Glu Gln Arg Gln Leu Pro Thr Val Gly Ser
        435                 440                 445

Ser Pro Pro Ile Ser Ile Ser Gly Phe Glu Pro Lys Lys Gln Pro Ser
    450                 455                 460

Leu Ser Asn Ser Pro Pro Leu Ser Asn Leu Gly Phe Glu Gln Arg Leu
465                 470                 475                 480

Gln Pro Met Ser Asn Ala Ser Pro Ile Ser Asn Leu Pro Phe Glu Gln
                485                 490                 495

Gln Arg Gln Gln Ala Thr Met Ser Asn Thr Arg Ser Ala Glu Pro Asp
            500                 505                 510

Ser Val Glu Ser Thr Thr Lys Trp Pro Leu Arg Met Asp Gly Ala Ile
        515                 520                 525

Gly Gly Cys Ala Gly Leu Pro Ser Ser Gln Lys Ala Pro Val Ile Met
    530                 535                 540

Gln Pro Glu Thr Gly Thr Met Lys Cys Pro Ile Pro Arg Thr Met Pro
545                 550                 555                 560

Ser Asn Ala Lys Ala Cys Pro Ala Val Gln Asn Ala Asn Ser Val Asn
                565                 570                 575

Lys Arg Pro Leu Thr Val Asp Asp Lys Asp Gln Thr Gly Ser Met Asn
            580                 585                 590

Lys Lys Ser Met Gln Lys Phe Leu Gly Pro Gln Gly Cys Ser Arg Leu
        595                 600                 605

Glu Ser Ile Ser Ala Leu Ala His Gln Lys Val Ser Gln Ser Thr Thr
    610                 615                 620

Ser Gly Arg Ala Leu Gly Pro Ala Leu Asn Thr Asn Leu Lys Pro Arg
625                 630                 635                 640

Ala Arg Gln Gly Ser Ala Asn Asp Pro Gln Ser Ile Ala Ala Arg Val
                645                 650                 655

Arg Arg Glu Arg Ile Ser Glu Arg Leu Lys Val Leu Gln Ala Leu Ile
            660                 665                 670

Pro Asn Gly Asp Lys Val Asp Met Val Thr Met Leu Glu Lys Ala Ile
        675                 680                 685

Ser Tyr Val Gln Cys Leu Glu Phe Gln Ile Lys Met Leu Lys Asn Asp
    690                 695                 700

Ser Leu Trp Pro Lys Ala Leu Gly Pro Leu Pro Asn Thr Leu Gln Glu
705                 710                 715                 720

Leu Leu Glu Leu Ala Gly Pro Glu Phe Ala Gly Ile Asp Gly Lys Asn
                725                 730                 735

Thr Glu Glu Ser Ser Glu Lys Pro Lys Lys Ser Ala Leu Glu Val Ile
            740                 745                 750

Glu Leu Asp Gly Asn Gln Pro Ser Ala Asp
        755                 760

<210>  27
<211>  2289
<212>  DNA
<213>  Physcomitrella patens

<400> 27
atggcaggtc cagcaggagc tttatggagt acttgtgatc cacagcctat tcaacaggca 60
gagatattta gtggtcctga caaccaagct ggtttgatgt cttttcatgt ggataccccg 120
ttccattggg gatctgaacc atgggctctc cactctcggt cagatgacat cgccttgatg 180
tccccctcgc ttgttcacga catatcacct tatgattctg tcttgcatct ttccggagtg 240
tctggggatg tgcaagattt agtttgcggg aatcccaaat ttcgccaaag tgggcaatgg 300
gggcagagcg agttttcata ctctgttcag gacaacatgc aagatctcct aaccaaccag 360
ttcataccgt acaacacatc ttcattgggt ttaaatcatc tctccccgaa tttcaccgac 420
ttggattgcg caccggtata caatgatacc aaggcttttg gcactgttac acacaacagg 480
gcagtcccga gcactaatac ccagagtgct cagcacggga gttcgtctat ggtttcaagt 540
aacaggccaa tcactagcac agcttctcct actactcagt atggaggtcc gaggactcca 600
tcccaaacca cccagtacgg gggttcatct atggttacca actcgatgga aatgtttgct 660
tcagctgcac ctcagggtat tatgactaca tctggcttga gtggcggttg caactcagac 720
ttgatgcatc tgccgaagcg ccagcatgct cactctcttc ctcctaccac tggcagagat 780
ttaactgcat ctgaagtggt atctggaaat tcgatatcaa acatttccgg ggttggatct 840
tttaacagca gccagaaaag cagtgcatcc gtgatgatgt ctcctttagc tgcttcttct 900
cacatgcaca aggctgctgc tgtatctgaa gaacttaaga tggcaagttt caaccctggt 960
ccattcgtac ctacgcagaa aaagcagcaa catgagcagc aggatacgat gacctctaat 1020
cgtatatggg cggataagaa caacttggga aaaattagtt catcgcccat tccgatcatg 1080
gggtttgagc agagtcaaca gcaatccatg agcaattcct cccctgttac cagtttgggg 1140
tttgagcaaa ggcaaaaaat gtccatgggt agctctccct ccatcacgat cattggattt 1200
gagcaaagac agaagcaacc tatgagtagt tcttccccca tttcaaacat ggtttttgaa 1260
ccaagacaaa aacagccaat gagtagctct tctcctatct ctaatattgt ctttgagcaa 1320
agacaactcc caactgtggg tagctctcct ccgatttcaa tctcaggatt tgagccaaag 1380
aaacaaccat ctttgagcaa ttctcctccc ctctctaatc tgggttttga gcaaaggcta 1440
caacccatga gtaatgcatc tcctatttcc aacttaccct ttgagcaaca aagacaacaa 1500
gcaaccatga gtaacaccag atctgcagaa cccgattctg tcgagtctac cacgaagtgg 1560
cccttgcgga tggatggtgc cataggtgga tgtgctggct taccaagcag tcagaaagct 1620
cctgttatca tgcagcctga gactgggact atgaagtgtc ctattccgag gaccatgccc 1680
agcaatgcta aggcttgccc agctgtgcag aatgctaatt ccgtaaacaa gcgccctctt 1740
acggttgatg acaaggacca aactggatcg atgaataaga agtcgatgca aaagtttttg 1800

ggacctcaag gttgtagcag acttgaaagt atcagtgctt tagctcacca aaaagtgagt 1860

caaagtacaa caagcggtcg tgctctaggg cctgctttga acaccaatct caagcctcgt 1920

gcacgccaag ggagtgccaa tgatccgcag agcattgctg ctagggtgcg aagagaaaga 1980

ataagtgagc ggctcaaagt tttgcaagcc ttgataccta acggtgataa agtggatatg 2040

gtcaccatgc tggagaaggc tatcagctac gtgcagtgtt tggaatttca gattaagatg 2100

ttaaaaaatg actctttgtg gcctaaggcg cttggccctc taccgaacac tttgcaagag 2160

cttctcgaac ttgctgggcc agagtttgcc ggcatagatg gcaagaatac tgaggagtcg 2220

tcagagaaac cgaagaaatc tgctcttgaa gtaattgagt tggacggcaa tcagccttct 2280

gctgactaa 2289

<210> 28
<211> 173
<212> PRT
<213> Physcomitrella patens

<400> 28

Met Asn Lys Lys Pro Met Gln Lys Ala Leu Gly Pro Gln Gly Cys Ser
1               5                   10                  15

Arg Leu Glu Ser Ile Ser Ala Leu Ala His Gln Lys Val Ser Gln Ser
            20                  25                  30

Ala Ser Gly Arg Ala Leu Gly Pro Ala Leu Asn Thr Asn Leu Lys Pro
        35                  40                  45

Arg Ala Arg Gln Gly Ser Ala Asn Asp Pro Gln Ser Ile Ala Ala Arg
    50                  55                  60

Val Arg Arg Glu Arg Ile Ser Glu Arg Leu Lys Val Leu Gln Ala Leu
65                  70                  75                  80

Ile Pro Asn Gly Asp Lys Val Asp Met Val Thr Met Leu Glu Lys Ala
                85                  90                  95

Ile Thr Tyr Val Gln Cys Leu Glu Leu Gln Ile Lys Met Leu Lys Asn
            100                 105                 110

Asp Ser Ile Trp Pro Lys Ala Leu Gly Pro Leu Pro Asn Thr Leu Gln
        115                 120                 125

Glu Leu Leu Glu Leu Ala Gly Pro Glu Phe Ser Gly Thr Glu Ser Lys
    130                 135                 140

Asn Val Glu Glu Pro Pro Ala Lys Pro Lys Lys Ser Ala Pro Asp Val
145                 150                 155                 160

Ile Glu Phe Asp Gly Asn Gln Pro Ser Ala Asp Lys Glu
            165                 170

<210> 29
<211> 522
<212> DNA
<213> Physcomitrella patens

<400> 29
atgaataaga agcctatgca aaaagctttg ggacctcaag gatgcagcag gctagaaagc     60

atcagtgctt tagctcatca aaaagtgagt cagagtgcaa gtggtcgtgc actagggcct    120

gctctgaaca ccaacctcaa gcctcgtgct cgtcaaggga gtgccaatga cccacagagc    180

attgccgcta gggttcgaag agaaaggata agtgagcggc tgaaagtttt gcaagccttg    240

atacctaatg gtgataaggt agatatggtg accatgctgg agaaggctat cacctacgtg    300

cagtgtctgg aactccagat taagatgtta aagaatgatt ctatctggcc caaggcgctt    360

ggacctctac caaacactct tcaagagctt ctggagcttg ctggaccaga attttctgga    420

acggaaagca agaatgtaga ggagccccca gcgaagccaa agaaatcagc tcctgacgtt    480

attgagttcg acggcaatca accttctgcc gacaaagagt ag                       522

<210> 30
<211> 67
<212> PRT
<213> Physcomitrella patens

<400> 30

Gly Ser Ala Thr Asp Pro Gln Ser Val Tyr Ala Arg His Arg Arg Glu
1               5                   10                  15

Lys Ile Asn Glu Arg Leu Lys Thr Leu Gln His Leu Val Pro Asn Gly
            20                  25                  30

Ala Lys Val Asp Ile Val Thr Met Leu Asp Glu Ala Ile His Tyr Val
        35                  40                  45

Gln Phe Leu Gln Leu Gln Val Thr Leu Leu Lys Ser Asp Glu Tyr Trp
    50                  55                  60

Met Tyr Ala
65

<210> 31
<211> 201
<212> DNA
<213> Physcomitrella patens

<400> 31
ggttcagcga ctgatccgca gagtgtatat gccaggcata gaagggagaa gatcaacgag 60

cgcttgaaga cattacagca cttggtacca aatggagcta aggtagacat cgtgaccatg 120

cttgacgaag ccattcacta cgtccaattt ctgcagctcc aagtgacgct gttgaagtcg 180

gatgaatatt ggatgtacgc c 201


<210> 32
<211> 538
<212> PRT
<213> Physcomitrella patens

<400> 32

Met Thr Asp Leu Ile Ser Ile Leu Glu Ser Ser Gly Ser Ser Arg Glu
1               5                   10                  15


Glu Met Cys Pro Val Ala Val Pro Ser Ser Val Ala Ser Ser Cys Glu
            20                  25                  30


Arg Leu Ile Trp Glu Gly Trp Thr Ala Gln Pro Ser Pro Val Glu Glu
        35                  40                  45


Ser Thr Thr Ser Lys Leu Leu Pro Lys Leu Leu Pro Glu Leu Glu Thr
    50                  55                  60


Ser Ser Tyr Ser Ala Leu Thr Leu Gln Gln Pro Asp Ala Leu Ser Ser
65                  70                  75                  80


Ile Leu Ser Val Leu His Pro Phe Ser His Tyr Ser Ser Ala Ser Leu
                85                  90                  95


Glu Leu Ala Arg Asn Pro Asp Trp Ser Leu Lys Ser Ser Asn Pro Leu
            100                 105                 110


Arg Glu Ser Ser Ser Glu Ala Gly Ile Arg Thr Ser Ser Phe Glu Gly
        115                 120                 125


Leu Tyr Ser Gly Gln His Thr Thr Lys Lys Ile His Leu Gly Val Ile
    130                 135                 140


Pro Tyr His Leu Ser Glu Asp Gln Arg Gln Cys Ala Val Ser Pro Pro
145                 150                 155                 160

Glu Asn Glu Cys Arg Leu Leu Ser Ala Asn Ser Ser Gly Ser Leu His
                165                 170                 175

Trp Trp His Ser Ile Gly Pro Glu Ser Pro Ser Ser Thr Leu Ala Phe
            180                 185                 190

His Asn Ile Gly Ile Gln His Ser Thr Phe Glu Lys Cys Glu Pro Arg
        195                 200                 205

Gly Gln Ser His Ser Ser Trp Pro Ala Ala Ser Gly Thr Ser Pro Thr
    210                 215                 220

Val Gln Tyr Phe His Ala His Ser Ala Asp Asn Glu Gly Val Glu Val
225                 230                 235                 240

Val Lys Gln Asp Asp Ser Gln Ile Ser Lys Ala Leu Ala Thr Tyr Gln
                245                 250                 255

Pro His Gly Asp His Ser Leu Val Leu Asn Ser Asp Arg Ile Ala Ser
            260                 265                 270

Thr Thr Ser His Ser Glu Asp Pro Cys Gly Pro Lys Pro Gly Arg Arg
        275                 280                 285

Pro Ala Ala Ser Tyr Asp Thr Glu Met Ile Leu Ser Pro Ser Glu Ser
    290                 295                 300

Phe Leu Thr Thr Pro Asn Met Leu Ser Thr Leu Glu Cys Val Ile Ser
305                 310                 315                 320

Gly Ala Ser Asn Ile Ser Asp Gln Tyr Met Asn Phe Val Arg Glu Pro
                325                 330                 335

Gln Glu Gln Arg Leu Ser Ser Ile Ser Asp Leu Ser Leu Ile Pro Asp
            340                 345                 350

Ser His Ala Asp Pro His Ser Ile Gly Phe Ile Ser Gly Thr Phe Arg
        355                 360                 365

Thr Asp Ser His Gly Thr Gly Ile Arg Lys Asn Arg Ile Phe Leu Ser
    370                 375                 380

Asp Glu Glu Ser Asp Phe Leu Pro Lys Lys Arg Ser Lys Tyr Thr Val
385                 390                 395                 400

Arg Gly Asp Phe Gln Met Asp Arg Phe Asp Ala Val Trp Gly Asn Thr
                405                 410                 415

Gly Leu Arg Gly Ser Ser Cys Pro Gly Asn Ser Val Ser Gln Met Met
            420                 425                 430

Ala Ile Tyr Glu Phe Gly Pro Ala Leu Asn Arg Asn Gly Arg Pro Arg
        435                 440                 445

Val Gln Arg Gly Ser Ala Thr Asp Pro Gln Ser Val His Ala Arg Ala
    450                 455                 460

Arg Arg Glu Lys Ile Ala Glu Arg Leu Arg Lys Leu Gln His Leu Ile
465                 470                 475                 480

Pro Asn Gly Gly Lys Val Asp Ile Val Thr Met Leu Asp Glu Ala Val
                485                 490                 495

His Tyr Val Gln Phe Leu Lys Arg Gln Val Thr Leu Leu Lys Ser Asp
            500                 505                 510

Glu Tyr Trp Met Tyr Ala Thr Pro Thr Ser Tyr Arg Ser Lys Phe Asp
        515                 520                 525

Asp Cys Ser Leu Val Pro Gly Glu Asn Asn
    530                 535

<210> 33
<211> 1617
<212> DNA
<213> Physcomitrella patens

<400> 33
atgaccgatc tgatttcgat cttggagtca tcagggtcat cacgagagga gatgtgccct 60

gttgctgtgc caagctccgt ggcttcttct tgtgaaaggt tgatatggga ggggtggact 120

gcacaaccat ctcctgtcga agaaagcacc accagcaagt tacttccaaa gctacttcca 180

gagctcgaga catcatccta ctctgcactc acccttcagc aacctgatgc gctctccagc 240

atactttcag tcctccaccc tttttctcat tacagttcgg ccagtttaga actcgctcgc 300

aatcctgact ggagcttgaa atcttcaaat cctctgcggg aaagcagctc ggaggctggc 360

atccgaacct catctttcga aggcttgtac tctggtcagc acaccaccaa aaagattcat 420

ttgggggtca taccctacca cttgtccgaa gatcagcgcc agtgcgctgt cagtcctccg 480

gaaaatgagt gccgcctact gtctgcaaat tcctctggat cccttcactg gtggcattcc 540

ataggccccg agtctccttc ctctactctt gcattccata atattgggat ccaacactct 600

accttcgaaa agtgtgagcc taggggccag tcgcactcat catggccagc ggccagcggc 660

acgtcgccaa cagttcaata ctttcatgcc cattctgcag ataatgaagg tgtcgaggtc 720

gtcaagcaag atgactcgca gatatccaag gctctggcga cctatcaacc ccacggcgac 780

catagtctcg tgctaaattc agaccgcatt gcaagcacaa ccagccactc agaagatcct 840

tgcggcccta aacctggacg cagaccagct gcatcatacg acaccgagat gattcttagc 900

ccaagtgaga gtttcttgac aactcccaat atgttatcaa cgttggagtg cgtaatatcc 960

ggtgcaagta acatatctga tcagtatatg aacttcgtca gagaaccgca ggagcaaagg 1020

ctgtcctcta tctccgatct gtcccttatt cctgacagcc acgcggatcc gcacagtatc 1080

ggatttatct ctgggacctt tagaacagac tcccacggaa ctggaataag aaagaaccgc 1140

atctttctca gtgatgagga atccgacttc ttgcctaaga agcgatccaa gtacacggtc 1200

cgcggcgatt ttcagatgga tcgcttcgac gcagtttggg ggaataccgg tcttcgggga 1260

tctagctgtc ctggaaattc agtatcccag atgatggcga tttacgaatt cggacccgca 1320

ctgaacagga acggcaggcc gcgagtacaa cgtggttcgg cgactgatcc gcagagtgta 1380

cacgccaggg cgcggaggga gaaaatcgcc gagcgcttga gaaagttgca gcacctcatt 1440

ccaaacggcg ggaaggtgga catcgtaacc atgctcgacg aagccgttca ctatgttcag 1500

tttttgaagc gacaagttac gcttctgaaa tccgacgagt attggatgta cgccacgccg 1560

acctcgtacc ggagcaaatt cgacgactgc agtctggttc ccggcgagaa caactga 1617

<210> 34
<211> 539
<212> PRT
<213> Physcomitrella patens

<400> 34

Met Val Gln Leu Tyr Met Ser Ser Val Glu Glu Gln Arg Glu Thr Met
1               5                   10                  15

Val Gln Pro Tyr Val Ser Ser Met Asp Ser Gly Ser Thr Ser Gly Arg
            20                  25                  30

Gln Thr Pro Ser Cys Val Val Gln Gln Gly Ser Asn Thr Phe Glu Thr
        35                  40                  45

Ser Asn Leu Trp Glu Glu Trp Thr Gln Ala Ser Asn Gly Asp Asp Thr
    50                  55                  60

Val Ser Thr Ser Asn Phe Leu Pro Glu Ile Ser Ser Phe Thr Ser Ser
65                  70                  75                  80

Arg Leu Ser Phe Gln Gln Ser Asp Ser Leu Thr Thr Trp Met Ser Gly
                85                  90                  95

Phe Pro Pro Leu Ser Gln Thr Ala Leu Ser Pro Asp Leu Ser His Ser
            100                 105                 110

Ser Asp Pro Val Asp His Pro Pro Ala Phe Met Gln Glu Gly Leu Gly
        115                 120                 125

Pro Gly Asp Ser Ile Leu Asp Tyr Ser Pro Ala Leu Thr Glu Met Tyr
    130                 135                 140

Pro Lys Ser Ser Ser Lys His Asn Ser Ser Asp Cys Leu Pro Tyr Pro
145                 150                 155                 160

Ala Ala Ser Ala Pro Asp Lys Lys Met Thr Asp His Glu Leu Gly Ser
                165                 170                 175

Ala Ile Ser Leu Ala Tyr Asp Arg Gly Thr Val Ser Arg Gln Leu Leu
            180                 185                 190

Arg Ala Leu Gly Pro Leu Ser Pro Ser Ser Pro Leu Ala Leu Gln Asn
        195                 200                 205

Gly Leu Gln Asn Pro Leu Gly Asp Pro Trp Asp Ala Ser Pro Ser Ala
    210                 215                 220

Met Pro Trp Pro Met Ala Thr Thr Gly His Ala Tyr Gly Pro Gly Ala
225                 230                 235                 240

Thr Arg Thr Ser Ile Pro Asp His Leu Ala Asn Ala Ile Asn His Leu
                245                 250                 255

Glu Gly Ile Ala Pro Ser Ser Ala Ser His Ala Ser Lys Pro Arg His
            260                 265                 270

Thr Asp Ile Phe Ile Ala Pro Asn Gly Thr Phe Asp Ser Thr Pro Gly
        275                 280                 285

Gly Trp Thr Pro Gln Tyr Tyr Asp Gly Ser Val Thr Thr Asp Glu Ser
    290                 295                 300

Val Lys Ala Met Lys Leu Ile Ala Ser Leu Arg Glu Ala Gly His Ala
305                 310                 315                 320

Glu Ala Thr Ile Gly Phe Cys Thr Glu Ser Lys Pro Ser Phe Leu Arg
                325                 330                 335

Gly Gly Asp Arg Thr Thr Ser Pro Val Asp Ser Phe Phe Gly Lys Cys
            340                 345                 350

Val Gly Ala Lys Thr Ser Ile Lys Gln Ala Cys Ser Gly Lys His Pro
        355                 360                 365

Leu Glu Leu Glu Glu Ile Val Asp Ser Glu Asn Ser Glu Leu Asn Pro
    370                 375                 380

Thr Gln Leu Lys Arg Ser Lys Leu Phe Glu Asn His Pro Asn Ala Leu
385                 390                 395                 400

Trp Ser Asp Gln Ser Met Asn Gly Arg Glu Leu Arg Ser Tyr Ser His
                405                 410                 415

Leu Val Gly Ser Ser Leu Thr Ala Ser Gln Pro Met Asp Ile Ile Ala
            420                 425                 430

Ile Gly Pro Ala Leu Asn Thr Asp Gly Lys Pro Arg Ala Lys Arg Gly
        435                 440                 445

Ser Ala Thr Asp Pro Gln Ser Val Tyr Ala Arg His Arg Arg Glu Lys
    450                 455                 460

Ile Asn Glu Arg Leu Lys Ser Leu Gln Asn Leu Val Pro Asn Gly Ala
465                 470                 475                 480

Lys Val Asp Ile Val Thr Met Leu Asp Glu Ala Ile His Tyr Val Lys
                485                 490                 495

Phe Leu Gln Asn Gln Val Glu Leu Leu Lys Ser Asp Glu Leu Trp Ile
            500                 505                 510

Tyr Ala Thr Pro Asn Lys Tyr Asn Gly Met Asp Ile Ser Asp Leu Ser
        515                 520                 525

Asp Met Tyr Leu Gln Glu Leu Glu Ser Arg Ala
    530                 535

<210> 35
<211> 1620
<212> DNA
<213> Physcomitrella patens

<400> 35
atggtgcagt tatacatgtc ctcagttgaa gagcagcggg aaacaatggt acagccatac 60
gtctcaagca tggactcagg ctcaacgtcg gggcgccaga cgccatcttg cgtcgttcag 120
cagggaagta acacatttga gacttcgaat ctgtgggagg aatggacgca agcatcgaac 180
ggcgacgata cagtctccac cagcaatttc ctccccgaaa tcagttcctt cacgtcgagt 240
cgtctctcct tccagcaaag cgactctctc accacttgga tgtcagggtt ccctcccctc 300
tcccaaactg ccttgagccc ggatcttagt cactcctccg accccgtgga tcatccccca 360
gcattcatgc aggagggttt aggccccggt gattctattc tggactattc ccccgctctc 420
acagagatgt acccgaaaag tagctccaaa cataattcct cggattgttt accttaccct 480
gcggccagtg caccagacaa aaaaatgact gatcacgaac taggttcggc tatttccctc 540
gcgtatgata gaggcaccgt ttcccgccag cttcttcgag ccttgggccc attgtcgcct 600
tcatcgcctc tagcattgca gaatgggctg caaaacccgc ttggggaccc ctgggatgct 660
tctccatctg caatgccgtg gccaatggca acaaccggtc atgcttatgg accaggcgcc 720
accaggactt ctattccaga tcacttagca aatgcaatta atcacctgga gggcattgca 780
ccgtccagtg ccagtcatgc atcgaaacct cgtcacactg atattttcat tgcacccaat 840
ggcacgttcg attcgacgcc gggaggttgg acaccgcagt attacgatgg gtccgtgacg 900
acagatgagt ctgtgaaggc gatgaagctg attgcgtccc tacgtgaagc aggccacgca 960
gaggctacaa ttggattctg tacagagagc aagcctagtt ttctcagggg tggggacaga 1020
acaacctcgc cagtggacag cttcttcggc aaatgtgtag gggccaaaac gagtataaag 1080
caagcctgtt ctgggaaaca ccctcttgaa cttgaggaga tcgttgatag tgaaaacagt 1140
gaattaaatc ccacccagct caaacgctct aaactttttg agaatcatcc gaatgccttg 1200
tggagcgatc agagtatgaa tggaagagaa ctgagatcgt actctcattt ggttggcagc 1260
agtcttactg catcgcagcc catggacata attgcaattg gcccagcgct caacactgat 1320
ggcaaaccac gagcaaagcg gggttcagca accgatcctc agagtgttta cgctagacat 1380
aggagagaaa aaatcaacga acgattgaag agtttacaaa acctagtacc taatggagcc 1440
aaggttgaca tagtaaccat gctggacgaa gctatacatt acgtcaaatt tttacaaaat 1500
caagttgagc tgctgaagtc cgacgagttg tggatttacg caacaccaaa taagtacaac 1560
ggcatggaca tttccgacct ctctgacatg tatttgcagg agctggagtc acgtgcgtga 1620

<210> 36
<211> 538
<212> PRT
<213> Physcomitrella patens

<400> 36

Met Val Arg Phe Asn Tyr Met Tyr Pro Val Gln Glu Gln Leu Glu Ala
1               5                   10                  15

Met Thr Asp Gln His Thr Pro Ser Met Asp Ser Val Ser Ser Ala Gly
            20                  25                  30

Glu Lys Thr Ser Ser Cys Ile Val Gln Gln Gly Gly Asn Ala Ser Glu
        35                  40                  45

Thr Ser Asn Leu Trp Glu Glu Trp Thr Gln Gly Ser Asn Gly Asp Asp
    50                  55                  60

Ser Val Ser Thr Ser Asn Phe Leu Pro Glu Leu Asn Ser Ser Thr Ser
65                  70                  75                  80

Ser Arg Leu Ala Phe His Gln Ser Asp Ile Leu Ser Thr Trp Ile Ser
                85                  90                  95

Gly Tyr His Pro Leu Ser Gln Ser Ser Leu Ser Ser Glu Phe Ser His
            100                 105                 110

Thr Ser Asp Arg Glu Asn His Pro Pro Ala Phe Met Gln Glu Gly Leu
        115                 120                 125

Ile Pro Ser Gly Leu Ile Leu Asp Ser Asp Pro Ala Leu Thr Asp Ile
    130                 135                 140

Tyr Thr Arg Ser Ser Ser Ser Asp Ser Leu Pro Tyr Pro Thr Ala Arg
145                 150                 155                 160

Ile Met Asp Lys Ala Leu Thr Asp His Glu Leu Glu Ser Ala Val Pro
                165                 170                 175

Leu Ala Tyr Glu Lys Gly Cys Val Pro Pro Gln Val Leu Arg Asn Leu
            180                 185                 190

Gly Pro Leu Ser Pro Ser Ser Pro Leu Ala Phe Gln Asn Gly Leu Leu
        195                 200                 205

Asn Pro Leu Arg Asp Pro Trp Asp Ser Cys Pro Ser Ala Leu Pro Trp
    210                 215                 220

Ser Asn Val Thr Thr Ala Ser Gln Thr Tyr Gly Gln Val Thr Thr Arg
225                 230                 235                 240

Thr Phe Ile Pro Asp His Ser Ala Ser Ala Ile Asp Lys Leu Glu Ala
                245                 250                 255

Val Ala Thr Ile Thr Ala Gly Tyr Gly Ala Ser Lys Pro Gln His Thr
            260                 265                 270

Asp Val Phe Ile Glu Pro Asn Gly Thr Phe Gln Ser Thr Pro Ala Gly
        275                 280                 285

Trp Ala Pro Gln Phe Tyr Asp Gly Ser Glu Ala Thr Gly Leu Leu Val
    290                 295                 300

Lys Pro Met Arg Ala Ile Ala Ser Leu Gly Glu Ala Gly Cys Gly Glu
305                 310                 315                 320

Ala Thr Ser Glu Phe Cys Thr Lys Thr Lys Pro Gly Leu Leu Lys Gly
                325                 330                 335

Gly Asp Thr Ile Thr Ser Pro Val Gly Ser Leu Leu Gly Asp Cys Lys
            340                 345                 350

Lys Ala Glu Ser Ser Met Lys Gln Val Trp Pro Gly Lys His Arg Leu
        355                 360                 365

Glu Leu Val Glu Leu Val Asp Gly Glu Asp Thr Lys Ser Ser Pro Thr
    370                 375                 380

Gln Leu Lys Arg Pro Lys His Ser Thr Asp Tyr Ala Asn Val Leu Leu
385                 390                 395                 400

Ser Asp His Ile Leu Lys Gly Ala Glu Leu Arg Ser Tyr Phe His Ser
                405                 410                 415

Gly Asp Val Gly Leu Asn Ala Ser Gln Ala Met Asp Ile Ile Val Ile
            420                 425                 430

Gly Pro Ala Leu Asn Thr Asn Gly Lys Pro Arg Ala Lys Arg Gly Ser
        435                 440                 445

Ala Thr Asp Pro Gln Ser Val Tyr Ala Arg His Arg Arg Glu Lys Ile
    450                 455                 460

Asn Glu Arg Leu Lys Asn Leu Gln Asn Leu Val Pro Asn Gly Ala Lys
465                 470                 475                 480

Val Asp Ile Val Thr Met Leu Asp Glu Ala Ile His Tyr Val Lys Phe
                485                 490                 495

Leu Gln Thr Gln Val Glu Leu Leu Lys Ser Asp Glu Phe Trp Met Phe
            500                 505                 510

Ala Asn Pro His Asn Tyr Asn Gly Ile Asp Ile Ser Asp Pro Ser Ser
        515                 520                 525

Met His Ser Pro Glu Leu Glu Ser Asn Ile
    530                 535

<210> 37
<211> 1617
<212> DNA
<213> Physcomitrella patens

<400> 37
atggtgcggt ttaactacat gtacccggtt caagagcagc tggaagccat gacggaccaa 60

cacaccccaa gcatggattc ggtctcgtcg gccggagaga agacatcctc ttgcatcgtc 120

cagcagggag gaaatgcatc cgaaacttca aacttgtggg aagaatggac acaagggtcg 180

aacggcgacg attctgtctc taccagcaac ttcctccccg aactgaattc ctccacctcc 240

agtcgtctcg cattccacca aagcgacatt ctttccactt ggatctcagg ctaccaccca 300

ctctcgcaaa gcagcctgag ttccgaattc agccacacct ccgaccgcga gaatcacccc 360

ccagcattca tgcaagaggg tttaatcccc agtggtttaa ttcttgactc tgatcctgct 420

ctcacagata tttatacgag aagcagctcc tcggactctt tgccataccc cacggctagg 480

atcatggaca aagcattgac cgatcacgag cttgagtctg ctgtcccact tgcatatgaa 540

aaaggctgcg ttcctcccca ggttctgcgt aacctagggc cattgtcacc ttcttcgcct 600

ctggcattcc agaatggact gctaaacccc ctcagggacc cttgggattc gtgtccatct 660

gcattgccat ggtcaaatgt gaccacagcc agccagactt acggtcaagt gacaaccagg 720

actttcattc cagatcactc tgcaagtgca atcgacaagt tggaggccgt cgcaacgatc 780

actgccggat acggcgcgtc gaaaccacaa catactgacg tcttcataga acccaacggg 840

acgtttcagt cgactccggc agggtgggca ccgcagtttt acgatggatc cgaggcgacg 900

ggcctgttgg tcaagccaat gagggccatc gcatctctgg gtgaagccgg ctgtggggag 960

gccactagtg aattctgcac aaagaccaag ccaggacttc tcaaaggtgg ggacacaata 1020

acctcgccgg tgggtagcct gttgggcgat tgcaaaaaag ctgagtcaag tatgaagcaa 1080

gtttggcctg gaaaacaccg tcttgaactc gtggaactag tcgatggtga agacaccaaa 1140

tcaagtccca cccagctcaa acggccgaaa cattctacgg attatgcgaa tgtcctgttg 1200

agcgatcata ttctgaaagg agcggagctg cggtcctact tccattctgg tgatgttggt 1260

ctaaatgcat ctcaagcgat ggacattatt gtaattggcc cagccttgaa tactaatggc 1320

aagccgcgag ctaaacgggg ttcagccacc gatccccaga gtgtgtacgc tagacatagg 1380

cgagaaaaaa tcaacgaacg actgaagaat ttacaaaatc tcgtgccaaa tggagccaag 1440

gttgacattg tgaccatgct agacgaagcc atacactacg tcaaattctt gcaaactcaa 1500

gttgagctgc tgaaatccga cgagttctgg atgttcgcaa atccacacaa ctacaacggc 1560

atagatatct ccgatccctc tagcatgcat tcgccggagc tggagtcgaa tatttag 1617

<210> 38
<211> 67
<212> PRT
<213> Physcomitrella patens

<400> 38

Gly Ser Ala Thr Asp Pro Gln Ser Val Tyr Ala Arg His Arg Arg Glu
1               5                   10                  15

Lys Ile Asn Glu Arg Leu Lys Thr Leu Gln Arg Leu Val Pro Asn Gly
            20                  25                  30

Glu Gln Val Asp Ile Val Thr Met Leu Glu Glu Ala Ile His Phe Val
        35                  40                  45

Lys Phe Leu Glu Phe Gln Leu Glu Leu Leu Arg Ser Asp Asp Arg Trp
    50                  55                  60

Met Phe Ala
65

<210> 39
<211> 201
<212> DNA
<213> Physcomitrella patens

<400> 39
gggtcagcta ctgatcctca gagtgtgtac gcaaggcatc gccgggagaa gattaacgag 60

cgcctaaaga cattgcagcg gttggttcct aacggagaac aggtcgacat tgtgaccatg 120

ctggaagaag ccattcactt tgtcaaattt ttggagttcc aactggagct gttgcgatcc 180

gatgatcgct ggatgttcgc c 201

<210> 40
<211> 80

<212> PRT
<213> Selaginella moelendorfii

<400> 40

Leu Asn Thr Asn Leu Lys Pro Arg Ala Lys Gln Gly Cys Ala Asn Asp
1               5                   10                  15

Pro Gln Ser Ile Ala Ala Arg Gln Arg Arg Glu Arg Ile Ser Asp Arg
            20                  25                  30

Leu Lys Ile Leu Gln Glu Leu Ile Pro Asn Gly Ser Lys Val Asp Leu
        35                  40                  45

Val Thr Met Leu Glu Lys Ala Ile Asn Tyr Val Lys Phe Leu Gln Leu
    50                  55                  60

Gln Val Lys Val Leu Met Asn Asp Glu Tyr Trp Pro Pro Lys Gly Asp
65                  70                  75                  80

<210> 41
<211> 240
<212> DNA
<213> Selaginella moelendorfii

<400> 41
ctcaacacta atcttaagcc gcgagcaaag caaggttgtg ctaatgatcc acaaagcatt     60

gctgccagac aacgaagaga acggataagt gaccggctta aaatcctgca ggagctcata    120

ccaaatggat ccaaggtcga tctggtaacc atgctggaga aggccatcaa ctacgtcaag    180

ttcttgcaat tgcaagtcaa agttcttatg aacgatgagt attggccacc aaagggagat    240

<210> 42
<211> 80
<212> PRT
<213> Selaginella moelendorfii

<400> 42

Leu Asn Thr Asn Leu Lys Pro Arg Ala Lys Gln Gly Cys Ala Asn Asp
1               5                   10                  15

Pro Gln Ser Ile Ala Ala Arg Gln Arg Arg Glu Arg Ile Ser Asp Arg
            20                  25                  30

Leu Lys Ile Leu Gln Glu Leu Ile Pro Asn Gly Ser Lys Val Asp Leu
        35                  40                  45

Val Thr Met Leu Glu Lys Ala Ile Asn Tyr Val Lys Phe Leu Gln Leu
    50                  55                  60

Gln Val Lys Val Leu Met Asn Asp Glu Tyr Trp Pro Pro Lys Gly Asp
65                  70                  75                  80

<210> 43
<211> 240
<212> DNA
<213> Selaginella moelendorfii

<400> 43
ctcaacacta atcttaagcc gcgagcaaag caaggttgtg ctaatgatcc acaaagcatt     60

gctgccagac aacgaagaga acggataagt gaccggctta aaatcctgca ggagctcata    120

ccaaatggat ccaaggtcga tctggtaacc atgttggaga aggccatcaa ctacgtcaag    180

ttcttgcaat tgcaagtcaa agttcttatg aacgatgagt attggccacc aaagggagat    240

<210> 44
<211> 76
<212> PRT
<213> Selaginella moelendorfii

<400> 44

Leu Asn Thr Asn Phe Lys Pro Arg Ala Arg Gln Gly Ser Ala Asn Asp
1               5                   10                  15

Pro Gln Ser Ile Ala Ala Arg His Arg Arg Glu Arg Ile Ser Asp Arg
            20                  25                  30

Leu Lys Ile Leu Gln Glu Leu Val Pro Asn Ser Thr Lys Val Asp Leu
        35                  40                  45

Val Thr Met Leu Glu Lys Ala Ile Asn Tyr Val Lys Phe Leu Gln Leu
    50                  55                  60

Gln Val Lys Val Leu Thr Ser Asp Asp Tyr Trp Pro
65                  70                  75

<210> 45
<211> 228
<212> DNA
<213> Selaginella moelendorfii

<400> 45
ctcaacacca atttcaagcc tcgagccagg cagggaagcg ccaatgatcc ccagagcatc     60

gctgctagac atcgccggga gaggatcagt gacaggctca agatcttgca agagctcgtt    120

ccaaacagca caaaggttga tctagtgacg atgctggaga aggccatcaa ttacgtcaag    180

ttcctccagc tgcaagttaa ggtgcttacg tcggacgact actggcca                 228

<210> 46
<211> 76
<212> PRT
<213> Selaginella moelendorfii

<400> 46

Leu Asn Thr Asn Phe Lys Pro Arg Ala Arg Gln Gly Ser Ala Asn Asp
1               5                   10                  15

Pro Gln Ser Ile Ala Ala Arg His Arg Arg Glu Arg Ile Ser Asp Arg
            20                  25                  30

Leu Lys Ile Leu Gln Glu Leu Val Pro Asn Ser Thr Lys Val Asp Leu
        35                  40                  45

Val Thr Met Leu Glu Lys Ala Ile Asn Tyr Val Lys Phe Leu Gln Leu
    50                  55                  60

Gln Val Lys Val Leu Thr Ser Asp Asp Tyr Trp Pro
65                  70                  75

<210> 47
<211> 228
<212> DNA
<213> Selaginella moelendorfii

<400> 47
ctcaacacca atttcaagcc tcgagccagg cagggaagcg ccaatgatcc ccagagcatc     60

gctgctagac atcgccggga gaggatcagt gacaggctca agatcttgca agagctcgtt    120

ccaaacagca caaaggttga tctagtgacg atgctggaga aggccatcaa ttacgtcaag    180

ttcctccagc tgcaagttaa ggtgcttacg tcggacgact attggcca                 228

<210> 48
<211> 80
<212> PRT
<213> Selaginella moelendorfii

<400> 48

Leu Asn Thr Asp Gly Lys Pro Arg Ala Lys Arg Gly Ser Ala Thr Asp
1               5                   10                  15

Pro Gln Ser Ile Tyr Ala Arg Gln Arg Arg Glu Arg Ile Asn Glu Arg
            20                  25                  30

Leu Arg Ala Leu Gln Gly Leu Val Pro Asn Gly Ala Lys Val Asp Ile
        35                  40                  45

Val Thr Met Leu Glu Glu Ala Ile Asn Tyr Val Lys Phe Leu Gln Leu
50                  55                  60

Gln Val Lys Leu Leu Ser Ser Asp Glu Tyr Trp Met Tyr Ala Pro Thr
65                  70                  75                  80

<210>  49
<211>  240
<212>  DNA
<213>  Selaginella moelendorfii

<400>  49
ctaaacaccg acggaaagcc acgcgcaaag cgtggatctg ccacggaccc gcaaagcatc     60

tacgctcggc aaagaagaga aaggatcaac gagcgtttga gagcgctaca aggactcgta    120

ccaaacggag cgaaggttga cattgtgacg atgctcgagg aagccatcaa ctatgtcaag    180

tttttgcagc tgcaagtaaa gctgctcagc tcggacgagt attggatgta cgcccccaca    240

<210>  50
<211>  80
<212>  PRT
<213>  Selaginella moelendorfii

<400>  50

Leu Asn Thr Asn Gly Lys Pro Arg Ala Lys Arg Gly Ser Ala Thr Asp
1               5                   10                  15

Pro Gln Ser Val Tyr Ala Arg His Arg Arg Glu Arg Ile Asn Glu Arg
            20                  25                  30

Leu Lys Thr Leu Gln His Leu Val Pro Asn Gly Ala Lys Val Asp Ile
        35                  40                  45

Val Thr Met Leu Glu Glu Ala Ile His Tyr Val Lys Phe Leu Gln Leu
    50                  55                  60

Gln Val Asn Met Leu Ser Ser Asp Glu Tyr Trp Ile Tyr Ala Pro Thr
65                  70                  75                  80

<210>  51
<211>  240
<212>  DNA
<213>  Selaginella moelendorfii

<400>  51
ctcaacacga atggcaagcc cagagcaaag cgtggatctg caacagatcc ccaaagcgtt     60

tacgcaaggc accggagaga gaggatcaac gagaggctca aaactttaca acaccttgtt    120

ccaaatggtg caaaggttga catagtgaca atgcttgaag aagcaataca ttacgtgaag 180

tttctacagc tgcaagtcaa catgttaagc tctgatgagt actggattta tgcacccaca 240

<210> 52
<211> 80
<212> PRT
<213> Selaginella moelendorfii

<400> 52

Leu Asn Thr Asn Gly Lys Pro Arg Ala Lys Arg Gly Ser Ala Thr Asp
1               5                   10                  15

Pro Gln Ser Val Tyr Ala Arg His Arg Arg Glu Arg Ile Asn Glu Arg
            20                  25                  30

Leu Lys Thr Leu Gln His Leu Val Pro Asn Gly Ala Lys Val Asp Ile
        35                  40                  45

Val Thr Met Leu Glu Glu Ala Ile His Tyr Val Lys Phe Leu Gln Leu
    50                  55                  60

Gln Val Asn Met Leu Ser Ser Asp Glu Tyr Trp Thr Tyr Ala Pro Thr
65                  70                  75                  80

<210> 53
<211> 240
<212> DNA
<213> Selaginella moelendorfii

<400> 53
ctcaacacga atggcaagcc ccgagcaaag cgtggatctg caacagatcc ccaaagcgtt 60

tatgcaaggc accggagaga gaggatcaac gagaggctca aaactttaca acaccttgtt 120

ccaaatggtg caaaggttga cattgtgaca atgcttgaag aagcaataca ttacgtgaag 180

tttctacagc tgcaagtcaa catgttaagc tctgatgagt actggactta tgcacccaca 240

<210> 54
<211> 80
<212> PRT
<213> Selaginella moelendorfii

<400> 54

Leu Asn Thr Asp Gly Lys Pro Arg Ala Lys Arg Gly Ser Ala Thr Asp
1               5                   10                  15

Pro Gln Ser Ile Tyr Ala Arg Gln Arg Arg Glu Arg Ile Asn Glu Arg
            20                  25                  30

Leu Arg Ala Leu Gln Gly Leu Val Pro Asn Gly Ala Lys Val Asp Ile
        35                  40                  45

Val Thr Met Leu Glu Glu Ala Ile Asn Tyr Val Lys Phe Leu Gln Leu
    50                  55                  60

Gln Val Lys Leu Leu Ser Ser Asp Glu Tyr Trp Met Tyr Ala Pro Thr
65                  70                  75                  80

<210> 55
<211> 240
<212> DNA
<213> Selaginella moelendorfii

<400> 55
ctaaacaccg acggaaagcc acgcgcaaag cgtggatctg ccacggaccc gcaaagtatc 60

tacgctcggc aaagaagaga aaggatcaac gagcgtttga gagcgctaca aggactcgta 120

ccaaacggag cgaaggttga cattgtgacg atgctcgagg aagccatcaa ctatgtcaag 180

tttttgcagc tgcaagtaaa gctgctcagc tcggacgagt attggatgta cgcccccaca 240

<210> 56
<211> 295
<212> PRT
<213> Oryza sativa

<400> 56

Met Met Ala Ala Gln Ala Ser Ser Lys Arg Gly Met Leu Leu Pro Arg
1               5                   10                  15

Glu Ala Val Leu Tyr Asp Asp Glu Pro Ser Met Pro Leu Glu Ile Leu
            20                  25                  30

Gly Tyr His Gly Asn Gly Val Gly Gly Gly Gly Cys Val Asp Ala Asp
        35                  40                  45

Tyr Tyr Tyr Ser Trp Ser Gly Ser Ser Ser Ser Ser Ser Ser Ser Val
    50                  55                  60

Leu Ser Phe Asp Gln Ala Ala Val Gly Gly Ser Gly Gly Gly Cys Ala
65                  70                  75                  80

Arg Gln Leu Ala Phe His Pro Gly Gly Asp Asp Asp Asp Cys Ala Met
                85                  90                  95

Trp Met Asp Ala Ala Ala Gly Ala Met Val Glu Asn Thr Ser Val Val

            100                 105                 110

Ala Gly Gly Gly Asn Asn Tyr Cys His Arg Leu Gln Phe His Gly Gly
        115                 120                 125

Ala Ala Gly Phe Gly Leu Ala Ser Pro Gly Ser Ser Val Val Asp Asn
    130                 135                 140

Gly Leu Glu Ile His Glu Ser Asn Val Ser Lys Pro Pro Pro Pro Ala
145                 150                 155                 160

Ala Lys Lys Arg Ala Cys Pro Ser Gly Glu Ala Arg Ala Ala Gly Lys
                165                 170                 175

Lys Gln Cys Arg Lys Gly Ser Lys Pro Asn Lys Ala Ala Ser Ala Ser
            180                 185                 190

Ser Pro Ser Pro Ser Pro Ser Pro Ser Pro Ser Pro Asn Lys Glu Gln
        195                 200                 205

Pro Gln Ser Ala Ala Ala Lys Val Arg Arg Glu Arg Ile Ser Glu Arg
    210                 215                 220

Leu Lys Val Leu Gln Asp Leu Val Pro Asn Gly Thr Lys Val Asp Leu
225                 230                 235                 240

Val Thr Met Leu Glu Lys Ala Ile Asn Tyr Val Lys Phe Leu Gln Leu
                245                 250                 255

Gln Val Lys Val Leu Ala Thr Asp Glu Phe Trp Pro Ala Gln Gly Gly
            260                 265                 270

Lys Ala Pro Glu Leu Ser Gln Val Lys Asp Ala Leu Asp Ala Ile Leu
        275                 280                 285

Ser Ser Gln His Pro Asn Lys
    290                 295

<210> 57
<211> 888
<212> DNA
<213> Oryza sativa

<400> 57
atgatggcag ctcaggcaag cagcaagcgc ggcatgctgc tgccacggga ggcggtgctc 60

tacgacgacg agccctccat gccgctggag atcttgggct accacggcaa tggcgtcggc 120

ggcggtggct gcgttgacgc cgattactac tacagctggt cggggtccag ctccagctcc 180

agctcgtcgg tgctcagctt tgaccaggcg gcggtcggcg gcagcggcgg cggctgcgcc 240

cggcagctgg ctttccatcc cggcggcgac gacgacgact gcgccatgtg gatggacgcc 300

gccgccggcg ccatggtcga gaacacgtct gtcgtcgccg gcggcggcaa caactactgt 360

catcgcctgc agttccacgg cggcgccgcc ggtttcggac tcgcgagccc aggctcgtcg 420

gtcgttgaca acggcctcga aatccacgag agcaacgtca gcaagccgcc accgccggca 480

gccaagaagc gcgcatgccc gagcggcgag gcgagagcag cggggaagaa gcagtgcagg 540

aaagggagca agccaaacaa ggctgcttct gcttcttctc cttctccttc tccttctcct 600

tctccttctc ctaacaagga acaacctcaa agcgccgctg caaaggtaag aagagagcgg 660

atcagtgaga ggctcaaagt tcttcaggat ctcgtgccta atggcacaaa ggtagacttg 720

gtcaccatgc tagaaaaggc gatcaactac gtcaaattcc tccagctgca agtgaaggtt 780

ttggctactg atgagttctg gccggcacaa ggagggaaag caccagagct ctctcaagtc 840

aaggacgcct tggacgccat cctatcttct cagcatccaa acaaatga 888

<210> 58
<211> 300
<212> PRT
<213> Oryza sativa

<400> 58

Met Arg Met Ala Leu Val Arg Glu Arg Ala Met Val Tyr Gly Gly Gly
1               5                   10                  15

Cys Asp Ala Glu Ala Phe Gly Gly Gly Phe Glu Ser Ser Gln Met Gly
            20                  25                  30

Tyr Gly His Asp Ala Leu Leu Asp Ile Asp Ala Ala Ala Leu Phe Gly
        35                  40                  45

Gly Tyr Glu Ala Ala Ala Ser Ala Gly Cys Ala Leu Val Gln Asp Gly
    50                  55                  60

Ala Ala Gly Trp Ala Gly Ala Gly Ala Ser Ser Ser Val Leu Ala Phe
65                  70                  75                  80

Asp Arg Ala Ala Gln Ala Glu Glu Ala Glu Cys Asp Ala Trp Ile Glu
                85                  90                  95

Ala Met Asp Gln Ser Tyr Gly Ala Gly Gly Glu Ala Ala Pro Tyr Arg
            100                 105                 110

Ser Thr Thr Ala Val Ala Phe Asp Ala Ala Thr Gly Cys Phe Ser Leu
        115                 120                 125

Thr Glu Arg Ala Thr Gly Gly Gly Gly Gly Ala Gly Gly Arg Gln Phe
    130                 135                 140

Gly Leu Leu Phe Pro Ser Thr Ser Gly Gly Gly Val Ser Pro Glu Arg
145                 150                 155                 160

Ala Ala Pro Ala Pro Ala Pro Arg Gly Ser Gln Lys Arg Ala His Ala
                165                 170                 175

Glu Ser Ser Gln Ala Met Ser Pro Ser Lys Lys Gln Cys Gly Ala Gly
            180                 185                 190

Arg Lys Ala Gly Lys Ala Lys Ser Ala Pro Thr Thr Pro Thr Lys Asp
        195                 200                 205

Pro Gln Ser Leu Ala Ala Lys Asn Arg Arg Glu Arg Ile Ser Glu Arg
    210                 215                 220

Leu Arg Ile Leu Gln Glu Leu Val Pro Asn Gly Thr Lys Val Asp Leu
225                 230                 235                 240

Val Thr Met Leu Glu Lys Ala Ile Ser Tyr Val Lys Phe Leu Gln Leu
                245                 250                 255

Gln Val Lys Val Leu Ala Thr Asp Glu Phe Trp Pro Ala Gln Gly Gly
            260                 265                 270

Lys Ala Pro Glu Ile Ser Gln Val Lys Glu Ala Leu Asp Ala Ile Leu
        275                 280                 285

Ser Ser Ser Ser Pro Leu Met Gly Gln Leu Met Asn
    290                 295                 300

<210> 59
<211> 903
<212> DNA
<213> Oryza sativa

<400> 59
atgcgcatgg cgctggtgcg ggagcgcgcg atggtgtacg gtggagggtg cgacgccgag 60

gcgttcggcg gcgggttcga gtcgtcccag atggggtacg gccacgacgc gctgctcgac 120

atcgacgcgg cggcgctgtt cggggggtac gaggcggccg ccagcgccgg gtgcgccctc 180

gtgcaggacg gcgccgcggg gtgggcgggc gcgggcgcgt cgtcctcggt gctggcgttc    240

gaccgcgccg ctcaggcgga ggaggccgag tgcgacgcgt ggatcgaagc catggaccag    300

agctacggcg ccggcggcga ggcggcgccg taccggtcga cgacggccgt cgccttcgac    360

gcggccaccg gctgcttcag cctgacggag agagccaccg gcggcggcgg cggcgcgggt    420

gggcggcagt tcgggctgct gttcccgagc acgtcgggcg gcggcgtctc ccccgaacgc    480

gccgcgccgg cgccggcgcc ccgcggctcg cagaagcggg cccacgcgga gtcgtcgcag    540

gccatgagcc ctagcaagaa gcagtgcggc gccggcagga aggcgggcaa ggccaagtcg    600

gcgccgacca ccccaaccaa ggacccgcaa agcctcgcgg ccaagaatcg gcgcgagagg    660

atcagcgagc ggctgcggat cctgcaggag ctcgtgccca acggcaccaa ggtcgacctc    720

gtcaccatgc tcgagaaggc catcagctac gtcaagttcc tccagcttca agtcaaggtt    780

cttgcgacgg acgagttctg gccggcgcag ggagggaagg cgccggagat atcccaggtg    840

aaggaggcgc tcgacgccat cttgtcgtcg tcgtcgccgc tgatgggaca actcatgaac    900

tga                                                                903

<210>  60
<211>  294
<212>  PRT
<213>  Oryza sativa

<400>  60

Met Ala Met Val Ala Gly Asp Glu Ala Met Ser Val Pro Trp His Asp
1               5                   10                  15

Val Gly Val Val Val Asp Pro Glu Ala Ala Gly Thr Ala Pro Phe Asp
            20                  25                  30

Ala Gly Ala Gly Tyr Val Pro Ser Tyr Gly Gln Cys Gln Tyr Tyr Tyr
        35                  40                  45

Tyr Tyr Asp Asp His His His His Pro Cys Ser Thr Glu Leu Ile His
    50                  55                  60

Ala Gly Asp Ala Gly Ser Ala Val Ala Val Ala Tyr Asp Gly Val Asp
65                  70                  75                  80

Gly Trp Val His Ala Ala Ala Ala Ala Thr Ser Pro Ser Ser Ser Ser
                85                  90                  95

Ala Leu Thr Phe Asp Gly His Gly Ala Glu Glu His Ser Ala Val Ser
            100                 105                 110

Trp Met Asp Met Asp Met Asp Ala His Gly Ala Ala Pro Pro Leu Ile
        115                 120                 125

Gly Tyr Gly Pro Thr Ala Ala Thr Ser Ser Pro Ser Ser Cys Phe Ser
    130                 135                 140

Ser Gly Gly Ser Gly Asp Ser Gly Met Val Met Val Thr Thr Thr Thr
145                 150                 155                 160

Pro Arg Ser Ala Ala Ala Ser Gly Ser Gln Arg Arg Ala Arg Pro Pro
                165                 170                 175

Pro Ser Pro Leu Gln Gly Ser Glu Leu His Glu Tyr Ser Lys Lys Gln
            180                 185                 190

Arg Ala Asn Asn Lys Glu Thr Gln Ser Ser Ala Ala Lys Ser Arg Arg
        195                 200                 205

Glu Arg Ile Ser Glu Arg Leu Arg Ala Leu Gln Glu Leu Val Pro Ser
    210                 215                 220

Gly Gly Lys Val Asp Met Val Thr Met Leu Asp Arg Ala Ile Ser Tyr
225                 230                 235                 240

Val Lys Phe Met Gln Met Gln Leu Arg Val Leu Glu Thr Asp Ala Phe
                245                 250                 255

Trp Pro Ala Ser Asp Gly Ala Thr Pro Asp Ile Ser Arg Val Lys Asp
            260                 265                 270

Ala Leu Asp Ala Ile Ile Leu Ser Ser Ser Ser Pro Ser Gln Lys Ala
        275                 280                 285

Ser Pro Pro Arg Ser Gly
    290

<210> 61
<211> 885
<212> DNA
<213> Oryza sativa

<400> 61
atggctatgg tggccggcga cgaggcgatg tcagtgccat ggcacgacgt cggcgtcgtc 60

gtcgaccccg aggcggccgg gacggcgccg ttcgacgccg gcgccggcta tgtcccatcg 120

tacggtcagt gccaatacta ctactactac gacgaccacc accaccaccc gtgcagcacg 180

gagctgatcc acgcgggcga cgctggcagt gcggttgcgg ttgcgtacga cggcgtcgac 240

ggctgggttc acgccgccgc cgcagccacc tccccgtcct cgtcatctgc gctcaccttc 300

gatggtcacg gcgccgagga gcacagcgca gtgtcgtgga tggacatgga catggacgcg 360

cacggcgccg cgcctcccct aatcggctac ggcccgacgg cggcgacctc ctccccctcc 420

tcctgcttca gctccggcgg ctccggcgac agcggcatgg tgatggtgac caccaccacc 480

ccgaggagcg ccgccgcctc tggttcgcag aggcgggcac gcccgccgcc gtcgccgttg 540

cagggatcag agctgcacga gtactccaag aagcagcgcg ccaacaacaa ggagacacag 600

agctcagctg ccaagagccg gcgggagagg atcagcgagc ggctgagggc gctgcaggag 660

ctggtgccga gcggcgggaa ggtggacatg gtgaccatgc tggacagggc catcagctac 720

gtcaagttca tgcagatgca gctcagggtg ctggagaccg acgcgttctg gccggcgtcc 780

gacggcgcca cgccggacat ctcccgggtc aaggacgcgc tcgacgccat catcctctcc 840

tcgtcctcgc cctcgcaaaa ggcttctcct cctcggtcgg gctag 885

<210> 62
<211> 324
<212> PRT
<213> Oryza sativa

<400> 62

Met Glu Asp Ser Glu Ala Met Ala Gln Leu Leu Gly Val Gln Tyr Phe
1               5                   10                  15

Gly Asn Asp Gln Glu Gln Gln Gln Pro Ala Ala Ala Ala Pro Pro Ala
            20                  25                  30

Met Tyr Trp Pro Ala His Asp Ala Ala Asp Gln Tyr Tyr Gly Ser Ala
        35                  40                  45

Pro Tyr Cys Tyr Met Gln Gln Gln Gln His Tyr Gly Cys Tyr Asp Gly
    50                  55                  60

Gly Ala Met Val Ala Gly Gly Asp Phe Phe Val Pro Glu Glu Gln Leu
65                  70                  75                  80

Val Ala Asp Pro Ser Phe Met Val Asp Leu Asn Leu Glu Phe Glu Asp
                85                  90                  95

Gln His Gly Gly Asp Ala Gly Gly Ala Gly Ser Ser Ala Ala Ala Ala
            100                 105                 110

Ala Ala Ala Thr Lys Met Thr Pro Ala Cys Lys Arg Lys Val Glu Asp

        115                 120                 125

His Lys Asp Glu Ser Cys Thr Asp Asn Val Ala Arg Lys Lys Ala Arg
    130                 135                 140

Ser Thr Ala Ala Thr Val Val Gln Lys Lys Gly Asn Lys Asn Ala Gln
145                 150                 155                 160

Ser Lys Lys Ala Gln Lys Gly Ala Cys Ser Arg Ser Ser Asn Gln Lys
                165                 170                 175

Glu Ser Asn Gly Gly Gly Asp Gly Gly Asn Val Gln Ser Ser Ser Thr
            180                 185                 190

Asn Tyr Leu Ser Asp Asp Asp Ser Leu Ser Leu Glu Met Thr Ser Cys
        195                 200                 205

Ser Asn Val Ser Ser Ala Ser Lys Lys Ser Ser Leu Ser Ser Pro Ala
    210                 215                 220

Thr Gly His Gly Gly Ala Lys Ala Arg Ala Gly Arg Gly Ala Ala Thr
225                 230                 235                 240

Asp Pro Gln Ser Leu Tyr Ala Arg Lys Arg Arg Glu Arg Ile Asn Glu
                245                 250                 255

Arg Leu Lys Ile Leu Gln Asn Leu Ile Pro Asn Gly Thr Lys Val Asp
            260                 265                 270

Ile Ser Thr Met Leu Glu Glu Ala Val His Tyr Val Lys Phe Leu Gln
        275                 280                 285

Leu Gln Ile Lys Leu Leu Ser Ser Asp Asp Met Trp Met Phe Ala Pro
    290                 295                 300

Ile Ala Tyr Asn Gly Val Asn Val Gly Leu Asp Leu Lys Ile Ser Pro
305                 310                 315                 320

Pro Gln Gln Gln

<210> 63
<211> 975
<212> DNA
<213> Oryza sativa

<400> 63

atggaggact cggaggcgat ggcgcagctg ctcggcgtgc agtacttcgg caatgaccag 60

gagcagcagc agccggcggc ggcggcgccg ccggcgatgt actggccggc gcacgacgcg 120

gccgaccagt actacggctc ggcgccatac tgctacatgc agcagcagca gcattacggg 180

tgctacgacg gcggcgcgat ggtggccggc ggcgacttct tcgtgccgga ggagcagctg 240

gtggccgacc cgagcttcat ggtggacctg aacctcgagt tcgaggacca gcacggcggc 300

gatgctggcg gcgctgggag cagcgccgcc gccgccgccg ccgccaccaa gatgacaccg 360

gcgtgcaaga ggaaggttga ggatcacaag gatgagagct gcacggacaa cgtcgcgagg 420

aagaaggcgc gctccacggc agcaacagtg gtgcagaaga agggtaataa gaacgcgcag 480

tcaaagaagg cgcagaaggg cgcgtgcagc cggagcagca accagaagga gagcaatggc 540

ggcggcgacg gcggcaatgt gcagagctcg agcaccaact acctctctga tgacgactcg 600

ctgtcgctgg agatgacttc gtgcagcaac gtgagctcgg cgtccaagaa gtcgtcgttg 660

tcatcgccgg cgaccgggca cggcggcgcg aaggcgaggg ccgggcgcgg ggcggcgacc 720

gatccgcaaa gcctctatgc caggaagagg agagaaagga tcaatgaacg gctaaagata 780

ctgcagaatc ttatcccaaa tggaaccaag gtggacatca gcacgatgct tgaagaagca 840

gttcactacg tcaagttctt gcagctccaa atcaagcttc tgagctcgga tgatatgtgg 900

atgttcgcgc cgatcgcgta caacggggtc aacgtcgggc tcgacctcaa gatctctcca 960

ccgcagcagc aatga 975

<210> 64
<211> 310
<212> PRT
<213> Oryza sativa

<400> 64
   Met Glu Ser Gly Gly Val Ile Ala Glu Ala Gly Trp Ser Ser Leu Asp
   1 5 10 15
   Met Ser Ser Gln Ala Glu Glu Ser Glu Met Met Ala Gln Leu Leu Gly
   20 25 30
   Thr Cys Phe Pro Ser Asn Gly Glu Asp Asp His His Gln Glu Leu Pro
   35 40 45
   Trp Ser Val Asp Thr Pro Ser Ala Tyr Tyr Leu His Cys Asn Gly Gly
   50 55 60
   Ser Ser Ser Ala Tyr Ser Ser Thr Thr Ser Ser Asn Ser Ala Ser Gly
   65 70 75 80
   Ser Phe Thr Leu Ile Ala Pro Arg Ser Glu Tyr Glu Gly Tyr Tyr Val
   85 90 95
   Ser Asp Ser Asn Glu Ala Ala Leu Gly Ile Ser Ile Gln Glu Gln Gly
   100 105 110
   Ala Ala Gln Phe Met Asp Ala Ile Leu Asn Arg Asn Gly Asp Pro Gly
   115 120 125
   Phe Asp Asp Leu Ala Asp Ser Ser Val Asn Leu Leu Asp Ser Ile Gly
   130 135 140
   Ala Ser Asn Lys Arg Lys Ile Gln Glu Gln Gly Arg Leu Asp Asp Gln
   145 150 155 160
   Thr Lys Ser Arg Lys Ser Ala Lys Lys Ala Gly Ser Lys Arg Gly Lys
   165 170 175
   Lys Ala Ala Gln Cys Glu Gly Glu Asp Gly Ser Ile Ala Val Thr Asn
   180 185 190
   Arg Gln Ser Leu Ser Cys Cys Thr Ser Glu Asn Asp Ser Ile Gly Ser
   195 200 205
   Gln Glu Ser Pro Val Ala Ala Lys Ser Asn Gly Lys Ala Gln Ser Gly
   210 215 220
   His Arg Ser Ala Thr Asp Pro Gln Ser Leu Tyr Ala Arg Lys Arg Arg
   225 230 235 240
   Glu Arg Ile Asn Glu Arg Leu Lys Ile Leu Gln Asn Leu Val Pro Asn
   245 250 255
   Gly Thr Lys Val Asp Ile Ser Thr Met Leu Glu Glu Ala Met His Tyr
   260 265 270
   Val Lys Phe Leu Gln Leu Gln Ile Lys Leu Leu Ser Ser Asp Glu Met
   275 280 285
   Trp Met Tyr Ala Pro Ile Ala Tyr Asn Gly Met Asn Ile Gly Ile Asp
   290 295 300
   Leu Asn Leu Ser Gln His
   305 310

<210> 65
<211> 933
<212> DNA
<213> Oryza sativa

<400> 65
atggagtccg gaggggtgat cgcggaggcg gggtggagct cgctcgacat gtcgtcgcag 60

gccgaggagt cggagatgat ggcgcagctg cttggaacct gcttcccctc caatggcgag 120

gatgatcatc accaagagct tccttggtcg gttgacaccc ccagtgccta ctacctccat 180

tgcaatggag gtagctcaag tgcatacagc tctaccacta gcagcaacag tgctagtggt 240

agcttcactc tcattgcacc aagatctgag tatgaggggt actatgtgag tgactctaat 300

gaggcggccc tcgggatcag catccaggag caaggtgcag ctcagttcat ggatgccatt 360

ctcaaccgga acggcgatcc gggcttcgat gatctcgctg actcgagcgt taatctgctg 420

gattccatcg gcgcttctaa caagagaaag attcaggagc aaggcaggct agatgaccaa 480

acgaaaagta ggaaatctgc gaagaaggct ggctcgaagc ggggaaagaa ggcggcgcaa 540

tgtgaaggtg aagatggcag cattgctgtc accaacaggc aaagcttgag ctgctgcacc 600

tctgaaaatg attcgattgg ttctcaagaa tctcctgttg ctgctaagtc gaatggcaag 660

gctcaatctg gccatcggtc agcaaccgat ccccagagcc tctatgcaag gaaaagaaga 720

gagaggatca atgagaggct caagattctg cagaaccttg taccaaatgg aaccaaagta 780

gatatcagca ctatgcttga agaggcaatg cattacgtga agttcttgca gcttcaaatc 840

aagctcctca gctctgatga aatgtggatg tacgcaccga ttgcttacaa cgggatgaac 900

atcgggatcg atttgaacct ctctcagcat tga 933

<210> 66
<211> 246
<212> PRT
<213> Oryza sativa

<400> 66

Met Asp Ala Arg Cys Ala Asn Ile Trp Ser Ser Ala Asp Ala Arg Ser
1               5                   10                  15

Glu Glu Ser Glu Met Ile Asp Gln Leu Lys Ser Met Phe Trp Ser Ser
            20                  25                  30

Thr Asp Ala Glu Ile Asn Phe Tyr Ser Pro Asp Ser Ser Val Asn Ser
        35                  40                  45

Cys Val Thr Thr Ser Thr Met Pro Ser Ser Leu Phe Leu Pro Leu Met
    50                  55                  60

Asp Asp Glu Gly Phe Gly Thr Val Gln Leu Met His Gln Val Ile Thr
65                  70                  75                  80

Gly Asn Lys Arg Met Phe Pro Met Asp Glu His Phe Glu Gln Gln Gln
                85                  90                  95

Lys Lys Pro Lys Lys Lys Thr Arg Thr Ser Arg Ser Val Ser Ser Ser
            100                 105                 110

Ser Thr Ile Thr Asp Tyr Glu Thr Ser Ser Glu Leu Val Asn Pro Ser
        115                 120                 125

Cys Ser Ser Gly Ser Ser Val Gly Glu Asp Ser Ile Ala Ala Thr Asp
    130                 135                 140

Gly Ser Val Val Leu Lys Gln Ser Asp Asn Ser Arg Gly His Lys Gln
145                 150                 155                 160

Cys Ser Lys Asp Thr Gln Ser Leu Tyr Ala Lys Arg Arg Arg Glu Arg
                165                 170                 175

Ile Asn Glu Arg Leu Arg Ile Leu Gln Gln Leu Val Pro Asn Gly Thr
            180                 185                 190

Lys Val Asp Ile Ser Thr Met Leu Glu Glu Ala Val Gln Tyr Val Lys
        195                 200                 205

Phe Leu Gln Leu Gln Ile Lys Leu Leu Ser Ser Asp Asp Thr Trp Met
    210                 215                 220

Phe Ala Pro Leu Ala Tyr Asn Gly Met Asn Met Asp Leu Gly His Thr
225                 230                 235                 240

Leu Ala Glu Asn Gln Glu
                245

<210>  67
<211>  741
<212>  DNA
<213>  Oryza sativa

<400>  67
atggatgcaa ggtgtgcaaa catctggagc tctgctgatg caaggagtga ggaatctgag      60

atgattgatc aactaaagtc catgttctgg agcagcactg atgctgaaat caacttttat     120

tctcctgaca gtagtgtaaa ttcttgtgtc acaactagca caatgcctag cagcttgttt     180

cttcctctga tggatgatga gggatttggc acagtgcaat tgatgcatca ggtcatcact 240

gggaacaaga ggatgttccc catggatgag cactttgagc agcagcagaa gaagccgaag 300

aagaaaaccc gaacttctcg ctcggtatca agtagttcaa ccattactga ctatgagact 360

agctctgaac ttgtcaatcc tagctgttcc tccgggagca gcgtcggaga ggattcaatt 420

gctgcaactg atggatctgt agtgctgaaa caaagtgaca attcaagagg ccataagcag 480

tgctccaagg atacacaaag cctctatgct aagaggagaa gggaaaggat taatgagaga 540

ctgagaatac ttcagcagct tgttcccaat ggcactaaag ttgacatcag cacaatgctg 600

gaggaagcag ttcagtatgt caagtttttg cagttgcaaa taaagctatt gagctctgac 660

gacacatgga tgtttgcgcc cctagcctat aatggcatga acatggatct cggtcatact 720

cttgctgaaa accaagaatg a 741

<210> 68
<211> 198
<212> PRT
<213> Oryza sativa

<400> 68

Met Glu Cys Ser Ser Phe Glu Ala Ile Cys Asn Glu Ser Glu Met Ile
1               5                   10                  15

Ala His Leu Gln Ser Leu Phe Trp Ser Ser Ser Asp Ala Asp Pro Cys
            20                  25                  30

Phe Gly Ser Ser Ser Phe Ser Leu Ile Ser Ser Glu Gly Tyr Asp Thr
        35                  40                  45

Met Thr Thr Glu Phe Val Asn Ser Ser Thr Asn Val Cys Phe Asp Tyr
    50                  55                  60

Gln Asp Asp Ser Phe Val Ser Ala Glu Glu Thr Thr Ile Gly Asn Lys
65                  70                  75                  80

Arg Lys Val Gln Met Asp Thr Glu Asn Glu Leu Met Thr Asn Arg Ser
                85                  90                  95

Lys Glu Val Arg Thr Lys Met Ser Val Ser Lys Ala Cys Lys His Ser
            100                 105                 110

Val Ser Ala Glu Ser Ser Gln Ser Tyr Tyr Ala Lys Asn Arg Arg Gln
        115                 120                 125

Arg Ile Asn Glu Arg Leu Arg Ile Leu Gln Glu Leu Ile Pro Asn Gly

    130                 135                 140

Thr Lys Val Asp Ile Ser Thr Met Leu Glu Glu Ala Ile Gln Tyr Val
145                 150                 155                 160

Lys Phe Leu His Leu Gln Ile Lys Leu Leu Ser Ser Asp Glu Met Trp
                165                 170                 175

Met Tyr Ala Pro Leu Ala Phe Asp Ser Gly Asn Asn Arg Leu Tyr Gln
            180                 185                 190

Asn Ser Leu Ser Gln Glu
        195

<210> 69
<211> 597
<212> DNA
<213> Oryza sativa

<400> 69
atggaatgca gctcctttga agcaatctgc aatgagtcgg agatgattgc gcatttgcag 60

tcattgttct ggagcagcag cgatgctgat ccttgttttg gtagctcatc attttctctc 120

atcagtagtg agggctacga cacaatgacc acagagtttg tgaatagcag cacaaatgta 180

tgttttgatt accaagatga tagcttcgtt tcagcagagg agactaccat tggtaacaag 240

agaaaagttc agatggatac tgagaatgag ctgatgacga accgcagcaa ggaagttcgc 300

accaagatgt cggtgtcaaa agcatgcaaa cattctgttt ctgcagagag ctcacagtct 360

tattatgcaa agaacaggag acagaggatc aatgagagat tgagaatact gcaagaactg 420

atccctaatg gaacaaaagt tgacatcagc acaatgttgg aggaagcaat tcagtatgtc 480

aagtttctac acctgcaaat caagctcttg agctctgatg aaatgtggat gtatgcgccc 540

cttgcttttg acagtggtaa caacaggctc tatcagaact ctctgtcaca agagtag 597

<210> 70
<211> 304
<212> PRT
<213> Oryza sativa

<400> 70

Met Glu Gly Gly Gly Leu Ile Ala Asp Met Ser Trp Thr Val Phe Asp
1               5                   10                  15

Leu Pro Ser His Ser Asp Glu Ser Glu Met Met Ala Gln Leu Phe Ser
            20                  25                  30

Ala Phe Pro Ile His Gly Glu Glu Glu Gly His Glu Gln Leu Pro Trp
        35                  40                  45

Phe Asp Gln Ser Ser Asn Pro Cys Tyr Tyr Ser Cys Asn Ala Ser Ser
    50                  55                  60

Thr Ala Tyr Ser Asn Ser Asn Ala Ser Ser Ile Pro Ala Pro Ser Glu
65                  70                  75                  80

Tyr Glu Gly Tyr Cys Phe Ser Asp Ser Asn Glu Ala Leu Gly Val Ser
                85                  90                  95

Ser Ser Ile Ala Pro His Asp Leu Ser Met Val Gln Val Gln Gly Ala
            100                 105                 110

Thr Glu Phe Leu Asn Val Ile Pro Asn His Ser Leu Asp Ser Phe Gly
        115                 120                 125

Asn Gly Glu Leu Gly His Glu Asp Leu Asp Ser Val Ser Gly Thr Asn
    130                 135                 140

Lys Arg Lys Gln Ser Ala Glu Gly Glu Phe Asp Gly Gln Thr Arg Gly
145                 150                 155                 160

Ser Lys Cys Ala Arg Lys Ala Glu Pro Lys Arg Ala Lys Lys Ala Lys
                165                 170                 175

Gln Thr Val Glu Lys Asp Ala Ser Val Ala Ile Pro Asn Gly Ser Cys
            180                 185                 190

Ser Ile Ser Asp Asn Asp Ser Ser Ser Ser Gln Glu Val Ala Asp Ala
        195                 200                 205

Gly Ala Thr Ser Lys Gly Lys Ser Arg Ala Gly Arg Gly Ala Ala Thr
    210                 215                 220

Asp Pro Gln Ser Leu Tyr Ala Arg Lys Arg Arg Glu Arg Ile Asn Glu
225                 230                 235                 240

Arg Leu Lys Thr Leu Gln Asn Leu Val Pro Asn Gly Thr Lys Val Asp
                245                 250                 255

Ile Ser Thr Met Leu Glu Glu Ala Val His Tyr Val Lys Phe Leu Gln
            260                 265                 270

Leu Gln Ile Lys Leu Leu Ser Ser Asp Glu Met Trp Met Tyr Ala Pro

        275                 280                 285

Ile Ala Tyr Asn Gly Met Asn Ile Gly Leu Asp Leu Asn Ile Asp Thr
    290                 295                 300

<210> 71
<211> 915
<212> DNA
<213> Oryza sativa

<400> 71
atggagggtg gaggactgat cgccgatatg agctggaccg tcttcgactt gccatcgcac 60

agcgatgagt cggagatgat ggcgcagctc ttcagtgcat tccccatcca tggtgaggag 120

gaaggccatg agcagctccc atggtttgat caatcttcca atccatgcta ctatagctgc 180

aatgctagca gcactgcata cagcaacagc aatgctagta gcattcctgc tccatctgag 240

tatgaaggat actgcttcag tgactcaaat gaggccctgg gtgtcagctc cagcattgca 300

ccacatgacc tgagcatggt ccaggtgcaa ggtgcaactg agtttctgaa tgtgatccca 360

aaccattccc ttgattcatt cggtaatggc gagctgggcc acgaggatct tgattcggtt 420

agtgggacta acaagagaaa acagtcggca gaaggagaat ttgatggcca aacaagaggt 480

tcaaaatgcg cgagaaaggc tgaaccgaag cgagcgaaga aggccaagca aactgtggag 540

aaggatgcaa gtgttgccat cccaaatggg agctgttcca tttctgacaa tgattccagt 600

tcatcccagg aggttgcaga tgctggtgct acttcgaaag gcaaatcccg ggctggccgc 660

ggagcagcca ctgatcccca gagcctctat gcaaggaaaa ggagagagag gatcaatgag 720

aggctcaaga cacttcagaa ccttgtgccc aatggcacca aagttgatat cagcaccatg 780

cttgaggagg cagtccacta tgtgaagttc ctgcagcttc agatcaagct cctcagctcc 840

gatgaaatgt ggatgtatgc gccaattgcg tacaacggga tgaacattgg gctcgatctg 900

aacattgata catga 915

<210> 72
<211> 279
<212> PRT
<213> Oryza sativa

<400> 72

Met Ala Gln Phe Leu Gly Ala His Gly Asp His Cys Phe Thr Tyr Glu
1               5                   10                  15

Gln Met Asp Glu Ser Met Glu Ala Met Ala Ala Met Phe Leu Pro Gly
            20                  25                  30

Leu Asp Thr Asp Ser Asn Ser Ser Ser Gly Cys Leu Asn Tyr Asp Val
        35                  40                  45

Pro Pro Gln Cys Trp Pro Gln His Gly His Ser Ser Ser Val Thr Ser
    50                  55                  60

Phe Pro Asp Pro Ala His Ser Tyr Gly Ser Phe Glu Phe Pro Val Met
65                  70                  75                  80

Asp Pro Phe Pro Ile Ala Asp Leu Asp Ala His Cys Ala Ile Pro Tyr
                85                  90                  95

Leu Thr Glu Asp Leu Ile Ser Pro Pro His Gly Asn His Pro Ser Ala
            100                 105                 110

Arg Val Glu Glu Ala Thr Lys Val Val Thr Pro Val Ala Thr Lys Arg
        115                 120                 125

Lys Ser Ser Ala Ala Met Thr Ala Ser Lys Lys Ser Lys Lys Ala Gly
    130                 135                 140

Lys Lys Asp Pro Ile Gly Ser Asp Glu Gly Gly Asn Thr Tyr Ile Asp
145                 150                 155                 160

Thr Gln Ser Ser Ser Ser Cys Thr Ser Glu Glu Gly Asn Leu Glu Gly
                165                 170                 175

Asn Ala Lys Pro Ser Ser Lys Lys Met Gly Thr Arg Ala Asn Arg Gly
            180                 185                 190

Ala Ala Thr Asp Pro Gln Ser Leu Tyr Ala Arg Lys Arg Arg Glu Arg
        195                 200                 205

Ile Asn Glu Arg Leu Arg Ile Leu Gln Asn Leu Val Pro Asn Gly Thr
    210                 215                 220

Lys Val Asp Ile Ser Thr Met Leu Glu Glu Ala Val Gln Tyr Val Lys
225                 230                 235                 240

Phe Leu Gln Leu Gln Ile Lys Leu Leu Ser Ser Asp Asp Thr Trp Met
                245                 250                 255

Tyr Ala Pro Ile Ala Tyr Asn Gly Val Asn Ile Ser Asn Ile Asp Leu
            260                 265                 270

Asn Ile Ser Ser Leu Gln Lys

        275

<210> 73
<211> 840
<212> DNA
<213> Oryza sativa

<400> 73
atggcgcagt ttcttggagc tcatggtgat cactgcttca cctacgagca aatggatgag 60

tccatggagg caatggcagc gatgttcttg cctggccttg acaccgactc caattcttct 120

tctggttgtc tcaactacga tgtgcctcca caatgctggc ctcagcatgg ccatagctct 180

agcgtcacca gcttccctga tccagctcat agctatggaa gctttgagtt cccggtcatg 240

gatccgttcc cgatcgccga tctcgacgcg cattgcgcca tcccctacct tactgaggat 300

ctgatcagcc ctccacatgg caaccatcca tcagcaagag tggaagaagc tacaaaggtt 360

gttacaccag tggctaccaa gaggaagtct agtgctgcca tgacggcatc aaagaagagc 420

aagaaggctg gcaaaaaaga tcctattggc agcgacgaag gcggcaacac ctacattgat 480

acgcaaagtt ctagcagttg cacctcagag gaaggaaacc tggagggcaa cgcgaagccg 540

agctcgaaga agatgggtac tagggccaac cgtggggcgg caaccgatcc ccagagtctc 600

tatgcaagga agaggagaga gaggatcaat gaaagattga ggatcctgca gaacttggtt 660

cccaatggaa caaaggttga catcagtaca atgctggagg aagcagtgca gtatgtcaaa 720

tttttgcaac ttcagattaa gttgctaagc tctgatgaca cgtggatgta tgcaccaatc 780

gcttacaatg gagtcaacat cagcaatatt gatctgaaca tctcttctct gcaaaaataa 840


<210> 74
<211> 334
<212> PRT
<213> Populus trichocarpa

<400> 74

Met Ala Leu Ala Lys Asp Arg Met Gly Ser Val Gln Thr Cys Pro Tyr
1               5                   10                  15


Asn Gly Asn Val Met Gly Asp Phe Ser Ser Met Gly Ser Tyr Gly Phe
            20                  25                  30


Asp Glu Tyr Gln Lys Val Ala Phe Tyr Glu Glu Gly Asn Ser Thr Phe
        35                  40                  45


Glu Lys Thr Ser Gly Leu Met Ile Lys Asn Leu Ala Met Thr Ser Ser
    50                  55                  60

Pro Ser Ser Leu Gly Ser Pro Ser Ser Ala Ile Ser Gly Glu Leu Val
65                  70                  75                  80

Phe Gln Ala Thr Asp His Gln Ala Glu Glu Ala His Ser Leu Ile Ser
                85                  90                  95

Phe Lys Gly Ile Gly Phe Asp Asn Ile Met His Asn Asn Gly Ser Leu
            100                 105                 110

Leu Ser Phe Glu Gln Ser Ser Arg Val Ser Gln Thr Ser Ser Gln Lys
        115                 120                 125

Asp Asp Tyr Ser Ala Trp Glu Gly Asn Leu Ser Tyr Asn Tyr Gln Trp
    130                 135                 140

Asn Glu Met Asn Pro Lys Cys Asn Thr Ser Pro Arg Leu Met Glu Asp
145                 150                 155                 160

Phe Asn Cys Phe Gln Arg Ala Gly Asn Phe Ile Ser Met Thr Gly Lys
                165                 170                 175

Glu Asn His Gly Asp Trp Leu Tyr Ala Glu Ser Thr Ile Val Ala Asp
            180                 185                 190

Ser Ile Gln Asp Ser Ala Thr Pro Asp Ala Ser Ser Phe His Lys Arg
        195                 200                 205

Pro Asn Met Gly Glu Ser Met Gln Ala Leu Lys Lys Gln Cys Asn Asn
    210                 215                 220

Ala Thr Lys Lys Pro Lys Pro Lys Ser Ala Ala Gly Pro Ala Lys Asp
225                 230                 235                 240

Leu Gln Ser Ile Ala Ala Lys Asn Arg Arg Glu Arg Ile Ser Glu Arg
                245                 250                 255

Leu Lys Val Leu Gln Asp Leu Val Pro Asn Gly Ser Lys Val Asp Leu
            260                 265                 270

Val Thr Met Leu Glu Lys Ala Ile Ser Tyr Val Lys Phe Leu Gln Leu
        275                 280                 285

Gln Val Lys Val Leu Ala Thr Asp Glu Leu Trp Pro Val Gln Gly Gly
    290                 295                 300

Lys Ala Pro Asp Ile Ser Gln Val Lys Glu Ala Ile Asp Ala Leu Leu

305                 310                 315                 320

Ser Ser Gln Thr Lys Asp Gly Asn Ser Ser Ser Ser Pro Lys
                325                 330

<210> 75
<211> 1005
<212> DNA
<213> Populus trichocarpa

<400> 75
atggcacttg ccaaggaccg tatgggatcg gttcaaactt gcccctataa tggaaatgtg 60

atgggggatt tttcctccat ggggtcttac ggatttgatg aatatcagaa ggtagcattt 120

tatgaagagg gaaatagcac ctttgagaaa accagtgggc ttatgatcaa gaatttagct 180

atgacctctt ctccttcttc tcttggcagt ccgagcagcg cgatttctgg tgaattagtg 240

tttcaggcta ctgaccatca agctgaggaa gctcattctt tgatcagctt caaaggtatc 300

ggattcgata acatcatgca taataatgga tctttgctta gctttgagca aagtagtagg 360

gtttctcaaa ctagtagcca gaaagatgac tactcagcct gggagggtaa tttgagttac 420

aactaccagt ggaacgaaat gaatccaaaa tgtaacacaa gtcctcggtt gatggaagat 480

tttaattgct ttcaaagagc tggcaacttc atttccatga ctggaaagga aaatcatggt 540

gattggttat acgctgaatc cacaattgtt gctgatagca ttcaggattc tgcaacacca 600

gatgccagca gcttccataa gcgtcctaat atgggagaga gtatgcaggc tctaaagaag 660

caatgcaaca atgcaacaaa aaagccaaaa ccgaagtccg cagcaggtcc agctaaggat 720

ctacagagta ttgctgccaa gaatcgacga gagaggatta gcgagaggct taaggtattg 780

caggatttag tccctaatgg ctcaaaggtt gatttggtta ctatgctaga gaaagccatt 840

agttatgtta agtttcttca attgcaagta aaggtgttag ccactgatga attatggcca 900

gttcaaggtg gtaaagctcc tgatatttct caagtaaagg aagccatcga tgccctactc 960

tcatctcaga ctaaagacgg aaactcaagc tcaagcccaa agtaa 1005

<210> 76
<211> 330
<212> PRT
<213> Populus trichocarpa

<400> 76

Met Ala Leu Ala Lys Asp Arg Met Asp Ser Val Gln Thr Cys Ala Leu
1               5                   10                  15

Tyr Gly Asn Val Met Gly Asp Leu Ser Ser Leu Gly Pro Asn Tyr Arg
            20                  25                  30

Phe Asp Glu Glu Gly Asp Arg Asn Phe Glu Lys Asn Ser Ala Leu Met
        35                  40                  45

Ile Lys Asn Leu Ala Met Ser Pro Ser Pro Pro Ser Leu Gly Ser Pro
    50                  55                  60

Ser Ser Ala Asn Ser Gly Glu Leu Val Phe Gln Ala Thr Asp Asn Gln
65                  70                  75                  80

Val Glu Glu Ala His Ser Leu Ile Asn Phe Lys Gly Thr Gly Phe Asp
                85                  90                  95

Ser Ile Met His Ala Asn Gly Ser Leu Ile Ser Phe Glu Gln Ser Asn
            100                 105                 110

Arg Val Ser Gln Thr Ser Ser His Lys Asp Asp Tyr Ser Ala Trp Glu
        115                 120                 125

Gly Asn Leu Ser Cys Asn Tyr Gln Trp Asn Gln Ile Asn Pro Lys Cys
    130                 135                 140

Asn Ala Asn Pro Arg Leu Met Glu Asp Leu Asn Cys Tyr Gln Ser Ala
145                 150                 155                 160

Ser Asn Phe Asn Ser Ile Thr Asn Ser Ala Glu Lys Glu Asn His Gly
                165                 170                 175

Asp Trp Leu Tyr Thr His Glu Ser Thr Ile Val Thr Asp Ser Ile Pro
            180                 185                 190

Asp Ser Ala Thr Pro Asp Ala Ser Ser Phe His Lys Arg Pro Asn Met
        195                 200                 205

Gly Glu Ser Met Gln Ala Leu Lys Lys Gln Arg Asp Ser Ala Thr Lys
    210                 215                 220

Lys Pro Lys Pro Lys Ser Ala Gly Pro Ala Lys Asp Pro Gln Ser Ile
225                 230                 235                 240

Ala Ala Lys Asn Arg Arg Glu Arg Ile Ser Glu Arg Leu Lys Met Leu
                245                 250                 255

Gln Asp Leu Val Pro Asn Gly Ser Lys Val Asp Leu Val Thr Met Leu
            260                 265                 270

Glu Lys Ala Ile Ser Tyr Val Lys Phe Leu Gln Leu Gln Val Lys Val
        275                 280                 285

Leu Ala Thr Asp Glu Phe Trp Pro Val Gln Gly Gly Lys Ala Pro Asp
    290                 295                 300

Ile Ser Gln Val Lys Gly Ala Ile Asp Ala Thr Leu Ser Ser Gln Thr
305                 310                 315                 320

Lys Asp Arg Asn Ser Asn Ser Ser Ser Lys
                325                 330

<210> 77
<211> 993
<212> DNA
<213> Populus trichocarpa

<400> 77
atggcacttg ccaaggaccg tatggattcg gttcaaactt gcgcccttta tggaaatgtg 60

atgggggatc tttcctcctt ggggcctaat tatagatttg atgaagaggg agataggaac 120

tttgagaaaa atagtgcgct tatgatcaag aatttagcta tgagcccttc tcctccttct 180

cttggcagtc caagcagtgc aaattctggt gaactagtgt ttcaggctac tgacaatcaa 240

gttgaggaag ctcattcttt gatcaacttc aaaggtaccg gatttgatag tatcatgcat 300

gctaatggat ctttgattag ctttgagcaa agtaataggg tttctcaaac tagtagtcac 360

aaagatgact actctgcttg ggagggtaat ttgagttgca attaccagtg gaaccaaatc 420

aatccaaaat gtaacgcaaa tcctcggttg atggaagatc ttaattgcta tcaaagtgca 480

agcaacttca actccataac caacagtgct gaaaaggaaa accatggtga ttggttatac 540

actcatgaat ccacaattgt tactgatagc attcccgatt ctgcaacacc agatgccagc 600

agcttccata agcgtcccaa tatgggagag agtatgcagg ctctaaagaa gcaacgcgac 660

agcgccacaa aaaagccgaa acccaagtct gctggtccag ctaaggatcc acaaagtatt 720

gctgccaaga atcgacgaga gcggattagc gagcgcctta agatgttgca ggatttagtc 780

cctaacggct ccaaggttga tttggttact atgctagaga aagccattag ttatgttaag 840

tttcttcaat tgcaagtaaa ggtgttggcc actgatgaat tctggccagt tcaaggtggt 900

aaagctcctg atatttctca agtaaaggga gccattgatg ccacactctc atctcagact 960

aaagacagaa attcaaactc aagctcaaag tga 993

<210> 78
<211> 354
<212> PRT

<213>  Populus trichocarpa

<400>  78

Met Ala Glu Gly Glu Trp Ser Ser Leu Gly Gly Met Tyr Thr Ser Glu
1               5                   10                  15

Glu Ala Asp Phe Met Ala Gln Leu Leu Gly Asn Cys Pro Asn Gln Val
            20                  25                  30

Asp Ser Ser Ser Asn Phe Gly Val Pro Ser Ser Phe Trp Pro Asn His
        35                  40                  45

Glu Pro Thr Thr Asp Met Glu Gly Ala Asn Glu Cys Leu Phe Tyr Ser
    50                  55                  60

Leu Asp Phe Ala Asn Ile Asn Leu His His Phe Ser Gln Gly Ser Ser
65                  70                  75                  80

Ser Tyr Ser Gly Gly Ser Gly Ile Leu Phe Pro Asn Thr Ser Gln Asp
                85                  90                  95

Ser Tyr Tyr Met Ser Asp Ser His Pro Ile Leu Ala Asn Asn Asn Ser
            100                 105                 110

Ser Met Ser Met Asp Phe Cys Met Gly Asp Ser Tyr Leu Val Glu Gly
        115                 120                 125

Asp Asp Cys Ser Asn Gln Glu Met Ser Asn Ser Asn Glu Glu Pro Gly
    130                 135                 140

Gly Asn Gln Thr Val Ala Ala Leu Pro Glu Asn Asp Phe Arg Ala Lys
145                 150                 155                 160

Arg Glu Pro Glu Met Pro Ala Ser Glu Leu Pro Leu Glu Asp Lys Ser
                165                 170                 175

Ser Asn Pro Pro Gln Ile Ser Lys Lys Arg Ser Arg Asn Ser Gly Asp
            180                 185                 190

Ala Gln Lys Asn Lys Arg Asn Ala Ser Ser Lys Lys Ser Gln Lys Val
        195                 200                 205

Ala Ser Thr Ser Asn Asn Asp Glu Gly Ser Asn Ala Gly Leu Asn Gly
    210                 215                 220

Pro Ala Ser Ser Gly Cys Cys Ser Glu Asp Glu Ser Asn Ala Ser His

225                 230                 235                 240

Glu Leu Asn Arg Gly Ala Ser Ser Ser Leu Ser Ser Lys Gly Thr Ala
            245                 250                 255

Thr Leu Asn Ser Ser Gly Lys Thr Arg Ala Ser Arg Gly Ala Ala Thr
        260                 265                 270

Asp Pro Gln Ser Leu Tyr Ala Arg Lys Arg Arg Glu Arg Ile Asn Glu
    275                 280                 285

Arg Leu Arg Ile Leu Gln Thr Leu Val Pro Asn Gly Thr Lys Val Asp
    290                 295                 300

Ile Ser Thr Met Leu Glu Glu Ala Val Gln Tyr Val Lys Phe Leu Gln
305                 310                 315                 320

Leu Gln Ile Lys Leu Leu Ser Ser Glu Asp Leu Trp Met Tyr Ala Pro
            325                 330                 335

Ile Ala Tyr Asn Gly Met Asp Ile Gly Leu Asp His Leu Lys Val Thr
        340                 345                 350

Ala Pro

<210>  79
<211>  1065
<212>  DNA
<213>  Populus trichocarpa

<400>  79
atggcagagg gagagtggag ttctcttggt ggaatgtaca ctagtgagga ggctgatttc     60

atggcacagt tgcttggtaa ctgtcctaat caggttgatt caagttcaaa ctttggagtt    120

ccatctagtt tctggcctaa ccacgaacca acaacggaca tggaaggggc taatgaatgt    180

ttattttatt ctttggattt tgctaatatt aatttgcacc atttttcaca agggagtagt    240

agttatagtg gtggcagtgg cattcttttt cccaacacaa gccaagatag ctactacatg    300

agtgattctc atccaatttt ggctaacaat aatagctcaa tgtcaatgga tttttgcatg    360

ggagactcat atctcgttga aggcgatgac tgctcaaacc aagaaatgag caatagcaat    420

gaggagcctg gtggaaacca gactgtagct gctcttcctg aaaacgattt tcgggccaag    480

agagaaccag agatgccagc ttctgaacta cccctggaag acaaaagcag caacccacct    540

cagatttcta agaaaagatc acgaaattca ggagatgctc aaaagaacaa gaggaatgca    600

agttcaaaga agagccagaa ggttgcctcg actagcaaca atgatgaagg aagtaatgct 660

ggccttaatg ggcctgcctc aagcggttgc tgctcagagg atgaatccaa tgcctctcat 720

gagctcaata gaggagcgag ttcaagtttg agctcgaaag ggactgcaac tctcaactca 780

agtggcaaaa caagagccag caggggggca gccactgatc cccagagtct ctatgcaagg 840

aaaagaagag aaagaataaa tgagaggctg agaattctac aaacccttgt ccccaacgga 900

acaaaggttg acattagcac aatgcttgaa gaagctgtcc agtatgtgaa gtttttgcaa 960

ctccaaatta agctgctaag ctctgaggac ttgtggatgt atgcgcctat cgcttacaac 1020

gggatggaca tcggtcttga tcatctgaag gttaccgcac catga 1065

<210> 80
<211> 317
<212> PRT
<213> Populus trichocarpa

<400> 80

Met Glu Pro Ile Gly Ala Thr Ala Glu Gly Glu Trp Ser Ser Leu Ser
1               5                   10                  15

Gly Met Tyr Thr Ser Glu Glu Ala Asp Phe Met Glu Gln Leu Leu Val
            20                  25                  30

Asn Cys Pro Pro Asn Gln Val Asp Ser Ser Ser Ser Phe Gly Val Pro
        35                  40                  45

Ser Ser Phe Trp Pro Asn His Glu Ser Thr Met Asn Met Glu Gly Ala
    50                  55                  60

Asn Glu Cys Leu Leu Tyr Ser Leu Asp Ile Ala Asp Thr Asn Leu Tyr
65                  70                  75                  80

His Phe Ser Gln Val Ser Ser Gly Tyr Ser Gly Glu Leu Ser Asn Gly
                85                  90                  95

Asn Val Glu Glu Ser Gly Gly Asn Gln Thr Val Ala Ala Leu Pro Glu
            100                 105                 110

Pro Glu Ser Asn Leu Gln Pro Lys Arg Glu Ser Lys Met Pro Ala Ser
        115                 120                 125

Glu Leu Pro Leu Glu Asp Lys Ser Arg Lys Pro Pro Glu Asn Ser Lys
    130                 135                 140

Lys Arg Ser Arg Arg Thr Gly Asp Ala Gln Lys Asn Lys Arg Asn Val

145                150                155                160

Arg Ser Lys Lys Ser Gln Lys Val Ala Ser Thr Gly Asn Asn Asp Glu
            165                170                175

Glu Ser Asn Gly Gly Leu Asn Gly Pro Val Ser Ser Gly Cys Cys Ser
        180                185                190

Glu Asp Glu Ser Asn Ala Ser Gln Glu Leu Asn Gly Gly Ala Ser Ser
    195                200                205

Ser Leu Ser Ser Lys Gly Thr Thr Thr Leu Asn Ser Ser Gly Lys Thr
    210                215                220

Arg Ala Ser Lys Gly Ala Ala Thr Asp Pro Gln Ser Leu Tyr Ala Arg
225                230                235                240

Lys Arg Arg Glu Arg Ile Asn Glu Arg Leu Arg Ile Leu Gln Asn Leu
            245                250                255

Val Pro Asn Gly Thr Lys Val Asp Ile Ser Thr Met Leu Glu Glu Ala
        260                265                270

Val Gln Tyr Val Lys Phe Leu Gln Leu Gln Ile Lys Leu Leu Ser Ser
    275                280                285

Glu Asp Leu Trp Met Tyr Ala Pro Ile Ala Tyr Asn Gly Met Asp Ile
    290                295                300

Gly Leu Asp His Leu Lys Leu Thr Thr Pro Arg Arg Leu
305                310                315

<210>  81
<211>  954
<212>  DNA
<213>  Populus trichocarpa

<400>  81
atggagccta ttggagccac tgcggaggga gagtggagtt ctcttagtgg aatgtacaca      60

agtgaggagg ctgatttcat ggaacagttg cttgtcaact gtcctcctaa tcaggttgat     120

tcaagttcaa gctttggagt tccatctagt tttggcctaa accatgaatc aacaatgaac     180

atggaagggg ccaatgaatg tttattgtat tctttggata ttgctgatac taatctgtac     240

catttttcac aagtgagcag tggttatagt ggtgaattga gcaatggaaa tgtggaagag     300

tctggtggaa accagactgt agctgctctt cctgaacctg aaagcaattt gcaacccaag     360

agagaatcaa agatgccagc atctgaacta cccctggaag ataaaagcag aaagccacct 420

gagaattcca agaaaagatc acgacgtacg ggagatgccc aaaagaacaa gaggaatgta 480

aggtcaaaga agagccagaa ggttgcctcg actggcaaca atgatgaaga aagcaatggt 540

ggccttaatg gtcctgtctc aagcggttgc tgctcagagg atgaatccaa tgcctcccag 600

gagctcaatg gaggagcgag ttcaagtttg agctcaaaag ggacaacaac tctcaactca 660

agtggcaaaa caagagccag taagggggca gccactgatc cccagagcct ctatgcaagg 720

aaaagaagag aaagaataaa tgagaggctg agaattctac aaaaccttgt ccccaatgga 780

acaaaggttg acattagcac aatgcttgaa gaggctgtcc agtatgtgaa gtttttgcaa 840

ctccaaatta agctgctaag ctctgaagac ctgtggatgt atgctcctat cgcgtacaat 900

ggtatggaca tcggtcttga tcatctgaag cttaccacac caagacgatt gtag 954

<210> 82
<211> 362
<212> PRT
<213> Populus trichocarpa

<400> 82

Met Asn Thr Gln Ala Met Glu Ala Phe Arg Asp Gly Glu Leu Trp Asn
1               5                   10                  15

Phe Ser Arg Met Phe Ser Met Glu Glu Pro Asp Cys Thr Pro Glu Leu
            20                  25                  30

Leu Gly Gln Cys Ser Phe Leu Gln Asp Thr Asp Glu Gly Leu His Phe
        35                  40                  45

Thr Ile Pro Ser Ala Phe Phe Pro Ala Pro Glu Ser Asp Ala Ser Met
    50                  55                  60

Ala Glu Asp Glu Ser Leu Phe Tyr Ser Trp His Thr Pro Asn Pro Asn
65                  70                  75                  80

Leu His Phe Asp Ser Gln Glu Ser Ser Asn Asn Ser Asn Ser Ser Ser
                85                  90                  95

Ser Val Phe Leu Pro Tyr Ser Ser His Glu Ser Tyr Phe Phe Asn Asp
            100                 105                 110

Ser Asn Pro Ile Gln Ala Thr Asn Asn Asn Ser Met Ser Met Asp Ile
        115                 120                 125

Met Asp Glu Glu Asn Ile Gly Leu Phe Met Pro Leu Phe Pro Glu Ile

    130                 135                 140

Ala Met Ala Glu Thr Ala Cys Met Asn Gly Asp Met Ser Gly Asp Lys
145                 150                 155                 160

Thr Gly Asp Leu Asp Asp Asn Leu Lys Pro Ala Ala Asn Asp Val Leu
                165                 170                 175

Ala Lys Gly Leu Gln Leu Lys Arg Lys Leu Asp Val Pro Glu Pro Ile
            180                 185                 190

Ala Asn Thr Leu Asp Asp Met Lys Lys Lys Ala Arg Val Thr Arg Asn
        195                 200                 205

Val Gln Lys Thr Arg Lys Val Gly Gln Ser Lys Lys Asn Gln Lys Asn
    210                 215                 220

Ala Pro Asp Ile Ser His Asp Glu Glu Glu Ser Asn Ala Gly Pro Asp
225                 230                 235                 240

Gly Gln Ser Ser Ser Ser Cys Ser Ser Glu Glu Asp Asn Ala Ser Gln
                245                 250                 255

Asp Ser Asp Ser Lys Val Ser Gly Val Leu Asn Ser Asn Gly Lys Thr
            260                 265                 270

Arg Ala Thr Arg Gly Ala Ala Thr Asp Pro Gln Ser Leu Tyr Ala Arg
        275                 280                 285

Lys Arg Arg Glu Arg Ile Asn Glu Arg Leu Lys Ile Leu Gln Asn Leu
    290                 295                 300

Val Pro Asn Gly Thr Lys Val Asp Ile Ser Thr Met Leu Glu Glu Ala
305                 310                 315                 320

Val His Tyr Val Asn Phe Leu Gln Leu Gln Ile Lys Leu Leu Ser Ser
                325                 330                 335

Asp Asp Leu Trp Met Tyr Ala Pro Leu Ala Tyr Asn Gly Ile Asp Ile
            340                 345                 350

Gly Leu Asn Gln Lys Leu Ser Met Phe Leu
        355                 360

<210> 83
<211> 1089

<212> DNA
<213> Populus trichocarpa

<400> 83
atgaatacgc aggctatgga agcctttcgt gatggagaat tatggaactt cagcagaatg 60

ttctccatgg aagagcctga ttgcacccca gaattacttg gtcagtgctc ttttcttcag 120

gatactgatg aaggattgca ttttacaatc ccatcagctt tcttccctgc tcctgaatcc 180

gacgcgagca tggctgagga cgagagtttg ttttattctt ggcatactcc caaccccaat 240

ttgcattttg attctcaaga aagtagtaat aacagtaatt ctagcagtag tgtatttctt 300

ccctattcca gccatgaatc ctacttcttc aatgattcta atcccattca agctacgaac 360

aataactcta tgtccatgga tattatggat gaggaaaata ttggcttgtt tatgccactt 420

tttcctgaaa ttgcaatggc agaaactgcc tgtatgaatg gagatatgag cggtgacaaa 480

acaggagatt tagatgataa tctgaagcca gcagctaatg atgttctggc caagggattg 540

cagctcaaaa ggaagcttga tgttccagaa ccaatagcca acacattgga cgacatgaag 600

aaaaaagccc gggttacaag aaatgtgcaa aagactagga aggttggaca gtcaaaaaaa 660

aatcagaaga acgcaccaga tattagccat gatgaagaag agagtaatgc tggaccagac 720

ggacaaagtt ccagcagttg tagttcagaa gaggacaatg cctctcagga ttctgattcc 780

aaggtttctg gagttctcaa ttccaatgga aaaacaagag ctactagggg agctgccaca 840

gacccccaga gcctttatgc aaggaaaaga agggagagga taaacgagag actgaaaatc 900

ttgcagaatc ttgtccctaa cggaaccaag gttgatatca gcacgatgct agaagaggca 960

gtccattacg taaacttttt gcagcttcaa atcaagcttt tgagctcgga tgatctatgg 1020

atgtatgcac ctctggctta caatggaata gatattggac tcaaccagaa gctctctatg 1080

tttctatga 1089

<210> 84
<211> 302
<212> PRT
<213> Musa acuminata

<400> 84

Met Ala Gln Glu Ser Thr Trp Ser Ser Phe Asp Ala Thr Met Leu Ala
1               5                   10                  15

Glu Glu Glu Ser Arg Met Ile Ala Gln Leu Leu Ser Asn Tyr Gln Cys
            20                  25                  30

Phe Gly Glu Gln Asp Arg Asp Val Gly Cys Cys Glu Leu Pro Pro Ser
        35                  40                  45

Ser Cys Cys Ser Ser His Ala Ala Asp Ser Cys Tyr Cys Trp Ser Ala
50                  55                  60

Asn Glu Asn Ser Asn Pro Gly Leu Cys Tyr Trp Ser Gln Ser Gly Asp
65                  70                  75                  80

Glu Ser Asp Gly Ala His Ala Ile Gly Thr Val Pro Val Phe Thr Asn
                85                  90                  95

His Cys Leu Val Gly Asp Gln Val Ala Val Asn Gln Thr Leu Ser Ile
            100                 105                 110

His Glu Pro Thr Ala Ala His Ala Glu Met Pro Lys Arg Lys Ile Glu
        115                 120                 125

Ser His Ala Ser Glu Asp Asp Phe Arg Arg Gln Ser Ser Lys Lys Lys
    130                 135                 140

Leu Gln Ala Pro Thr Asn Ala Leu Lys Ser Val Lys Lys Ala Arg Pro
145                 150                 155                 160

Gly Arg Asn Gln Lys Ser Ile Val Cys Gly Asp Glu Glu Glu Asn Asn
                165                 170                 175

Ala Arg Ser Ser Gly Arg Ser Cys Cys Ser Tyr Ser Ser Glu Glu Asp
            180                 185                 190

Ser Gln Ala Phe Gln Ala Asp Leu Asn Ala Lys Thr Arg Ser Asn Arg
        195                 200                 205

Trp Pro Ala Thr Asp Pro Gln Ser Leu Tyr Ala Lys Gln Arg Arg Glu
    210                 215                 220

Arg Ile Asn Ala Arg Leu Arg Thr Leu Gln Asn Leu Val Pro Asn Gly
225                 230                 235                 240

Thr Lys Val Asp Ile Ser Thr Met Leu Glu Glu Ala Val Arg Tyr Val
                245                 250                 255

Lys Phe Leu Gln Leu Gln Ile Lys Leu Leu Ser Ser Asp Glu Leu Trp
            260                 265                 270

Met Tyr Ala Pro Val Val His Ser Gly Met Ile Asp Gly Gln Val Asn
        275                 280                 285

Ser Glu Ile Phe Val Ser Ala Asn Thr Arg Asn Glu Trp Phe
    290                 295                 300

<210> 85
<211> 909
<212> DNA
<213> Musa acuminata

<400> 85
atggctcagg agtcaacttg gagctcgttt gatgctacaa tgcttgctga ggaggagtcc 60

cgaatgatcg cacaattgct cagcaactac cagtgttttg gcgagcaaga tcgagatgtt 120

ggatgctgtg aactcccgcc atcgtcttgt tgttcttctc atgcagctga ttcatgttac 180

tgttggtcag caaatgagaa cagtaacccg ggtttgtgct actggtctca gagtggagat 240

gaatccgatg gagcacatgc aatcggcact gtgccggtct tcacgaacca ttgcttggtg 300

ggagatcaag tcgctgtgaa tcaaactttg agcattcacg aacctactgc tgctcatgca 360

gagatgccaa agcgcaagat agagtctcat gcttctgaag atgatttccg tcgtcaaagt 420

tctaagaaaa agcttcaggc tccgacgaat gctctgaaga gcgtgaagaa ggcacgacct 480

gggaggaacc agaagagcat tgtgtgtggt gatgaggaag agaacaatgc caggagcagt 540

ggccggagtt gctgcagcta cagctctgag gaagactcac aagctttcca ggctgatctt 600

aatgcaaaaa cacgatcgaa tcgatggcca gccacagatc ctcaaagcct ctatgcaaag 660

caaagaaggg aaagaatcaa tgctagattg aggacattgc agaacctggt gcctaatgga 720

actaaagttg acattagcac aatgctcgaa gaagctgttc gttacgtcaa gttcttgcag 780

ctgcagataa agcttttgag ctcggatgag ctgtggatgt acgctcctgt tgtccacagt 840

gggatgattg atggccaagt caactcagag atatttgtgt ctgcaaatac tcgtaatgag 900

tggttctga 909

<210> 86
<211> 366
<212> PRT
<213> Medicago truncatula

<400> 86

Met Glu Pro Ile Gly Thr Phe Pro Glu Gly Glu Trp Asp Phe Phe Arg
1               5                   10                  15

Lys Met Phe Ala Ser Glu Asp His Glu Tyr Tyr Ser Gln Gln Phe Leu
            20                  25                  30

Asp Gln Asn Ser Leu Leu Leu Gly Glu Asn Asp Gly Leu Asn Asn Gly
        35                  40                  45

Thr Gln Ser Thr Phe Cys Thr Ala Glu Ile Gly Glu Asn Glu Arg Met
50                  55                  60

Phe Tyr Ser Phe Asp His Ala His Ile Gln Asn Ser Asn Tyr Ile Pro
65                  70                  75                  80

Gln Thr Gln Glu Asn Ser Tyr Asn Ser Asn Ser Ser Ala Ser Asp Asp
                85                  90                  95

Thr Asn Tyr Tyr Phe Ser Tyr Pro Asn His Val Leu Glu Asn Asn Ile
            100                 105                 110

Asn Asn Cys Ile Ser Asn Asp Phe Arg Met Asp Glu Asn Leu Phe Ala
        115                 120                 125

Ser Ser Val Pro Ser Leu Asn Glu Ile Val Met Glu Glu Asn Val Arg
    130                 135                 140

Met Asn Glu Asp Ser Ala Ser Asp Asp His Ile Val Glu Lys Asn Gly
145                 150                 155                 160

Tyr Asn Thr Gln Ile Met Glu Pro Phe Asp Leu His Thr Lys His Glu
                165                 170                 175

Met Gln Met Lys Leu Lys Arg Lys Leu Asp Val Ile Glu Val Glu Val
            180                 185                 190

Pro Val Glu Glu Lys Ile Asn Asn Asn Pro Lys Lys Lys Pro Arg Val
        195                 200                 205

Ser Asn Asp Gly Gln Gly Cys Met Lys Asn Ala Arg Ser Lys Lys Asn
    210                 215                 220

His Lys Val Ile Ala Ser His Glu Glu Glu Met Thr Glu Glu Ile Asn
225                 230                 235                 240

Arg Gly Ser Asn Gly Asn Ser Ser Ser Ser Asn Ile Ser Glu Asp Asp
                245                 250                 255

Asn Ala Ser Gln Glu Asn Ser Gly Gly Thr Thr Leu Asn Ser Asn Gly
            260                 265                 270

Lys Thr Arg Ala Ser Arg Gly Ser Ala Thr Asp Pro Gln Ser Leu Tyr
        275                 280                 285

Ala Arg Lys Arg Arg Glu Arg Ile Asn Glu Arg Leu Arg Val Leu Gln
290                 295                 300

Asn Leu Val Pro Asn Gly Thr Lys Val Asp Ile Ser Thr Met Leu Glu
305                 310                 315                 320

Glu Ala Val Asn Tyr Val Lys Phe Leu Gln Thr Gln Ile Lys Leu Leu
                325                 330                 335

Ser Ser Asp Asp Met Trp Met Tyr Ala Pro Leu Ala Tyr Asn Gly Leu
            340                 345                 350

Asp Leu Gly Leu Asn Leu Asn Leu Asn Ser Ser Leu Pro Leu
        355                 360                 365

<210> 87
<211> 1101
<212> DNA
<213> Medicago truncatula

<400> 87
atggaaccta taggtacttt ccctgaagga gaatgggatt tctttcgcaa aatgtttgca     60

agtgaagatc atgaatatta ctcacaacaa tttcttgatc aaaattcact tcttctaggg    120

gaaaatgatg ggttgaacaa tggaacacag tccacatttt gcactgctga aattggtgaa    180

aatgagcgta tgttttattc ttttgatcat gctcatatcc aaaactctaa ctatattcct    240

caaactcaag agaatagtta caatagcaat tctagtgcta gtgatgatac aaattactat    300

tttagttatc ctaatcatgt actagaaaat aatattaata attgtatatc caatgatttt    360

cgcatggatg agaatttgtt tgcttcttct gttccatccc ttaatgagat tgtaatggaa    420

gagaatgtga gaatgaatga agattctgca agtgatgatc atattgtgga gaaaaatggt    480

tacaatactc aaataatgga accttttgat cttcacacca agcatgagat gcaaatgaag    540

ctcaaaagga aacttgatgt gatagaagtg gaggttcccg ttgaagaaaa aattaacaac    600

aatccgaaga aaaaacctcg tgtttcgaat gatggccaag gatgcatgaa aaatgcaagg    660

tcaaagaaga accacaaagt tattgctagc catgaagagg agatgacaga agagattaat    720

agaggatcaa atggaaatag ttctagtagt aacatttctg aggatgataa tgcttctcaa    780

gaaaatagtg gaggaactac tctcaactca aatgggaaga caagagctag tagaggatct    840

gcaacagatc cccaaagtct atatgcaagg aaaagaagag agagaataaa tgaacgacta    900

agagtcttac aaaatcttgt accaaacgga acaaaggttg atatcagtac aatgcttgaa    960

gaggcagtca attatgtgaa atttttacag actcaaatca agcttttgag ctctgatgat   1020

atgtggatgt atgcaccact tgcttacaat ggacttgacc ttggactcaa tctcaacctc   1080

aacagctctc taccactatg a                                    1101

<210> 88
<211> 258
<212> PRT
<213> Glycine max

<220>
<221> misc_feature
<222> (1)..(1)
<223> Xaa is unknown

<400> 88

Xaa Phe Leu Cys Phe Ser Gln Gly Ser Ser Ser Ser Thr Asp Asn Ser
1               5                   10                  15

Gly Asn Asn Ile Phe Ser Ile Thr Ser Ser Gly Ala Tyr Ser Cys Asp
            20                  25                  30

Pro Glu Ala Asn Phe Asp Ser Val Ser Met Val Leu Cys Leu Gly Asp
        35                  40                  45

Ala Lys Phe Ser Pro His Ser Phe Gln Cys Asp Asp Asn Ser Asn Gln
    50                  55                  60

Gln Ile Asn Glu Asn Thr Asp Glu Glu Ser Ser Leu Asp Pro Trp Lys
65                  70                  75                  80

Leu Ala Ile Ala Asp Asn Asn Leu Gln Ala Lys Arg Glu Tyr Glu Met
                85                  90                  95

Met Val Ser Glu Pro Val Glu Val Asp Arg Ser Arg Asn Leu Glu Asn
            100                 105                 110

Leu Ala Lys Arg Leu Lys Ser Ser Ile Glu Val Ser Lys Thr Leu Arg
        115                 120                 125

Ser Ala Lys Ser Gly Lys Asn Ser Lys Ser Ala Ser Val Ser Asn Asp
    130                 135                 140

Glu Asp Asp Arg Ser Leu Ser Leu Gln Ala Gln Arg Asn Ser Cys Phe
145                 150                 155                 160

Ser Gln Ser Asp Ser Asn Ala Tyr Leu Glu Pro Asn Gly Gly Ala Ser
                165                 170                 175

Lys Asp Pro Ala Pro Pro Asn Leu His Arg Lys Ser Arg Ala Thr Thr

            180                 185                 190

Gly Ala Ala Thr Asp Pro Gln Ser Leu Tyr Ala Arg Lys Arg Arg Glu
        195                 200                 205

Arg Ile Asn Glu Arg Leu Arg Ile Leu Gln Asn Leu Val Pro Asn Gly
    210                 215                 220

Thr Lys Val Asp Ile Ser Thr Met Leu Glu Glu Ala Val Gln Tyr Val
225                 230                 235                 240

Lys Phe Leu Gln Leu Gln Ile Lys Leu Leu Ser Ser Asp Asp Leu Trp
                245                 250                 255

Met Tyr

<210> 89
<211> 773
<212> DNA
<213> Glycine max

<400> 89
attttttgtg tttctcacaa gggagtagct ccagtactga taatagtggt aataatatct 60

tttccattac aagtagtgga gcctactcct gtgatccaga agcaaacttt gattctgtgt 120

ccatggtttt gtgccttgga gatgccaaat ttagtcccca tagttttcaa tgtgatgaca 180

actcaaacca acagataaat gaaaacactg atgaagagtc aagtctagac ccatggaagt 240

tggctatagc tgacaataat ttgcaggcta agagggagta tgaaatgatg gtttctgaac 300

ctgtagaagt ggatagaagc agaaacctgg agaacctagc aaaaagacta aagagttcaa 360

tagaggtttc aaaaacattg aggagtgcta aatcagggaa aaattcaaaa tctgcttcag 420

tgagcaacga tgaagatgat agaagcttga gcctccaagc ccaaaggaat agctgttttt 480

cacagagtga ctctaatgct tatctggagc caaatggagg ggcatcaaaa gatcctgcac 540

ctcccaattt gcatagaaaa tcaagagcaa ctaccggtgc tgccactgat ccacagagcc 600

tctatgcaag aaagagaaga gaaagaataa atgaaaggtt gagaatactg caaaatcttg 660

ttcccaacgg aactaaggtg gatatcagca ccatgcttga ggaagctgtc caatacgtga 720

agtttttaca gctccaaatt aagcttctga gctctgacga tctgtggatg tat 773

<210> 90
<211> 176
<212> PRT
<213> Glycine max

<220>
<221> misc_feature
<222> (1)..(1)
<223> Xaa is unknown

<400> 90

Xaa Asn Leu Glu Asn Leu Pro Lys Arg Leu Lys Ser Ser Ile Glu Val
1               5                   10                  15

Pro Lys Thr Ser Arg Asn Ala Lys Ser Arg Lys Asn Ser Lys Ser Ala
            20                  25                  30

Ser Thr Ser Asn Asp Glu Asp Asp Arg Ser Leu Ser Leu Gln Val Gln
        35                  40                  45

Arg Asn Asn Ser Cys Phe Ser Gln Ser Asp Ser Asn Ala Tyr Leu Glu
    50                  55                  60

Pro Asn Gly Gly Ala Ser Lys Asp Pro Ala Pro Pro Asn Leu Asp Arg
65                  70                  75                  80

Lys Ser Arg Ala Thr Thr Ser Ala Ala Ala Asp Pro Gln Ser Leu Tyr
                85                  90                  95

Ala Arg Lys Arg Arg Glu Arg Ile Asn Glu Arg Leu Arg Ile Leu Gln
            100                 105                 110

Asn Leu Val Pro Asn Gly Thr Lys Val Asp Ile Ser Thr Met Leu Glu
        115                 120                 125

Glu Ala Val Gln Tyr Val Lys Phe Leu Gln Leu Gln Ile Lys Leu Leu
    130                 135                 140

Ser Ser Glu Asp Leu Trp Met Tyr Ala Pro Ile Val Tyr Asn Gly Ile
145                 150                 155                 160

Asn Ile Gly Leu Asp Leu Gly Ile Ser Pro Thr Lys Gly Arg Ser Met
                165                 170                 175

<210> 91
<211> 571
<212> DNA
<213> Glycine max

<400> 91
gaaacctgga gaacctacca aaaagactaa agagctcaat agaggtccca aaaacatcga      60

ggaatgctaa atcaaggaaa aattcaaaat ctgcttcaac tagcaacgat gaagatgata     120

gaagcttgag cctccaagtc caaaggaata atagctgttt ttcacagagt gactctaatg    180

cttatcttga gccaaatgga ggggcatcaa aagatcctgc acctcctaat ttggatagaa    240

aatcaagagc aactaccagt gccgccgctg atccacagag cctctatgca agaaagagaa    300

gagaaagaat aaatgaaagg ctgagaatac tgcaaaatct tgtccccaac ggaactaagg    360

tggatatcag caccatgctt gaagaagctg tccaatacgt taagttttta cagctccaaa    420

ttaagcttct gagctctgaa gatttgtgga tgtatgctcc aattgtttac aatggaataa    480

acattggact agacctcggt atttctccaa ccaaaggaag atcaatgtga tagcatagca    540

attaaagagg atataatatt tcattaactt a                                571

<210>  92
<211>  108
<212>  PRT
<213>  Lactuca saligna

<220>
<221>  misc_feature
<222>  (1)..(1)
<223>  Xaa is unknown

<400>  92

Xaa Arg Ser Lys Glu Ala Glu Ile Leu Ser Ser Asn Gly Lys Arg Lys
1               5                   10                  15

Ala Ser Arg Gly Ser Ala Thr Asp Pro Gln Ser Val Tyr Ala Arg Lys
            20                  25                  30

Arg Arg Glu Arg Ile Asn Glu Arg Leu Arg Ile Leu Gln Asn Leu Val
        35                  40                  45

Pro Asn Gly Thr Lys Val Asp Ile Ser Thr Met Leu Glu Glu Ala Val
    50                  55                  60

Glu Tyr Val Lys Phe Leu Gln Leu Gln Ile Lys Leu Leu Ser Ser Asp
65                  70                  75                  80

Asp Met Trp Met Tyr Ala Pro Ile Ala Tyr Asp Gly Met Asp Ile Gly
                85                  90                  95

Leu His Ser Thr Thr Ile Pro Ser Ser Ser Thr Arg
            100                 105

<210>  93
<211>  419
<212>  DNA
<213>  Lactuca saligna

<400> 93
tgagatcaaa agaggctgaa attctgagct caaatggcaa gagaaaagca agtagggggt 60

cagcaactga tccacaaagt gtctatgcac ggaaaagaag agaaagaatt aacgaacgtt 120

taagaatatt acaaaatctt gttcctaatg gtacaaaggt tgatataagc acaatgcttg 180

aagaggctgt tgagtacgtg aagtttttgc agcttcaaat caagctcttg agctccgatg 240

atatgtggat gtatgctccg attgcatacg atggaatgga cattgggctt cattcaacaa 300

ccatcccatc atcgtcaaca agataatgca aagttgggct atccatattg tcacattttt 360

gttgaataaa aggcaatcga taacaaaatt caaagtttat aaagagtaca catttatgc 419

<210> 94
<211> 274
<212> PRT
<213> Triticum aestivum

<400> 94

Met Ala Ser Lys Arg Ala Thr Thr Arg Glu Leu Arg Ala Met Tyr Asp
1               5                   10                  15

Asp Glu Pro Ser Ser Met Ser Leu Glu Leu Phe Gly Tyr His Gly Val
            20                  25                  30

Val Val Asp Gly Asp Asp Glu Asn Asp Asp Thr Ala Thr Ala Leu Pro
        35                  40                  45

Gln Leu Ser Phe Val Asp Asn Phe Lys Gly Gly Cys Gly Ser Ala Ala
    50                  55                  60

Asp Tyr Tyr Ser Trp Ala Tyr Asn Ala Ser Gly Gly Thr Pro Gly Ala
65                  70                  75                  80

Ser Ser Ser Ser Thr Ser Ser Val Leu Ser Phe Glu His Ala Gly Gly
                85                  90                  95

Ala Gly His Gln Leu Ala Tyr Asn Ser Gly Thr Gly Asp Asp Asp Cys
            100                 105                 110

Ala Leu Trp Met Asp Ser Met Ala Asp His Gln His Gly Ala Ala Arg
        115                 120                 125

Phe Gly Phe Met Asn Pro Gly Ser Ala Asp Val Val Pro Glu Ile Gln
    130                 135                 140

Glu Ser Ser Ile Lys Gln Pro Ala Lys Ser Ala Gln Lys Arg Ser Ser

145                 150                 155                 160

Ser Gly Gly Glu Ala Gln Ala Ala Ala Lys Lys Gln Cys Gly Gly Gly
                165                 170                 175

Arg Lys Ser Lys Ala Lys Val Val Pro Thr Lys Asp Pro Gln Ser Ala
            180                 185                 190

Val Ala Lys Val Arg Arg Glu Arg Ile Ser Glu Arg Leu Lys Val Leu
        195                 200                 205

Gln Asp Leu Val Pro Asn Gly Thr Lys Val Asp Met Val Thr Met Leu
    210                 215                 220

Glu Lys Ala Ile Thr Tyr Val Lys Phe Leu Gln Leu Gln Val Lys Val
225                 230                 235                 240

Leu Ala Thr Asp Glu Phe Trp Pro Val Gln Gly Gly Lys Ala Pro Glu
                245                 250                 255

Leu Ser Gln Val Lys Thr Ala Leu Asp Ala Ile Leu Ser Ser Gln Gln
            260                 265                 270

Gln Pro

<210>  95
<211>  825
<212>  DNA
<213>  Triticum aestivum

<400>  95
atggcgagca agcgggccac cacgcgggag ctccgggcga tgtacgacga cgagccctcc      60

tccatgtccc tcgagctctt cggctaccat ggcgtggtcg tcgacggtga cgatgaaaac     120

gacgacactg ccaccgccct gccccagctc tccttcgtcg acaacttcaa aggtgggtgc     180

gggtcggcgg cggactacta cagctgggcg tacaacgcct ccggcgggac gccgggcgcc     240

tcctccagct ccacctcgtc ggtgctcagc tttgagcatg ccggcggtgc cggtcatcag     300

ctggcttata attccggcac aggcgacgat gactgcgcgc tctggatgga cagcatggcc     360

gatcatcagc acggcgcggc caggtttggg ttcatgaacc cagggtcggc cgatgtcgtc     420

ccagaaatcc aggagagcag catcaagcag ccggccaagt ctgcgcagaa gcgctcgagc     480

tcgggtggtg aggcgcaagc agcggcgaag aagcagtgtg gaggaggcag gaagagcaag     540

gccaaagttg tccctaccaa ggatcctcag agcgctgttg caaaggtccg aagagagcgc     600

atcagtgaga ggctcaaagt tctgcaggat cttgtaccca acggcacgaa ggtggacatg    660

gtcaccatgc tcgagaaggc aatcacctat gtcaagttcc tgcagctgca agtcaaggtg    720

ttggcgaccg acgagttctg gccggtgcaa ggagggaagg cgccggagct ctcccaagtg    780

aagaccgcgc tggacgccat cctttcttcc cagcagcaac cctag                825

<210> 96
<211> 18
<212> PRT
<213> Carthamus tinctorius

<400> 96

Asp Ser Gln Ile Ile His Pro Met Pro Cys Asp Glu Leu His Lys Ser
1               5                   10                  15

Leu Ile

<210> 97
<211> 769
<212> DNA
<213> Carthamus tinctorius

<400> 97
gattcacaga taatccaccc tatgccgtgt gatgaactcc acaaatcctt aatttaattg    60

taccacatca ggcgacgtta tccatattgg gtgttcactg atggtgaaag cacatctttc    120

gcgcgacctc tactcaatga ctcaagaatt agaggtgaac tattgcttac actatctact    180

actaaacatt gtaaagtgac tgccagttct atgagacgtt cgtatagcat gatgcatgat    240

catgagaaaa gctaaaagat acagcgcaga aagagccaga agctcgtttc taaaggcaac    300

gaaagtgaag ctgaccatga tgcagttttt gggcaaataa tgaaaatgtg tggatctgac    360

aatgactcga attggcctcg ggagtcgagc acaagtccaa gaccaaaaga ggctgcaaat    420

ctgaactcaa atgggaagac aaaagcaaat agggggtcag caacggatcc acaaagtgtc    480

tacgcacgga agagaagaga acgaattaat gaacggttaa gaatactaca gagtctggtt    540

cctaatggta caaaggttga tataagcaca atgcttgaag atgctgtcca gtatgtgaaa    600

tttttgcagc tccaaatcaa gccgttgagc tctgatgatc tgtggatgta tgcccccatc    660

gcgtacaacg ggatggagac ggggcttgat tctacgatcc cctcgccaag gtgaagacta    720

tccaaagttg ccgcatcttt tttcttgaaa aaagggaagc ctggggcaa            769

<210> 98
<211> 309
<212> PRT
<213> Brachypodium distachyon

<400> 98

Met Ala Leu Val Arg Glu Pro Met Val Leu Tyr Asp Gly Gly Phe Asp
1               5                   10                  15

Ala Ser Glu Ala Ser Ala Phe Asp Ser Ile Gly Cys Phe Gly His Gly
            20                  25                  30

His Gly His Asp Ala Leu Leu Gly Gly Val Asp Ala Ala Ala Leu Phe
        35                  40                  45

Gly Gly Tyr Ala His Asp Glu Pro Ala Gly Ala Ser Ala Ser Ala Tyr
    50                  55                  60

Val Lys Asp Gly Ser His Trp Ala Gly Val Gly Ala Ser Val Leu Ala
65                  70                  75                  80

Phe Asp Arg Ala Ala Arg Gly His Gly Ala Gln Ala Met Ala Thr Ala
                85                  90                  95

Ala Ala Gln Glu Glu Glu Glu Cys Asp Ala Trp Ile Asp Ala Met Asp
            100                 105                 110

Glu Asp Asn Gly Glu Ala Ala Pro Ala Pro Ser Ile Gly Phe Asp Pro
        115                 120                 125

Ala Thr Gly Cys Phe Ser Leu Thr Gln Arg Pro Gly Ala Gly Ala Arg
    130                 135                 140

Arg Pro Phe Gly Leu Leu Phe Pro Ser Ala Ser Gly Gly Ala Pro Ser
145                 150                 155                 160

Pro Asp Ser Ala Ala Pro Ala Pro Ala Ser Arg Gly Ser Gln Lys Arg
                165                 170                 175

Pro Ser Ala Gly Ile Ala Arg Ala Gln Asp Ala Glu Pro Arg Ala Ser
            180                 185                 190

Lys Lys Gln Cys Gly Ala Ser Arg Lys Thr Thr Ala Lys Ala Lys Ser
        195                 200                 205

Pro Ala Pro Ala Ile Thr Ser Pro Lys Asp Pro Gln Ser Leu Ala Ala
    210                 215                 220

Lys Asn Arg Arg Glu Lys Ile Ser Glu Arg Leu Arg Thr Leu Gln Glu
225                 230                 235                 240

Met Val Pro Asn Gly Thr Lys Val Asp Met Val Thr Met Leu Glu Lys
                245                 250                 255

Ala Ile Ser Tyr Val Lys Phe Leu Gln Leu Gln Val Lys Val Leu Ala
            260                 265                 270

Thr Asp Glu Phe Trp Pro Ala Gln Gly Gly Met Ala Pro Glu Ile Ser
        275                 280                 285

Gln Val Lys Glu Ala Leu Asp Ala Ile Leu Ser Ser Gln Arg Gly Gln
    290                 295                 300

Phe Asn Cys Ser Ser
305

<210> 99
<211> 930
<212> DNA
<213> Brachypodium distachyon

<400> 99
atggcattag tgcgggagcc gatggtactg tatgacggcg gtttcgacgc ctcggaggcg 60

tcggcattcg actccatcgg ctgcttcggc cacggccacg gccacgacgc gctcctaggc 120

ggcgtcgacg cggccgcgct gttcgggggc tacgcgcacg acgagccggc cggcgccagc 180

gccagcgcct acgtgaagga cggctcgcac tgggccggcg tgggtgcgtc cgtgctcgcg 240

ttcgaccgtg ccgctcgggg ccacggcgcg caggccatgg cgaccgcggc cgctcaggag 300

gaggaagaat gcgacgcgtg gatcgacgcc atggacgagg acaatggcga ggcggcgccg 360

gcgccgtcca tcggcttcga cccggccacg ggctgcttca gcctcacgca gcggcccggc 420

gccggcgcgc ggcgcccgtt cgggctcctg ttcccgagcg cgtccggtgg cgcgccctcg 480

cccgacagcg ccgcgccagc gccggcatcc cgcggttccc agaagcggcc atccgccggg 540

attgcgcgcg cgcaggacgc ggagccgcgg gccagcaaga agcagtgcgg cgcgagcagg 600

aagacgacgg ccaaggcgaa gtcgcctgcg cctgccatca cctcgcccaa ggacccgcag 660

agcctcgctg caaagaaccg gagggagaag atcagcgagc ggctccggac gttgcaggag 720

atggtgccca acggcaccaa ggtggacatg gtcaccatgc tcgagaaggc catcagctac 780

gtcaagttcc tgcagctgca agtcaaggtg ctcgcgacgg acgagttctg gccggcgcag 840

ggagggatgg cgccggagat ctcccaggtg aaggaggcgc tcgacgccat cctgtcgtcg 900

cagaggggcc aattcaactg ctccagctag 930

<210> 100
<211> 272
<212> PRT
<213> Brachypodium distachyon

<400> 100

Met Ala Ser Arg His Ala Thr Thr Arg Glu Pro His Leu Arg Thr Met
1               5                   10                  15

Tyr Asp Asp Glu Pro Ser Met Ser Leu Glu Leu Phe Gly Tyr His Gly
            20                  25                  30

Val Val Val Asp Gly Asp Asp Asp Gly Asp Thr Ala Thr Asp Leu Pro
        35                  40                  45

Gln Leu Thr Phe Val Asp Asn Phe Lys Gly Gly Cys Gly Ser Ala Asp
    50                  55                  60

Tyr Tyr Gly Trp Ala Tyr Ser Ala Ser Gly Gly Ala Ser Gly Ala Cys
65                  70                  75                  80

Ser Ser Ser Ser Ser Ser Val Leu Ser Phe Glu Gln Ala Gly Gly Ala
                85                  90                  95

Gly His Gln Leu Ala Tyr Asn Ala Gly Thr Gly Asp Asp Asp Cys Ala
            100                 105                 110

Leu Trp Met Asp Gly Met Ala Asp Gln His Asp Thr Ala Lys Phe Gly
        115                 120                 125

Phe Met Asp Pro Gly Met Ser Asp Val Ser Leu Glu Ile Gln Glu Ser
    130                 135                 140

Ser Met Lys Pro Pro Ala Lys Met Ala Gln Lys Arg Ala Cys Gln Gly
145                 150                 155                 160

Gly Glu Thr Gln Ala Ala Ala Lys Lys Gln Cys Gly Gly Ser Lys Lys
                165                 170                 175

Ser Lys Ala Lys Ala Ala Pro Ala Lys Asp Pro Gln Ser Ala Val Ala
            180                 185                 190

Lys Val Arg Arg Glu Arg Ile Ser Glu Arg Leu Lys Val Leu Gln Asp
        195                 200                 205

Leu Val Pro Asn Gly Thr Lys Val Asp Met Val Thr Met Leu Glu Lys
    210                 215                 220

Ala Ile Thr Tyr Val Lys Phe Leu Gln Leu Gln Val Lys Val Leu Ala
225                 230                 235                 240

Thr Asp Asp Phe Trp Pro Val Gln Gly Gly Lys Ala Pro Glu Leu Ser
                245                 250                 255

Gln Val Lys Asp Ala Leu Asp Ala Ile Leu Ser Ser Gln Asn Gln Ser
            260                 265                 270

<210> 101
<211> 819
<212> DNA
<213> Brachypodium distachyon

<400> 101
atggcaagca ggcacgccac tacacgggag ccacacctcc ggaccatgta cgacgacgag      60

ccatccatgt ccctcgagct cttcggctac catggcgtcg tcgtcgacgg tgacgacgat     120

ggcgacaccg ccaccgacct tccccagctc acctttgttg acaacttcaa aggcgggtgt     180

gggtcagccg actactacgg ctgggcgtac agcgcctccg gtggtgcgtc aggcgcctgc     240

tccagctcca gctcgtcggt gctcagcttt gagcaggcgg gtggtgccgg tcatcagctg     300

gcttataacg ccggcacagg tgacgatgac tgcgcgctct ggatggacgg catggctgac     360

cagcatgaca cagccaagtt tgggttcatg gacccaggca tgtctgatgt cagcctagaa     420

atccaggaga gcagcatgaa accgccggcc aagatggcac agaagcgcgc ttgccagggt     480

ggtgagacgc aagcagcggc gaagaagcag tgtggaggaa gcaagaagag caaggcaaaa     540

gctgcccctg ccaaggatcc tcaaagcgcc gttgcaaagg tccgaagaga gcgcatcagc     600

gagaggctca aagttctgca ggatctcgtg cccaatggca caaaggttga catggtcacc     660

atgctcgaaa aggcaatcac ctatgtcaag ttcctgcagc tgcaagtcaa ggtattggcg     720

actgatgact tctggccggt gcaaggaggg aaagctccgg agctctccca agtgaaggac     780

gctctggacg cgatcctgtc ttcccagaat caatcctag                            819

<210> 102
<211> 240
<212> PRT
<213> Brachypodium distachyon

<400> 102

Met Ala Leu Val Gly Gln Ala Thr Lys Leu Cys Tyr Asp Gly Phe Ala
1               5                   10                  15

Gly Asp Gly Val Pro Pro Phe Met Asp Ala Ala Cys Leu Ala Phe Asp

20                  25                  30

His Gly Tyr Asp Tyr Asn Asn Pro His Ala Trp Glu Phe Pro Thr Gly
        35                  40                  45

Ala Glu Pro Gly Asn Ser Ser Ala Phe Asp Val Ala Trp Thr Gly Val
    50                  55                  60

Ser Ser Thr Ser Pro Val Leu Thr Phe Asp Ala Ala Glu Trp Met Asp
65                  70                  75                  80

Ala Thr Ala Thr Asp Arg Leu Ser Ser Tyr Ser Pro Ser Ala Ala Thr
                85                  90                  95

Val Pro Ala Ser Tyr Lys Arg Pro Arg Ala His Val Gln Pro Gln Gln
            100                 105                 110

Glu Ala Glu Glu Gln Glu Ser Ile Thr Pro Asn Pro Lys Lys Gln Cys
        115                 120                 125

Gly Asp Gly Lys Val Val Ile Lys Ser Ser Ala Ala Ala Thr Gly Thr
    130                 135                 140

Ser Pro Arg Lys Glu Pro Gln Ser Gln Ala Ala Lys Ser Arg Arg Glu
145                 150                 155                 160

Arg Ile Gly Glu Arg Leu Arg Ala Leu Gln Glu Leu Val Pro Asn Gly
                165                 170                 175

Ser Lys Val Asp Met Val Thr Met Leu Asp Lys Ala Ile Thr Tyr Val
            180                 185                 190

Lys Phe Met Gln Leu Gln Leu Thr Val Leu Glu Thr Asp Ala Phe Trp
        195                 200                 205

Pro Ala Gln Gly Gly Ala Ala Pro Glu Ile Ser Gln Val Lys Ala Ala
    210                 215                 220

Leu Asp Ala Ile Ile Leu Ser Ser Ser Gln Lys Pro Arg Gln Trp Ser
225                 230                 235                 240

<210>  103
<211>  723
<212>  DNA
<213>  Brachypodium distachyon

<400>  103

atggctctag tgggtcaggc aacgaagctc tgctacgacg gcttcgccgg agacggtgtg 60

ccgccgttca tggacgcagc ttgtctggca ttcgaccacg ggtatgatta caacaatccc 120

cacgcatggg aattccccac cggcgccgag ccaggcaaca gcagcgcgtt cgacgttgcc 180

tggaccggcg tctcctccac ttctccggtg ctcacattcg acgccgccga gtggatggac 240

gccacggcca cggaccggct gagctcctac agcccgtctg cggccaccgt gccggcctct 300

tacaagcggc ctcgtgcgca cgtgcagcca cagcaggaag cagaagaaca ggaaagcatt 360

actcccaatc ccaagaagca gtgcggcgat gggaaagtag ttatcaagtc atcggcggcg 420

gctaccggca ccagtccacg caaggaaccc caaagccaag ctgccaagag ccgtcgtgag 480

cggatcggcg agcggctgag agcgctgcag gagctggtgc ccaacggcag caaggtggac 540

atggtcacca tgctcgacaa ggccatcact tatgtcaagt tcatgcagct ccagctcacg 600

gtgctcgaga cagacgcgtt ctggcctgcg cagggtggcg cggcgccgga gatctcccag 660

gtgaaggcgg cgctcgacgc catcatcctc tcctcgtcgc agaagcctcg tcagtggagc 720

tag 723

<210> 104
<211> 821
<212> PRT
<213> Brachypodium distachyon

<400> 104

Met Glu Ala Gly Gly Leu Ile Ser Glu Ala Gly Trp Thr Met Phe Asp
1               5                   10                  15

Phe Pro Ser Gln Gly Glu Glu Ser Glu Ile Met Ser Gln Leu Leu Gly
            20                  25                  30

Ala Phe Pro Ser His Leu Glu Glu Gly His Gln Asp Leu Pro Trp Tyr
        35                  40                  45

Gln Ala Ser Asp Pro Ser Tyr Tyr Asp Cys Asn Leu Asn Thr Ser Ser
    50                  55                  60

Glu Ser Asn Ala Ser Ser Leu Ala Val Pro Ser Glu Cys Met Gly Tyr
65                  70                  75                  80

Tyr Leu Gly Asp Ser Ser Glu Ser Leu Asp Leu Ser Ser Cys Ile Ala
                85                  90                  95

Pro Asn Asp Leu Asn Leu Val Gln Glu Gln Asp Ala Thr Glu Phe Leu
            100                 105                 110

Asn Met Thr Pro Asn Leu Ser Leu Asp Leu Arg Gly Asn Gly Glu Ser
        115                 120                 125

Ser Cys Glu Asp Leu Thr Ser Val Gly Pro Thr Asn Lys Arg Lys His
    130                 135                 140

Ser Ser Ala Glu Glu Gly Ile Asp Cys Gln Ala Arg Gly Gln Lys Phe
145                 150                 155                 160

Ala Arg Lys Ala Glu Pro Lys Arg Thr Lys Lys Thr Lys Gln Ser Gly
                165                 170                 175

Trp Glu Val Ala Val Ala Thr Arg Asn Gly Ser Thr Ala Ser Cys Cys
            180                 185                 190

Thr Ser Asp Asp Asp Ser Asn Ala Ser Gln Glu Ser Ala Asp Thr Gly
        195                 200                 205

Val Cys Pro Lys Gly Lys Ala Arg Ala Ala Arg Gly Ala Ser Thr Asp
    210                 215                 220

Pro Gln Ser Leu Tyr Ala Arg Lys Arg Arg Glu Arg Ile Asn Glu Arg
225                 230                 235                 240

Leu Lys Thr Leu Gln Thr Leu Val Pro Asn Gly Thr Lys Val Asp Met
                245                 250                 255

Ser Thr Met Leu Glu Glu Ala Val His Tyr Val Lys Phe Leu Gln Leu
            260                 265                 270

Gln Ile Lys Val Leu Ser Ser Asp Asp Met Trp Met Tyr Ala Pro Leu
        275                 280                 285

Ala Tyr Asn Gly Met Asn Ile Gly Leu Asp Leu Asn Ile Tyr Thr Pro
    290                 295                 300

Glu Arg Trp Arg Thr Ala Ser Ala Ala Pro Ser Thr Glu Gly Arg Glu
305                 310                 315                 320

Tyr Ala Gly Val Asp Arg Ile Ser Asp Leu Pro Asp Gly Ile Leu Gly
                325                 330                 335

Asp Ile Val Ser Leu Leu Pro Thr Ala Glu Gly Ala Arg Thr Gln Ile
            340                 345                 350

Leu Lys Arg Arg Trp Arg His Ile Trp Arg Cys Ser Ala Pro Leu Asn
        355                 360                 365

Leu Asp Cys Cys Thr Leu Val Ala Arg Gly Gly Gly Arg Glu Ala Glu
    370                 375                 380

Asp Glu Leu Val Gly Leu Ile Pro Ser Ile Leu Ser Ser His Gln Gly
385                 390                 395                 400

Thr Gly Arg Arg Phe His Val Pro Ser Ser Arg His Ser Asp Arg Ala
                405                 410                 415

Ala Thr Ile Glu Ala Trp Leu Gln Ser Ala Ala Leu Asp Asn Leu Gln
            420                 425                 430

Glu Leu Asp Leu Trp Cys Thr His Thr Tyr Leu Tyr Asp Tyr Val Pro
        435                 440                 445

Leu Pro Pro Ala Val Phe Arg Phe Ser Ala Thr Val Arg Val Val Thr
    450                 455                 460

Ile Ala Asn Cys Asn Leu Arg Asp Ser Ala Val Gln Gly Leu Gln Phe
465                 470                 475                 480

Pro Gln Leu Lys Gln Leu Gly Phe Lys Asp Ile Ile Ile Met Glu Asp
                485                 490                 495

Ser Leu His His Met Ile Ala Ala Cys Pro Asp Leu Glu Cys Leu Met
            500                 505                 510

Ile Glu Arg Ser Leu Gly Phe Ala Cys Val Arg Ile Asn Ser Leu Ser
        515                 520                 525

Leu Arg Ser Ile Gly Val Ser Thr Asp His Pro His Pro His Glu Leu
    530                 535                 540

Gln Phe Val Glu Leu Val Ile Asp Asn Ala Pro Cys Leu Lys Arg Leu
545                 550                 555                 560

Leu His Leu Glu Met Cys Tyr His Leu Asp Met His Ile Thr Val Ile
                565                 570                 575

Ser Ala Pro Lys Leu Glu Thr Leu Ser Cys Cys Ser Ser Val Ser Arg
            580                 585                 590

Ser Ser Thr Lys Leu Ser Phe Gly Ser Ala Ala Ile Gln Gly Leu His

595                600                605

Ile Asp Ser Leu Thr Thr Val Val Arg Thr Val Gln Ile Leu Ala Val
    610                615                620

Glu Met His Ser Leu Cys Leu Asp Thr Ile Ile Asp Phe Met Lys Cys
625                630                635                640

Phe Pro Cys Leu Gln Lys Leu Tyr Ile Lys Ser Phe Val Ser Gly Asn
                645                650                655

Asn Trp Trp Gln Arg Lys His Arg Asn Val Ile Lys Ser Leu Asp Ile
            660                665                670

Arg Leu Lys Thr Ile Ala Leu Glu Ser Tyr Gly Gly Asn Gln Ser Asp
        675                680                685

Ile Asn Phe Val Thr Phe Phe Val Leu Asn Ala Arg Val Leu Glu Leu
    690                695                700

Met Thr Phe Asp Val Cys Ser Glu His Tyr Thr Val Glu Phe Leu Ala
705                710                715                720

Glu Gln Tyr Arg Lys Leu Gln Leu Asp Lys Arg Ala Ser Arg Ala Ala
                725                730                735

Arg Phe His Phe Thr Ser Asn Arg Cys Val Arg Gly Ile Pro Tyr Ile
            740                745                750

Gly Arg Ala Glu Leu Phe Leu Pro Ile Lys Cys Ser His Val Asp Thr
        755                760                765

Ser Pro Asn Leu Ser Ser Phe Arg Leu Ser Ala Val Phe Ser Val Cys
    770                775                780

Ile Thr Arg Asn Leu Leu Arg Leu Lys Lys Ala Met Trp Val Ile Ser
785                790                795                800

Leu Tyr Tyr Ser Pro Glu Phe Thr Lys Gln Val Ala Val His Asn Pro
                805                810                815

Asn Glu Met Pro Phe
            820

<210>  105
<211>  2466

<212> DNA
<213> Brachypodium distachyon

<400> 105
atggaggctg gagggctgat ttctgaggct ggctggacca tgtttgactt cccgtcgcaa 60

ggcgaggaat cagagatcat gtcgcagctg ctaggcgcct tcccctccca tcttgaggaa 120

ggccatcagg atctgccttg gtaccaggct tctgacccat cctactatga ctgtaatctt 180

aatacaagta gtgaaagcaa tgctagtagt cttgctgttc catccgagtg tatgggctac 240

tatttgggtg attcaagtga gtccctggac ctgagctcct gcattgcacc aaatgacctg 300

aacttggtcc aggagcaaga tgcaactgag tttctgaata tgacaccaaa tctttccctt 360

gatttacgtg ggaatggtga gtcgagctgc gaggatctca cttcggtcgg tcctactaac 420

aagcgaaagc actcctcggc agaagaagga atcgactgcc aagcaagagg ccagaaattc 480

gccagaaagg ctgaaccgaa gcgaacaaag aagaccaagc aaagcggatg ggaggttgct 540

gttgccacca ggaatggaag cacagcgagc tgctgcacct ctgatgatga ctcaaacgct 600

tctcaagaat ctgcagatac cggtgtttgt ccgaaaggca aggctcgggc tgcccgtggc 660

gcatcaactg atccccagag cctctatgca aggaaaagga gggaaaggat caatgagaga 720

ctgaagacac tgcagaccct tgtgcccaat ggaaccaaag tagatatgag caccatgctt 780

gaggaggcag tccactacgt gaagttcctg cagcttcaga tcaaggtctt gagctctgat 840

gatatgtgga tgtatgcgcc gctagcatac aacgggatga acattgggct tgatctgaac 900

atatatactc cggagaggtg gaggacagcg tccgcggcgc cctcaaccga agggcgtgaa 960

tacgccggcg tcgaccgcat cagcgacctc cccgacggca tcctcggcga catcgtctcg 1020

ttgctcccca ccgccgaagg agcccgcacc cagatcctca agcgcaggtg gcgccacatc 1080

tggcgctgct ccgcccctct caacctcgat tgctgtacct tggtcgcccg tggcggcggc 1140

cgtgaggctg aagatgaact cgtcggtctc ataccgtcca tcctttcttc tcaccaaggc 1200

accggccgcc gcttccacgt cccctcgtcg cgccactctg accgagctgc taccattgaa 1260

gcctggctcc aatctgctgc cctcgacaat ctccaggagc tcgatttatg gtgcacccac 1320

acctatcttt acgactatgt tccgctgcca cccgccgtct ttcgcttctc cgccaccgtc 1380

cgtgttgtca ccatcgcaaa ttgtaacctc cgtgacagcg ccgtccaagg ccttcaattc 1440

ccacaactta aacagctcgg attcaaagat atcatcatca tggaggattc gctgcaccac 1500

atgattgctg cgtgtccaga tctcgagtgc ttgatgattg aaaggagctt aggttttgct 1560

tgcgtccgga tcaattccct tagtcttaga agcatcggtg tgagcactga ccaccctcac 1620

ccacatgagc tccagtttgt ggaactcgtc attgataatg caccttgtct taagagattg 1680

ctccatcttg aaatgtgtta tcaccttgac atgcatataa cagtaatctc cgcgcctaaa 1740

ctggagacct tgagctgctg ttcttctgtg agtcgctcct ccaccaaact ctcgtttggc 1800

tccgcggcca ttcagggatt gcacattgat agcctaacaa cagtggtgcg cactgtccaa 1860

attttagctg tagagatgca ttctctttgt ctagacacaa ttattgactt catgaaatgc 1920

tttccatgtc tgcagaagtt gtacattaag tcatttgtaa gtggaaacaa ttggtggcaa 1980

cgtaaacacc ggaacgttat caaatccctt gacatccgtc tcaagacaat agcgttggaa 2040

agttatgggg gcaatcagtc tgacatcaac tttgtcacat tctttgtctt gaacgcgaga 2100

gtgctagagt tgatgacatt tgacgtttgt tctgagcatt acactgtgga gttcttggca 2160

gagcaatata ggaagcttca gctagataag agggcttcaa gagccgctcg gttccatttt 2220

acaagtaacc gatgtgtccg tggtattccg tatatcggac gtgccgagct attcttgcct 2280

atcaaatgtt ctcatgttga caccagtcca aacttgagta gtttccgttt gtctgcagta 2340

ttttcagttt gtattacccg gaaccttttg cgtttaaaaa aagctatgtg ggtcattagt 2400

ttgtattatt ctccagaatt tacaaaacaa gtggccgtgc acaatcccaa tgaaatgccg 2460

ttttag 2466

<210> 106
<211> 256
<212> PRT
<213> Brachypodium distachyon

<400> 106

Met Glu Ala Lys Cys Gly Ala Ile Trp Ser Ser Ile Asp Ala Arg Ser
1               5                   10                  15

Glu Asp Ser Glu Met Ile Ala His Leu Gln Ser Met Phe Trp Ser Asn
            20                  25                  30

Ser Asp Val Ala Leu Asn Leu Cys Ser Ser Asn Thr Ser Gly Asn Ser
        35                  40                  45

Cys Val Thr Ala Ser Thr Leu Pro Ser Ser Leu Phe Leu Pro Leu Val
    50                  55                  60

Asp Asn Glu Ser Tyr Gly Ala Ala Pro Ser Val Asp Thr Gly Met Asp
65                  70                  75                  80

Ser Cys Phe Asp His Gln His Gln Ser Ile Thr Gly His Lys Arg Ile
                85                  90                  95

Ser His Met Asp Glu Gln Met Lys Lys Thr Arg Lys Lys Ser Arg Thr
            100                 105                 110

Val Pro Ser Val Ser Lys Ala Leu Gly Ser Ser Leu Val Asp Asn Gln
        115                 120                 125

Met Asn Ala Asp Ile Phe Asn Gln Ser Ser Ser Cys Cys Ser Ser Gly
    130                 135                 140

Glu Asp Ser Ile Gly Thr Ser Glu Lys Ser Ile Val Ala Asn Gln Ser
145                 150                 155                 160

Asp Asn Thr Ser Gly Cys Lys Arg Pro Ser Lys Asn Met Gln Ser Leu
                165                 170                 175

Tyr Ala Lys Lys Arg Arg Glu Arg Ile Asn Glu Lys Leu Arg Val Leu
            180                 185                 190

Gln Gln Leu Ile Pro Asn Gly Thr Lys Val Asp Ile Ser Thr Met Leu
        195                 200                 205

Glu Glu Ala Val Gln Tyr Val Lys Phe Leu Gln Leu Gln Ile Lys Val
    210                 215                 220

Leu Ser Ser Asp Glu Thr Trp Met Tyr Ala Pro Leu Ala Tyr Asn Gly
225                 230                 235                 240

Met Asp Ile Gly Leu Thr Leu Ala Leu Arg Thr Ala Ala Asn Gln Glu
                245                 250                 255

<210> 107
<211> 771
<212> DNA
<213> Brachypodium distachyon

<400> 107
atggaggcca agtgtggagc tatttggagc tctatcgatg cgaggagcga ggactctgag 60

atgattgctc acctgcagtc catgttctgg agcaacagtg atgttgctct caacctctgt 120

tcgtcaaaca ccagtggcaa ttcttgtgtc acagctagca cattgcctag cagcttgttc 180

cttcctcttg tcgataatga gagctatggt gcagcgccat cggtggacac cggcatggat 240

tcatgctttg atcaccagca tcagagcatt actggtcaca agaggatatc gcacatggat 300

gagcagatga agaagacgag aaagaagtcc cggactgttc catcggtatc aaaggctctg 360

ggttccagcc tagtcgataa tcagatgaat gctgacattt tcaatcagag ctcctcctgc 420

tgcagctcgg gagaagattc aattggaaca tctgagaaat ccattgttgc aaaccagagt 480

gacaatacga gtggttgtaa gcggccttca aagaatatgc aaagccttta tgcaaagaag 540

agaagagaga ggatcaacga gaagttgaga gtactgcagc agctgattcc caatggcacc 600

aaagttgaca tcagcacaat gttggaggaa gcagttcagt atgtcaagtt tctgcagctg 660

caaataaagg tcttaagctc tgacgagaca tggatgtatg cgcccctcgc ctacaatggt 720

atggacatcg gtctcactct cgctctgaga actgctgcaa accaagagtg a 771

<210> 108
<211> 283
<212> PRT
<213> Zea mays

<400> 108

Met Ala Leu Val Arg Glu His Gly Gly Tyr Tyr Gly Gly Phe Asp Ser
1               5                   10                  15

Val Glu Ala Ala Ala Phe Asp Thr Leu Gly Tyr Gly His Gly Ala Ser
            20                  25                  30

Leu Gly Phe Asp Ala Ser Ser Ala Leu Phe Gly Glu Gly Gly Tyr Ala
        35                  40                  45

Ala Gly Gly Gly Asp Ala Trp Ala Gly Ala Gly Ala Ser Thr Val Leu
    50                  55                  60

Ala Phe Asn Arg Thr Thr Ala Ala Ala Ala Val Gly Val Glu Glu Glu
65                  70                  75                  80

Glu Glu Glu Cys Asp Ala Trp Ile Asp Ala Met Asp Glu Asp Asp Gln
                85                  90                  95

Ser Ser Gly Pro Ala Ala Ala Ala Pro Glu Ala Arg His Ala Leu Thr
            100                 105                 110

Ala Ser Val Gly Phe Asp Ala Ser Thr Gly Cys Phe Thr Leu Thr Glu
        115                 120                 125

Arg Ala Ser Ser Ser Ser Gly Gly Ala Gly Arg Pro Phe Gly Leu Leu
    130                 135                 140

Phe Pro Ser Thr Ser Ser Ser Gly Gly Thr Pro Glu Arg Thr Ala Pro
145                 150                 155                 160

Val Arg Val Pro Gln Lys Arg Thr Tyr Gln Ala Val Ser Pro Asn Lys
                165                 170                 175

Lys His Cys Gly Ala Gly Arg Lys Ala Ser Lys Ala Lys Leu Ala Ser
            180                 185                 190

Thr Ala Pro Thr Lys Asp Pro Gln Ser Leu Ala Ala Lys Gln Asn Arg
        195                 200                 205

Arg Glu Arg Ile Ser Glu Arg Leu Arg Ala Leu Gln Glu Leu Val Pro
    210                 215                 220

Asn Gly Thr Lys Val Asp Leu Val Thr Met Leu Glu Lys Ala Ile Ser
225                 230                 235                 240

Tyr Val Lys Phe Leu Gln Leu Gln Val Lys Val Leu Ala Thr Asp Glu
                245                 250                 255

Phe Trp Pro Ala Gln Gly Gly Lys Ala Pro Glu Ile Ser Gln Val Arg
            260                 265                 270

Glu Ala Leu Asp Ala Ile Leu Ser Ser Ala Ser
        275                 280

<210> 109
<211> 849
<212> DNA
<213> Zea mays

<400> 109
atggcgttgg tgagggagca cggtgggtac tacggaggct tcgacagcgt cgaggcggcg 60

gccttcgaca cgctcggcta cggccacggc gcgtcgctgg gctttgacgc gtcgtcggcg 120

ctgttcgggg aaggcggtta tgcggcgggc ggcggggacg cctggcgggg cgcgggggcg 180

tcgaccgtcc tggcgttcaa ccgcacaacg gcagcggcgg ccgtgggtgt ggaagaggag 240

gaggaggagt gcgacgcgtg gatcgacgct atggacgagg acgaccagag ctccggcccc 300

gccgcggcgg cgccagaggc gcgccacgcg ctgacggcct ccgtgggttt cgacgcctcc 360

acggggtgct tcaccctgac ggagagggcg tcgtcgtcgt caggcggagc ggggcgcccg 420

ttcggcctgc tgttcccgag cacgtcgtcg tcgggcggca cgcccgagcg cacggcgccg 480

gtgcgcgtcc cgcagaaacg gacctaccag gctgtgagcc ccaacaagaa gcactgcggc 540

gcgggcagga aggcgagcaa ggccaagctc gcgtccacag ccccaaccaa agatccccag 600

agcctcgcgg ccaagcagaa ccggcgcgag cggatcagcg agcggctgcg ggcgctgcag 660

gagctggtgc ccaacggcac caaggtcgac ctggtcacca tgctcgagaa ggccatcagc 720

tacgttaagt tcctccagtt gcaagtcaag gttctggcaa cagacgaatt ctggccggca 780

cagggaggga aggcgccgga gatctcccag gtgagggagg cgctcgacgc catcttgtcg 840

tcggcgtcg                                                    849

<210> 110
<211> 279
<212> PRT
<213> Zea mays

<400> 110

Met Ala Gln Phe Leu Gly Ala Ala Asp Asp His Cys Phe Thr Tyr Glu
1               5                   10                  15

Tyr Glu His Val Asp Glu Ser Met Glu Ala Ile Ala Ala Leu Phe Leu
            20                  25                  30

Pro Thr Leu Asp Thr Asp Ser Ala Asn Phe Ser Ser Ser Cys Phe Asn
        35                  40                  45

Tyr Ala Val Pro Pro Gln Cys Trp Pro Gln Pro Asp His Ser Ser Ser
    50                  55                  60

Val Thr Ser Leu Leu Asp Pro Ala Glu Asn Phe Glu Phe Pro Val Arg
65                  70                  75                  80

Asp Pro Leu Pro Pro Ser Gly Phe Asp Pro His Cys Ala Val Ala Tyr
                85                  90                  95

Leu Thr Glu Asp Ser Ser Pro Leu His Gly Lys Arg Ser Ser Val Ile
            100                 105                 110

Glu Glu Glu Ala Ala Asn Ala Ala Pro Ala Ala Lys Lys Arg Lys Ala
        115                 120                 125

Gly Ala Ala Met Gln Gly Ser Lys Lys Ser Arg Lys Ala Ser Lys Lys
    130                 135                 140

Asp Asn Ile Gly Asp Ala Asp Asp Asp Gly Gly Tyr Ala Cys Val Asp
145                 150                 155                 160

Thr Gln Ser Ser Ser Ser Cys Thr Ser Glu Asp Gly Asn Phe Glu Gly
                165                 170                 175

Asn Thr Asn Ser Ser Ser Lys Lys Thr Cys Ala Arg Ala Ser Arg Gly
            180                 185                 190

Ala Ala Thr Glu Pro Gln Ser Leu Tyr Ala Arg Lys Arg Arg Glu Arg
        195                 200                 205

Ile Asn Glu Arg Leu Arg Ile Leu Gln Asn Leu Val Pro Asn Gly Thr
210                 215                 220

Lys Val Asp Ile Ser Thr Met Leu Glu Glu Ala Ala Gln Tyr Val Lys
225                 230                 235                 240

Phe Leu Gln Leu Gln Ile Lys Leu Leu Ser Cys Asp Asp Thr Trp Met
                245                 250                 255

Tyr Ala Pro Ile Ala Tyr Asn Gly Ile Asn Ile Gly Asn Val Asp Leu
            260                 265                 270

Asn Ile Tyr Ser Leu Gln Lys
        275

<210>  111
<211>  840
<212>  DNA
<213>  Zea mays

<400>  111
atggctcagt ttcttggggc ggctgatgat cactgcttca cctacgagta tgagcatgtg     60

gatgagtcca tggaagcaat agcagccctg ttcttgccta cccttgacac cgactccgcc    120

aacttctcct ctagctgttt caactatgct gtccctccac agtgctggcc tcagccagac    180

catagctcta gcgttaccag tttgcttgat ccagccgaga actttgagtt tccagtcagg    240

gacccgctcc ccccaagcgg cttcgatcca cattgcgctg tcgcctacct cactgaggat    300

tcgagccctc tgcatggcaa acgttcatca gtcattgagg aagaagcagc caacgccgca    360

cctgctgcta agaagaggaa ggctggtgct gcaatgcagg gatcaaagaa atccaggaag    420

gcgagcaaaa aggataacat cggcgacgcc gacgatgatg gcggctatgc ctgtgttgac    480

acgcaaagct ccagtagctg cacctccgag gacgggaact tcgaaggaaa tacgaattca    540

agctccaaga agacctgcgc cagggccagc cgcggagcag caactgaacc tcagagtctc    600

tatgcaagga agaggagaga gaggatcaac gaaaggttga gaatcttgca gaacttggtt    660

ccaaatggaa caaaagtaga cattagcacg atgctcgagg aagcggcgca gtatgtcaag    720

tttttacagc tccagattaa gctgttgagc tgtgacgaca catggatgta tgcgccaatc    780

gcgtacaatg gaattaacat cggcaatgtt gatctgaaca tctactctct gcaaaagtaa    840

<210>  112
<211>  307
<212>  PRT
<213>  Zea mays

<220>
<221> misc_feature
<222> 7
<223> Xaa is Ile or Val

<400> 112

Met Glu Asp Gly Gly Leu Xaa Ser Glu Ala Gly Ala Trp Ala Glu Leu
1               5                   10                  15

Gly Thr Gly Gly Asp Glu Ser Glu Glu Leu Val Ala Gln Leu Leu Gly
            20                  25                  30

Ala Phe Phe Arg Ser His Gly Glu Glu Gly Arg His Gln Leu Leu Trp
        35                  40                  45

Ser Asp Asp Gln Ala Ser Ser Asp Asp Val His Gly Asp Gly Ser Leu
    50                  55                  60

Ala Val Pro Leu Ala Tyr Asp Gly Cys Cys Gly Tyr Leu Ser Tyr Ser
65                  70                  75                  80

Gly Ser Asn Ser Asp Glu Leu Pro Leu Gly Ser Ser Ser Arg Ala Ala
                85                  90                  95

Pro Ala Gly Gly Pro Pro Glu Glu Leu Leu Gly Ala Ala Glu Thr Glu
            100                 105                 110

Tyr Leu Asn Asn Val Ala Ala Ala Asp His Pro Phe Phe Lys Trp Cys
        115                 120                 125

Gly Asn Gly Glu Gly Leu Asp Gly Pro Thr Ser Val Val Gly Thr Leu
    130                 135                 140

Gly Leu Gly Ser Gly Arg Lys Arg Ala Arg Lys Lys Ser Gly Asp Glu
145                 150                 155                 160

Asp Glu Asp Pro Ser Thr Ala Ile Ala Ser Gly Ser Gly Pro Thr Ser
                165                 170                 175

Cys Cys Thr Thr Ser Asp Ser Asp Ser Asn Ala Ser Pro Leu Glu Ser
            180                 185                 190

Ala Asp Ala Gly Ala Arg Arg Pro Lys Gly Asn Glu Asn Ala Arg Ala
        195                 200                 205

Ala Gly Arg Gly Ala Ala Ala Ala Thr Thr Thr Thr Ala Glu Pro Gln

210                215                220

Ser Ile Tyr Ala Arg Val Arg Arg Glu Arg Ile Asn Glu Arg Leu Lys
225                230                235                240

Val Leu Gln Ser Leu Val Pro Asn Gly Thr Lys Val Asp Met Ser Thr
                245                250                255

Met Leu Glu Glu Ala Val His Tyr Val Lys Phe Leu Gln Leu Gln Ile
            260                265                270

Arg Val Leu Gln Leu Leu Ser Ser Asp Asp Thr Trp Met Tyr Ala Pro
        275                280                285

Ile Ala Tyr Asn Gly Met Gly Ile Gly Ile Asp Leu Arg Met His Gly
    290                295                300

Gln Asp Arg
305

<210>  113
<211>  924
<212>  DNA
<213>  Zea mays

<400>  113
atggaggacg gagggttgrt cagcgaggcc ggcgcctggg ccgagctcgg caccggcggc     60

gacgagtcgg aggagctggt ggcgcagctg ctgggcgcct tcttccggtc ccacggcgag    120

gaaggccggc accagctgct ttggtctgac gaccaagctt cttccgacga cgtgcacggc    180

gacggcagcc ttgccgtgcc gctcgcatac gacggctgct gcggctatct gagctactca    240

ggtagcaact cggacgagct ccccctcggg agcagctccc gcgctgcgcc agcaggtggc    300

ccaccggagg agctgctcgg tgcagctgag actgagtacc tgaataatgt ggccgccgca    360

gaccatccct tcttcaaatg gtgtgggaat ggtgagggtc tggatggtcc gacgagcgtc    420

gtgggcacgc ttgggcttgg ctcgggccgg aaacgcgcgc gcaagaagag cggggacgaa    480

gacgaagacc cgagcacggc catcgccagc ggaagcggcc ccacgagctg ctgcactacc    540

tccgacagcg actcaaacgc gtctcctctg gagtccgcgg acgccggcgc tcgtcgcccc    600

aagggcaacg agaatgcccg ggcagctggc cgcggcgcgg cggcggcgac gacgacgaca    660

gcggagcccc agagcatcta cgcaagggta cggagggagc ggatcaacga gaggctcaag    720

gtgctgcaga gcctggtgcc caacggcacc aaggtggaca tgagcaccat gctcgaggag    780

gccgtccact acgtcaagtt cctgcagctt cagatcaggg tgctgcagct cctgagctcc    840

gacgacacgt ggatgtacgc gcccatcgcg tacaacggga tgggcatcgg gatcgacctc 900

cgcatgcatg gacaggacag atga 924

<210> 114
<211> 141
<212> PRT
<213> Zea mays

<400> 114

Ser Lys Lys Ser Arg Lys Ala Ser Lys Lys Asp Cys Ile Val Asp Asp
1               5                   10                  15

Asp Asp Val Tyr Val Asp Pro Gln Ser Ser Gly Ser Cys Thr Ser Glu
            20                  25                  30

Glu Gly Asn Phe Glu Gly Asn Thr Tyr Ser Ser Ala Lys Lys Thr Cys
        35                  40                  45

Thr Arg Ala Ser Arg Gly Gly Ala Thr Asp Pro Gln Ser Leu Tyr Ala
    50                  55                  60

Arg Lys Arg Arg Glu Arg Ile Asn Glu Arg Leu Arg Ile Leu Gln Asn
65                  70                  75                  80

Leu Val Pro Asn Gly Thr Lys Val Asp Ile Ser Thr Met Leu Glu Glu
                85                  90                  95

Ala Ala Gln Tyr Val Lys Phe Leu Gln Leu Gln Ile Lys Leu Leu Ser
            100                 105                 110

Ser Asp Asp Met Trp Met Tyr Ala Pro Ile Ala Tyr Asn Gly Ile Asn
        115                 120                 125

Ile Ser Asn Val Asp Leu Asn Ile Pro Ala Leu Gln Lys
    130                 135                 140

<210> 115
<211> 414
<212> DNA
<213> Zea mays

<400> 115
tcaaagaaat ccaggaaggc gagcaaaaaa gattgtattg tcgatgacga cgatgtctat 60

gttgacccgc aaagctccgg tagctgcacc tccgaggagg ggaattttga agggaatacg 120

tattcaagcg cgaaaaagac ctgcaccagg gccagccgcg gaggagcaac tgatcctcag 180

agtctctatg caaggaagag gagagagagg atcaatgaaa ggttgagaat cttgcagaac 240

ttggtcccca atggaacaaa ggttgacatt agtacgatgc tcgaggaagc agcacagtat 300

gtcaaatttt tacagcttca gattaagctg ttgagctctg acgacatgtg gatgtatgcg 360

ccaatcgcgt acaatgggat caacatcagc aatgttgatc tgaacatccc tgca 414

<210> 116
<211> 65
<212> PRT
<213> Carthamus tinctorius

<400> 116

Leu Tyr His Ile Arg Arg Arg Tyr Pro Tyr Trp Val Phe Thr Asp Gly
1               5                   10                  15

Glu Ser Thr Ser Phe Ala Arg Pro Leu Leu Asn Asp Ser Arg Ile Arg
            20                  25                  30

Gly Glu Leu Leu Leu Thr Leu Ser Thr Thr Lys His Cys Lys Val Thr
        35                  40                  45

Ala Ser Ser Met Arg Arg Ser Tyr Ser Met Met His Asp His Glu Lys
    50                  55                  60

Ser
65

<210> 117
<211> 152
<212> PRT
<213> Carthamus tinctorius

<400> 117

Lys Ile Gln Arg Arg Lys Ser Gln Lys Leu Val Ser Lys Gly Asn Glu
1               5                   10                  15

Ser Glu Ala Asp His Asp Ala Val Phe Gly Gln Ile Met Lys Met Cys
            20                  25                  30

Gly Ser Asp Asn Asp Ser Asn Trp Pro Arg Glu Ser Ser Thr Ser Pro
        35                  40                  45

Arg Pro Lys Glu Ala Ala Asn Leu Asn Ser Asn Gly Lys Thr Lys Ala
    50                  55                  60

Asn Arg Gly Ser Ala Thr Asp Pro Gln Ser Val Tyr Ala Arg Lys Arg
65                  70                  75                  80

Arg Glu Arg Ile Asn Glu Arg Leu Arg Ile Leu Gln Ser Leu Val Pro
                85                  90                  95

Asn Gly Thr Lys Val Asp Ile Ser Thr Met Leu Glu Asp Ala Val Gln
            100                 105                 110

Tyr Val Lys Phe Leu Gln Leu Gln Ile Lys Pro Leu Ser Ser Asp Asp
        115                 120                 125

Leu Trp Met Tyr Ala Pro Ile Ala Tyr Asn Gly Met Glu Thr Gly Leu
    130                 135                 140

Asp Ser Thr Ile Pro Ser Pro Arg
145                 150

<210> 118
<211> 18
<212> PRT
<213> Carthamus tinctorius

<400> 118

Arg Leu Ser Lys Val Ala Ala Ser Phe Phe Leu Lys Lys Gly Lys Pro
1               5                   10                  15

Gly Ala

<210> 119
<211> 30
<212> DNA
<213> Artificial sequence

<220>
<223> Synthetic sequence: Primer

<400> 119
ccaggatcca tggcactcgt taatgaccat 30

<210> 120
<211> 31
<212> DNA
<213> Artificial sequence

<220>
<223> Synthetic sequence: Primer

<400> 120
ccagtcgact taattggtga tcagattcga a 31

<210> 121

<211> 29
<212> DNA
<213> Artificial sequence

<220>
<223> Synthetic sequence: Primer

<400> 121
ccaggatcca tgggagaatg gagcaacaa 29


<210> 122
<211> 28
<212> DNA
<213> Artificial sequence

<220>
<223> Synthetic sequence: Primer

<400> 122
ccagtcgact catctcggtg agctgaga 28


<210> 123
<211> 28
<212> DNA
<213> Artificial sequence

<220>
<223> Synthetic sequence: Primer

<400> 123
cggggtacca tggaagccat gggagaat 28


<210> 124
<211> 30
<212> DNA
<213> Artificial sequence

<220>
<223> Synthetic sequence: Primer

<400> 124
cgcggatcct catctggtca gtgcattgag 30


<210> 125
<211> 30
<212> DNA
<213> Artificial sequence

<220>
<223> Synthetic sequence: Primer

<400> 125
cggggtacca tggacgtttt tgttgatggt 30


<210> 126
<211> 30

<212> DNA
<213> Artificial sequence

<220>
<223> Synthetic sequence: Primer

<400> 126
cgcggatcct cacataagcc gagacaaaag 30


<210> 127
<211> 67
<212> PRT
<213> Arabidopsis thaliana

<400> 127

Arg Ile Ser Asp Asp Pro Gln Thr Val Val Ala Arg Arg Arg Arg Glu
1               5                   10                  15


Arg Ile Ser Glu Lys Ile Arg Ile Leu Lys Arg Ile Val Pro Gly Gly
            20                  25                  30


Ala Lys Met Asp Thr Ala Ser Met Leu Asp Glu Ala Ile Arg Tyr Thr
        35                  40                  45


Lys Phe Leu Lys Arg Gln Val Arg Ile Leu Gln Pro His Ser Gln Ile
    50                  55                  60


Gly Ala Pro
65


<210> 128
<211> 67
<212> PRT
<213> Physcomitrella patens

<400> 128

Arg Ile Ser Lys Asp Pro Gln Ser Val Ala Ala Arg His Arg Arg Glu
1               5                   10                  15


Arg Ile Ser Asp Arg Ile Arg Val Leu Gln Arg Leu Val Pro Gly Gly
            20                  25                  30


Thr Lys Met Asp Thr Ala Ser Met Leu Asp Glu Ala Ile His Tyr Val
        35                  40                  45


Lys Phe Leu Lys Leu Gln Leu Gln Val Cys Asp Thr Cys Asn Leu Val
    50                  55                  60


Pro Val Asp

65

<210> 129
<211> 72
<212> PRT
<213> Arabidopsis thaliana

<400> 129

Lys Thr Lys Pro Lys Pro Thr Thr Ser Pro Lys Asp Pro Gln Ser Leu
1               5                   10                  15

Ala Ala Lys Asn Arg Arg Glu Arg Ile Ser Glu Arg Leu Lys Ile Leu
            20                  25                  30

Gln Glu Leu Val Pro Asn Gly Thr Lys Val Asp Leu Val Thr Met Leu
        35                  40                  45

Glu Lys Ala Ile Ser Tyr Val Lys Phe Leu Gln Val Gln Val Lys Val
    50                  55                  60

Leu Ala Thr Asp Glu Phe Trp Pro
65                  70

<210> 130
<211> 72
<212> PRT
<213> Arabidopsis thaliana

<400> 130

Lys Ile Lys Pro Lys Ala Thr Thr Ser Pro Lys Asp Pro Gln Ser Leu
1               5                   10                  15

Ala Ala Lys Asn Arg Arg Glu Arg Ile Ser Glu Arg Leu Lys Val Leu
            20                  25                  30

Gln Glu Leu Val Pro Asn Gly Thr Lys Val Asp Leu Val Thr Met Leu
        35                  40                  45

Glu Lys Ala Ile Gly Tyr Val Lys Phe Leu Gln Val Gln Val Lys Val
    50                  55                  60

Leu Ala Ala Asp Glu Phe Trp Pro
65                  70

<210> 131
<211> 71
<212> PRT
<213> Arabidopsis thaliana

<400>  131

Lys Thr Arg Ala Ser Arg Gly Ala Ala Thr Asp Pro Gln Ser Leu Tyr
1               5                   10                  15

Ala Arg Lys Arg Arg Glu Arg Ile Asn Glu Arg Leu Arg Ile Leu Gln
            20                  25                  30

Asn Leu Val Pro Asn Gly Thr Lys Val Asp Ile Ser Thr Met Leu Glu
        35                  40                  45

Glu Ala Val His Tyr Val Lys Phe Leu Gln Leu Gln Ile Lys Leu Leu
    50                  55                  60

Ser Ser Asp Asp Leu Trp Met
65                  70

<210>  132
<211>  71
<212>  PRT
<213>  Arabidopsis thaliana

<400>  132

Lys Thr Arg Ala Ser Arg Gly Ala Ala Thr Asp Pro Gln Ser Leu Tyr
1               5                   10                  15

Ala Arg Lys Arg Arg Glu Arg Ile Asn Glu Arg Leu Arg Ile Leu Gln
            20                  25                  30

His Leu Val Pro Asn Gly Thr Lys Val Asp Ile Ser Thr Met Leu Glu
        35                  40                  45

Glu Ala Val Gln Tyr Val Lys Phe Leu Gln Leu Gln Ile Lys Leu Leu
    50                  55                  60

Ser Ser Asp Asp Leu Trp Met
65                  70

<210>  133
<211>  71
<212>  PRT
<213>  Arabidopsis thaliana

<400>  133

Lys Thr Arg Ala Thr Lys Gly Thr Ala Thr Asp Pro Gln Ser Leu Tyr
1               5                   10                  15

Ala Arg Lys Arg Arg Glu Lys Ile Asn Glu Arg Leu Lys Thr Leu Gln

            20                  25                  30

Asn Leu Val Pro Asn Gly Thr Lys Val Asp Ile Ser Thr Met Leu Glu
        35                  40                  45

Glu Ala Val His Tyr Val Lys Phe Leu Gln Leu Gln Ile Lys Leu Leu
    50                  55                  60

Ser Ser Asp Asp Leu Trp Met
65                  70

<210> 134
<211> 71
<212> PRT
<213> Arabidopsis thaliana

<400> 134

Lys Ala Lys Ala Asn Arg Gly Ile Ala Ser Asp Pro Gln Ser Leu Tyr
1               5                   10                  15

Ala Arg Lys Arg Arg Glu Arg Ile Asn Asp Arg Leu Lys Thr Leu Gln
            20                  25                  30

Ser Leu Val Pro Asn Gly Thr Lys Val Asp Ile Ser Thr Met Leu Glu
        35                  40                  45

Asp Ala Val His Tyr Val Lys Phe Leu Gln Leu Gln Ile Lys Leu Leu
    50                  55                  60

Ser Ser Glu Asp Leu Trp Met
65                  70

<210> 135
<211> 72
<212> PRT
<213> Brachypodium distachyon

<400> 135

Lys Ser Pro Ala Pro Ala Ile Thr Ser Pro Lys Asp Pro Gln Ser Leu
1               5                   10                  15

Ala Ala Lys Asn Arg Arg Glu Lys Ile Ser Glu Arg Leu Arg Thr Leu
            20                  25                  30

Gln Glu Met Val Pro Asn Gly Thr Lys Val Asp Met Val Thr Met Leu
        35                  40                  45

Glu Lys Ala Ile Ser Tyr Val Lys Phe Leu Gln Leu Gln Val Lys Val
    50                  55                  60

Leu Ala Thr Asp Glu Phe Trp Pro
65                  70

<210> 136
<211> 71
<212> PRT
<213> Brachypodium distachyon

<400> 136

Lys Ser Lys Ala Lys Ala Ala Pro Ala Lys Asp Pro Gln Ser Ala Val
1               5                   10                  15

Ala Lys Val Arg Arg Glu Arg Ile Ser Glu Arg Leu Lys Val Leu Gln
            20                  25                  30

Asp Leu Val Pro Asn Gly Thr Lys Val Asp Met Val Thr Met Leu Glu
        35                  40                  45

Lys Ala Ile Thr Tyr Val Lys Phe Leu Gln Leu Gln Val Lys Val Leu
    50                  55                  60

Ala Thr Asp Asp Phe Trp Pro
65                  70

<210> 137
<211> 74
<212> PRT
<213> Brachypodium distachyon

<400> 137

Lys Ser Ser Ala Ala Ala Thr Gly Thr Ser Pro Arg Lys Glu Pro Gln
1               5                   10                  15

Ser Gln Ala Ala Lys Ser Arg Arg Glu Arg Ile Gly Glu Arg Leu Arg
            20                  25                  30

Ala Leu Gln Glu Leu Val Pro Asn Gly Ser Lys Val Asp Met Val Thr
        35                  40                  45

Met Leu Asp Lys Ala Ile Thr Tyr Val Lys Phe Met Gln Leu Gln Leu
    50                  55                  60

Thr Val Leu Glu Thr Asp Ala Phe Trp Pro
65                  70

<210> 138
<211> 71
<212> PRT
<213> Brachypodium distachyon

<400> 138

Lys Ala Arg Ala Ala Arg Gly Ala Ser Thr Asp Pro Gln Ser Leu Tyr
1               5                   10                  15

Ala Arg Lys Arg Arg Glu Arg Ile Asn Glu Arg Leu Lys Thr Leu Gln
            20                  25                  30

Thr Leu Val Pro Asn Gly Thr Lys Val Asp Met Ser Thr Met Leu Glu
        35                  40                  45

Glu Ala Val His Tyr Val Lys Phe Leu Gln Leu Gln Ile Lys Val Leu
    50                  55                  60

Ser Ser Asp Asp Met Trp Met
65                  70

<210> 139
<211> 72
<212> PRT
<213> Brachypodium distachyon

<400> 139

Asp Asn Thr Ser Gly Cys Lys Arg Pro Ser Lys Asn Met Gln Ser Leu
1               5                   10                  15

Tyr Ala Lys Lys Arg Arg Glu Arg Ile Asn Glu Lys Leu Arg Val Leu
            20                  25                  30

Gln Gln Leu Ile Pro Asn Gly Thr Lys Val Asp Ile Ser Thr Met Leu
        35                  40                  45

Glu Glu Ala Val Gln Tyr Val Lys Phe Leu Gln Leu Gln Ile Lys Val
    50                  55                  60

Leu Ser Ser Asp Glu Thr Trp Met
65                  70

<210> 140
<211> 71
<212> PRT
<213> Carthamus tinctorius

<400> 140

Lys Thr Lys Ala Asn Arg Gly Ser Ala Thr Asp Pro Gln Ser Val Tyr
1               5                   10                  15

Ala Arg Lys Arg Arg Glu Arg Ile Asn Glu Arg Leu Arg Ile Leu Gln
            20                  25                  30

Ser Leu Val Pro Asn Gly Thr Lys Val Asp Ile Ser Thr Met Leu Glu
        35                  40                  45

Asp Ala Val Gln Tyr Val Lys Phe Leu Gln Leu Gln Ile Lys Pro Leu
    50                  55                  60

Ser Ser Asp Asp Leu Trp Met
65                  70

<210>  141
<211>  71
<212>  PRT
<213>  Glycine max

<400>  141

Lys Ser Arg Ala Thr Thr Gly Ala Ala Thr Asp Pro Gln Ser Leu Tyr
1               5                   10                  15

Ala Arg Lys Arg Arg Glu Arg Ile Asn Glu Arg Leu Arg Ile Leu Gln
            20                  25                  30

Asn Leu Val Pro Asn Gly Thr Lys Val Asp Ile Ser Thr Met Leu Glu
        35                  40                  45

Glu Ala Val Gln Tyr Val Lys Phe Leu Gln Leu Gln Ile Lys Leu Leu
    50                  55                  60

Ser Ser Asp Asp Leu Trp Met
65                  70

<210>  142
<211>  71
<212>  PRT
<213>  Glycine max

<400>  142

Lys Ser Arg Ala Thr Thr Ser Ala Ala Ala Asp Pro Gln Ser Leu Tyr
1               5                   10                  15

Ala Arg Lys Arg Arg Glu Arg Ile Asn Glu Arg Leu Arg Ile Leu Gln
            20                  25                  30

Asn Leu Val Pro Asn Gly Thr Lys Val Asp Ile Ser Thr Met Leu Glu
        35                  40                  45

Glu Ala Val Gln Tyr Val Lys Phe Leu Gln Leu Gln Ile Lys Leu Leu
    50                  55                  60

Ser Ser Glu Asp Leu Trp Met
65                  70

<210> 143
<211> 71
<212> PRT
<213> Lactuca saligna

<400> 143

Lys Arg Lys Ala Ser Arg Gly Ser Ala Thr Asp Pro Gln Ser Val Tyr
1               5                   10                  15

Ala Arg Lys Arg Arg Glu Arg Ile Asn Glu Arg Leu Arg Ile Leu Gln
            20                  25                  30

Asn Leu Val Pro Asn Gly Thr Lys Val Asp Ile Ser Thr Met Leu Glu
        35                  40                  45

Glu Ala Val Glu Tyr Val Lys Phe Leu Gln Leu Gln Ile Lys Leu Leu
    50                  55                  60

Ser Ser Asp Asp Met Trp Met
65                  70

<210> 144
<211> 71
<212> PRT
<213> Musa acuminata

<400> 144

Lys Thr Arg Ser Asn Arg Trp Pro Ala Thr Asp Pro Gln Ser Leu Tyr
1               5                   10                  15

Ala Lys Gln Arg Arg Glu Arg Ile Asn Ala Arg Leu Arg Thr Leu Gln
            20                  25                  30

Asn Leu Val Pro Asn Gly Thr Lys Val Asp Ile Ser Thr Met Leu Glu
        35                  40                  45

Glu Ala Val Arg Tyr Val Lys Phe Leu Gln Leu Gln Ile Lys Leu Leu

    50                  55                  60

Ser Ser Asp Glu Leu Trp Met
65                  70

<210> 145
<211> 71
<212> PRT
<213> Medicago truncatula

<400> 145

Lys Thr Arg Ala Ser Arg Gly Ser Ala Thr Asp Pro Gln Ser Leu Tyr
1               5                   10                  15

Ala Arg Lys Arg Arg Glu Arg Ile Asn Glu Arg Leu Arg Val Leu Gln
            20                  25                  30

Asn Leu Val Pro Asn Gly Thr Lys Val Asp Ile Ser Thr Met Leu Glu
        35                  40                  45

Glu Ala Val Asn Tyr Val Lys Phe Leu Gln Thr Gln Ile Lys Leu Leu
    50                  55                  60

Ser Ser Asp Asp Met Trp Met
65                  70

<210> 146
<211> 72
<212> PRT
<213> Oryza sativa

<400> 146

Ser Pro Ser Pro Ser Pro Ser Pro Asn Lys Glu Gln Pro Gln Ser Ala
1               5                   10                  15

Ala Ala Lys Val Arg Arg Glu Arg Ile Ser Glu Arg Leu Lys Val Leu
            20                  25                  30

Gln Asp Leu Val Pro Asn Gly Thr Lys Val Asp Leu Val Thr Met Leu
        35                  40                  45

Glu Lys Ala Ile Asn Tyr Val Lys Phe Leu Gln Leu Gln Val Lys Val
    50                  55                  60

Leu Ala Thr Asp Glu Phe Trp Pro
65                  70

<210> 147
<211> 72
<212> PRT
<213> Oryza sativa

<400> 147

Lys Ala Lys Ser Ala Pro Thr Thr Pro Thr Lys Asp Pro Gln Ser Leu
1               5                   10                  15

Ala Ala Lys Asn Arg Arg Glu Arg Ile Ser Glu Arg Leu Arg Ile Leu
            20                  25                  30

Gln Glu Leu Val Pro Asn Gly Thr Lys Val Asp Leu Val Thr Met Leu
        35                  40                  45

Glu Lys Ala Ile Ser Tyr Val Lys Phe Leu Gln Leu Gln Val Lys Val
    50                  55                  60

Leu Ala Thr Asp Glu Phe Trp Pro
65                  70

<210> 148
<211> 71
<212> PRT
<213> Oryza sativa

<400> 148

Tyr Ser Lys Lys Gln Arg Ala Asn Asn Lys Glu Thr Gln Ser Ser Ala
1               5                   10                  15

Ala Lys Ser Arg Arg Glu Arg Ile Ser Glu Arg Leu Arg Ala Leu Gln
            20                  25                  30

Glu Leu Val Pro Ser Gly Gly Lys Val Asp Met Val Thr Met Leu Asp
        35                  40                  45

Arg Ala Ile Ser Tyr Val Lys Phe Met Gln Met Gln Leu Arg Val Leu
    50                  55                  60

Glu Thr Asp Ala Phe Trp Pro
65                  70

<210> 149
<211> 71
<212> PRT
<213> Oryza sativa

<400> 149

Lys Ala Arg Ala Gly Arg Gly Ala Ala Thr Asp Pro Gln Ser Leu Tyr
1               5                   10                  15

Ala Arg Lys Arg Arg Glu Arg Ile Asn Glu Arg Leu Lys Ile Leu Gln
            20                  25                  30

Asn Leu Ile Pro Asn Gly Thr Lys Val Asp Ile Ser Thr Met Leu Glu
        35                  40                  45

Glu Ala Val His Tyr Val Lys Phe Leu Gln Leu Gln Ile Lys Leu Leu
    50                  55                  60

Ser Ser Asp Asp Met Trp Met
65                  70

<210>  150
<211>  71
<212>  PRT
<213>  Oryza sativa

<400>  150

Lys Ala Gln Ser Gly His Arg Ser Ala Thr Asp Pro Gln Ser Leu Tyr
1               5                   10                  15

Ala Arg Lys Arg Arg Glu Arg Ile Asn Glu Arg Leu Lys Ile Leu Gln
            20                  25                  30

Asn Leu Val Pro Asn Gly Thr Lys Val Asp Ile Ser Thr Met Leu Glu
        35                  40                  45

Glu Ala Met His Tyr Val Lys Phe Leu Gln Leu Gln Ile Lys Leu Leu
    50                  55                  60

Ser Ser Asp Glu Met Trp Met
65                  70

<210>  151
<211>  71
<212>  PRT
<213>  Oryza sativa

<400>  151

Asn Ser Arg Gly His Lys Gln Cys Ser Lys Asp Thr Gln Ser Leu Tyr
1               5                   10                  15

Ala Lys Arg Arg Arg Glu Arg Ile Asn Glu Arg Leu Arg Ile Leu Gln
            20                  25                  30

Gln Leu Val Pro Asn Gly Thr Lys Val Asp Ile Ser Thr Met Leu Glu
        35                  40                  45

Glu Ala Val Gln Tyr Val Lys Phe Leu Gln Leu Gln Ile Lys Leu Leu
    50                  55                  60

Ser Ser Asp Asp Thr Trp Met
65                  70

<210> 152
<211> 71
<212> PRT
<213> Oryza sativa

<400> 152

Lys Ala Cys Lys His Ser Val Ser Ala Glu Ser Ser Gln Ser Tyr Tyr
1               5                   10                  15

Ala Lys Asn Arg Arg Gln Arg Ile Asn Glu Arg Leu Arg Ile Leu Gln
            20                  25                  30

Glu Leu Ile Pro Asn Gly Thr Lys Val Asp Ile Ser Thr Met Leu Glu
        35                  40                  45

Glu Ala Ile Gln Tyr Val Lys Phe Leu His Leu Gln Ile Lys Leu Leu
    50                  55                  60

Ser Ser Asp Glu Met Trp Met
65                  70

<210> 153
<211> 71
<212> PRT
<213> Oryza sativa

<400> 153

Lys Ser Arg Ala Gly Arg Gly Ala Ala Thr Asp Pro Gln Ser Leu Tyr
1               5                   10                  15

Ala Arg Lys Arg Arg Glu Arg Ile Asn Glu Arg Leu Lys Thr Leu Gln
            20                  25                  30

Asn Leu Val Pro Asn Gly Thr Lys Val Asp Ile Ser Thr Met Leu Glu
        35                  40                  45

Glu Ala Val His Tyr Val Lys Phe Leu Gln Leu Gln Ile Lys Leu Leu
    50                  55                  60

Ser Ser Asp Glu Met Trp Met
65                70

<210> 154
<211> 71
<212> PRT
<213> Oryza sativa

<400> 154

Gly Thr Arg Ala Asn Arg Gly Ala Ala Thr Asp Pro Gln Ser Leu Tyr
1               5                   10                  15

Ala Arg Lys Arg Arg Glu Arg Ile Asn Glu Arg Leu Arg Ile Leu Gln
            20                  25                  30

Asn Leu Val Pro Asn Gly Thr Lys Val Asp Ile Ser Thr Met Leu Glu
        35                  40                  45

Glu Ala Val Gln Tyr Val Lys Phe Leu Gln Leu Gln Ile Lys Leu Leu
    50                  55                  60

Ser Ser Asp Asp Thr Trp Met
65                  70

<210> 155
<211> 71
<212> PRT
<213> Physcomitrella patens

<400> 155

Lys Pro Arg Ala Arg Gln Gly Ser Ala Asn Asp Pro Gln Ser Ile Ala
1               5                   10                  15

Ala Arg Val Arg Arg Glu Arg Ile Ser Glu Arg Leu Lys Val Leu Gln
            20                  25                  30

Ala Leu Ile Pro Asn Gly Asp Lys Val Asp Met Val Thr Met Leu Glu
        35                  40                  45

Lys Ala Ile Ser Tyr Val Gln Cys Leu Glu Phe Gln Ile Lys Met Leu
    50                  55                  60

Lys Asn Asp Ser Leu Trp Pro
65                  70

<210> 156

<211> 71
<212> PRT
<213> Physcomitrella patens

<400> 156

Lys Pro Arg Ala Arg Gln Gly Ser Ala Asn Asp Pro Gln Ser Ile Ala
1               5                   10                  15

Ala Arg Val Arg Arg Glu Arg Ile Ser Glu Arg Leu Lys Val Leu Gln
            20                  25                  30

Ala Leu Ile Pro Asn Gly Asp Lys Val Asp Met Val Thr Met Leu Glu
        35                  40                  45

Lys Ala Ile Thr Tyr Val Gln Cys Leu Glu Leu Gln Ile Lys Met Leu
    50                  55                  60

Lys Asn Asp Ser Ile Trp Pro
65                  70

<210> 157
<211> 71
<212> PRT
<213> Physcomitrella patens

<400> 157

Lys Pro Arg Ala Arg Arg Gly Ser Ala Thr Asp Pro Gln Ser Val Tyr
1               5                   10                  15

Ala Arg His Arg Arg Glu Lys Ile Asn Glu Arg Leu Lys Thr Leu Gln
            20                  25                  30

His Leu Val Pro Asn Gly Ala Lys Val Asp Ile Val Thr Met Leu Asp
        35                  40                  45

Glu Ala Ile His Tyr Val Gln Phe Leu Gln Leu Gln Val Thr Leu Leu
    50                  55                  60

Lys Ser Asp Glu Tyr Trp Met
65                  70

<210> 158
<211> 71
<212> PRT
<213> Physcomitrella patens

<400> 158

Arg Pro Arg Val Gln Arg Gly Ser Ala Thr Asp Pro Gln Ser Val His

1               5                   10                  15

Ala Arg Ala Arg Arg Glu Lys Ile Ala Glu Arg Leu Arg Lys Leu Gln
            20                  25                  30

His Leu Ile Pro Asn Gly Gly Lys Val Asp Ile Val Thr Met Leu Asp
        35                  40                  45

Glu Ala Val His Tyr Val Gln Phe Leu Lys Arg Gln Val Thr Leu Leu
    50                  55                  60

Lys Ser Asp Glu Tyr Trp Met
65                  70

<210>  159
<211>  71
<212>  PRT
<213>  Physcomitrella patens

<400>  159

Lys Pro Arg Ala Lys Arg Gly Ser Ala Thr Asp Pro Gln Ser Val Tyr
1               5                   10                  15

Ala Arg His Arg Arg Glu Lys Ile Asn Glu Arg Leu Lys Ser Leu Gln
            20                  25                  30

Asn Leu Val Pro Asn Gly Ala Lys Val Asp Ile Val Thr Met Leu Asp
        35                  40                  45

Glu Ala Ile His Tyr Val Lys Phe Leu Gln Asn Gln Val Glu Leu Leu
    50                  55                  60

Lys Ser Asp Glu Leu Trp Ile
65                  70

<210>  160
<211>  71
<212>  PRT
<213>  Physcomitrella patens

<400>  160

Lys Pro Arg Ala Lys Arg Gly Ser Ala Thr Asp Pro Gln Ser Val Tyr
1               5                   10                  15

Ala Arg His Arg Arg Glu Lys Ile Asn Glu Arg Leu Lys Asn Leu Gln
            20                  25                  30

Asn Leu Val Pro Asn Gly Ala Lys Val Asp Ile Val Thr Met Leu Asp
        35                  40                  45

Glu Ala Ile His Tyr Val Lys Phe Leu Gln Thr Gln Val Glu Leu Leu
    50                  55                  60

Lys Ser Asp Glu Phe Trp Met
65                  70

<210> 161
<211> 71
<212> PRT
<213> Physcomitrella patens

<400> 161

Arg Pro Arg Ala Lys Arg Gly Ser Ala Thr Asp Pro Gln Ser Val Tyr
1               5                   10                  15

Ala Arg His Arg Arg Glu Lys Ile Asn Glu Arg Leu Lys Thr Leu Gln
            20                  25                  30

Arg Leu Val Pro Asn Gly Glu Gln Val Asp Ile Val Thr Met Leu Glu
        35                  40                  45

Glu Ala Ile His Phe Val Lys Phe Leu Glu Phe Gln Leu Glu Leu Leu
    50                  55                  60

Arg Ser Asp Asp Arg Trp Met
65                  70

<210> 162
<211> 71
<212> PRT
<213> Populus trichocarpa

<400> 162

Lys Pro Lys Ser Ala Ala Gly Pro Ala Lys Asp Leu Gln Ser Ile Ala
1               5                   10                  15

Ala Lys Asn Arg Arg Glu Arg Ile Ser Glu Arg Leu Lys Val Leu Gln
            20                  25                  30

Asp Leu Val Pro Asn Gly Ser Lys Val Asp Leu Val Thr Met Leu Glu
        35                  40                  45

Lys Ala Ile Ser Tyr Val Lys Phe Leu Gln Leu Gln Val Lys Val Leu
    50                  55                  60

Ala Thr Asp Glu Leu Trp Pro
65                  70

<210>  163
<211>  72
<212>  PRT
<213>  Populus trichocarpa

<400>  163

Lys Pro Lys Pro Lys Ser Ala Gly Pro Ala Lys Asp Pro Gln Ser Ile
1               5                   10                  15

Ala Ala Lys Asn Arg Arg Glu Arg Ile Ser Glu Arg Leu Lys Met Leu
            20                  25                  30

Gln Asp Leu Val Pro Asn Gly Ser Lys Val Asp Leu Val Thr Met Leu
        35                  40                  45

Glu Lys Ala Ile Ser Tyr Val Lys Phe Leu Gln Leu Gln Val Lys Val
    50                  55                  60

Leu Ala Thr Asp Glu Phe Trp Pro
65                  70

<210>  164
<211>  71
<212>  PRT
<213>  Populus trichocarpa

<400>  164

Lys Thr Arg Ala Ser Arg Gly Ala Ala Thr Asp Pro Gln Ser Leu Tyr
1               5                   10                  15

Ala Arg Lys Arg Arg Glu Arg Ile Asn Glu Arg Leu Arg Ile Leu Gln
            20                  25                  30

Thr Leu Val Pro Asn Gly Thr Lys Val Asp Ile Ser Thr Met Leu Glu
        35                  40                  45

Glu Ala Val Gln Tyr Val Lys Phe Leu Gln Leu Gln Ile Lys Leu Leu
    50                  55                  60

Ser Ser Glu Asp Leu Trp Met
65                  70

<210>  165
<211>  71

<212> PRT
<213> Populus trichocarpa

<400> 165

Lys Thr Arg Ala Ser Lys Gly Ala Ala Thr Asp Pro Gln Ser Leu Tyr
1               5                   10                  15

Ala Arg Lys Arg Arg Glu Arg Ile Asn Glu Arg Leu Arg Ile Leu Gln
            20                  25                  30

Asn Leu Val Pro Asn Gly Thr Lys Val Asp Ile Ser Thr Met Leu Glu
        35                  40                  45

Glu Ala Val Gln Tyr Val Lys Phe Leu Gln Leu Gln Ile Lys Leu Leu
    50                  55                  60

Ser Ser Glu Asp Leu Trp Met
65                  70

<210> 166
<211> 71
<212> PRT
<213> Populus trichocarpa

<400> 166

Lys Thr Arg Ala Thr Arg Gly Ala Ala Thr Asp Pro Gln Ser Leu Tyr
1               5                   10                  15

Ala Arg Lys Arg Arg Glu Arg Ile Asn Glu Arg Leu Lys Ile Leu Gln
            20                  25                  30

Asn Leu Val Pro Asn Gly Thr Lys Val Asp Ile Ser Thr Met Leu Glu
        35                  40                  45

Glu Ala Val His Tyr Val Asn Phe Leu Gln Leu Gln Ile Lys Leu Leu
    50                  55                  60

Ser Ser Asp Asp Leu Trp Met
65                  70

<210> 167
<211> 71
<212> PRT
<213> Selaginella moelendorfii

<400> 167

Lys Pro Arg Ala Lys Gln Gly Cys Ala Asn Asp Pro Gln Ser Ile Ala
1               5                   10                  15

Ala Arg Gln Arg Arg Glu Arg Ile Ser Asp Arg Leu Lys Ile Leu Gln
            20                  25                  30

Glu Leu Ile Pro Asn Gly Ser Lys Val Asp Leu Val Thr Met Leu Glu
        35                  40                  45

Lys Ala Ile Asn Tyr Val Lys Phe Leu Gln Leu Gln Val Lys Val Leu
    50                  55                  60

Met Asn Asp Glu Tyr Trp Pro
65                  70

<210> 168
<211> 71
<212> PRT
<213> Selaginella moelendorfii

<400> 168

Lys Pro Arg Ala Lys Gln Gly Cys Ala Asn Asp Pro Gln Ser Ile Ala
1               5                   10                  15

Ala Arg Gln Arg Arg Glu Arg Ile Ser Asp Arg Leu Lys Ile Leu Gln
            20                  25                  30

Glu Leu Ile Pro Asn Gly Ser Lys Val Asp Leu Val Thr Met Leu Glu
        35                  40                  45

Lys Ala Ile Asn Tyr Val Lys Phe Leu Gln Leu Gln Val Lys Val Leu
    50                  55                  60

Met Asn Asp Glu Tyr Trp Pro
65                  70

<210> 169
<211> 71
<212> PRT
<213> Selaginella moelendorfii

<400> 169

Lys Pro Arg Ala Arg Gln Gly Ser Ala Asn Asp Pro Gln Ser Ile Ala
1               5                   10                  15

Ala Arg His Arg Arg Glu Arg Ile Ser Asp Arg Leu Lys Ile Leu Gln
            20                  25                  30

Glu Leu Val Pro Asn Ser Thr Lys Val Asp Leu Val Thr Met Leu Glu

        35                  40                  45

Lys Ala Ile Asn Tyr Val Lys Phe Leu Gln Leu Gln Val Lys Val Leu
    50                  55                  60

Thr Ser Asp Asp Tyr Trp Pro
65                  70

<210> 170
<211> 71
<212> PRT
<213> Selaginella moelendorfii

<400> 170

Lys Pro Arg Ala Arg Gln Gly Ser Ala Asn Asp Pro Gln Ser Ile Ala
1               5                   10                  15

Ala Arg His Arg Arg Glu Arg Ile Ser Asp Arg Leu Lys Ile Leu Gln
            20                  25                  30

Glu Leu Val Pro Asn Ser Thr Lys Val Asp Leu Val Thr Met Leu Glu
        35                  40                  45

Lys Ala Ile Asn Tyr Val Lys Phe Leu Gln Leu Gln Val Lys Val Leu
    50                  55                  60

Thr Ser Asp Asp Tyr Trp Pro
65                  70

<210> 171
<211> 71
<212> PRT
<213> Selaginella moelendorfii

<400> 171

Lys Pro Arg Ala Lys Arg Gly Ser Ala Thr Asp Pro Gln Ser Ile Tyr
1               5                   10                  15

Ala Arg Gln Arg Arg Glu Arg Ile Asn Glu Arg Leu Arg Ala Leu Gln
            20                  25                  30

Gly Leu Val Pro Asn Gly Ala Lys Val Asp Ile Val Thr Met Leu Glu
        35                  40                  45

Glu Ala Ile Asn Tyr Val Lys Phe Leu Gln Leu Gln Val Lys Leu Leu
    50                  55                  60

Ser Ser Asp Glu Tyr Trp Met
65                  70

<210>  172
<211>  71
<212>  PRT
<213>  Selaginella moelendorfii

<400>  172

Lys Pro Arg Ala Lys Arg Gly Ser Ala Thr Asp Pro Gln Ser Val Tyr
1               5                   10                  15

Ala Arg His Arg Arg Glu Arg Ile Asn Glu Arg Leu Lys Thr Leu Gln
            20                  25                  30

His Leu Val Pro Asn Gly Ala Lys Val Asp Ile Val Thr Met Leu Glu
        35                  40                  45

Glu Ala Ile His Tyr Val Lys Phe Leu Gln Leu Gln Val Asn Met Leu
    50                  55                  60

Ser Ser Asp Glu Tyr Trp Ile
65                  70

<210>  173
<211>  71
<212>  PRT
<213>  Selaginella moelendorfii

<400>  173

Lys Pro Arg Ala Lys Arg Gly Ser Ala Thr Asp Pro Gln Ser Val Tyr
1               5                   10                  15

Ala Arg His Arg Arg Glu Arg Ile Asn Glu Arg Leu Lys Thr Leu Gln
            20                  25                  30

His Leu Val Pro Asn Gly Ala Lys Val Asp Ile Val Thr Met Leu Glu
        35                  40                  45

Glu Ala Ile His Tyr Val Lys Phe Leu Gln Leu Gln Val Asn Met Leu
    50                  55                  60

Ser Ser Asp Glu Tyr Trp Thr
65                  70

<210>  174
<211>  71
<212>  PRT

<213>  Selaginella moelendorfii

<400>  174

Lys Pro Arg Ala Lys Arg Gly Ser Ala Thr Asp Pro Gln Ser Ile Tyr
1               5                   10                  15

Ala Arg Gln Arg Arg Glu Arg Ile Asn Glu Arg Leu Arg Ala Leu Gln
            20                  25                  30

Gly Leu Val Pro Asn Gly Ala Lys Val Asp Ile Val Thr Met Leu Glu
        35                  40                  45

Glu Ala Ile Asn Tyr Val Lys Phe Leu Gln Leu Gln Val Lys Leu Leu
    50                  55                  60

Ser Ser Asp Glu Tyr Trp Met
65                  70

<210>  175
<211>  71
<212>  PRT
<213>  Triticum aestivum

<400>  175

Lys Ser Lys Ala Lys Val Val Pro Thr Lys Asp Pro Gln Ser Ala Val
1               5                   10                  15

Ala Lys Val Arg Arg Glu Arg Ile Ser Glu Arg Leu Lys Val Leu Gln
            20                  25                  30

Asp Leu Val Pro Asn Gly Thr Lys Val Asp Met Val Thr Met Leu Glu
        35                  40                  45

Lys Ala Ile Thr Tyr Val Lys Phe Leu Gln Leu Gln Val Lys Val Leu
    50                  55                  60

Ala Thr Asp Glu Phe Trp Pro
65                  70

<210>  176
<211>  73
<212>  PRT
<213>  Zea mays

<400>  176

Lys Ala Lys Leu Ala Ser Thr Ala Pro Thr Lys Asp Pro Gln Ser Leu
1               5                   10                  15

Ala Ala Lys Gln Asn Arg Arg Glu Arg Ile Ser Glu Arg Leu Arg Ala
            20                  25                  30

Leu Gln Glu Leu Val Pro Asn Gly Thr Lys Val Asp Leu Val Thr Met
        35                  40                  45

Leu Glu Lys Ala Ile Ser Tyr Val Lys Phe Leu Gln Leu Gln Val Lys
    50                  55                  60

Val Leu Ala Thr Asp Glu Phe Trp Pro
65                  70

<210> 177
<211> 71
<212> PRT
<213> Zea mays

<400> 177

Cys Ala Arg Ala Ser Arg Gly Ala Ala Thr Glu Pro Gln Ser Leu Tyr
1               5                   10                  15

Ala Arg Lys Arg Arg Glu Arg Ile Asn Glu Arg Leu Arg Ile Leu Gln
            20                  25                  30

Asn Leu Val Pro Asn Gly Thr Lys Val Asp Ile Ser Thr Met Leu Glu
        35                  40                  45

Glu Ala Ala Gln Tyr Val Lys Phe Leu Gln Leu Gln Ile Lys Leu Leu
    50                  55                  60

Ser Cys Asp Asp Thr Trp Met
65                  70

<210> 178
<211> 81
<212> PRT
<213> Zea mays

<400> 178

Asn Ala Arg Ala Ala Gly Arg Gly Ala Ala Ala Ala Thr Thr Thr Thr
1               5                   10                  15

Ala Glu Pro Gln Ser Ile Tyr Ala Arg Val Arg Arg Glu Arg Ile Asn
            20                  25                  30

Glu Arg Leu Lys Val Leu Gln Ser Leu Val Pro Asn Gly Thr Lys Val
        35                  40                  45

Asp Met Ser Thr Met Leu Glu Glu Ala Val His Tyr Val Lys Phe Leu
50                  55                  60

Gln Leu Gln Ile Arg Val Leu Gln Leu Leu Ser Ser Asp Asp Thr Trp
65                  70                  75                  80

Met

<210>  179
<211>  71
<212>  PRT
<213>  Zea mays

<400>  179

Cys Thr Arg Ala Ser Arg Gly Gly Ala Thr Asp Pro Gln Ser Leu Tyr
1               5                   10                  15

Ala Arg Lys Arg Arg Glu Arg Ile Asn Glu Arg Leu Arg Ile Leu Gln
            20                  25                  30

Asn Leu Val Pro Asn Gly Thr Lys Val Asp Ile Ser Thr Met Leu Glu
        35                  40                  45

Glu Ala Ala Gln Tyr Val Lys Phe Leu Gln Leu Gln Ile Lys Leu Leu
    50                  55                  60

Ser Ser Asp Asp Met Trp Met
65                  70

<210>  180
<211>  72
<212>  PRT
<213>  Arabidopsis thaliana

<400>  180

Lys Pro Asn Arg Arg Asn Val Arg Ile Ser Asp Asp Pro Gln Thr Val
1               5                   10                  15

Val Ala Arg Arg Arg Arg Glu Arg Ile Ser Glu Lys Ile Arg Ile Leu
            20                  25                  30

Lys Arg Ile Val Pro Gly Gly Ala Lys Met Asp Thr Ala Ser Met Leu
        35                  40                  45

Asp Glu Ala Ile Arg Tyr Thr Lys Phe Leu Lys Arg Gln Val Arg Ile

    50                  55                  60

Leu Gln Pro His Ser Gln Ile Gly
65                  70

<210> 181
<211> 25
<212> DNA
<213> Artificial sequence

<220>
<223> Synthetic sequence: Primer

<400> 181
ggattgattt aattaccata tttat 25

<210> 182
<211> 18
<212> DNA
<213> Artificial sequence

<220>
<223> Synthetic sequence: Primer

<400> 182
caagtatcaa acgatgtg 18

<210> 183
<211> 25
<212> DNA
<213> Artificial sequence

<220>
<223> Synthetic sequence: Primer

<400> 183
caccggtacc gaccgttacc gaccg 25

<210> 184
<211> 24
<212> DNA
<213> Artificial sequence

<220>
<223> Synthetic sequence: Primer

<400> 184
taccggtacc gaaaacgaac ggga 24

<210> 185
<211> 23
<212> DNA
<213> Artificial sequence

<220>

<223> Synthetic sequence: Primer

<400> 185
gttcccaatg gcaccaaggt aca 23

<210> 186
<211> 25
<212> DNA
<213> Artificial sequence

<220>
<223> Synthetic sequence: Primer

<400> 186
cccatttgga cgtgaatgta gacac 25

<210> 187
<211> 19
<212> DNA
<213> Artificial sequence

<220>
<223> Synthetic sequence: Primer

<400> 187
cgtgtggacc gacgtctga 19

<210> 188
<211> 29
<212> DNA
<213> Artificial sequence

<220>
<223> Synthetic sequence: Primer

<400> 188
cattttataa taacgctgcg gacatctac 29

<210> 189
<211> 58
<212> DNA
<213> Artificial sequence

<220>
<223> Synthetic sequence: Primer

<400> 189
gggacaactt tgtatagaaa agttgttctc aaagagggac aagaccaaag cccatgac 58

<210> 190
<211> 61
<212> DNA
<213> Artificial sequence

<220>
<223> Synthetic sequence: Primer

<400> 190
ggggactgct tttttgtaca aacttgctag acactaataa gtttgataag tgattttttg 60

t 61

<210> 191
<211> 59
<212> DNA
<213> Artificial sequence

<220>
<223> Synthetic sequence: Primer

<400> 191
ggggacagct ttcttgtaca aagtggccat ggcactcgtt aatgaccatc ccaacgaga 59

<210> 192
<211> 57
<212> DNA
<213> Artificial sequence

<220>
<223> Synthetic sequence: Primer

<400> 192
ggggacaact ttgtataata aagttgctga taaatcgaga tcttaggtat gtcgtcc 57

<210> 193
<211> 53
<212> DNA
<213> Artificial sequence

<220>
<223> Synthetic sequence: Primer

<400> 193
ggggacaact ttgtatagaa aagttgtgat aatggattgg agaaaaatta aag 53

<210> 194
<211> 52
<212> DNA
<213> Artificial sequence

<220>
<223> Synthetic sequence: Primer

<400> 194
ggggactgct tttttgtaca aacttgtatt gcaatgttcg ttaatgagtg ac 52

<210> 195
<211> 53
<212> DNA
<213> Artificial sequence

<220>

<223> Synthetic sequence: Primer

<400> 195
ggggacagct ttcttgtaca aagtgggtaa ttacatctca accccaaatt ctt 53

<210> 196
<211> 49
<212> DNA
<213> Artificial sequence

<220>
<223> Synthetic sequence: Primer

<400> 196
acaactttgt ataataaagt tgatgtataa tttccgaaga tgcttaaaa 49

<210> 197
<211> 27
<212> DNA
<213> Artificial sequence

<220>
<223> Synthetic sequence: Primer

<400> 197
ccaggatcca tggcaggtcc agcagga 27

<210> 198
<211> 30
<212> DNA
<213> Artificial sequence

<220>
<223> Synthetic sequence: Primer

<400> 198
ccagtcgact tagtcagcag aaggctgatt 30

<210> 199
<211> 30
<212> DNA
<213> Artificial sequence

<220>
<223> Synthetic sequence: Primer

<400> 199
cctctagaag tacttgtgat ccacagccta 30

<210> 200
<211> 25
<212> DNA
<213> Artificial sequence

<220>
<223> Synthetic sequence: Primer

<400> 200
ggctcgagcc gtactgggtg gtttg                                    25

<210> 201
<211> 33
<212> DNA
<213> Artificial sequence

<220>
<223> Synthetic sequence: Primer

<400> 201
ccgttaactt ctacatgttg cgttatttat ggt                           33

<210> 202
<211> 43
<212> DNA
<213> Artificial sequence

<220>
<223> Synthetic sequence: Primer

<400> 202
ccggcgcgcc aatatttata taaataagca taatacactt cga                43

<210> 203
<211> 29
<212> DNA
<213> Artificial sequence

<220>
<223> Synthetic sequence: Primer

<400> 203
gcaacgcgtg ggtttgatca aagacggaa                                29

<210> 204
<211> 29
<212> DNA
<213> Artificial sequence

<220>
<223> Synthetic sequence: Primer

<400> 204
gctactagtc gtcaacctaa cccaaacat                                29

<210> 205
<211> 29
<212> DNA
<213> Artificial sequence

<220>
<223> Synthetic sequence: Primer

<400> 205
gagggatccg tgaggtgaaa gcagtgaaa 29

<210> 206
<211> 28
<212> DNA
<213> Artificial sequence

<220>
<223> Synthetic sequence: Primer

<400> 206
ccaaagctta ggcctgtgaa ctcggaca 28

<210> 207
<211> 26
<212> DNA
<213> Artificial sequence

<220>
<223> Synthetic sequence: Primer

<400> 207
gctgctaggg taacataaac attctt 26

<210> 208
<211> 21
<212> DNA
<213> Artificial sequence

<220>
<223> Synthetic sequence: Primer

<400> 208
ctggacactg gaatgaacct a 21

<210> 209
<211> 20
<212> DNA
<213> Artificial sequence

<220>
<223> Synthetic sequence: Primer

<400> 209
cccatggagt caaagattca 20

<210> 210
<211> 19
<212> DNA
<213> Artificial sequence

<220>
<223> Synthetic sequence: Primer

<400> 210

ccgcgaattc gagctcggt 19

<210> 211
<211> 24
<212> DNA
<213> Artificial sequence

<220>
<223> Synthetic sequence: Primer

<400> 211
cccaaatatg catttttaat cttt 24

<210> 212
<211> 22
<212> DNA
<213> Artificial sequence

<220>
<223> Synthetic sequence: Primer

<400> 212
gcgacaatcc agcagcctct at 22

<210> 213
<211> 20
<212> DNA
<213> Artificial sequence

<220>
<223> Synthetic sequence: Primer

<400> 213
gtaggagggc gtggatatgt 20

<210> 214
<211> 15
<212> DNA
<213> Artificial sequence

<220>
<223> Synthetic sequence: Primer

<400> 214
cgagtgctgg ggcgt 15

<210> 215
<211> 20
<212> DNA
<213> Artificial sequence

<220>
<223> Synthetic sequence: Primer

<400> 215
gggacctcaa ggatgcagca 20

<210> 216
<211> 20
<212> DNA
<213> Artificial sequence

<220>
<223> Synthetic sequence: Primer

<400> 216
cgaactcaat aacgtcagga                                20


<210> 217
<211> 22
<212> DNA
<213> Artificial sequence

<220>
<223> Synthetic sequence: Primer

<400> 217
tcccagaatc gctaagattg cc                             22


<210> 218
<211> 18
<212> DNA
<213> Artificial sequence

<220>
<223> Synthetic sequence: Primer

<400> 218
cctttccctt aagctctg                                  18


<210> 219
<211> 18
<212> DNA
<213> Artificial sequence

<220>
<223> Synthetic sequence: Primer

<400> 219
ctcacacggg agagagca                                  18


<210> 220
<211> 19
<212> DNA
<213> Artificial sequence

<220>
<223> Synthetic sequence: Primer

<400> 220
cttcgattct tggctgcta                                 19

<210> 221
<211> 21
<212> DNA
<213> Artificial sequence

<220>
<223> Synthetic sequence: Primer

<400> 221
gcctagcagc caagaaccga a 21

<210> 222
<211> 22
<212> DNA
<213> Artificial sequence

<220>
<223> Synthetic sequence: Primer

<400> 222
ctcatcggct gcaagtacct ta 22

<210> 223
<211> 24
<212> DNA
<213> Artificial sequence

<220>
<223> Synthetic sequence: Primer

<400> 223
ctggttggtt aggagatctt gcat 24

<210> 224
<211> 24
<212> DNA
<213> Artificial sequence

<220>
<223> Synthetic sequence: Primer

<400> 224
gttgtaattt ggtccatttc tgct 24

<210> 225
<211> 20
<212> DNA
<213> Artificial sequence

<220>
<223> Synthetic sequence: Primer

<400> 225
gggacctcaa ggatgcagca 20

<210> 226
<211> 20
<212> DNA
<213> Artificial sequence

<220>
<223> Synthetic sequence: Primer

<400> 226
cgaactcaat aacgtcagga 20

<210> 227
<211> 26
<212> DNA
<213> Artificial sequence

<220>
<223> Synthetic sequence: Primer

<400> 227
gagataggag catctgtacc gcttgt 26

<210> 228
<211> 18
<212> DNA
<213> Artificial sequence

<220>
<223> Synthetic sequence: Primer

<400> 228
cgcatggtgg gatcggct 18